(19) Europäisches Patentamt
European Patent Office
Office européen des brevets

(11) EP 3 174 903 B1

(12) EUROPEAN PATENT SPECIFICATION

(45) Date of publication and mention of the grant of the patent:
**10.04.2024 Bulletin 2024/15**

(21) Application number: **15753907.3**

(22) Date of filing: **31.07.2015**

(51) International Patent Classification (IPC):
***C07K 16/28*** *(2006.01)* ***C07K 16/30*** *(2006.01)*

(52) Cooperative Patent Classification (CPC):
**C07K 16/30; A61P 31/12; A61P 35/00; A61P 37/02; A61P 37/04; C07K 16/2803; C07K 16/2809;** C07K 2317/31; C07K 2317/622; C07K 2319/31

(86) International application number:
**PCT/EP2015/067627**

(87) International publication number:
**WO 2016/016415 (04.02.2016 Gazette 2016/05)**

# (54) BISPECIFIC SINGLE CHAIN ANTIBODY CONSTRUCT WITH ENHANCED TISSUE DISTRIBUTION

BISPEZIFISCHER EINKETTIGER ANTIKÖRPER MIT VERBESSERTEM VERTEILUNG IM GEWEBE

MOLÉCULES D'ANTICORPS À CHAÎNE UNIQUE BISPÉCIFIQUES AVEC DISTRIBUTION AMELIORES DANS LES TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.07.2014 US 201462031777 P**

(43) Date of publication of application:
**07.06.2017 Bulletin 2017/23**

(73) Proprietor: **Amgen Research (Munich) GmbH**
**81477 München (DE)**

(72) Inventors:
- **KUFER, Peter**
  **81477 Munich (DE)**
- **HOFFMANN, Patrick**
  **81477 Munich (DE)**
- **MUNZ, Markus**
  **81477 Munich (DE)**
- **KLINGER, Matthias**
  **81477 Munich (DE)**
- **DOPFER, Elaine-Pashupati**
  **79102 Freiburg (DE)**
- **NAHRWOLD, Elisabeth**
  **81477 Munich (DE)**

(74) Representative: **Schiweck Weinzierl Koch Patentanwälte Partnerschaft mbB**
**Ganghoferstraße 68 B**
**80339 München (DE)**

(56) References cited:
**WO-A1-2014/114800 WO-A1-2014/114801**
**WO-A1-2014/125273 WO-A2-2006/071441**

- **J. T. SOCKOLOSKY ET AL: "Engineering neonatal Fc receptor-mediated recycling and transcytosis in recombinant proteins by short terminal peptide extensions", PROCEEDINGS OF THE NATIONAL ACADEMY OF SCIENCES, vol. 109, no. 40, 2 October 2012 (2012-10-02), pages 16095-16100, XP055067064, ISSN: 0027-8424, DOI: 10.1073/pnas.1208857109**
- **BERTUCCI FRANOIS ET AL: "Gene expression profiling of human melanoma cell lines with distinct metastatic potential identifies new progression markers", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 27, no. 5A, 1 September 2007 (2007-09-01), pages 3441-3449, XP009154071, ISSN: 0250-7005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The complete document including Reference Table(s) and the Sequence Listing(s) can be downloaded from the EPO website

## Description

### Field of the Invention

The present invention relates to a bispecific single chain antibody construct binding to a target cell surface antigen via a first binding domain and to the T cell surface antigen CD3 via a second binding domain, the construct comprising two FcRn binding peptides as defined in the appended claims. Moreover, the invention provides a nucleic acid molecule encoding the antibody construct, a vector comprising said nucleic acid molecule and a host cell transformed or transfected with said vector as defined in the appended claims. Furthermore, the invention provides a process for the production of the antibody construct of the invention, a medical use of said antibody construct and a kit comprising said antibody construct as defined in the appended claims.

### Background of the Invention

A decisive question during the development of a pharmaceutical composition is whether the active compound quantitatively reaches its target to show efficacy. For therapeutic approaches which make use of bispecific T cell engaging antibody derived compounds e.g. in the treatment of cancer, viral or inflammatory diseases, the corresponding question is whether the antibody derived compound reaches a sufficient concentration in the compartment of the target cells. Bispecific molecules such as BiTE® (bispecific T cell engager) antibodies are recombinant protein constructs made of two flexibly linked antibody derived binding domains. One binding domain of BiTE® antibodies is specific for a selected tumor-associated surface antigen on target cells; the second binding domain is specific for CD3, a subunit of the T cell receptor complex on T cells. By their particular design BiTE® antibodies are uniquely suited to transiently connect T cells with target cells and, at the same time, potently activate the inherent cytolytic potential of T cells against target cells. BiTE® antibodies are small proteins with a molecular weight which allows those types of compounds to infiltrate into compartments behind the endothelial barrier via paracellular diffusion. However, the downside of small proteins such as BiTE® antibodies is a molecular weight below the renal cut-off that could likely result in a shorter half-life, a feature that BiTE® antibodies share with many other antibody formats.

An increased half-life is generally useful in *in vivo* applications of immunoglobulins, especially antibodies and most especially antibody fragments of small size. Although such antibody constructs based on antibody fragments (Fvs, disulphide bonded Fvs, Fabs, scFvs, dAbs) are able to rapidly reach most parts of the body, those antibody constructs are likely to suffer from rapid clearance from the body. Strategies described in the art for extending the half-life of antibody constructs such as sinlge-chain diabodies include the conjugation of polyethylene glycol chains (PEGylation), the fusion to the IgG Fc region or to an albumin-binding domain form streptococcal protein G (see Stork 2009, JBC 284(38), p 25612). All those approaches have different issues, which must be separately addressed. The PEGylation may either be irreversible, or the process may impair the biologic activity and/or tissue distribution of the antibody construct. The fusion of a bispecific antibody construct to an IgG Fc region may result in a trifuncional molecule as described for Catumaxomab, which is able to recruit a further cell type via the Fc-FcR function. Furthermore, the fusion of an albumin-binding domain of a bacterial source may increase the immunogenicity of the antibody construct, and it is generally agreed within the art that the immunogenicity must be minimized in order to allow for a long-lasting efficacy of a protein based compound in a patient.

WO 2006/071441 A2 discloses fully human monoclonal antibodies that specifically bind to GPNMB, and uses thereof, as well as immunoconjugates comprising anti-GPNMB antibodies and bispecific antibodies comprising an anti-GPNMB antibody component and an anti-CD3 component, and methods of using such bispecific antibodies. Bertucci et al., Anticancer Research 27:3441-3450 (2007) discloses that gene expression profiling of human melanoma cell lines with distinct metastatic potential identifies new progression markers. Sockolosky et al., PNAS 109(40):16095-16100 (2012) discloses engineering of neonatal Fc receptor-mediated recycling and transcytosis in recombinant proteins by short terminal peptide extensions.

Thus, while it is on the one hand desirable to have a small binding molecule, since it can for example quickly reach its designated location in the body and can also reach most parts of the body, the small "size" of such a binding molecule is on the other hand not favorable as regards, in particular, renal clearance. The price for the strategy of sizing up the molecular weight of an antibody construct above the threshold for renal clearance may be the loss of appreciated tissue distribution or sterical impairment of the antibody function. Thus, it is a balancing act between small size, renal clearance, stability/functionality and tissue distribution.

### Definitions

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series.

The term "and/or" wherever used herein includes the meaning of "and", "or" and "all or any other combination of the elements connected by said term".

The term "about" or "approximately" as used herein means within ±20%, preferably within ±15%, more preferably within ±10%, and most preferably within ±5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

## Detailed description of the Invention

As described herein above, a preferred strategy to increase the distribution volume of a bispecific single chain antibody construct is the attachment of small peptide domains, which allow for the preferred tissue distribution characteristics but avoid impairing the T cell engaging function of the molecule due to steric effects. One possible approach in this context was described for spherical flurescent recombinant protein by Sockolosky (PNAS 2012, 109(40, p 16095). This approach describes the fusion of short terminal peptide extensions binding to the neonatal Fc receptor (FcRn). The recombinant protein (mKate) used by Sockolosky were modified in order to extend the half-live of those proteins by making use of the FcRn-mediated recycling and transcytosis system. However, the use of mKate, a far-red fluorescent protein of the anemone *Entacmaea quadricolor*, in combination with FcRn binding peptides is a very simplified model system. In contrast to the mKate protein, a T cell engaging bispecific antibody construct is a much more complex structure, which requires for its functionality the correct formation of the binding domain to allow binding to its specific epitope. This binding is a prerequisite to engage the patients' own T cells with those cells carrying the target epitope (which is the epitope of the target binding domain), which results in the cytotoxic elimination of the target cell. Of course, an additional decisive factor for the functionality of a bispecific T cell engaging antibody construct is the question whether the construct is still producible in line with industrial standards for pharmaceutical utility. In view of the above described technical field, the problem underlying the present invention is to provide functional bispecific single chain antibody constructs which show an enhanced tissue distribution compared with the bispecific T cell engaging antibody constructs known in the art.

The present invention provides a bispecific single chain antibody construct binding to a target cell surface antigen via a first binding domain and to the T cell surface antigen CD3 via a second binding domain, the construct comprising two FcRn binding peptides, wherein:

(a) a first FcRn binding peptide consists of the amino acid sequence **QRFVTGHFGGL$X_1$PANG** (SEQ ID NO: 1) whereas $X_1$ is Y or H; and
(b) a second FcRn binding peptide consists of the amino acid sequence **TGHFGGLHP** (SEQ ID NO: 4);

wherein the bispecific single chain antibody construct does not have an amino acid sequence as depicted in SEQ ID NOs: **132-135.**

In one embodiment of the antibody construct of the invention the second FcRn binding peptide consists of the amino acid sequence **QRFVTGHFGGLHPANG** (SEQ ID NO: 3) or **QRFCTGHFGGLHPCNG** (SEQ ID NO: 5).

The term "antibody construct" refers to a molecule in which the structure and/or function is/are based on the structure and/or function of an antibody, e.g. of a full-length or whole immunoglobulin molecule. An antibody construct is hence capable of binding to its specific target or antigen. Furthermore, an antibody construct according to the invention comprises the minimum structural requirements of an antibody which allow for the target binding. This minimum requirement may e.g. be defined by the presence of at least the three light chain CDRs (i.e. CDR1, CDR2 and CDR3 of the VL region) and/or the three heavy chain CDRs (i.e. CDR1, CDR2 and CDR3 of the VH region). The antibodies on which the constructs according to the invention are based include for example monoclonal, recombinant, chimeric, deimmunized, humanized and human antibodies.

Within the definition of "antibody constructs" according to the invention are full-length or whole antibodies including camelid antibodies and other immunoglobulin antibodies generated by biotechnological or protein engineering methods or processes. These full-length antibodies may be for example monoclonal, recombinant, chimeric, deimmunized, humanized and human antibodies. Also within the definition of "antibody constructs" are fragments of full-length antibodies, such as VH, VHH, VL, (s)dAb, Fv, Fd, Fab, Fab', F(ab')2 or "r IgG" ("half antibody"). Antibody constructs according to the invention may also be modified fragments of antibodies, also called antibody variants, such as scFv, di-scFv or bi(s)-scFv, scFv-Fc, scFv-zipper, scFab, Fab2, Fab3, diabodies, single chain diabodies, tandem diabodies (Tandab's), tandem di-scFv, tandem tri-scFv, "minibodies" exemplified by a structure which is as follows: (VH-VL-CH3)2, (scFv-CH3)2 or (scFv-CH3-scFv)2, multibodies such as triabodies or tetrabodies, and single domain antibodies such as nanobodies or single variable domain antibodies comprising merely one variable domain, which might be VHH, VH or VL, that specifically bind an antigen or epitope independently of other V regions or domains.

Furthermore, the definition of the term "antibody constructs" includes monovalent, bivalent and polyvalent / multivalent constructs and, thus, monospecific constructs, specifically binding to only one antigenic structure, as well as bispecific and polyspecific / multispecific constructs, which specifically bind more than one antigenic structure, e.g. two, three or more, through distinct binding domains. Moreover, the definition of the term "antibody constructs" includes molecules consisting of only one polypeptide chain as well as molecules consisting of more than one polypeptide chain, which chains can be either identical (homodimers, homotrimers or homo oligomers) or different (heterodimer, heterotrimer or heterooligomer). Examples for the above identified antibodies and variants or derivatives thereof are described *inter alia* in Harlow and Lane, Antibodies a laboratory manual, CSHL Press (1988) and Using Antibodies: a laboratory manual, CSHL Press (1999), Kontermann and Dübel, Antibody Engineering, Springer, 2nd ed. 2010 and Little, Recombinant Antibodies for Immunotherapy, Cambridge University Press 2009.

The antibody constructs of the present invention are preferably *"in vitro* generated antibody constructs". This term refers to an antibody construct according to the above definition where all or part of the variable region (e.g., at least one CDR) is generated in a non-immune cell selection, *e.g.,* an *in vitro* phage display, protein chip or any other method in which candidate sequences can be tested for their ability to bind to an antigen. This term thus preferably excludes sequences generated solely by genomic rearrangement in an immune cell in an animal. A "recombinant antibody" is an antibody made through the use of recombinant DNA technology or genetic engineering.

The present invention is directed to "single chain antibody constructs". Accordingly, those single chain antibody constructs only include those embodiments of the above described antibody constructs which consist of a single polypeptide chain.

The term "monoclonal antibody" (mAb) or monoclonal antibody construct as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site or determinant on the antigen, in contrast to conventional (polyclonal) antibody preparations which typically include different antibodies directed against different determinants (or epitopes). In addition to their specificity, the monoclonal antibodies are advantageous in that they are synthesized by the hybridoma culture, hence uncontaminated by other immunoglobulins. The modifier "monoclonal" indicates the character of the antibody as being obtained from a substantially homogeneous population of antibodies, and is not to be construed as requiring production of the antibody by any particular method.

For the preparation of monoclonal antibodies, any technique providing antibodies produced by continuous cell line cultures can be used. For example, monoclonal antibodies to be used may be made by the hybridoma method first described by Koehler et al., Nature, 256: 495 (1975), or may be made by recombinant DNA methods (see, e.g., U.S. Patent No. 4,816,567). Examples for further techniques to produce human monoclonal antibodies include the trioma technique, the human B-cell hybridoma technique (Kozbor, Immunology Today 4 (1983), 72) and the EBV-hybridoma technique (Cole et al., Monoclonal Antibodies and Cancer Therapy, Alan R. Liss, Inc. (1985), 77-96).

Hybridomas can then be screened using standard methods, such as enzyme-linked immunosorbent assay (ELISA) and surface plasmon resonance (BIACORE™) analysis, to identify one or more hybridomas that produce an antibody that specifically binds with a specified antigen. Any form of the relevant antigen may be used as the immunogen, e.g., recombinant antigen, naturally occurring forms, any variants or fragments thereof, as well as an antigenic peptide thereof. Surface plasmon resonance as employed in the BIAcore system can be used to increase the efficiency of phage antibodies which bind to an epitope of a target antigen, such as the target cell surface antigen or CD3 epsilon (Schier, Human Antibodies Hybridomas 7 (1996), 97-105; Malmborg, J. Immunol. Methods 183 (1995), 7-13).

Another exemplary method of making monoclonal antibodies includes screening protein expression libraries, e.g., phage display or ribosome display libraries. Phage display is described, for example, in Ladner et al., U.S. Patent No. 5,223,409; Smith (1985) Science 228:1315-1317, Clackson et al., Nature, 352: 624-628 (1991) and Marks et al., J. Mol. Biol., 222: 581-597 (1991).

In addition to the use of display libraries, the relevant antigen can be used to immunize a non-human animal, e.g., a rodent (such as a mouse, hamster, rabbit or rat). For example, it is possible to engineer mouse strains deficient in mouse antibody production with large fragments of the human Ig (immunoglobulin) loci. Using the hybridoma technology, antigen-specific monoclonal antibodies derived from the genes with the desired specificity may be produced and selected. See, e.g., XENOMOUSE™, Green et al. (1994) Nature Genetics 7:13-21, US 2003-0070185, WO 96/34096, and WO96/33735.

A monoclonal antibody can also be obtained from a non-human animal, and then modified, e.g., humanized, deimmunized, rendered chimeric etc., using recombinant DNA techniques known in the art. Examples of modified antibody constructs include humanized variants of non-human antibodies, "affinity matured" antibodies (see, *e.g.* Hawkins et al. J. Mol. Biol. 254, 889-896 (1992) and Lowman et al., Biochemistry 30, 10832- 10837 (1991)) and antibody mutants with altered effector function(s) (see, *e.g.,* US Patent 5,648,260, Kontermann and Dübel (2010), *loc. cit.* and Little (2009), *loc. cit.*).

In immunology, affinity maturation is the process by which B cells produce antibodies with increased affinity for antigen during the course of an immune response. With repeated exposures to the same antigen, a host will produce antibodies of successively greater affinities. Like the natural prototype, the *in vitro* affinity maturation is based on the principles of mutation and selection. The *in vitro* affinity maturation has successfully been used to optimize antibodies, antibody constructs, and antibody fragments. Random mutations inside the CDRs are introduced using radiation, chemical mutagens or error-prone PCR. In addition, the genetical diversity can be increased by chain shuffling. Two or three rounds of mutation and selection using display methods like phage display usually results in antibody fragments with affinities in the low nanomolar range.

A preferred type of an amino acid substitutional varianation of the antibody constructs involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e. g. 6-7 sites) are mutated to generate all possible amino acid substitutions at each site. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the binding domain and, e.g., human the target cell surface antigen. Such contact residues and neighbouring residues are candidates for substitution according to the techniques elaborated herein. Once such variants are generated, the panel of variants is subjected to screening as described herein and antibodies with superior properties in one or more relevant assays may be selected for further development.

The monoclonal antibodies and antibody constructs of the present invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is/are identical with or homologous to corresponding sequences in antibodies derived from another species or belonging to another antibody class or subclass, as well as fragments of such antibodies, so long as they exhibit the desired biological activity (U.S. Patent No. 4,816, 567; Morrison et al., Proc. Natl. Acad. Sci. USA, 81: 6851-6855 (1984)). Chimeric antibodies of interest herein include "primitized" antibodies comprising variable domain antigen-binding sequences derived from a non-human primate (e.g., Old World Monkey, Ape etc.) and human constant region sequences. A variety of approaches for making chimeric antibodies have been described. See *e.g.,* Morrison et al., Proc. Natl. Acad. ScL U.S.A. 81:6851 , 1985; Takeda et al., Nature 314:452, 1985, Cabilly et al., U.S. Patent No. 4,816,567; Boss et al., U.S. Patent No. 4,816,397; Tanaguchi et al., EP 0171496; EP 0173494; and GB 2177096.

An antibody, antibody construct or antibody fragment may also be modified by specific deletion of human T cell epitopes (a method called "deimmunization") by the methods disclosed in WO 98/52976 and WO 00/34317. Briefly, the heavy and light chain variable domains of an antibody can be analyzed for peptides that bind to MHC class II; these peptides represent potential T cell epitopes (as defined in WO 98/52976 and WO 00/34317). For detection of potential T cell epitopes, a computer modeling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences are disclosed e.g. in Tomlinson, et al. (1992) J. Mol. Biol. 227:776-798; Cook, G.P. et al. (1995) Immunol. Today Vol. 16 (5): 237-242; and Tomlinson et al. (1995) EMBO J. 14:

14:4628-4638. The V BASE directory provides a comprehensive directory of human immunoglobulin variable region sequences (compiled by Tomlinson, LA. et al. MRC Centre for Protein Engineering, Cambridge, UK). These sequences can be used as a source of human sequence, e.g., for framework regions and CDRs. Consensus human framework regions can also be used, for example as described in US Patent No. 6,300,064.

"Humanized" antibodies, antibody constructs or fragments thereof (such as Fv, Fab, Fab', F(ab')2 or other antigen-binding subsequences of antibodies) are antibodies or immunoglobulins of mostly human sequences, which contain (a) minimal sequence(s) derived from non-human immunoglobulin. For the most part, humanized antibodies are human immunoglobulins (recipient antibody) in which residues from a hypervariable region (also CDR) of the recipient are replaced by residues from a hypervariable region of a non-human (e.g., rodent) species (donor antibody) such as mouse, rat, hamster or rabbit having the desired specificity, affinity, and capacity. In some instances, Fv framework region (FR) residues of the human immunoglobulin are replaced by corresponding non-human residues. Furthermore, "humanized antibodies" as used herein may also comprise residues which are found neither in the recipient antibody nor the donor antibody. These modifications are made to further refine and optimize antibody performance. The humanized antibody may also comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Those methods include isolating, manipulating, and expressing the nucleic acid molecules that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

Humanized antibodies may also be produced using transgenic animals such as mice that express human heavy and light chain genes, but are incapable of expressing the endogenous mouse immunoglobulin heavy and light chain genes. Winter describes an exemplary CDR grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

A humanized antibody can be optimized by the introduction of conservative substitutions, consensus sequence substitutions, germline substitutions and/or back mutations. Such altered immunoglobulin molecules can be made by any of several techniques known in the art, (e.g., Teng et al., Proc. Natl. Acad. Sci. U.S.A., 80: 7308-7312, 1983; Kozbor et al., Immunology Today, 4: 7279, 1983; Olsson et al., Meth. Enzymol., 92: 3-16, 1982, and EP 239 400.

The term "human antibody", "human antibody construct" and "human binding domain" includes antibodies, antibody constructs and binding domains having antibody regions such as variable and constant regions or domains which correspond substantially to human germline immunoglobulin sequences known in the art, including, for example, those described by Kabat *et al.* (1991) (*loc. cit.*). The human antibodies, antibody constructs or binding domains of the invention may include amino acid residues not encoded by human germline immunoglobulin sequences (*e.g.*, mutations introduced by random or site-specific mutagenesis *in vitro* or by somatic mutation *in vivo*), for example in the CDRs, and in particular, in CDR3. The human antibodies, antibody constructs or binding domains can have at least one, two, three, four, five, or more positions replaced with an amino acid residue that is not encoded by the human germline immunoglobulin sequence. The definition of human antibodies, antibody constructs and binding domains as used herein also contemplates fully human antibodies, which include only non-artificially and/or genetically altered human sequences of antibodies as those can be derived by using technologies or systems such as the Xenomouse.

In some embodiments, the antibody constructs of the invention are "isolated" or "substantially pure" antibody constructs. "isolated" or "substantially pure" when used to describe the antibody construct disclosed herein means an antibody construct that has been identified, separated and/or recovered from a component of its production environment. Preferably, the antibody construct is free or substantially free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. The antibody constructs may e.g constitute at least about 5%, or at least about 50% by weight of the total protein in a given sample. It is understood that the isolated protein may constitute from 5% to 99.9% by weight of the total protein content, depending on the circumstances. The polypeptide may be made at a significantly higher concentration through the use of an inducible promoter or high expression promoter, such that it is made at increased concentration levels. The definition includes the production of an antibody construct in a wide variety of organisms and/or host cells that are known in the art. In preferred embodiments, the antibody construct will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody construct will be prepared by at least one purification step.

The term "binding domain" characterizes in connection with the present invention a domain which (specifically) binds to / interacts with / recognizes a given target epitope or a given target site on the target molecules (antigens) and CD3, respectively. The structure and function of the first binding domain (recognizing the target cell surface antigen), and preferably also the structure and/or function of the second binding domain (CD3), is/are based on the structure and/or function of an antibody, e.g. of a full-length or whole immunoglobulin molecule. According to the invention, the first binding domain is characterized by the presence of three light chain CDRs (i.e. CDR1, CDR2 and CDR3 of the VL region) and three heavy chain CDRs (i.e. CDR1, CDR2 and CDR3 of the VH region). The second binding domain preferably also comprises the minimum structural requirements of an antibody which allow for the target binding. More preferably, the second binding domain comprises at least three light chain CDRs (i.e. CDR1, CDR2 and CDR3 of the VL region) and/or three heavy chain CDRs (i.e. CDR1, CDR2 and CDR3 of the VH region). It is envisaged that the first and/or second binding domain is produced by or obtainable by phage-display or library screening methods rather than by grafting CDR sequences from a pre-existing (monoclonal) antibody into a scaffold.

According to the present invention, binding domains are preferably in the form of polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids). The term "polypeptide" as used herein describes a group of molecules, which usually consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "peptide", "polypeptide" and "protein" also refer to naturally modified peptides / polypeptides / proteins wherein the modification is effected e.g. by post-translational modifications like glycosylation, acetylation, phosphorylation and the like. A "peptide", "polypeptide" or "protein" when referred to herein may also be chemically modified such as pegylated. Such modifications are well known in the art and described herein below.

As mentioned above, a binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of (modified) antigen-binding antibody fragments include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv) , the latter being preferred (for example, derived from an scFV library).

Antibodies and antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. In order to avoid the use of rodent derived antibodies or antibody constructs, human or fully human antibodies / antibody constructs can be generated through the introduction of human antibody function into a rodent so that the rodent produces fully human antibodies.

The ability to clone and reconstruct megabase-sized human loci in YACs and to introduce them into the mouse germline provides a powerful approach to elucidating the functional components of very large or crudely mapped loci as well as generating useful models of human disease. Furthermore, the use of such technology for substitution of mouse loci with their human equivalents could provide unique insights into the expression and regulation of human gene products during development, their communication with other systems, and their involvement in disease induction and progression.

An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B-cell development. Furthermore, such a strategy could provide an ideal source for production of fully human monoclonal antibodies (mAbs) - an important milestone towards fulfilling the promise of antibody therapy in human disease. Fully human antibodies or antibody constructs are expected to minimize the immunogenic and allergic responses intrinsic to mouse or mouse-derivatized mAbs and thus to increase the efficacy and safety of the administered antibodies / antibody constructs. The use of fully human antibodies or antibody constructs can be expected to provide a substantial advantage in the treatment of chronic and recurring human diseases, such as inflammation, autoimmunity, and cancer, which require repeated compound administrations.

One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against any antigen of interest, including human antigens. Using the hybridoma technology, antigen-specific human mAbs with the desired specificity could be readily produced and selected. This general strategy was demonstrated in connection with the generation of the first XenoMouse mouse strains (see Green et al. Nature Genetics 7:13-21 (1994)). The XenoMouse strains were engineered with yeast artificial chromosomes (YACs) containing 245 kb and 190 kb-sized germline configuration fragments of the human heavy chain locus and kappa light chain locus, respectively, which contained core variable and constant region sequences. The human Ig containing YACs proved to be compatible with the mouse system for both rearrangement and expression of antibodies and were capable of substituting for the inactivated mouse Ig genes. This was demonstrated by their ability to induce B cell development, to produce an adult-like human repertoire of fully human antibodies, and to generate antigen-specific human mAbs. These results also suggested that introduction of larger portions of the human Ig loci containing greater numbers of V genes, additional regulatory elements, and human Ig constant regions might recapitulate substantially the full repertoire that is characteristic of the human humoral response to infection and immunization. The work of Green et al. was recently extended to the introduction of greater than approximately 80% of the human antibody repertoire through introduction of megabase sized, germline configuration YAC fragments of the human heavy chain loci and kappa light chain loci, respectively. See Mendez et al. Nature Genetics 15:146-156 (1997) and U.S. patent application Ser. No. 08/759,620.

The production of the XenoMouse mice is further discussed and delineated in U.S. patent applications Ser. No. 07/466,008, Ser. No. 07/610,515, Ser. No. 07/919,297, Ser. No. 07/922,649, Ser. No. 08/031,801, Ser. No. 08/112,848, Ser. No. 08/234,145, Ser. No. 08/376,279, Ser. No. 08/430,938, Ser. No. 08/464,584, Ser. No. 08/464,582, Ser. No. 08/463,191, Ser. No. 08/462,837, Ser. No. 08/486,853, Ser. No. 08/486,857, Ser. No. 08/486,859, Ser. No. 08/462,513, Ser. No. 08/724,752, and Ser. No. 08/759,620; and U.S. Pat. Nos. 6,162,963, 6,150,584, 6,114,598, 6,075,181, and 5,939,598 and Japanese Patent Nos. 3 068 180 B2, 3 068 506 B2, and 3 068 507 B2. See also Mendez et al. Nature Genetics 15:146-156 (1997) and Green and Jakobovits J. Exp. Med. 188:483-495 (1998), EP 0 463 151 B1, WO 94/02602, WO 96/34096, WO 98/24893, WO 00/76310, and WO 03/47336.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more VH genes, one or more DH genes, one or more JH genes, a mu constant region, and a second constant region (preferably a gamma constant region) are formed into a construct for insertion into an animal. This approach is described in U.S. Pat. No. 5,545,807 to Surani et al. and U.S. Pat. Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Pat. Nos. 5,591,669 and 6,023.010 to Krimpenfort and Berns, U.S. Pat. Nos. 5,612,205, 5,721,367, and 5,789,215 to Berns et al., and U.S. Pat. No. 5,643,763 to Choi and Dunn, and GenPharm International U.S. patent application Ser. No. 07/574,748, Ser. No. 07/575,962, Ser. No. 07/810,279, Ser. No. 07/853,408, Ser. No. 07/904,068, Ser. No. 07/990,860, Ser. No. 08/053,131, Ser. No. 08/096,762, Ser. No. 08/155,301, Ser. No. 08/161,739, Ser. No. 08/165,699, Ser. No. 08/209,741. See also EP 0 546 073 B1, WO 92/03918, WO 92/22645, WO 92/22647, WO 92/22670, WO 93/12227, WO 94/00569, WO 94/25585, WO 96/14436, WO 97/13852, and WO 98/24884 and U.S. Pat. No. 5,981,175. See further Taylor *et al.* (1992), Chen *et al.* (1993), Tuaillon *et al.* (1993), Choi *et al.* (1993), Lonberg *et al.* (1994), Taylor *et al.* (1994), and Tuaillon *et al.* (1995), Fishwild *et al.* (1996).

Kirin has also demonstrated the generation of human antibodies from mice in which, through microcell fusion, large pieces of chromosomes, or entire chromosomes, have been introduced. See European Patent Application Nos. 773 288 and 843 961. Xenerex Biosciences is developing a technology for the potential generation of human antibodies. In this technology, SCID mice are reconstituted with human lymphatic cells, e.g., B and/or T cells. Mice are then immunized with an antigen and can generate an immune response against the antigen. See U.S. Pat. Nos. 5,476,996; 5,698,767; and 5,958,765.

Human anti-mouse antibody (HAMA) responses have led the industry to prepare chimeric or otherwise humanized antibodies. It is however expected that certain human anti-chimeric antibody (HACA) responses will be observed, particularly in chronic or multi-dose utilizations of the antibody. Thus, it would be desirable to provide antibody constructs comprising a fully human binding domain against the target cell surface antigen and a fully human binding domain against CD3 in order to vitiate concerns and/or effects of HAMA or HACA response.

The terms "(specifically) binds to", (specifically) recognizes", "is (specifically) directed to", and "(specifically) reacts with" mean in accordance with this invention that a binding domain interacts or specifically interacts with one or more, preferably at least two, more preferably at least three and most preferably at least four amino acids of an epitope located on the target protein or antigen (the target cell surface antigen / CD3).

The term "epitope" refers to a site on an antigen to which a binding domain, such as an antibody or immunoglobulin or derivative or fragment of an antibody or of an immunoglobulin, specifically binds. An "epitope" is antigenic and thus the term epitope is sometimes also referred to herein as "antigenic structure" or "antigenic determinant". Thus, the binding domain is an "antigen interaction site". Said binding/interaction is also understood to define a "specific recognition".

"Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically a conformational epitope comprises an increased number of amino acids relative to a linear epitope. With regard to recognition of conformational epitopes, the binding domain recognizes a three-dimensional structure of the antigen, preferably a peptide or protein or fragment thereof (in the context of the present invention, the antigen for one of the binding domains is comprised within the the target cell surface antigen protein). For example, when a protein molecule folds to form a three-dimensional structure, certain amino acids and/or the polypeptide backbone forming the conformational epitope become juxtaposed enabling the antibody to recognize the epitope. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy.

The interaction between the binding domain and the epitope or epitope cluster implies that a binding domain exhibits appreciable affinity for the epitope or epitope cluster on a particular protein or antigen (here: the target cell surface antigen and CD3, respectively) and, generally, does not exhibit significant reactivity with proteins or antigens other than the target cell surface antigen or CD3. "Appreciable affinity" includes binding with an affinity of about $10^{-6}$ M (KD) or stronger. Preferably, binding is considered specific when the binding affinity is about $10^{-12}$ to $10^{-8}$ M, $10^{-12}$ to $10^{-9}$ M, $10^{-12}$ to $10^{-10}$ M, $10^{-11}$ to $10^{-8}$ M, preferably of about $10^{-11}$ to $10^{-9}$ M. Whether a binding domain specifically reacts with or binds to a target can be tested readily by, *inter alia,* comparing the reaction of said binding domain with a target protein or antigen with the reaction of said binding domain with proteins or antigens other than the target cell surface antigen or CD3. Preferably, a binding domain of the invention does not essentially or substantially bind to proteins or antigens other than the target cell surface antigen or CD3 (i.e., the first binding domain is not capable of binding to proteins other than the target cell surface antigen and the second binding domain is not capable of binding to proteins other than CD3).

The term "does not essentially / substantially bind" or "is not capable of binding" means that a binding domain of the present invention does not bind a protein or antigen other than the target cell surface antigen or CD3, *i.e.*, does not show reactivity of more than 30%, preferably not more than 20%, more preferably not more than 10%, particularly preferably not more than 9%, 8%, 7%, 6% or 5% with proteins or antigens other than the target cell surface antigen or CD3, whereby binding to the target cell surface antigen or CD3, respectively, is set to be 100%.

Specific binding is believed to be effected by specific motifs in the amino acid sequence of the binding domain and the antigen. Thus, binding is achieved as a result of their primary, secondary and/or tertiary structure as well as the result of secondary modifications of said structures. The specific interaction of the antigen-interaction-site with its specific antigen may result in a simple binding of said site to the antigen. Moreover, the specific interaction of the antigen-interaction-site with its specific antigen may alternatively or additionally result in the initiation of a signal, e.g. due to the induction of a change of the conformation of the antigen, an oligomerization of the antigen, etc.

The term "variable" refers to the portions of the antibody or immunoglobulin domains that exhibit variability in their sequence and that are involved in determining the specificity and binding affinity of a particular antibody (i.e., the "variable domain(s)"). The pairing of a variable heavy chain (VH) and a variable light chain (VL) together forms a single antigen-binding site. The CH domain most proximal to VH is designated as CH1. Each light (L) chain is linked to a heavy (H) chain by one covalent disulfide bond, while the two H chains are linked to each other by one or more disulfide bonds depending on the H chain isotype.

Variability is not evenly distributed throughout the variable domains of antibodies; it is concentrated in sub-domains of each of the heavy and light chain variable regions. These sub-domains are called "hypervariable regions" or "complementarity determining regions" (CDRs). The more conserved (i.e., non-hypervariable) portions of the variable domains are called the "framework" regions (FRM) and provide a scaffold for the six CDRs in three dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat *et al*., *loc. cit*.). The constant domains are not directly involved in antigen binding, but exhibit various effector functions, such as, for example, antibody-dependent, cell-mediated cytotoxicity and complement activation.

The terms "CDR", and its plural "CDRs", refer to the complementarity determining region of which three make up the binding character of a light chain variable region (CDR-L1, CDR-L2 and CDR-L3) and three make up the binding character of a heavy chain variable region (CDR-H1, CDR-H2 and CDR-H3). CDRs contain most of the residues responsible for specific interactions of the antibody with the antigen and hence contribute to the functional activity of an antibody molecule: they are the main determinants of antigen specificity.

The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat (an approach based on cross-species sequence variability), Chothia (an approach based on crystallographic studies of antigen-antibody complexes), and/or MacCallum (Kabat *et al., loc. cit*.; Chothia et al., J. Mol. Biol, 1987, 196: 901-917; and MacCallum et al., J. Mol. Biol, 1996, 262: 732). Still another standard for characterizing the antigen binding site is the AbM definition used by Oxford Molecular's AbM antibody modeling software. See, e.g., Protein Sequence and Structure Analysis of Antibody Variable Domains. In: Antibody Engineering Lab Manual (Ed.: Duebel, S. and Kontermann, R., Springer-Verlag, Heidelberg). To the extent that two residue identification techniques define regions of overlapping, but not identical regions, they can be combined to define a hybrid CDR. However, the numbering in accordance with the so-called Kabat system is preferred.

Typically, CDRs form a loop structure that can be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues.

The term "canonical structure" may also include considerations as to the linear sequence of the antibody, for example, as catalogued by Kabat (Kabat *et al.,* loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al and/or revealed by other techniques, for example, crystallography and two- or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia *et al.,* loc. cit. and their implications for construing canonical aspects of antibody structure, are described in the literature. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988.

The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. *In vitro* selection schemes in which CDR3 alone is varied can be used to vary the binding properties of an antibody or determine which residues contribute to the binding of an antigen. Hence, CDR3 is typically the greatest source of molecular diversity within the antibody-binding site. H3, for example, can be as short as two amino acid residues or greater than 26 amino acids.

The sequence of antibody genes after assembly and somatic mutation is highly varied, and these varied genes are estimated to encode $10^{10}$ different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., *in vitro* stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

The term "bispecific" as used herein refers to an antibody construct which is "at least bispecific", i.e., it comprises at least a first binding domain and a second binding domain, wherein the first binding domain binds to one antigen or target, and the second binding domain binds to another antigen or target (here: CD3). Accordingly, antibody constructs according to the invention comprise specificities for at least two different antigens or targets. The term "bispecific antibody construct" of the invention also encompasses multispecific antibody constructs such as trispecific antibody constructs, the latter ones including three binding domains, or constructs having more than three (e.g. four, five...) specificites.

Given that the antibody constructs according to the invention are (at least) bispecific, they do not occur naturally and they are markedly different from naturally occurring products. A "bispecific" antibody construct or immunoglobulin is hence an artificial hybrid antibody or immunoglobulin having at least two distinct binding sites with different specificities. Bispecific antibodies can be produced by a variety of methods including fusion of hybridomas or linking of Fab' fragments. See, e.g., Songsivilai & Lachmann, Clin. Exp. Immunol. 79:315-321 (1990).

The at least two binding domains and the variable domains of the antibody construct of the present invention may or may not comprise peptide linkers (spacer peptides). The term "peptide linker" defines in accordance with the present invention an amino acid sequence by which the amino acid sequences of one (variable and/or binding) domain and another (variable and/or binding) domain of the antibody construct of the invention are linked with each other. An essential technical feature of such peptide linker is that said peptide linker does not comprise any polymerization activity. Among the suitable peptide linkers are those described in U.S. Patents 4,751,180 and 4,935,233 or WO 88/09344.

In the event that a linker is used, this linker is preferably of a length and sequence sufficient to ensure that each of the first and second domains can, independently from one another, retain their differential binding specificities. For peptide linkers which connect the at least two binding domains in the antibody construct of the invention (or two variable domains), those peptide linkers are preferred which comprise only a few number of amino acid residues, e.g. 12 amino acid residues or less. Thus, peptide linker of 12, 11, 10, 9, 8, 7, 6 or 5 amino acid residues are preferred. An envisaged peptide linker with less than 5 amino acids comprises 4, 3, 2 or one amino acid(s) wherein Gly-rich linkers are preferred. A particularly preferred "single" amino acid in context of said "peptide linker" is Gly. Accordingly, said peptide linker may consist of the single amino acid Gly. Another preferred embodiment of a peptide linker is characterized by the amino acid sequence Gly-Gly-Gly-Gly-Ser, i.e. $Gly_4Ser$, or polymers thereof, i.e. $(Gly_4Ser)x$, where x is an integer of 1 or greater. The characteristics of said peptide linker, which comprise the absence of the promotion of secondary structures are known in the art and are described e.g. in Dall'Acqua et al. (Biochem. (1998) 37, 9266-9273), Cheadle et al. (Mol Immunol (1992) 29, 21-30) and Raag and Whitlow (FASEB (1995) 9(1), 73-80). Peptide linkers which also do not promote any secondary structures are preferred. The linkage of said domains to each other can be provided by, e.g. genetic engineering, as described in the examples. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).

Bispecific single chain molecules are known in the art and are described in WO 99/54440, Mack, J. Immunol. (1997), 158, 3965-3970, Mack, PNAS, (1995), 92, 7021-7025, Kufer, Cancer Immunol. Immunother., (1997), 45, 193-197, Löffler, Blood, (2000), 95, 6, 2098-2103, Brühl, Immunol., (2001), 166, 2420-2426, Kipriyanov, J. Mol. Biol., (1999), 293, 41-56. Techniques described for the production of single chain antibodies (see, *inter alia,* US Patent 4,946,778, Kontermann and Dübel (2010), *loc. cit.* and Little (2009), *loc. cit*.) can be adapted to produce single chain antibody constructs specifically recognizing (an) elected target(s).

Bivalent (also called divalent) or bispecific single-chain variable fragments (bi-scFvs or di-scFvs having the format $(scFv)_2$) can be engineered by linking two scFv molecules. If these two scFv molecules have the same binding specificity, the resulting $(scFv)_2$ molecule will preferably be called bivalent (*i.e.* it has two valences for the same target epitope). If the two scFv molecules have different binding specificities, the resulting $(scFv)_2$ molecule will preferably be called bispecific. The linking can be done by producing a single peptide chain with two VH regions and two VL regions, yielding tandem scFvs (see *e.g.* Kufer P. et al., (2004) Trends in Biotechnology 22(5):238-244). Another possibility is the creation of scFv molecules with linker peptides that are too short for the two variable regions to fold together (e.g. about five amino acids), forcing the scFvs to dimerize. This type is known as diabodies (see e.g. Hollinger, Philipp et al., (July 1993) Proceedings of the National Academy of Sciences of the United States of America 90 (14): 6444-8.).

Single domain antibodies comprise merely one (monomeric) antibody variable domain which is able to bind selectively to a specific antigen, independently of other V regions or domains. The first single domain antibodies were engineered from havy chain antibodies found in camelids, and these are called $V_HH$ fragments. Cartilaginous fishes also have heavy chain antibodies (IgNAR) from which single domain antibodies called $V_{NAR}$ fragments can be obtained. An alternative approach is to split the dimeric variable domains from common immunoglobulins e.g. from humans or rodents into monomers, hence obtaining VH or VL as a single domain Ab. Although most research into single domain antibodies is currently based on heavy chain variable domains, nanobodies derived from light chains have also been shown to bind specifically to target epitopes. Examples of single domain antibodies are called sdAb, nanobodies or single variable domain antibodies.

A (single domain mAb)$_2$ is hence a monoclonal antibody construct composed of (at least) two single domain monoclonal antibodies, which are individually selected from the group comprising VH, VL, $V_HH$ and $V_{NAR}$. The linker is preferably in the form of a peptide linker. Similarly, an "scFv-single domain mAb" is a monoclonal antibody construct composed of at least one single domain antibody as described above and one scFv molecule as described above. Again, the linker is preferably in the form of a peptide linker.

It is also envisaged that the antibody construct of the invention has, in addition to its function to bind to the target antigen and CD3, a further function. In this format, the antibody construct is a trifunctional or multifunctional antibody construct by targeting target cells through binding to the target antigen, mediating cytotoxic T cell activity through CD3 binding and providing a further function such as a label (fluorescent etc.), a therapeutic agent such as a toxin or radionuclide, etc.

Covalent modifications of the antibody constructs are also included within the scope of this invention, and are generally, but not always, done post-translationally. For example, several types of covalent modifications of the antibody construct are introduced into the molecule by reacting specific amino acid residues of the antibody construct with an organic derivatizing agent that is capable of reacting with selected side chains or the N- or C-terminal residues.

Cysteinyl residues are most commonly reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0. Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using $^{125}I$ or $^{131}I$ to prepare labeled proteins for use in radioimmunoassay, the chloramine T method described above being suitable.

Carboxyl side groups (aspartyl or glutamyl) are selectively modified by reaction with carbodiimides (R'-N=C=N--R'), where R and R' are optionally different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking the antibody constructs of the present invention to a water-insoluble support matrix or surface for use in a variety of methods. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the $\alpha$-amino groups of lysine, arginine, and histidine side chains (T. E. Creighton, Proteins: Structure and Molecular Properties, W. H. Freeman & Co., San Francisco, 1983, pp. 79-86), acetylation of the N-terminal amine, and amidation of any C-terminal carboxyl group.

Another type of covalent modification of the antibody constructs included within the scope of this invention comprises altering the glycosylation pattern of the protein. As is known in the art, glycosylation patterns can depend on both the sequence of the protein (e.g., the presence or absence of particular glycosylation amino acid residues, discussed below), or the host cell or organism in which the protein is produced. Particular expression systems are discussed below.

Glycosylation of polypeptides is typically either N-linked or O-linked. N-linked refers to the attachment of the carbohydrate moiety to the side chain of an asparagine residue. The tri-peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri-peptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Addition of glycosylation sites to the antibody construct is conveniently accomplished by altering the amino acid sequence such that it contains one or more of the above-described tri-peptide sequences (for N-linked glycosylation sites). The alteration may also be made by the addition of, or substitution by, one or more serine or threonine residues to the starting sequence (for O-linked glycosylation sites). For ease, the amino acid sequence of an antibody construct is preferably altered through changes at the DNA level, particularly by mutating the DNA encoding the polypeptide at preselected bases such that codons are generated that will translate into the desired amino acids.

Another means of increasing the number of carbohydrate moieties on the antibody construct is by chemical or enzymatic coupling of glycosides to the protein. These procedures are advantageous in that they do not require production of the protein in a host cell that has glycosylation capabilities for N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330, and in Aplin and Wriston, 1981, CRC Crit. Rev. Biochem., pp. 259-306.

Removal of carbohydrate moieties present on the starting antibody construct may be accomplished chemically or enzymatically. Chemical deglycosylation requires exposure of the protein to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al., 1987, Arch. Biochem. Biophys. 259:52 and by Edge et al., 1981, Anal. Biochem. 118:131. Enzymatic cleavage of carbohydrate moieties on polypeptides can be achieved by the use of a variety of endo- and exo-glycosidases as described by Thotakura et al., 1987, Meth. Enzymol. 138:350. Glycosylation at potential glycosylation sites may be prevented by the use of the compound tunicamycin as described by Duskin et al., 1982, J. Biol. Chem. 257:3105. Tunicamycin blocks the formation of protein-N-glycoside linkages.

Other modifications of the antibody construct are contemplated herein. For example, another type of covalent modification of the antibody construct comprises linking the antibody construct to various non-proteinaceous polymers, including, but not limited to, various polyols such as polyethylene glycol, polypropylene glycol, polyoxyalkylenes, or copolymers of polyethylene glycol and polypropylene glycol, in the manner set forth in U.S. Pat. Nos. 4,640,835; 4,496,689; 4,301,144; 4,670,417; 4,791,192 or 4,179,337. In addition, as is known in the art, amino acid substitutions may be made in various positions within the antibody construct, e.g. in order to facilitate the addition of polymers such as PEG.

In some embodiments, the covalent modification of the antibody constructs of the invention comprises the addition of one or more labels. The labelling group may be coupled to the antibody construct *via* spacer arms of various lengths to reduce potential steric hindrance. Various methods for labelling proteins are known in the art and can be used in performing the present invention. The term "label" or "labelling group" refers to any detectable label. In general, labels fall into a variety of classes, depending on the assay in which they are to be detected - the following examples include, but are not limited to:

a) isotopic labels, which may be radioactive or heavy isotopes, such as radioisotopes or radionuclides (*e.g.,* $^{3}H$, $^{14}C$, $^{15}N$, $^{35}S$, $^{89}Zr$, $^{90}Y$, $^{99}Tc$, $^{111}In$, $^{125}I$, $^{131}I$)
b) magnetic labels (*e.g.,* magnetic particles)
c) redox active moieties
d) optical dye (including, but not limited to, chromophores, phosphors and fluorophores) such as fluorescent groups (*e.g.,* FITC, rhodamine, lanthanide phosphors), chemiluminescent groups, and fluorophores which can be either "small molecule" fluores or proteinaceous fluores
e) enzymatic groups (*e.g.* horseradish peroxidase, β-galactosidase, luciferase, alkaline phosphatase)
f) biotinylated groups
g) predetermined polypeptide epitopes recognized by a secondary reporter (*e.g.,* leucine zipper pair sequences, binding sites for secondary antibodies, metal binding domains, epitope tags, etc.)

By "fluorescent label" is meant any molecule that may be detected *via* its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes, Eugene, OR), FITC, Rhodamine, and Texas Red (Pierce, Rockford, IL), Cy5, Cy5.5, Cy7 (Amersham Life Science, Pittsburgh, PA). Suitable optical dyes, including fluorophores, are described in Molecular Probes Handbook by Richard P. Haugland.

Suitable proteinaceous fluorescent labels also include, but are not limited to, green fluorescent protein, including a Renilla, Ptilosarcus, or Aequorea species of GFP (Chalfie et al., 1994, Science 263:802-805), EGFP (Clontech Laboratories, Inc., Genbank Accession Number U55762), blue fluorescent protein (BFP, Quantum Biotechnologies, Inc. 1801 de Maisonneuve Blvd. West, 8th Floor, Montreal, Quebec, Canada H3H 1J9; Stauber, 1998, Biotechniques 24:462-471; Heim et al., 1996, Curr. Biol. 6:178-182), enhanced yellow fluorescent protein (EYFP, Clontech Laboratories, Inc.), luciferase (Ichiki et al., 1993, J. Immunol. 150:5408-5417), β galactosidase (Nolan et al., 1988, Proc. Natl. Acad. Sci. U.S.A. 85:2603-2607) and Renilla (WO92/15673, WO95/07463, WO98/14605, WO98/26277, WO99/49019, U.S. Patent Nos. 5292658, 5418155, 5683888, 5741668, 5777079, 5804387, 5874304, 5876995, 5925558).

Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al., 1994, FEBS Letters 344:191. The use of a modified leucine zipper that allows for stable trimerization of a heterologous protein fused thereto is described in Fanslow et al., 1994, Semin. Immunol. 6:267-78. In one approach, recombinant fusion proteins comprising the target antigen antibody fragment or derivative fused to a leucine zipper peptide are expressed in suitable host cells, and the soluble oligomeric target antigen antibody fragments or derivatives that form are recovered from the culture supernatant.

The antibody construct of the invention may also comprise additional domains, which are *e.g.* helpful in the isolation of the molecule or relate to an adapted pharmacokinetic profile of the molecule. Domains helpful for the isolation of an antibody construct may be selected from peptide motives or secondarily introduced moieties, which can be captured in an isolation method, e.g. an isolation column. Non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. StrepII-tag) and His-tag. All herein disclosed antibody constructs characterized by the identified CDRs are preferred to comprise a His-tag domain, which is generally known as a repeat of consecutive His residues in the amino acid sequence of a molecule, preferably of six His residues.

T cells or T lymphocytes are a type of lymphocyte (itself a type of white blood cell) that play a central role in cell-mediated immunity. There are several subsets of T cells, each with a distinct function. T cells can be distinguished from other lymphocytes, such as B cells and NK cells, by the presence of a T cell receptor (TCR) on the cell surface. The TCR is responsible for recognizing antigens bound to major histocompatibility complex (MHC) molecules and is composed of two different protein chains. In 95% of the T cells, the TCR consists of an alpha (α) and beta (β) chain. When the TCR engages with antigenic peptide and MHC (peptide / MHC complex), the T lymphocyte is activated through a series of biochemical events mediated by associated enzymes, co-receptors, specialized adaptor molecules, and activated or released transcription factors

The CD3 receptor complex is a protein complex and is composed of four chains. In mammals, the complex contains a CD3γ (gamma) chain, a CD3δ (delta) chain, and two CD3ε (epsilon) chains. These chains associate with the T cell receptor (TCR) and the so-called ζ (zeta) chain to form the T cell receptor CD3 complex and to generate an activation signal in T lymphocytes. The CD3γ (gamma), CD3δ (delta), and CD3ε (epsilon) chains are highly related cell-surface proteins of the immunoglobulin superfamily containing a single extracellular immunoglobulin domain. The intracellular tails of the CD3 molecules contain a single conserved motif known as an immunoreceptor tyrosine-based activation motif or ITAM for short, which is essential for the signaling capacity of the TCR. The CD3 epsilon molecule is a polypeptide which in humans is encoded by the *CD3E* gene which resides on chromosome 11. The sequence of a preferred human CD3 epsilon extracellular domain is shown in SEQ ID NO: **610**, and the most preferred CD3 binding epitope corresponding to amino acid residues 1-27 of the human CD3 epsilon extracellular domain is represented in SEQ ID NO: 7.

The redirected lysis of target cells via the recruitment of T cells by a multispecific, at least bispecific, antibody construct involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

Cytotoxicity mediated by bispecific antibody constructs can be measured in various ways. Effector cells can be e.g. stimulated enriched (human) CD8 positive T cells or unstimulated (human) peripheral blood mononuclear cells (PBMC). If the target cells are of macaque origin or express or are transfected with macaque target cell antigen, the effector cells should also be of macaque origin such as a macaque T cell line, e.g. 4119LnPx. The target cells should express (at least the extracellular domain of) target cell antigen, e.g. human or macaque target cell antigen. Target cells can be a cell line (such as CHO) which is stably or transiently transfected with target cell antigen, e.g. human or macaque target cell antigen. Alternatively, the target cells can be a target cell antigen positive natural expresser cell line, such as a human cancer cell line. Usually EC50 values are expected to be lower with target cell lines expressing higher levels of target cell antigen on the cell surface. The effector to target cell (E:T) ratio is usually about 10:1, but can also vary. Cytotoxic activity of bispecific antibody constructs can be measured in a $^{51}$chromium release assay (incubation time of about 18 hours) or in a in a FACS-based cytotoxicity assay (incubation time of about 48 hours). Modifications of the assay incubation time (cytotoxic reaction) are also possible. Other methods of measuring cytotoxicity are well-known to the skilled person and comprise MTT or MTS assays, ATP-based assays including bioluminescent assays, the sulforhodamine B (SRB) assay, WST assay, clonogenic assay and the ECIS technology.

The cytotoxic activity mediated by bispecific antibody constructs of the present invention is preferably measured in a cell-based cytotoxicity assay. It is represented by the $EC_{50}$ value, which corresponds to the half maximal effective concentration (concentration of the antibody construct which induces a cytotoxic response halfway between the baseline and maximum). Preferably, the $EC_{50}$ value of the bispecific antibody constructs is ≤20.000 pg/ml, more preferably ≤5000 pg/ml, even more preferably ≤1000 pg/ml, even more preferably ≤500 pg/ml, even more preferably ≤350 pg/ml, even more preferably ≤250 pg/ml, even more preferably ≤100 pg/ml, even more preferably ≤50 pg/ml, even more preferably ≤10 pg/ml, and most preferably ≤5 pg/ml.

Any of the above given $EC_{50}$ values can be combined with any one of the indicated scenarios of a cell-based cytotoxicity assay, e.g. in line with the method described in the appended example. For example, when (human) CD8 positive T cells or a macaque T cell line are used as effector cells, the $EC_{50}$ value of the target cell antigen/CD3 bispecific antibody construct is preferably ≤1000 pg/ml, more preferably ≤500 pg/ml, even more preferably ≤250 pg/ml, even more preferably ≤100 pg/ml, even more preferably ≤50 pg/ml, even more preferably ≤10 pg/ml, and most preferably ≤5 pg/ml. If in this assay the target cells are (human or macaque) target cell antigen transfected cells such as CHO cells, the $EC_{50}$ value of the target cell antigen/CD3 bispecific antibody construct is preferably ≤150 pg/ml, more preferably ≤100 pg/ml, even more preferably ≤50 pg/ml, even more preferably ≤30 pg/ml, even more preferably ≤10 pg/ml, and most preferably ≤5 pg/ml. If the target cells are a target cell antigen positive natural expresser cell line, then the $EC_{50}$ value is preferably ≤350 pg/ml, more preferably ≤250 pg/ml, even more preferably ≤200 pg/ml, even more preferably ≤100 pg/ml, even more preferably ≤150 pg/ml, even more preferably ≤100 pg/ml, and most preferably ≤50 pg/ml, or lower. When (human) PBMCs are used as effector cells, the $EC_{50}$ value of the target cell antigen/CD3 bispecific antibody construct is preferably ≤1000 pg/ml, more preferably ≤750 pg/ml, more preferably ≤500 pg/ml, even more preferably ≤350 pg/ml, even more preferably ≤250 pg/ml, even more preferably ≤100 pg/ml, and most preferably ≤50 pg/ml, or lower.

Preferably, the bispecific antibody constructs of the present invention do not induce / mediate lysis or do not essentially induce / mediate lysis of target cell antigen negative cells such as CHO cells. The term "do not induce lysis", "do not essentially induce lysis", "do not mediate lysis" or "do not essentially mediate lysis" means that an antibody constructs of the present invention does not induce or mediate lysis of more than 30%, preferably not more than 20%, more preferably not more than 10%, particularly preferably not more than 9%, 8%, 7%, 6% or 5% of target cell antigen negative cells, whereby lysis of a target cell antigen positive cell line is set to be 100%. This usually applies for concentrations of the antibody construct of up to 500 nM. The skilled person knows how to measure cell lysis without further ado. Moreover, the present specification teaches specific instructions how to measure cell lysis.

The difference in cytotoxic activity between the monomeric and the dimeric isoform of individual bispecific antibody constructs is referred to as "potency gap". This potency gap can e.g. be calculated as ratio between $EC_{50}$ values of the molecule's monomeric and dimeric form. Potency gaps of the bispecific antibody constructs of the present invention are preferably ≤ 5, more preferably ≤ 4, even more preferably ≤ 3, even more preferably ≤ 2 and most preferably ≤ 1.

The first and/or the second (or any further) binding domain(s) of the antibody construct of the invention is/are preferably cross-species specific for members of the mammalian order of primates. Cross-species specific CD3 binding domains are, for example, described in WO 2008/119567. According to one embodiment, the first and/or second binding domain, in addition to binding to a target cell antigen and human CD3, respectively, will also bind to the target cell antigen / CD3 of primates including (but not limited to) new world primates (such as *Callithrix jacchus, Saguinus Oedipus* or *Saimiri sciureus*), old world primates (such baboons and macaques), gibbons, and non-human *homininae.*

In one aspect of the invention, the first binding domain binds to human CDH19 (SEQ ID NO: 611) and further binds to macaque CDH19, such as CDH19 of *Macaca fascicularis* (SEQ ID NO: 612). The affinity of the first binding domain for macaque CDH19 is preferably ≤15 nM, more preferably ≤10 nM, even more preferably ≤5 nM, even more preferably ≤1 nM, even more preferably ≤05 nM, even more preferably ≤0.1 nM, and most preferably ≤0.05 nM or even ≤0.01 nM.

Preferably the affinity gap of the antibody constructs according to the invention for binding to their specific macaque versus human target antigen, e.g. macaque CDH19 versus human CDH19 [maCDH19:huCDH19], is between 0.1 and 10, more preferably between 0.2 and 5, even more preferably between 0.3 and 2.5, even more preferably between 0.4 and 2, and most preferably between 0.5 and 1.

In a further embodiment of the antibody construct of the invention, the antibody construct comprises an FcRn binding peptide at the N-terminus and an FcRn binding peptide at the C-terminus selected from the group consisting of:

(a) the FcRn binding peptide at the N-terminus comprises the amino acid sequence **QRFVTGHFGGLYPANG** (SEQ ID NO: 2) and the one at the C-terminus comprises the amino acid sequence **QRFCTGHFGGLHPCNG** (SEQ ID NO: 5);
(b) the FcRn binding peptide at the N-terminus comprises the amino acid sequence **QRFVTGHFGGLYPANG** (SEQ ID NO: 2) and the one at the C-terminus comprises the amino acid sequence **QRFVTGHFGGLHPANG** (SEQ ID NO: 3);
(c) the FcRn binding peptide at the N-terminus comprises the amino acid sequence **QRFVTGHFGGLHPANG** (SEQ ID NO: 3) and the one at the C-terminus comprises the amino acid sequence **QRFCTGHFGGLHPCNG** (SEQ ID NO: 5);
(d) the FcRn binding peptide at the N-terminus comprises the amino acid sequence **QRFVTGHFGGLHPANG** (SEQ ID NO: 3) and the one at the C-terminus comprises the amino acid sequence **QRFVTGHFGGLHPANG** (SEQ ID NO: 3);
(e) the FcRn binding peptide at the N-terminus comprises the amino acid sequence **QRFCTGHFGGLHPCNG** (SEQ ID NO: 5) and the one at the C-terminus comprises the amino acid sequence **QRFVTGHFGGLYPANG** (SEQ ID NO: 2); and
(f) the FcRn binding peptide at the N-terminus comprises the amino acid sequence **QRFCTGHFGGLHPCNG** (SEQ ID NO: 5) and the one at the C-terminus comprises the amino acid sequence **QRFVTGHFGGLHPANG** (SEQ ID NO: 3).

As apparent form the data presented in Example 1, it has been surprisingly found that cyclic peptides, *i.e.* peptides comprising a cys-loop, are on the one hand critical for the production e.g. in the cell culture (upstream production criteria) and the isolation of bispecific single chain constructs (downstream production criteria). On the other hand, the example shows that the positon of such cyclic peptide within the protein chain is decisive to the production and isolation of bispecific single chain constructs. The bispecific single chain antibody constructs of the invention comprising the identified combinations of FcRn binding peptides at the N-terminus and at the C-terminus of the construct allow the provision of an antibody construct with preferred tissue distribution characteristics while retaining their industrially acceptable upstream and downstream production criteria.

In a further embodiment of the antibody construct of the invention the FcRn binding peptides are linked to the antibody construct via peptide linker. It is preferred that the peptide linker has an amino acid sequence of $(GGGGS)_n$ (SEQ ID NO: $6)_n$ wherein "n" is an integer in the range of 1 to 5. Further preferred is an integer "n" in the range of 1 to 3, and most preferably "n" is 1 or 2.

In one embodiment of the invention the construct comprises a further domain binding to serum albumin.

A particularly preferred albumin binding domain is e.g. the peptide having the sequence RDWDFDVFGGGTPVGG (SEQ ID NO: 609). However, domain binding to albumin which are of liniear structur and do not comprise structures such cys-loops are preferred since such additional cys-loop structure in an antibody construct of the invention is assumed to be connected production issues.

Also in one embodiment of the antibody construct of the invention the target cell surface antigen, is a tumor antigen. It is preferred that this tumor antigen is selected from the group of consisting CDH19 (cadherin 19), MSLN (mesothelin, also described as megagaryocyte-potentiating factor [MPF]), DLL3, FLT3 (FMS-related tyrosine kinase 3, also described as stem cell tyrosine kinase 1 [STK1]) or FLK2), CD33 (also known as sialic acid-binding immunoglobuline-like lectin 3 [SIGLEC3]), CD20 (also known as B-lymphocytes surface antigen B1 [B1] or MS4A1) and EGFRvIII.

In an embodiment of the antibody construct of the invention the second binding domain binds to an epitope of human and *Callithrix jacchus, Saguinus oedipus* or *Saimiri sciureus* CD3ε chain, wherein the epitope is part of an amino acid sequence comprised in the group consisting of SEQ ID NO: 7 (human), SEQ ID NO: 8 (*Callithrix jacchus*), SEQ ID NO: 9 (*Saguinus oedipus*), and SEQ ID NO: 10 (*Saimiri sciureus*) and comprises at least the amino acid sequence Gln-Asp-Gly-Asn-Glu (SEQ ID NO: 11). *Callithrix jacchus and Saguinus oedipus* are both new world primate belonging to the family of *Callitrichidae,* while *Saimiri sciureus* is a new world primate belonging to the family of *Cebidae.*

In one embodiment the second binding domain comprises a VL region having CDR-L1-L3 and a VH region having CDR-H1 -H3 selected from the group consisting of:

(a) CDR-L1-L3 as depicted in SEQ ID NOs:12-14 and CDR-H1-H3 as depicted in SEQ ID NOs:15-17;
(b) CDR-L1-L3 as depicted in SEQ ID NOs:24-26 and CDR-H1-H3 as depicted in SEQ ID NOs:27-29;
(c) CDR-L1-L3 as depicted in SEQ ID NOs:36-38 and CDR-H1-H3 as depicted in SEQ ID NOs:39-41;
(d) CDR-L1-L3 as depicted in SEQ ID NOs:48-50 and CDR-H1-H3 as depicted in SEQ ID NOs:51-53;
(e) CDR-L1-L3 as depicted in SEQ ID NOs:60-62 and CDR-H1-H3 as depicted in SEQ ID NOs:63-65;
(f) CDR-L1-L3 as depicted in SEQ ID NOs:72-74 and CDR-H1-H3 as depicted in SEQ ID NOs:75-77;
(g) CDR-L1-L3 as depicted in SEQ ID NOs:84-86 and CDR-H1-H3 as depicted in SEQ ID NOs:87-89;
(h) CDR-L1-L3 as depicted in SEQ ID NOs:96-98 and CDR-H1-H3 as depicted in SEQ ID NOs:99-101;
(i) CDR-L1-L3 as depicted in SEQ ID NOs:108-110 and CDR-H1-H3 as depicted in SEQ ID NOs:111-113;
(j) CDR-L1-L3 as depicted in SEQ ID NOs:120-122 and CDR-H1-H3 as depicted in SEQ ID NOs:123-125;
(k) CDR-L1-L3 as depicted in SEQ ID NOs: 616-618 and CDR-H1-H3 as depicted in SEQ ID NOs: 613-615;
(l) CDR-L1-L3 as depicted in SEQ ID NOs: 626-628 and CDR-H1-H3 as depicted in SEQ ID NOs: 623-625;
(m) CDR-L1-L3 as depicted in SEQ ID NOs: 636-638 and CDR-H1-H3 as depicted in SEQ ID NOs: 633-635;
(n) CDR-L1-L3 as depicted in SEQ ID NOs: 646-648 and CDR-H1-H3 as depicted in SEQ ID NOs: 643-645;
(o) CDR-L1-L3 as depicted in SEQ ID NOs: 656-658 and CDR-H1-H3 as depicted in SEQ ID NOs: 653-655;
(p) CDR-L1-L3 as depicted in SEQ ID NOs: 666-668 and CDR-H1-H3 as depicted in SEQ ID NOs: 663-665;
(q) CDR-L1-L3 as depicted in SEQ ID NOs: 676-678 and CDR-H1-H3 as depicted in SEQ ID NOs: 673-675; and
(r) CDR-L1-L3 as depicted in SEQ ID NOs: 686-688 and CDR-H1-H3 as depicted in SEQ ID NOs: 683-685.

In one embodiment the second binding domain comprises pairs of VH and VL chains selected from the group consisting of:

(a) a VH-chain as depicted in SEQ ID NO: 18 and a VL-chain as depicted in SEQ ID NO: 20;
(b) a VH-chain as depicted in SEQ ID NO: 30 and a VL-chain as depicted in SEQ ID NO: 32;
(c) a VH-chain as depicted in SEQ ID NO: 42 and a VL-chain as depicted in SEQ ID NO: 44;
(d) a VH-chain as depicted in SEQ ID NO: 54 and a VL-chain as depicted in SEQ ID NO: 56;
(e) a VH-chain as depicted in SEQ ID NO: 66 and a VL-chain as depicted in SEQ ID NO: 68;
(f) a VH-chain as depicted in SEQ ID NO: 78 and a VL-chain as depicted in SEQ ID NO: 80;
(g) a VH-chain as depicted in SEQ ID NO: 90 and a VL-chain as depicted in SEQ ID NO: 92;
(h) a VH-chain as depicted in SEQ ID NO: 102 and a VL-chain as depicted in SEQ ID NO: 104;
(i) a VH-chain as depicted in SEQ ID NO: 114 and a VL-chain as depicted in SEQ ID NO: 116;
(j) a VH-chain as depicted in SEQ ID NO: 126 and a VL-chain as depicted in SEQ ID NO: 12;8
(k) a VH-chain as depicted in SEQ ID NO: 619 and a VL-chain as depicted in SEQ ID NO: 620;
(l) a VH-chain as depicted in SEQ ID NO: 629 and a VL-chain as depicted in SEQ ID NO: 630;
(m) a VH-chain as depicted in SEQ ID NO: 639 and a VL-chain as depicted in SEQ ID NO: 640;
(n) a VH-chain as depicted in SEQ ID NO: 649 and a VL-chain as depicted in SEQ ID NO: 650;
(o) a VH-chain as depicted in SEQ ID NO: 659 and a VL-chain as depicted in SEQ ID NO: 660;
(p) a VH-chain as depicted in SEQ ID NO: 669 and a VL-chain as depicted in SEQ ID NO: 670;
(q) a VH-chain as depicted in SEQ ID NO: 679 and a VL-chain as depicted in SEQ ID NO: 680; and
(r) a VH-chain as depicted in SEQ ID NO: 689 and a VL-chain as depicted in SEQ ID NO: 690.

Also in one embodiment of the antibody construct of the invention the second binding domain comprises an amino acid sequence as depicted in SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 621, SEQ ID NO: 631, SEQ ID NO: 641, SEQ ID NO: 651, SEQ ID NO: 661, SEQ ID NO: 671, SEQ ID NO: 681, or SEQ ID NO: 691.

In a preferred embodiment of the antibody construct of the invention the domains are arranged from the N-terminus to the C-terminus a follows:

(FcRn binding peptide comprising SEQ ID NO: 1, whereas $X_1$ is Y or H) - (VH chain of the first binding domain) - (VL chain of the first binding domain) - (VH chain of the second binding domain) - (VL chain of the second binding domain) - (FcRn binding peptide comprising SEQ ID NO: 4)

or

(FcRn binding peptide comprising SEQ ID NO: 4) - (VH chain of the first binding domain) - (VL chain of the first binding domain) - (VH chain of the second binding domain) - (VL chain of the second binding domain) - (FcRn binding peptide comprising SEQ ID NO: 1, whereas $X_1$ is Y or H)

Amino acid sequence modifications of the antibody constructs described herein are also contemplated. For example, it may be desirable to improve the binding affinity and/or other biological properties of the antibody construct. Amino acid sequence variants of the antibody constructs are prepared by introducing appropriate nucleotide changes into the antibody constructs nucleic acid, or by peptide synthesis. All of the below described amino acd sequence modifications should result in an antibody construct which still retains the desired biological activity (binding to target cell antigen and to CD3) of the unmodified parental molecule.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

Amino acid modifications include, for example, deletions from, and/or insertions into, and/or substitutions of, residues within the amino acid sequences of the antibody constructs. Any combination of deletion, insertion, and substitution is made to arrive at the final construct, provided that the final construct possesses the desired characteristics. The amino acid changes also may alter post-translational processes of the antibody constructs, such as changing the number or position of glycosylation sites.

For example, 1, 2, 3, 4, 5, or 6 amino acids may be inserted or deleted in each of the CDRs (of course, dependent on their length), while 1, 2, 3, 4, 5, 6, 7, 8, 9, 10, 11, 12, 13, 14, 15, 16, 17, 18, 19, 20, or 25 amino acids may be inserted or deleted in each of the FRs. Preferably, amino acid sequence insertions include amino- and/or carboxyl-terminal fusions ranging in length from 1, 2, 3, 4, 5, 6, 7, 8, 9 or 10 residues to polypeptides containing a hundred or more residues, as well as intra-sequence insertions of single or multiple amino acid residues. An insertional variant of the antibody construct of the invention includes the fusion to the N-terminus or to the C-terminus of the antibody construct to an enzyme or a fusion to a polypeptide which increases the serum half-life of the antibody construct.

The sites of greatest interest for substitutional mutagenesis include the CDRs of the heavy and/or light chain, in particular the hypervariable regions, but FR alterations in the heavy and/or light chain are also contemplated. The substitutions are preferably conservative substitutions as described herein. Preferably, 1, 2, 3, 4, 5, 6, 7, 8, 9, or 10 amino acids may be substituted in a CDR, while 1, 2, 3, 4, 5, 6, 7, 8, 9, 10, 11, 12, 13, 14, 15, 16, 17, 18, 19, 20, or 25 amino acids may be substituted in the framework regions (FRs), depending on the length of the CDR or FR. For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

A useful method for identification of certain residues or regions of the antibody constructs that are preferred locations for mutagenesis is called "alanine scanning mutagenesis" as described by Cunningham and Wells in Science, 244: 1081-1085 (1989). Here, a residue or group of target residues within the antibody construct is/are identified (e.g. charged residues such as arg, asp, his, lys, and glu) and replaced by a neutral or negatively charged amino acid (most preferably alanine or polyalanine) to affect the interaction of the amino acids with the epitope.

Those amino acid locations demonstrating functional sensitivity to the substitutions then are refined by introducing further or other variants at, or for, the sites of substitution. Thus, while the site or region for introducing an amino acid sequence variation is predetermined, the nature of the mutation *per se* needs not to be predetermined. For example, to analyze or optimize the performance of a mutation at a given site, alanine scanning or random mutagenesis may be conducted at a target codon or region, and the expressed antibody construct variants are screened for the optimal combination of desired activity. Techniques for making substitution mutations at predetermined sites in the DNA having a known sequence are well known, for example, M13 primer mutagenesis and PCR mutagenesis. Screening of the mutants is done using assays of antigen binding activities, such as e.g. CDH19 binding.

Generally, if amino acids are substituted in one or more or all of the CDRs of the heavy and/or light chain, it is preferred that the then-obtained "substituted" sequence is at least 60%, more preferably 65%, even more preferably 70%, particularly preferably 75%, more particularly preferably 80% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the antibody construct may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions" listed in Table 1, below) is envisaged as long as the antibody construct retains its capability to bind to target cell antigen via the first binding domain and to CD3 epsilon via the second binding domain and/or its CDRs have an identity to the then substituted sequence (at least 60%, more preferably 65%, even more preferably 70%, particularly preferably 75%, more particularly preferably 80% identical to the "original" CDR sequence).
Conservative substitutions are shown in Table 1 under the heading of "preferred substitutions". If such substitutions result in a change in biological activity, then more substantial changes, denominated "exemplary substitutions" in Table A, or as further described below in reference to amino acid classes, may be introduced and the products screened for a desired characteristic.

Table A: Amino acid substitutions

| Original | Exemplary Substitutions | Preferred Substitutions |
|---|---|---|
| Ala (A) | val, leu, ile | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | Ala | ala |
| His (H) | asn, gln, lys, arg | arg |
| Ile (I) | leu, val, met, ala, phe | leu |
| Leu (L) | norleucine, ile, val, met, ala | ile |
| Lys (K) | arg, gln, asn | arg |
| Met (M) | leu, phe, ile | leu |
| Phe (F) | leu, val, ile, ala, tyr | tyr |
| Pro (P) | Ala | ala |
| Ser (S) | Thr | thr |
| Thr (T) | Ser | ser |
| Trp (W) | tyr, phe | tyr |
| Tyr (Y) | trp, phe, thr, ser | phe |
| Val (V) | ile, leu, met, phe, ala | leu |

Substantial modifications in the biological properties of the antibody construct of the present invention are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr; (3) acidic: asp, glu; (4) basic: asn, gin, his, lys, arg; (5) residues that influence chain orientation: gly, pro; and (6) aromatic : trp, tyr, phe.
Non-conservative substitutions will entail exchanging a member of one of these classes for another class. Any cysteine residue not involved in maintaining the proper conformation of the antibody construct may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).
For amino acid sequences, sequence identity and/or similarity is determined by using standard techniques known in the art, including, but not limited to, the local sequence identity algorithm of Smith and Waterman, 1981, Adv.

Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al., 1984, Nucl. Acid Res. 12:387-395, preferably using the default settings, or by inspection. Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, 1987, J. Mol. Evol. 35:351-360; the method is similar to that described by Higgins and Sharp, 1989, CABIOS 5:151-153. Useful PILEUP parameters including a default gap weight of 3.00, a default gap length weight of 0.10, and weighted end gaps.

Another example of a useful algorithm is the BLAST algorithm, described in: Altschul et al., 1990, J. Mol. Biol. 215:403-410; Altschul et al., 1997, Nucleic Acids Res. 25:3389-3402; and Karin et al., 1993, Proc. Natl. Acad. Sci. U.S.A. 90:5873-5787. A particularly useful BLAST program is the WU-BLAST-2 program which was obtained from Altschul et al., 1996, Methods in Enzymology 266:460-480. WU-BLAST-2 uses several search parameters, most of which are set to the default values. The adjustable parameters are set with the following values: overlap span=1, overlap fraction=0.125, word threshold (T)=ll. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., 1993, Nucl. Acids Res. 25:3389-3402. Gapped BLAST uses BLOSUM-62 substitution scores; threshold T parameter set to 9; the two-hit method to trigger ungapped extensions, charges gap lengths of k a cost of 10+k; Xu set to 16, and Xg set to 40 for database search stage and to 67 for the output stage of the algorithms. Gapped alignments are triggered by a score corresponding to about 22 bits.

Generally, the amino acid homology, similarity, or identity between individual variant CDRs are at least 60% to the sequences depicted herein, and more typically with preferably increasing homologies or identities of at least 65% or 70%, more preferably at least 75% or 80%, even more preferably at least 85%, 90%, 91%, 92%, 93%, 94%, 95%, 96%, 97%, 98%, 99%, and almost 100%. In a similar manner, "percent (%) nucleic acid sequence identity" with respect to the nucleic acid sequence of the binding proteins identified herein is defined as the percentage of nucleotide residues in a candidate sequence that are identical with the nucleotide residues in the coding sequence of the antibody construct. A specific method utilizes the BLASTN module of WU-BLAST-2 set to the default parameters, with overlap span and overlap fraction set to 1 and 0.125, respectively.

Generally, the nucleic acid sequence homology, similarity, or identity between the nucleotide sequences encoding individual variant CDRs and the nucleotide sequences depicted herein are at least 60%, and more typically with preferably increasing homologies or identities of at least 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99%, and almost 100%. Thus, a "variant CDR" is one with the specified homology, similarity, or identity to the parent CDR of the invention, and shares biological function, including, but not limited to, at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent CDR.

It is furthermore envisaged that the bispecific antibody constructs of the present invention exhibit therapeutic efficacy or anti-tumor activity. This can e.g. be assessed in a study as disclosed in the following example of an advanced stage human tumor xenograft model:

On day 1 of the study, $5 \times 10^6$ cells of a human target cell antigen positive cancer cell line are subcutaneously injected in the right dorsal flank of female NOD/SCID mice. When the mean tumor volume reaches about 100 $mm^3$, in vitro expanded human CD3 positive T cells are transplanted into the mice by injection of about $2 \times 10^7$ cells into the peritoneal cavity of the animals. Mice of vehicle control group 1 do not receive effector cells and are used as an untransplanted control for comparison with vehicle control group 2 (receiving effector cells) to monitor the impact of T cells alone on tumor growth. The antibody treatment starts when the mean tumor volume reaches about 200 $mm^3$. The mean tumor size of each treatment group on the day of treatment start should not be statistically different from any other group (analysis of variance). Mice are treated with 0.5 mg/kg/day of a target cell antigen/CD3 bispecifc antibody construct by intravenous bolus injection for about 15 to 20 days. Tumors are measured by caliper during the study and progress evaluated by intergroup comparison of tumor volumes (TV). The tumor growth inhibition T/C [%] is determined by calculating TV as T/C% = 100 $\times$ (median TV of analyzed group) / (median TV of control group 2).

The skilled person knows how to modify or adapt certain parameters of this study, such as the number of injected tumor cells, the site of injection, the number of transplanted human T cells, the amount of bispecific antibody constructs to be administered, and the timelines, while still arriving at a meaningful and reproducible result. Preferably, the tumor growth inhibition T/C [%] is ≤ 70 or ≤ 60, more preferably ≤ 50 or ≤ 40, even more preferably ≤ 30 or ≤ 20 and most preferably ≤ 10 or ≤ 5 or even ≤ 2.5.

In one embodiment, the bispecific antibody constructs of the present invention exhibit high monomer yields under standard research scale conditions, e.g., in a standard two-step purification process. Preferably the monomer yield of the antibody constructs according to the invention is ≥ 0.25 mg/L supernatant, more preferably ≥ 0.5 mg/L, even more preferably ≥ 1 mg/L, and most preferably ≥ 3 mg/L supernatant.

Likewise, the yield of the dimeric antibody construct isoforms and the monomer percentage (i.e., monomer: (monomer+dimer)) of the antibody constructs can be determined. The productivity of monomeric and dimeric antibody constructs and the calculated monomer percentage can e.g. be obtained in the SEC purification step of culture supernatant from standardized research-scale production in roller bottles. In one embodiment, the monomer percentage of the antibody constructs is ≥ 80%, more preferably ≥ 85%, even more preferably ≥ 90%, and most preferably ≥ 95%.

In a further embodiment, the percentage of identity to human germline of the antibody constructs according to the invention is ≥ 70% or ≥ 75%, more preferably ≥ 80% or ≥ 85%, even more preferably ≥ 90%, and most preferably ≥ 95%. Identity to human antibody germline gene products is thought to be an important feature to reduce the risk of therapeutic proteins to elicit an immune response against the drug in the patient during treatment. Hwang & Foote ("Immunogenicity of engineered antibodies"; Methods 36 (2005) 3-10) demonstrate that the reduction of non-human portions of drug antibody constructs leads to a decrease of risk to induce anti-drug antibodies in the patients during treatment. By comparing an exhaustive number of clinically evaluated antibody drugs and the respective immunogenicity data, the trend is shown that humanization of the V-regions of antibodies makes the protein less immunogenic (average 5.1 % of patients) than antibodies carrying unaltered non-human V regions (average 23.59 % of patients). A higher degree of identity to human sequences is hence desirable for V-region based protein therapeutics in the form of antibody constructs. For this purpose of determining the germline identity, the V-regions of VL can be aligned with the amino acid sequences of human germline V segments and J segments (http://vbase.mrc-cpe.cam.ac.uk/) using Vector NTI software and the amino acid sequence calculated by dividing the identical amino acid residues by the total number of amino acid residues of the VL in percent. The same can be for the VH segments (http://vbase.mrc-cpe.cam.ac.uk/) with the exception that the VH CDR3 may be excluded due to its high diversity and a lack of existing human germline VH CDR3 alignment partners. Recombinant techniques can then be used to increase sequence identity to human antibody germline genes.

In one embodiment, the antibody constructs have a preferred plasma stability (ratio of EC50 with plasma to EC50 w/o plasma) of ≤ 5, more preferably ≤ 4, even more preferably ≤ 3, and most preferably ≤ 2. The plasma stability of an antibody construct can be tested by incubation of the construct in human plasma at 37°C for 24 hours followed by EC50 determination in a 51chromium release cytotoxicity assay. The effector cells in the cytotoxicity assay can be stimulated enriched human CD8 positive T cells. Target cells can e.g. be CHO cells transfected with human trget cell antigen. The effector to target cell (E:T) ratio can be chosen as 10:1. The human plasma pool used for this purpose is derived from the blood of healthy donors collected by EDTA coated syringes. Cellular components are removed by centrifugation and the upper plasma phase is collected and subsequently pooled. As control, antibody constructs are diluted immediately prior to the cytotoxicity assay in RPMI-1640 medium. The plasma stability is calculated as ratio of EC50 (after plasma incubation) to EC50 (control).

It is preferred that the monomer to dimer conversion of antibody constructs of the invention is low. The conversion can be measured under different conditions and analyzed by high performance size exclusion chromatography. For example, incubation of the monomeric isoforms of the antibody constructs can be carried out for 7 days at 37°C and concentrations of e.g. 100 μg/ml or 250 μg/ml in an incubator. Under these conditions, it is preferred that the antibody constructs of the invention show a dimer percentage that is ≤5%, more preferably ≤4%, even more preferably ≤3%, even more preferably ≤2.5%, even more preferably ≤2%, even more preferably ≤1.5%, and most preferably ≤1%.

It is also preferred that the bispecific antibody constructs of the present invention present with very low dimer conversion after a number of freeze/thaw cycles. For example, the antibody construct monomer is adjusted to a concentration of 250 μg/ml e.g. in SEC running buffer and subjected to three freeze/thaw cycles (freezing at -80°C for 30 min followed by thawing for 30 min at room temperature), followed by high performance SEC to determine the percentage of initially monomeric antibody construct, which had been converted into dimeric antibody construct. Preferably the dimer percentages of the bispecific antibody constructs are ≤5%, more preferably ≤4%, even more preferably ≤3%, even more preferably ≤2.5%, even more preferably ≤2%, even more preferably ≤1.5%, and most preferably ≤1%, for example after three freeze/thaw cycles.

The bispecific antibody constructs of the present invention preferably show a favorable thermostability with melting temperatures above 60°C. This parameter can be determined as follows: Temperature melting curves are determined by Differential Scanning Calorimetry (DSC) to determine intrinsic biophysical protein stabilities of the antibody constructs. These experiments are performed using a MicroCal LLC (Northampton, MA, U.S.A) VP-DSC device. The energy uptake of a sample containing an antibody construct is recorded from 20°C to 90°C compared to a sample containing only the formulation buffer. The antibody constructs are adjusted to a final concentration of 250 μg/ml e.g.

in SEC running buffer. For recording of the respective melting curve, the overall sample temperature is increased stepwise. At each temperature T energy uptake of the sample and the formulation buffer reference is recorded. The difference in energy uptake Cp (kcal/mole/°C) of the sample minus the reference is plotted against the respective temperature. The melting temperature is defined as the temperature at the first maximum of energy uptake.

In a further embodiment the antibody construct according to the invention is stable at acidic pH. The more tolerant the antibody construct behaves at unphysiologic pH such as pH 5.5 (a pH which is required to run e.g. a cation exchange chromatography), the higher is the recovery of the antibody construct eluted from an ion exchange column relative to the total amount of loaded protein. Recovery of the antibody construct from an ion (e.g., cation) exchange column at pH 5.5 is preferably ≥ 30%, more preferably ≥ 40%, more preferably ≥ 50%, even more preferably ≥ 60%, even more preferably ≥ 70%, even more preferably ≥ 80%, and most preferably ≥ 90%.

In an alternative embodiment, the invention provides a polynucleotide encoding an antibody construct of the invention.

A polynucleotide is a biopolymer composed of 13 or more nucleotide monomers covalently bonded in a chain. DNA (such as cDNA) and RNA (such as mRNA) are examples of polynucleotides with distinct biological function. Nucleotides are organic molecules that serve as the monomers or subunits of nucleic acid molecules like DNA or RNA. The nucleic acid molecule or polynucleotide can be double stranded and single stranded, linear and circular. It is preferably comprised in a vector which is preferably comprised in a host cell. Said host cell is, e.g. after transformation or transfection with the vector or the polynucleotide of the invention, capable of expressing the antibody construct. For that purpose the polynucleotide or nucleic acid molecule is operatively linked with control sequences.

The genetic code is the set of rules by which information encoded within genetic material (nucleic acids) is translated into proteins. Biological decoding in living cells is accomplished by the ribosome which links amino acids in an order specified by mRNA, using tRNA molecules to carry amino acids and to read the mRNA three nucleotides at a time. The code defines how sequences of these nucleotide triplets, called codons, specify which amino acid will be added next during protein synthesis. With some exceptions, a three-nucleotide codon in a nucleic acid sequence specifies a single amino acid. Because the vast majority of genes are encoded with exactly the same code, this particular code is often referred to as the canonical or standard genetic code. While the genetic code determines the protein sequence for a given coding region, other genomic regions can influence when and where these proteins are produced.

Furthermore, the invention provides a vector comprising a polynucleotide / nucleic acid molecule of the invention.

A vector is a nucleic acid molecule used as a vehicle to transfer (foreign) genetic material into a cell. The term "vector" encompasses - but is not restricted to - plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

"Transfection" is the process of deliberately introducing nucleic acid molecules or polynucleotides (including vectors) into target cells. The term is mostly used for non-viral methods in eukaryotic cells. Transduction is often used to describe virus-mediated transfer of nucleic acid molecules or polynucleotides. Transfection of animal cells typically involves opening transient pores or "holes" in the cell membrane, to allow the uptake of material. Transfection can be carried out using calcium phosphate, by electroporation, by cell squeezing or by mixing a cationic lipid with the material to produce liposomes, which fuse with the cell membrane and deposit their cargo inside.

The term "transformation" is used to describe non-viral transfer of nucleic acid molecules or polynucleotides (including vectors) into bacteria, and also into non-animal eukaryotic cells, including plant cells. Transformation is hence the genetic alteration of a bacterial or non-animal eukaryotic cell resulting from the direct uptake through the cell membrane(s) from its surroundings and subsequent incorporation of exogenous genetic material (nucleic acid molecules).

Transformation can be effected by artificial means. For transformation to happen, cells or bacteria must be in a state of competence, which might occur as a time-limited response to environmental conditions such as starvation and cell density.

Moreover, the invention provides a host cell transformed or transfected with the polynucleotide / nucleic acid molecule or with the vector of the invention.

As used herein, the terms "host cell" or "recipient cell" are intended to include any individual cell or cell culture that can be or has/have been recipients of vectors, exogenous nucleic acid molecules, and polynucleotides encoding the antibody construct of the present invention; and/or recipients of the antibody construct itself. The introduction of the respective material into the cell is carried out by way of transformation, transfection and the like. The term "host cell" is also intended to include progeny or potential progeny of a single cell. Because certain modifications may occur in succeeding generations due to either natural, accidental, or deliberate mutation or due to environmental influences, such progeny may not, in fact, be completely identical (in morphology or in genomic or total DNA complement) to the parent cell, but is still included within the scope of the term as used herein. Suitable host cells include prokaryotic or eukaryotic cells, and also include but are not limited to bacteria, yeast cells, fungi cells, plant cells, and animal cells such as insect cells and mammalian cells, e.g., murine, rat, macaque or human.

The antibody construct of the invention can be produced in bacteria. After expression, the antibody construct of the invention is isolated from the *E. coli* cell paste in a soluble fraction and can be purified through, e.g., affinity chromatography and/or size exclusion. Final purification can be carried out similar to the process for purifying antibody expressed e.g., in CHO cells.

In addition to prokaryotes, eukaryotic microbes such as filamentous fungi or yeast are suitable cloning or expression hosts for the antibody construct of the invention. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe,* Kluyveromyces hosts such as *K. lactis, K. fragilis* (ATCC 12424), *K. bulgaricus* (ATCC 16045), *K. wickeramii* (ATCC 24178), *K. waltii* (ATCC 56500), *K. drosophilarum* (ATCC 36906), *K. thermotolerans,* and *K. marxianus;* yarrowia (EP 402 226); *Pichia pastoris* (EP 183 070); Candida; *Trichoderma reesia* (EP 244 234); *Neurospora crassa;* Schwanniomyces such as *Schwanniomyces occidentalis;* and filamentous fungi such as Neurospora, Penicillium, Tolypocladium, and Aspergillus hosts such as *A. nidulans* and *A. niger*.

Suitable host cells for the expression of glycosylated antibody construct of the invention are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruit fly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, Arabidopsis and tobacco can also be used as hosts. Cloning and expression vectors useful in the production of proteins in plant cell culture are known to those of skill in the art. See *e.g.* Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al. , J. Gen Virol. 36 : 59 (1977)); baby hamster kidney cells (BHK, ATCC CCL 10); Chinese hamster ovary cells/-DHFR (CHO, Urlaub et al., Proc. Natl. Acad. Sci. USA 77: 4216 (1980)); mouse sertoli cells (TM4, Mather, Biol. Reprod. 23: 243-251 (1980)); monkey kidney cells (CVI ATCC CCL 70); African green monkey kidney cells (VERO-76, ATCC CRL1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. (1982) 383: 44-68); MRC 5 cells; FS4 cells; and a human hepatoma line (Hep G2).

The invention also provides a process for the production of an antibody construct of the invention, said process comprising culturing a host cell of the invention under conditions allowing the expression of the antibody construct of the invention and recovering the produced antibody construct from the culture.

As used herein, the term "culturing" refers to the *in vitro* maintenance, differentiation, growth, proliferation and/or propagation of cells under suitable conditions in a medium. The term "expression" includes any step involved in the production of an antibody construct of the invention including, but not limited to, transcription, post-transcriptional modification, translation, post-translational modification, and secretion.

When using recombinant techniques, the antibody construct can be produced intracellularly, in the periplasmic space, or directly secreted into the medium. If the antibody construct is produced intracellularly, as a first step, the particulate debris, either host cells or lysed fragments, are removed, for example, by centrifugation or ultrafiltration.

Carter et al., Bio/Technology 10: 163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody construct of the invention prepared from the host cells can be recovered or purified using, for example, hydroxylapatite chromatography, gel electrophoresis, dialysis, and affinity chromatography. Other techniques for protein purification such as fractionation on an ion-exchange column, ethanol precipitation, Reverse Phase HPLC, chromatography on silica, chromatography on heparin SEPHAROSE™, chromatography on an anion or cation exchange resin (such as a polyaspartic acid column), chromato-focusing, SDS-PAGE, and ammonium sulfate precipitation are also available depending on the antibody to be recovered. Where the antibody construct of the invention comprises a CH3 domain, the Bakerbond ABX resin (J.T. Baker, Phillipsburg, NJ) is useful for purification.

Affinity chromatography is a preferred purification technique. The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose.

Affinity chromatography is a preferred purification technique. The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose.

In an alternative embodiment the invention provides a pharmaceutical composition comprising an antibody construct of the invention or an antibody construct produced according to the process of the invention.

One embodiment relates to an antibody construct of the invention or an antibody construct produced according to the process of the invention for use in the prevention, treatment or amelioration of a disease selected from a proliferative disease, a tumorous disease, a viral disease or an immunological disorder.

The formulations described herein are useful as pharmaceutical compositions in the treatment, amelioration and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

The term "amelioration" as used herein refers to any improvement of the disease state of a patient, by the administration of an antibody construct according to the invention to a subject in need thereof. Such an improvement may also be seen as a slowing or stopping of the progression patient's disease. The term "prevention" as used herein means the avoidance of the occurrence or re-occurrence of a patient having a disease as specified herein, by the administration of an antibody construct according to the invention to a subject in need thereof.

The term "disease" refers to any condition that would benefit from treatment with the antibody construct or the pharmaceutic composition described herein. This includes chronic and acute disorders or diseases including those pathological conditions that predispose the mammal to the disease in question.

The terms "subject in need" or those "in need of treatment" includes those already with the disorder, as well as those in which the disorder is to be prevented. The subject in need or "patient" includes human and other mammalian subjects that receive either prophylactic or therapeutic treatment.

The antibody construct of the invention will generally be designed for specific routes and methods of administration, for specific dosages and frequencies of administration, for specific treatments of specific diseases, with ranges of bio-availability and persistence, among other things. The materials of the composition are preferably formulated in concentrations that are acceptable for the site of administration.

Formulations and compositions thus may be designed in accordance with the invention for delivery by any suitable route of administration. In the context of the present invention, the routes of administration include, but are not limited to

- topical routes (such as epicutaneous, inhalational, nasal, opthalmic, auricular / aural, vaginal, mucosal);
- enteral routes (such as oral, gastrointestinal, sublingual, sublabial, buccal, rectal); and
- parenteral routes (such as intravenous, intraarterial, intraosseous, intramuscular, intracerebral, intracerebroventricular, epidural, intrathecal, subcutaneous, intraperitoneal, extra-amniotic, intraarticular, intracardiac, intradermal, intralesional, intrauterine, intravesical, intravitreal, transdermal, intranasal, transmucosal, intrasynovial, intraluminal).

The pharmaceutical compositions and the antibody construct of this invention are particularly useful for parenteral administration, e.g., subcutaneous or intravenous delivery, for example by injection such as bolus injection, or by infusion such as continuous infusion. Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

In particular, the present invention provides for an uninterrupted administration of the suitable composition. As a non-limiting example, uninterrupted or substantially uninterrupted, i.e. continuous administration may be realized by a small pump system worn by the patient for metering the influx of therapeutic agent into the body of the patient. The pharmaceutical composition comprising the antibody construct of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of the antibody constructs of the invention may be intravenous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may also be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

The compositions of the present invention can be administered to the subject at a suitable dose which can be determined e.g. by dose escalating studies by administration of increasing doses of the antibody construct of the invention exhibiting cross-species specificity described herein to non-chimpanzee primates, for instance macaques. As set forth above, the antibody construct of the invention exhibiting cross-species specificity described herein can be advantageously used in identical form in preclinical testing in non-chimpanzee primates and as drug in humans. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts or doses effective for this use will depend on the condition to be treated (the indication), the delivered antibody construct, the therapeutic context and objectives, the severity of the disease, prior therapy, the patient's clinical history and response to the therapeutic agent, the route of administration, the size (body weight, body surface or organ size) and/or condition (the age and general health) of the patient, and the general state of the patient's own immune system. The proper dose can be adjusted according to the judgment of the attending physician such that it can be administered to the patient once or over a series of administrations, and in order to obtain the optimal therapeutic effect.

A typical dosage may range from about 0.1 μg/kg to up to about 30 mg/kg or more, depending on the factors mentioned above. In specific embodiments, the dosage may range from 1.0 μg/kg up to about 20 mg/kg, optionally from 10 μg/kg up to about 10 mg/kg or from 100 μg/kg up to about 5 mg/kg.

A therapeutic effective amount of an antibody construct of the invention preferably results in a decrease in severity of disease symptoms, an increase in frequency or duration of disease symptom-free periods or a prevention of impairment or disability due to the disease affliction. For treating target cell antigen-expressing tumors, a therapeutically effective amount of the antibody construct of the invention, e.g. an anti-target cell antigen/anti-CD3 antibody construct, preferably inhibits cell growth or tumor growth by at least about 20%, at least about 40%, at least about 50%, at least about 60%, at least about 70%, at least about 80%, or at least about 90% relative to untreated patients. The ability of a compound to inhibit tumor growth may be evaluated in an animal model predictive of efficacy in human tumors.

The pharmaceutical composition can be administered as a sole therapeutic or in combination with additional therapies such as anti-cancer therapies as needed, e.g. other proteinaceous and non-proteinaceous drugs. These drugs may be administered simultaneously with the composition comprising the antibody construct of the invention as defined herein or separately before or after administration of said antibody construct in timely defined intervals and doses.

The term "effective and non-toxic dose" as used herein refers to a tolerable dose of an inventive antibody construct which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", *"in vivo* safety" or "tolerability" as used herein defines the administration of a drug without inducing severe adverse events directly after administration (local tolerance) and during a longer period of application of the drug. "Safety", *"in vivo* safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviations to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance hematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The above terms are also referred to *e.g.* in the Preclinical safety evaluation of biotechnology-derived pharmaceuticals S6; ICH Harmonised Tripartite Guideline; ICH Steering Committee meeting on July 16, 1997.

Finally, the invention provides a kit comprising an antibody construct of the invention or produced according to the process of the invention, a vector of the invention and/or a host cell of the invention.

In the context of the present invention, the term "kit" means two or more components - one of which corresponding to the antibody construct, the pharmaceutical composition, the vector or the host cell of the invention - packaged together in a container, recipient or otherwise. A kit can hence be described as a set of products and/or utensils that are sufficient to achieve a certain goal, which can be marketed as a single unit.

The kit may comprise one or more recipients (such as vials, ampoules, containers, syringes, bottles, bags) of any appropriate shape, size and material (preferably waterproof, e.g. plastic or glass) containing the antibody construct or the pharmaceutical composition of the present invention in an appropriate dosage for administration (see above). The kit may additionally contain directions for use (*e.g.* in the form of a leaflet or instruction manual), means for administering the antibody construct of the present invention such as a syringe, pump, infuser or the like, means for reconstituting the antibody construct of the invention and/or means for diluting the antibody construct of the invention.

The invention also provides kits for a single-dose administration unit. The kit of the invention may also contain a first recipient comprising a dried / lyophilized antibody construct and a second recipient comprising an aqueous formulation. In certain embodiments of this invention, kits containing single-chambered and multi-chambered pre-filled syringes (*e.g.*, liquid syringes and lyosyringes) are provided.

## Brief description of the drawings

**Figure 1:**
Eluation profiles for cation exchange column purification/isolation of BiTE antibody construct equipped with cyclic FcRnBP on both sites of the protein (panel A) compared to BiTE antibody construct with linaer FcRnBP on the n-terminus and cyclic FcRnBP on the c-terminus.

**Figure 2:**

FACS analysis of CDH19/CD3 bispecific antibodies on indicated cell lines: 1) CHO cells stably transfected with human CDH19, 2) human CD3 positive human T cell line HBP-ALL, 3) CHO cells stably transfected with cynomolgus CDH19, 4) macaque T cell line 4119 LnPx, 5) human melanoma cell line CHL-1 expressing native human CDH19, 6) untransfected CHO cells. Negative controls [1) to 6)]: detection antibodies without prior CDH19/CD3 bispecific antibody.

**Figure 3:**
Cytotoxic activity of CDH19/CD3 bispecific antibodies as measured in a 48-hour FACS-based cytotoxicity assay. Effector cells: CD3-expressing macaque T cell line 4119LnPx. Target cells: cynomolgus CDH19-transfected CHO cells. Effector to target cell (E:T)-ratio: 10:1. The figure shows the results for CDH19 2G6 302xI2C HALB, CDH19 2G6 302xI2C 156, CDH19 2G6 302xI2C LFcBY, CDH19 2G6 302xI2C LFcBY 156, CDH19 2G6 302xI2C D3 HALB and for a negative control.

**Figure 4:**
**Neonatal Fc Receptor (FcRn) is Expressed by Human Pulmonary Microvascular Endothelial Cells (HPMEC).**
Total cellular lysates of HPMEC and Jurkat E6.1 cells were separated by gel-electrophoresis. Anti-FcRn Western blotting showed a band at around 40 kDa in the HPMEC lysate (lane 1) which was less pronounced in the Jurkat E6.1 lysate (lane 2). As a control, anti-actin Western blotting showed a band at around 42 kDa in both HPMEC and Jurkat E6.1 total cellular lysates (lane 1 and 2) verifying equal amounts of protein were blotted.

**Figure 5:**
**Schematic Drawing of the *In-Vitro* Two-Chamber System.**
Human pulmonary microvascular endothelial cells (HPMEC) were plated onto a transwell insert membrane and grown until confluence. Prior to the experiment, integrity of the endothelial cell monolayer was confirmed by transendothelial electric resistance (TEER) measurement. At t = 0 h, analyte (BiTE antibody construct or Dextran) was given onto the endothelial cell monolayer inside of the transwell insert. The two-chamber system was then incubated for 4 h at 37°C. The amount of analyte that had passed through the endothelial cell monolayer into the lower chamber was quantified. After the experiment, the endothelial cell monolayer was stained and its integrity was checked microscopically.

**Figure 6:**
**Confluent Endothelial Cell Monolayers Exhibit Increased Transendothelial Electric Resistance (TEER) and Provide an Effective Barrier for Dextrans.**
Transwell inserts were either filled with assay medium alone (blank) or HPMEC were plated in triplicates (n = 3). **A.** After 24-30 h of cell growth, resistance (mean ± SD) of blanks and HPMEC monolayers was 199 ± 2 Ω and 249 ± 5 Ω, respectively resulting in a HPMEC TEER value (mean ± SD) of 16.67 ± 2.08 $\Omega{*}cm^2$. **B.** After 48 h of cell growth, FITC-Dextran 40 (2 mg/ml in assay medium containing 597 μM HSA) was given into the transwell inserts. After incubation for 4 h at 37°C, the mean fluorescence intensity (FI) ± SD of FITC-Dextran 40 which had passed through the membrane alone (blank) or the HPMEC monolayer into the lower chamber was 4256 ± 361 and 93 ± 15, respectively. C. After the experiment, HPMEC monolayers were stained and checked microscopically (10x magnification) for confluence (lower lane). Data were analyzed by GraphPad Prism 6 software for statistical significance using an unpaired t-test (****: $P \leq 0.0001$).

**Figure 7:**
**Half-Life-Extended BiTE Antibody construct CDH19-LfcBY Is Efficiently Transcytosed Across HPMEC Monolayers at Physiologic HSA Concentrations.**
HPMEC were plated onto transwell insert membranes in triplicates (n = 3) for each tested condition. After 24-30 h of cell growth, HPMEC TEER values ranged between 12 and 17 $\Omega{*}cm^2$ (data not shown). After 48 h of cell growth, 100 μl of the respective half-life-extended BiTE antibody construct (1 nM in assay medium containing 597 μM HSA) was given onto the endothelial cell monolayer inside of the transwell insert, while simultaneously filling 600 μl assay medium with HSA, but without BiTE antibody construct into the lower chamber. After incubation for 4 h at 37°C, the amount of BiTE antibody construct that had passed through the endothelial cell monolayer was quantified by electro-chemi-luminescence (ECL)-based ligand binding assay. Mean concentrations ± SD of recovered BiTE antibodies CDH19-156 (CDH19 2G6 302 x I2C-156-H6), CDH19-HALB (CDH19 2G6 302 x I2C-HALB-DY-H6), and CDH19-LfcBY (CDH19 2G6 302 x I2C-LfcBY-H6) were 0.424 ± 0.051 pM, 0.248 ± 0.026 pM, and 0.781 ± 0.069 pM, respectively. After the experiment, HPMEC monolayers were stained and checked microscopically (10x magnification) for confluence (data not shown). Data were analyzed by Microsoft Excel 2010, and GraphPad Prism 6 software for statistical significance by using one-way ANOVA combined with Tukey post-test (****: $P \leq 0.0001$; ***: $P \leq 0.001$;

*: $P \leq 0.05$).

**Figure 8:**

**Half-Life-Extended BiTE Antibody construct CDH19-LfcBY Is Efficiently Transcytosed Across HPMEC Monolayers in the Presence of 2% Human Serum.**

HPMEC were plated onto transwell insert membranes in triplicates (n = 3) for each tested condition. After 24-30 h of cell growth, HPMEC TEER values ranged between 19 and 31 $\Omega$*$cm^2$ (data not shown). After 48 h of cell growth, 100 $\mu$l of the respective half-life-extended BiTE antibody construct (1 nM in assay medium containing 2% pooled human serum) was given onto the endothelial cell monolayer inside of the transwell insert, while simultaneously filling 600 $\mu$l assay medium with 2% pooled human serum, but without BiTE antibody construct into the lower chamber. After incubation for 4 h at 37°C, the amount of BiTE antibody construct that had passed through the endothelial cell monolayer was quantified by electro-chemi-luminescence (ECL)-based ligand binding assay. Mean concentrations $\pm$ SD of recovered BiTE antibodies CDH19-156 (CDH19 2G6 302 x I2C-156-H6), CDH19-HALB (CDH19 2G6 302 x I2C-HALB-DY-H6), and CDH19-LfcBY (CDH19 2G6 302 x I2C-LfcBY-H6) were 0.849 $\pm$ 0.130 pM, 0.658 $\pm$ 0.047 pM, and 1.751 $\pm$ 0.212 pM, respectively. After the experiment, HPMEC monolayers were stained and checked microscopically (10x magnification) for confluence (data not shown). Data were analyzed by Microsoft Excel 2010, and GraphPad Prism 6 software for statistical significance by using one-way ANOVA combined with Tukey post-test (***: $P \leq 0.001$; ns: $P > 0.05$).

**Figure 9:**

**Half-Life-Extended BiTE Antibody construct CDH19-LfcBY Is Efficiently Transcytosed Across HPMEC Monolayers in the Presence of 20% Human Serum.**

HPMEC were plated onto transwell insert membranes in triplicates (n = 3) for each tested condition. After 24-30 h of cell growth, HPMEC TEER values ranged between 19 and 31 $\Omega$*$cm^2$ (data not shown). After 48 h of cell growth, 100 $\mu$l of the respective half-life-extended BiTE antibody construct (1 nM in assay medium containing 20% pooled human serum) was given onto the endothelial cell monolayer inside of the transwell insert, while simultaneously filling 600 $\mu$l assay medium with 20% pooled human serum, but without BiTE antibody construct into the lower chamber. After incubation for 4 h at 37°C, the amount of BiTE antibody construct that had passed through the endothelial cell monolayer was quantified by electro-chemi-luminescence (ECL)-based ligand binding assay. Mean concentrations $\pm$ SD of recovered BiTE antibodies CDH19-156 (CDH19 2G6 302 x I2C-156-H6), CDH19-HALB (CDH19 2G6 302 x I2C-HALB-DY-H6), and CDH19-LfcBY (CDH19 2G6 302 x I2C-LfcBY-H6) were 0.758 $\pm$ 0.027 pM, 0.761 $\pm$ 0.027 pM, and 1.976 $\pm$ 0.355 pM, respectively. After the experiment, HPMEC monolayers were stained and checked microscopically (10x magnification) for confluence (data not shown). Data were analyzed by Microsoft Excel 2010, and GraphPad Prism 6 software for statistical significance by using one-way ANOVA combined with Tukey post-test (***: $P \leq 0.001$; ns: $P > 0.05$).

**Figure 10:**

Pharmacokinetics of BiTE antibody constructs

Four molecules named 1) 2G6-156; 2) 2G6-LFcBy; 3) 2G6-LFcBy-156; 4) 2G6-D3HSA were tested in the cynomolgus monkey in the context of a pharmacokinetic (PK) study. The figure shows the results in connection with Example 5.

**Figure 11:**

**Efficient Transcytosis of Half-Life-Extended BiTE-LfcBY is Target Independent**

**A)** HPMECs were plated onto transwell insert membranes. After 24h of cell growth, HPMEC TEER values ranged between 13 and 23 $\Omega$*$cm^2$. After 48 h of cell growth, 100 $\mu$l of the respective half-life-extended BiTE antibody construct (1 nM in assay medium containing 20% pooled human serum) was plated onto the endothelial cell monolayer inside the transwell insert (n = 4 up to n=10). 600 $\mu$l of assay medium with 20% pooled human serum without BiTE antibody construct was placed into the lower chamber. After incubation for 4 h at 37°C, the amount of BiTE antibody construct that had passed through the endothelial cell monolayer was quantified by electro-chemi-luminescence (ECL)-based ligand binding assay. BiTE antibody constructs containing -LfcBY tag were normalized to their respective BiTE-156 counterpart and bar diagrams represent fold change $\pm$ SD of recovered BiTE-LfcBY antibodies. CD33-LfcBY was transcytosed 2.9$\pm$0.7 fold more efficiently than CD33-156. CHD19-LfcBY was transcytosed 3.1$\pm$0.4 fold more efficiently than CDH19-156. A Cadherin-LfcBY BiTE construct was transcytosed 2.4$\pm$0.5 fold more efficiently than a Cadherin-156 BiTE. Data were analyzed by Microsoft Excel 2010, and GraphPad Prism 6 software for statistical significance by unpaired t-test (****: $P \leq 0.0001$). **B)** The bar diagram represents mean values of BiTE-LfcBY transcytosis over three independent targets normalized to their BiTE-156 counterpart. BiTE-LfcBY constructs were transcytosed 2.8$\pm$0.3 fold more efficiently than BiTE-156 constructs (***: $P \leq 0.001$).

**Figure 12:**

**Different Half-Life-Extended BiTE-LfcBY Permutations are Transcytosed Efficiently** HPMECs were plated onto transwell insert membranes. After 24h of cell growth, HPMEC TEER values ranged between 13 and 23 $\Omega$*cm$^2$. After 48 h of cell growth, 100 $\mu$l of the respective half-life-extended BiTE antibody construct (1 nM in assay medium containing 20% pooled human serum) was plated onto the endothelial cell monolayer inside the transwell insert (n = 4 up to n=10). 600 $\mu$l of assay medium with 20% pooled human serum without BiTE antibody construct was placed into the lower chamber. After incubation for 4 h at 37°C, the amount of BiTE antibody construct that had passed through the endothelial cell monolayer was quantified by electro-chemi-luminescence (ECL)-based ligand binding assay. BiTE antibody constructs containing -LfcBY permutations were normalized to their respective BiTE-156 counterpart and bar diagrams represent fold change $\pm$ SD of recovered BiTE-LfcBY permutation antibodies. Compared to CH19-156, CH19-LH-FcB-CH was transcytosed 1.8$\pm$0.4, CH19-LH-FcB-LH 2.0$\pm$0.1, CH19-CH-FcB-LH 2.8$\pm$0.4, CH19-LY-FcB-LH 2.9$\pm$0.6, CH19-CH-FcB-LY 2.6$\pm$0.4 and CH19-LY-FcB-CH 3.1$\pm$0.4 fold more efficiently. Data were analyzed by Microsoft Excel 2010, and GraphPad Prism 6 software using ANOVA ONE-WAY and Dunnett's multiple comparisons test (****: $P \leq 0.0001$; ***: $P \leq 0.001$; **: $P \leq 0.01$).

**Figure 13:**

**Different Half-Life-Extended BiTE-LfcBY Permutations are Transcytosed Efficiently** HPMECs were plated onto transwell insert membranes. After 24h of cell growth, HPMEC TEER values ranged between 13 and 23 $\Omega$*cm$^2$. After 48 h of cell growth, 100 $\mu$l of the respective half-life-extended BiTE antibody BiTE antibody construct (1 nM in assay medium containing 20% pooled human serum) was plated onto the endothelial cell monolayer inside the transwell insert (n = 4 up to n=10). 600 $\mu$l of assay medium with 20% pooled human serum without BiTE antibody BiTE antibody construct was placed into the lower chamber. After incubation for 4 h at 37°C, the amount of BiTE antibody BiTE antibody construct that had passed through the endothelial cell monolayer was quantified by electro-chemi-luminescence (ECL)-based ligand binding assay. BiTE antibody constructs containing -LfcBY permutations were normalized to their respective BiTE-156 counterpart and bar diagrams represent fold change $\pm$ SD of recovered BiTE-LfcBY permutation antibodies. Compared to Cad-156, Cad-LH-FcB-CH was transcytosed 3.1$\pm$0.4, Cad-LH-FcB-LH 3.2$\pm$1 Cad-CH-FcB-LH 2.9$\pm$0.4, Cad-LY-FcB-LH 1.8$\pm$0.1, Cad-CH-FcB-LY 2.4$\pm$0.2 and Cad-LY-FcB-CH 2.4$\pm$0.5 fold more efficiently. Data were analyzed by Microsoft Excel 2010, and GraphPad Prism 6 software using ANOVA ONE-WAY and Dunnett's multiple comparisons test (****: $P \leq 0.0001$; *: $P \leq 0.05$).

## Examples

The following examples are provided for the purpose of illustrating specific embodiments or features of the present invention. These examples should not be construed as to limit the scope of this invention. The examples are included for purposes of illustration, and the present invention is limited only by the claims.

### Example 1:

### 1 Production and purification of bispecific single chain antibody constructs

Standardized research scale production of CDH19 BiTE antibody constructs was performed in roller bottles. Harvested culture supernatant was subjected after filtration to a two step BiTE antibody construct purification based on immobilized metal affinity chromatography (IMAC) capture and subsequent size exclusion chromatography (SEC).

### 1.1 IMAC capture step of BiTE antibody constructs

Äkta® Explorer Systems (GE Healthcare) controlled by Unicorn@ Software were used for chromatography. Immobilized metal affinity chromatography (IMAC) was performed using Fractogel EMD chelate® (Merck, Darmstadt) which was loaded with ZnCl2 according to the protocol provided by the manufacturer. The column was equilibrated with buffer A (20 mM sodium phosphate buffer , 0.1 M NaCl, 10 mM imidazole, pH 7.2) and the cell culture supernatant (1000 ml) applied to the column (10 ml packing volume) at a flow rate of 4 ml/min. The column was washed with buffer A to remove unbound sample. Bound protein was eluted using a two step gradient of buffer B (20 mM sodium phosphate buffer, 0.1 M NaCl, 0.5 M imidazole, pH 7.2) according to the following procedure:

Step 1: 10% buffer B in 5 column volumes
Step 2: 100% buffer B in 5 column volumes

Eluted protein fractions from step 2 were pooled for further purification and concentrated to 3 ml final volume using Vivaspin (Sartorius-Stedim, Göttingen-Germany) centrifugation units with Polyethersulfon PES membran and a molecular weight cut-off of 10 kDa. All chemicals were of research grade and purchased from Merck (Darmstadt, Germany).

### 1.2 Size Exclusion Chromatography SEC

Size exclusion chromatography was performed on a HiLoad 16/60 Superdex 200 prep grade column (GE Healthcare) equilibrated with SEC buffer (20 mM NaCl, 30 mM NaH2PO4, 100 mM L-Arginin, pH 7.0) at a flow rate of 1 ml/min. BiTE antibody construct monomer and dimer fractions were pooled and a 24% trehalose stock solution was added to reach a final trehalose concentration of 4%.

Protein pools were measured at 280 nm in polycarbonate cuvettes with 1 cm lightpath (Eppendorf, Hamburg-Germany) and protein concentration was calculated on the base of the Vector NTI sequence analysis software calculated factor for each protein.

BiTE monomer pools were adjusted to 250 μg/ml with additional BiTE formulation buffer (20 mM NaCl, 30 mM NaH2PO4, 100 mM L-Arginin, 4% Trehalose, pH 7.0).

### 1.3 Comparison of FcRn BiTE monomer yield from research scale production

As apparent form table 1 BiTE monomer yield for the BiTE with cyclic FcRn Binding Peptide FcRnBP on both sides (n- and c-terminal, see SEQ ID NO:145) of the BiTE antibody construct showed a more then threefold less production rate compared to the BiTE equipped with the linear FcRnBP at the n-terminus and cyclic FcRnBP on the c-terminus of the BiTE protein (SEQ ID NO: 132).

Table 1: BiTE monomer yield

| CDH19 BiTE | N-terminal FcRnBP | C-terminal FcRnBP | BiTE Monomer Yield [μg/l SN] |
|---|---|---|---|
| CH19 2G6 302 x I2C x FcBY | Cyclic | Cyclic | 1963 |
| CH19 2G6 302 x I2C -LFcBY | Linear | Cyclic | 6386 |

Much less BiTE monomer yield of BiTE version equipped with cyclic FcRnBP on both sides compared to BiTE version with linear FcRnBP at the n-terminus and cyclic FcRnBP at the c-terminus

### 1.4 Recovery of FcRn BiTE monomer from Cation Exchange Column

A 1 ml BioPro SP column manufactured by YMC (YMC Europe GmbH, Dinslaken-Germany) with sulphpropyl groups coupled to solid beads was connected to an Äkta Micro FPLC (GE Healthcare) device.

For column equilibration, sample dilution and washing a buffer consisting of 20 mM sodium dihydrogen phosphate and 30 mM sodium chloride adjusted with sodium hydroxide to pH of 5.5 was used.

For elution a buffer consisting of 20 mM NaH2PO4 and 1000 mM NaCl adjusted with sodium hydroxide to a pH of 5.5 was used. 50 μg of BiTE antibody construct monomer were diluted with dilution buffer to 50 ml final volume.

After column equilibration 40 ml of the diluted protein solution was applied to the column followed by a wash step using column equillibration buffer.

Elution was carried out by a steadily increasing gradient with elution buffer from zero to 100% over a total volume corresponding to 200 column volumes. The whole run was monitored at 280 nm optical absorption.

In contrast to the BiTE antibody construct equipped with cyclic FcRnBP on both side of the BiTE protein the BiTE antibody construct equipped with a linear FcRnBP on the n-terminal side and cyclic FcRnBP on the c-terminal side of the BiTE showed elution from cation exchange column. This behavior indicates much preferable purification behavior and product yield in a scaled up production of a BiTE antibody construct Figure 1

**Example 2:**

**Bispecific binding and interspecies cross-reactivity:**

For confirmation of binding to human and cyno CDH19 and to human and macaque CD3, bispecific antibodies were tested by flow cytometry using indicated cell lines. CHO cells transfected with human CDH19, with cyno CDH19, the human melanoma cell line CHL-1 expressing native human CDH19, CD3-expressing human T cell leukemia cell line HPB-ALL (DSMZ, Braunschweig, ACC483) and the CD3-expressing macaque T cell line 4119LnPx (Knappe A, et al., Blood, 2000, 95, 3256-3261) were used as antigen positive cell lines. Moreover, untransfected CHO cells were used as negative control.

For flow cytometry 200,000 cells of the respective cell lines were incubated for 30 min on ice with 50 μl of purified bispecific antibody at a concentration of 5 μg/ml. The cells were washed twice in PBS/10% FCS and binding of the constructs was detected with a murine anti-His antibody (AbD Serotec; diluted 1:1000 in 50 μl PBS/10% FCS). After washing, bound anti-His antibodies were detected with an Fc gamma-specific antibody (Dianova) conjugated to phycoerythrin, diluted 1:100 in PBS/10% FCS. Samples were measured by flow cytometry on a FACSCanto II instrument and analyzed by FACSDiva software (both from Becton Dickinson).

The CDH19/CD3 bispecific antibodies stained CHO cells transfected with human CDH19, cyno CDH19, the human CDH19-expressing melanoma cell lines CHL-1 as well as human and macaque T cells. Moreover, there was no staining of untransfected CHO cells (see Figure 2).

**Example 3:**

**Cytotoxic activity**

**Target cell labeling**

For the analysis of cell lysis in flow cytometry assays, the fluorescent membrane dye $DiOC_{18}$ (DiO) (Molecular Probes, #V22886) was used to label cynomolgus CDH19 positive CHO cells - as target cells and distinguish them from effector cells. Briefly, cells were harvested, washed once with PBS and adjusted to $10^6$ cell/mL in PBS containing 2 % (v/v) FBS and the membrane dye DiO (5 μL/$10^6$ cells). After incubation for 3 min at 37°C, cells were washed twice in complete RPMI medium and the cell number adjusted to $1.25 \times 10^5$ cells/mL. The vitality of cells was determined using 0.5 % (v/v) isotonic EosinG solution (Roth, #45380).

**Flow cytometry based analysis**

This assay was designed to quantify the lysis of cynomolgus CDH19-transfected CHO cells in the presence of serial dilutions of CDH19 bispecific antibodies.

Equal volumes of DiO-labeled target cells and effector cells (i.e. CD3-expressing macaque T cell line 4119LnPx) were mixed, resulting in an E:T cell ratio of 10:1. 160 μL of this suspension were transferred to each well of a 96-well plate. 40 μL of serial dilutions of the CDH19 bispecific antibodies and a negative control bispecific (an CD3-based bispecific antibody recognizing an irrelevant target antigen) or RPMI complete medium as an additional negative control were added. The bispecific antibody-mediated cytotoxic reaction proceeded for 48 hours in a 7% $CO_2$ humidified incubator. Then cells were transferred to a new 96-well plate and loss of target cell membrane integrity was monitored by adding propidium iodide (PI) at a final concentration of 1 μg/mL. PI is a membrane impermeable dye that normally is excluded from viable cells, whereas dead cells take it up and become identifiable by fluorescent emission.

Samples were measured by flow cytometry on a FACSCanto II instrument and analyzed by FACSDiva software (both from Becton Dickinson).

Target cells were identified as DiO-positive cells. PI-negative target cells were classified as living target cells. Percentage of cytotoxicity was calculated according to the following formula:

$$\text{Cytotoxicity}\,[\%] = \frac{n_{\text{dead target cells}}}{n_{\text{target cells}}} \times 100$$

n = number of events

Using GraphPad Prism 6 software (Graph Pad Software, San Diego), the percentage of cytotoxicity was plotted against the corresponding bispecific antibody concentrations. Dose response curves were analyzed with the four parametric logistic regression models for evaluation of sigmoid dose response curves with fixed hill slope and $EC_{50}$ values

were calculated.

The cytotox results using the above described system for CDH19 2G6 302xI2C HALB, CDH19 2G6 302xI2C 156, CDH19 2G6 302xI2C LFcBY, CDH19 2G6 302xI2C LFcBY 156, CDH19 2G6 302xI2C D3 HALB and for a negative control are shown in figure 3

**Example 4:**

***In-vitro* two-chamber system to mimic transcytosis of half-life extended BiTE antibodies through a human endothelial cell layer**

**Cultivation and harvesting of endothelial cells**

Human pulmonary microvascular endothelial cells (HPMEC) (PromoCell, #C-12281) were used as endothelial cell model. Cryopreserved HPMEC at passage 2 (> $5\times10^5$ cells/vial) were cultivated in MV2 basal medium (PromoCell, #C-22221) supplemented with endothelial cell growth supplement (ECGS, 1:100) (ScienCell, #1052), 5-7.5% pooled human serum (AMGEN, internal lot: 140625DrM01), and 5-10 mg/ml human albumin (HSA) (Behring, #C66444411B) (further referred to as growth medium) in cell culture flasks (Sarstedt, #831.810.302) at 37°C and 5% $CO_2$ in a Heraeus Cytoperm 2 (Thermo Scientific). Sub-cultivation of HPMEC was performed with the Detach Kit (PromoCell, #C-41200) consisting of HEPES-buffered balanced salt solution (PromoCell, #C-40000), trypsin-EDTA solution (0.04%/0.03%) (PromoCell, #C-41000), and trypsin neutralization solution (TNS) (PromoCell, #C-41100). Briefly, the medium was aspirated from the HPMEC layer and cells were washed with 3 ml of HEPES-buffered balanced salt solution. Addition of 3 ml of trypsin-EDTA solution diluted 1:1 with PBS for 1-3 min at room temperature led to detachment of HPMEC from the flask bottom. Inactivation of trypsin-EDTA solution was performed by addition of 3 ml TNS to the cell suspension. Cells were centrifuged at 300 g for 3 min in a Heraeus Megafuge 40 (Thermo Scientific) and seeded at a cell density of -10,000 cells/$cm^2$ into cell culture flasks (Sarstedt, #831.810.302, #833.911.302).

**Detection of neonatal Fc receptor (FcRn) in HPMEC by Western blot**

HPMEC (PromoCell, #C-12282, lot: 1071302.1, P7) were detached with EDTA (Biochrom, #L2113) and washed with PBS (Biochrom, #L1820). Jurkat cells E6.1 (ECCC, #88042803) were harvested and washed with PBS. $3\times10^6$ cells each were lysed in 100 $\mu$l lysis buffer (20 mM Trizma base (Sigma Aldrich, #T1503) pH = 8, 137 mM NaCl (Sigma-Aldrich, #S6546-1L), 10% Glycerol (Sigma-Aldrich, #G5516), 2 mM EDTA (Sigma-Aldrich, #03620), 1% Triton X100 (Sigma-Aldrich, #X100), 1x Protease Inhibitor Cocktail (Sigma-Aldrich, #I3911-1BO), 100 mM NaF (Sigma-Aldrich, #919), 500 $\mu$M $Na_3VO_4$ (Sigma-Aldrich, #S6508)) for 15 min on ice. Non-soluble parts of the total cellular lysates were pelleted by centrifugation at 13,200 g for 15 min at 4°C (Sigma Laborzentrifugen, #1K15) and supernatants were transferred into fresh 1.5 ml Eppendorf reaction tubes. 7.5 $\mu$l of lysates were diluted with 2 $\mu$l NuPAGE LDS sample buffer (life technologies, #NP0007) and denatured for 10 min at 70°C. 9.5 $\mu$l of the lysates and 10 $\mu$l of pre-stained protein standard (life technologies, #LC5800) were loaded onto a 4-12% BisTris Gel (life technologies, #NP0336BOX) and protein separation was performed in 1x NuPAGE MES running buffer (life technologies, #NP0002-02) by applying 200 V for 35 min. Proteins were transferred onto Nitrocellulose membrane (PALL BioTrace NT, #66485) according to XCell II Blot Module instructions (life technologies). Membrane was blocked using blocking solution (Thermo Scientific, #37542) for 1 h at room temperature under agitation. Membrane was incubated overnight at 4°C in anti-FcRn antibody solution (Novus Biologicals, #NBP1-89128, diluted 1:100 in blocking buffer, supplemented with 0.05% Tween-20 (Sigma-Aldrich, #P1379)) under agitation. Membrane was washed 3 times for 5 min at room temperature with PBS-Tween (Biochrom, #L1820 with 0.05% Tween-20 (Sigma-Aldrich, #P1379)). Membrane was incubated for 1 h at room temperature in HRP-labelled secondary antibody solution (anti-rabbit IgG HRP antibody (Thermo Scientific, #31460), diluted 1:5000 in PBS-Tween and 3-5% milk (Fluka, #70166)) under agitation. Membrane was washed 2 times for 5 min at room temperature with PBS-Tween and 1 time for 5 min with PBS. Membrane was incubated in SuperSignalWest Pico Substrate (Thermo Scientific, #1856135) according to the manufacturer's instructions. Light sensitive film (CL-XPosure™ Film, Thermo Scientific, #34088) was exposed to the membrane and developed in the developer (VELPEX, EXTRA-X). Subsequently, membrane was incubated overnight at 4°C in anti-beta actin antibody (Thermo Scientific, #MA1-91399, diluted 1:1000 in blocking buffer, supplemented with 0.05% Tween-20 (Sigma-Aldrich, #P1379)). Membrane was washed 3 times for 5 min at room temperature with PBS-Tween. Membrane was incubated for 1 h at room temperature in HRP-labelled secondary anti-mouse IgG antibody (Jackson ImmunoResearch, #415-035-100, diluted 1:5000 in PBS-Tween and 3-5% milk) under agitation. Membrane was developed as described in detail above.

### *In-vitro* two-chamber system

Plating of HPMEC

Polyester (PET) Membrane Transwell-Clear Inserts (Corning, #3470) with a pore size of 0.4 μm and an effective growth surface area of 0.33 $cm^2$ were coated with 10 μg/$cm^2$ Fibronectin (Sigma, #F2006) in PBS for 6 h at room temperature. The remaining Fibronectin solution was aspirated and inserts were washed once with PBS. 600 μl of growth medium were added to the outer chamber of the 24-well transwell system (Corning, #3470) and plates were equilibrated at 37°C for 30 min. HPMEC were harvested using the Detach Kit (PromoCell, #C-41200) as described above. Cells were plated in 200 μl pre-warmed growth medium at a density of $8\times10^4/cm^2$ onto equilibrated Fibronectin-coated transwell inserts. 4 wells were filled with growth medium only and used as blank values. After 24 h, 100 μl growth medium was added to both the inner well and outer chamber of the transwell system. Cell layer confluence was determined by transendothelial electric resistance (TEER) measurement.

### Transendothelial electric resistance (TEER) measurement of an endothelial cell monolayer

Electric resistance of each endothelial cell monolayer was measured using the Millicell ERS-2 system (Millipore, #MERS00002) with an STX03 adjustable electrode (Millipore, #MERSSTX03) according to the manufacturer's instructions. In brief, the functionality of the Millicell ERS-2 was tested by connecting the STX04 test electrode to the input port and switching on the power; the MODE switch was set to Ohm and, if necessary, the display was adjusted to 1000 Ω with a screwdriver at the "R Adj" screw. The STX03 adjustable electrode was sterilized by immersing the electrode in 80% ethanol for 5 min. For electric resistance measurement the STX03 electrode was connected to the ERS-2 via the input port, the MODE switch was set to Ohm and the power switch was turned on. The electrode tips were immersed into the transwell insert in a 90 degree angle; the shorter tip inside the transwell insert, not touching the endothelial cell monolayer; the longer tip outside the transwell insert, and just touching the bottom of the outer well (Corning, #3470). Blank transwell inserts without cells but containing the same medium were measured to determine background resistance. Before electric resistance measurement, plates were allowed to cool down to room temperature; when all wells were measured once, measurement was started again from beginning and values were averaged ($(\Omega_{measurement1} + \Omega_{measurement2})/2 = \Omega_{sample}$). The actual resistance of an individual cell monolayer was subsequently calculated by subtracting the mean resistance of the blank transwell inserts (Resistance $\Omega = \Omega_{sample} - \Omega_{blank}$). For the determination of the Unit Area Resistance (TEER), the effective membrane area (0.33 $cm^2$) of the 24-well transwell system (Corning, #3470) was taken into account (TEER = Resistance Ω * Effective Membrane Area). HPMEC cell monolayers characterized by a TEER value greater than 10 Ω*$cm^2$ were considered confluent.

### FITC-Dextran permeability of an endothelial cell monolayer

After 48 h of cell growth, transwell inserts containing a confluent HPMEC monolayer were transferred into a 24-well wash plate (FALCON, #353047), with each well pre-loaded with 700 μl pre-warmed MV basal medium (PromoCell, #C-22220) in order to wash the outside of the transwell inserts. 600 μl of assay medium (MV2 phenol red-free medium (PromoCell, #C-22226), ECGS (1:100) (ScienCell, #1052) and 597 μM HSA (Behring, #C66444411B)) were filled into blocked (700 μl/well of a 1:1 dilution of Starting Block (TBS) buffer (Thermo Scientific, #37542) in PBS / 10% FBS overnight at 37°C) receiver plates (24-well plates with ultra-low attachment surface (Corning, #3473)). Transwell inserts containing a confluent HPMEC monolayer were transferred from the wash plate into a pre-warmed receiver plate. At the same time, growth medium was removed from the inner well and the cell monolayer was washed once with 100 μl FITC-Dextran 40 (2 mg/ml in assay medium) (Sigma, #FD40). Then, 100 μl FITC-Dextran 40 (2 mg/ml in assay medium) were placed onto the HPMEC monolayer and the two-chamber assay was incubated for 4 h at 37°C and 5% $CO_2$. FITC-Dextran 40 fluorescence recovered from the outer well of the two-chamber system was quantified by measuring at excitation wavelength 485 nm and emission wavelength 535 nm using a SPECTRAFluor Plus (Serial number: 94493; Firmware: V 6.00 06_07_2003 Spectra; XFLUOR4 Version: V 4.40). For the two-chamber assay, all plates were handled on a heating plate (minitube, #12055/0010) at 37°C.

### Staining of an endothelial cell monolayer

After the two-chamber assay, cell monolayers were washed once with assay medium without FITC-Dextran 40. Then, 100 μl assay medium were placed onto the cell monolayers and 100 μl Paraformaldehyde (PFA) (4% in PBS) were added. The cell monolayers were fixed for 1 h at 37°C or overnight at room temperature at a final PFA concentration of 2%. PFA solution was removed and the cell monolayers were stained with 100 μl cell stain solution (Crystal Violet solution (Sigma-Aldrich, #HT90132-1L), 1:20 in 4% PFA in PBS) for 10 min at room temperature. Subsequently, cell monolayers were washed twice with 200 μl $ddH_2O$. Pictures of the HPMEC monolayers were taken using a Nikon Eclipse E800 microscope equipped with a 10x objective.

**Transcytosis of half-life-extended BiTE antibodies through an endothelial cell monolayer**

The two-chamber assay was performed with various half-life-extended BiTE antibodies as described in detail above for the FITC-Dextran 40 permeability. Assay medium consisted of MV2 phenol red-free medium (PromoCell, #C-22226), ECGS (1:100) (ScienCell, #1052), and HSA (Behring, #C66444411B) or pooled human serum (inactivated for 30 min at 56°C, AMGEN) as indicated. Dilutions of BiTE antibodies in above described assay medium were performed in Protein Low Binding Tubes (Sarstedt, #72.706.600). Identical assay medium was used for BiTE dilutions (i.e., in the upper transwell insert) and in the lower chamber of the two-chamber system. Plates were handled on a heating plate (minitube, #12055/0010) at 37°C. Different half-life-extended BiTE antibodies were assayed in 45 min time shifts to minimize time-dependent nonspecific adherence to plate surfaces. BiTE antibodies recovered from the lower well of the two-chamber system were quantified as described in detail below.

**Quantification of half-life-extended BiTE antibodies**

Quantification of BiTE antibodies from transcytosis experiments was done using a highly sensitive electro-chemi-luminescence (ECL)-based ligand binding assay.

Calibrator samples to build the standard reference curve were prepared by spiking known concentrations of the different BiTE antibodies into the respective test matrix (assay medium) followed by 10 dilution steps (1:2 dilutions, resulting in a total of 11 calibrator samples spanning a concentration range of 0.0048 to 5.0 ng/ml). Formulation of calibrator samples was matched to the respective formulation of unknown samples. For each BiTE antibody construct, the same material (construct and batch) was used both for preparing calibrator samples and for transcytosis assays. The respective test matrix of unknown samples was adjusted to equal amounts of HSA (597 μM (Behring, #C66444411B) / Tween-80 (0.05% (J.T. Baker, #4117-04) or pooled human serum (20%, inactivated for 30 min at 56°C, AMGEN) before quantification.

In a first step, capture antibody 3E5A5 (specific for the anti-CD3 binding part of BiTE antibodies, AMGEN) was immobilized on a carbon microtiter plate equipped with two electrodes (standard plate, MSD, #L15XA). To this end, plates were coated with 25 μl/well of a 1 μg/ml antibody solution overnight at 5 ± 3°C and then blocked with 150 μl/well of a 5% BSA solution for at least 1 h.

Samples were prepared as described above. 25 μl of each calibrator or unknown sample were added to individual wells and incubated for 1 h at 25 ± 2°C on a shaker. Plates were then washed 3 times with PBS-Tween (0.05% Tween-80) using an automated plate washer. BiTE antibody construct bound to the capture antibody was detected via biotinylated anti-Penta-His antibody (Qiagen, #34440; 1 μg/ml, 25 μl/well, 30 min at 25 ± 2°C on a shaker) followed by Streptavidin-SulfoTag (MSD, #R32AD-1; 1 μg/ml, 25 μl/well, 30 min at 25 ± 2°C on a shaker) without washing between incubations. Plates were washed 3 times with PBS-Tween (0.05% Tween-80) after the second incubation using an automated plate washer. Subsequently, 150 μl/well 1x MSD Read buffer (MSD, #R92TC-1) was added and the plates were measured using a Sektor Imager 2400 device (MSD).

ECL signals of standard reference curve calibrator samples were plotted against the known BiTE antibody construct concentrations and fitted by a 5-PL fit (weighting 1/y2; Software: SoftMaxPro GxP 5.4). Unknown sample concentrations were back-calculated from this regression curve. All unknown samples were analyzed in triplicates with the respective means being reported. Molar concentrations were calculated from these results.

After the two-chamber assay, endothelial cell monolayers were stained as described in detail above and checked for their integrity by light microscopy.

Detection of CDH19- and CD33-BiTE antibody constucts was performed as described above. Detection of Cadherin-BiTE was performed as follows. In a first step, a recombinant soluble form of the BiTE target antigen (AMGEN) was immobilized on a carbon microtiter plate equipped with two electrodes (standard plate, MSD, #L15XA). To this end, plates were coated with 25 μl/well of a 1 μg/ml antibody solution overnight at 5 ± 3°C and then blocked with 150 μl/well of a 5% BSA solution for at least 1 h.

Samples were prepared as described above. 25 μl of each calibrator or unknown sample were added to individual wells and incubated for 1 h at 25 ± 2°C on a shaker. Plates were then washed 3 times with PBS-Tween (0.05% Tween-80) using an automated plate washer. BiTE antibody construct bound to the target antigen was detected via SulfoTag conjugated antibody, specific for the anti-CD3 binding part of BiTE antibodies (3E5.E1-Rut, AMGEN). The antibody was diluted to a final concentration of 1 μg/mL. 25 μL were added per well and incubated for 30 min at 25 ± 2°C on a shaker.

**Example 5**

**Pharmacokinetics of BiTE antibody constructs**

Four molecules named 1) 2G6-156; 2) 2G6-LFcBy; 3) 2G6-LFcBy-156; 4) 2G6-D3HSA were tested in the cynomolgus monkey in the context of a pharmacokinetic (PK) study. In this PK study a dose of 6 μg/kg was administered as a single intravenous bolus injection. For each of the above compounds, a group of 2 animals were used. Blood samples were collected and serum was prepared for determination of serum concentration for each drug in both animals. Serum drug levels were measured using an immunoassay. The serum concentration-time data were used to determine PK parameters. Blood sample was collected at the following time points: predose, 0.05, 0.25, 0.5, 1, 4, 8, 24, 48, 72, 168, 240, and 336 hours post dose. The PK parameters were determined using standard non-compartmental analysis (NCA) methods.

For all drugs tested, serum levels were quantifiable for the vast majority of time points in all animals after drug administration. The PK profiles showed a biphasic exponential decline for all drugs tested. Using NCA methods, the following parameters were estimated: $AUC_{inf}$ (Area under the serum concentration-time curve), $V_{ss}$ (volume of distribution at steady state), CL (systemic clearance), MRT (mean residence time), and Terminal $t_{1/2}$ (half-life estimated from terminal phase). The PK parameters (mean of n=2) of each compound tested are summarized below:

The $AUC_{inf}$ was 568 hr*ng/mL, 366 hr*ng/mL, 1796 hr*ng/mL, and 1383 hr*ng/mL respectively for compounds 1, 2, 3 and 4. The $V_{ss}$ was 446 mL/kg, 594 mL/kg, 193 mL/kg, and 80.7 mL/kg respectively for compounds 1, 2, 3 and 4. Systemic clearance was 11.3 mL/hr/kg, 16.1 mL/hr/kg, 4.3 mL/hr/kg, and 4.9 mL/hr/kg respectively for compounds 1, 2, 3 and 4. The MRT value for compounds 1, 2, 3 and 4 were 42.3 hr, 39.8 hr, 48.7 hr, and 18.1 hr respectively and that for terminal half-life was 44.3 hr, 31.2 hr, 40.3 hr, and 13.5 hr respectively for compounds 1, 2, 3 and 4.

The terminal half- life and MRT of each of compounds 1, 2 and 3 were much higher (> 2-folds) than those for compound 4. Compounds 1, 2 and 3 present a longer half-life version of BiTE antibody constructs.

Results see figure 10.

| SEQ ID NO: | Description | SEQUENCE |
|---|---|---|
| 1. | linear FcRn binding peptide | QRFVTGHFGGLXPANG |
| 2. | linear FcRn binding peptide Y | QRFVTGHFGGLYPANG |
| 3. | linear FcRn binding peptide H | QRFVTGHFGGLHPANG |
| 4. | core FcRn binding peptide H | TGHFGGLHP |
| 5. | cyclic FcRn binding peptide H | QRFCTGHFGGLHPCNG |
| 6. | Peptide linker | GGGS |
| 7. | human CD3 ε (1-27) | QDGNEEMGGITQTPYKVSISGTTVILT |
| 8. | Callithrix jacchus CD3 ε (1-27) | QDGNEEMGDTTQNPYKVSISGTTVTLT |
| 9. | Saguinus oedipus CD3 ε (1-27) | QDGNEEMGDTTQNPYKVSISGTTVTLT |
| 10. | Saimiri sciureus CD3 ε (1-27) | QDGNEEIGDTTQNPYKVSISGTTVTLT |
| 11. | N-terminus of CD3ε | QDGNE |
| 12. | CDR-L1 of F6A | GSSTGAVTSGYYPN |
| 13. | CDR-L2 of F6A | GTKFLAP |
| 14. | CDR-L3 of F6A | ALWYSNRWV |
| 15. | CDR-H1 of F6A | IYAMN |
| 16. | CDR-H2 of F6A | RIRSKYNNYATYYADSVKS |
| 17. | CDR-H3 of F6A | HGNFGNSYVSFFAY |
| 18. | VH of F6A | EVQLVESGGGLVQPGGSLKLSCAASGFTFNIYAMNWVRQAPGKGLEWVARIRSKYNNYATYYA<br>DSVKSRFTISRDDSKNTAYLQMNNLKTEDTAVYYCVRHGNFGNSYVSFFAYWGQGTLVTVSS |
| 19. | VH of F6A | GAGGTGCAGCTGGTCGAGTCTGGAGGAGGATTGGTGCAGCCTGGAGGGTCATTGAAACTCTCA<br>TGTGCAGCCTCTGGATTCACCTTCAATATCTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAAGCAGGTTCACCATCTCCAGAGATGATTCAAAAAACACTGCCTATCTACAA |

| | | |
|---|---|---|
| | | ATGAACAACTTGAAAACTGAGGACACTGCCGTGTACTACTGTGTGAGACATGGGAACTTCGGTAATAGCTACGTATCCTTCTTCGCTTACTGGGGCCAAGGGACTCTGGTCACCGTCTCCTCA |
| 20. | VL of F6A | QTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 21. | VL of F6A | CAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 22. | VH-VL of F6A | EVQLVESGGGLVQPGGSLKLSCAASGFTFNIYAMNWVRQAPGKGLEWVARIRSKYNNYATYYADSVKSRFTISRDDSKNTAYLQMNNLKTEDTAVYYCVRHGNFGNSYVSFFAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 23. | VH-VL of F6A | GAGGTGCAGCTGGTCGAGTCTGGAGGAGGATTGGTGCAGCCTGGAGGGTCATTGAAACTCTCATGTGCAGCCTCTGGATTCACCTTCAATATCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAAGCAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTATCCTTCTTCGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 24. | CDR-L1 of H2C | GSSTGAVTSGYYPN |
| 25. | CDR-L2 of H2C | GTKFLAP |
| 26. | CDR-L3 of H2C | ALWYSNRWV |
| 27. | CDR-H1 of H2C | KYAMN |
| 28. | CDR-H2 of H2C | RIRSKYNNYATYYADSVKD |
| 29. | CDR-H3 of H2C | HGNFGNSYISYWAY |

| | | |
|---|---|---|
| 30. | VH of H2C | EVQLVESGGGLVQPGGSLKLSCAASGFTFNKYAMNWVRQAPGKGLEWVARIRSKYNNYATYYA DSVKDRFTISRDDSKNTAYLQMNNLKTEDTAVYYCVRHGNFGNSYISYWAYWGQGTLVTVSS |
| 31. | VH of H2C | GAGGTGCAGCTGGTCGAGTCTGGAGGAGGATTGGTGCAGCCTGGAGGGTCATTGAAACTCTCA TGTGCAGCCTCTGGATTCACCTTCAATAAGTACGCCATGAACTGGGTCCGCCAGGCTCCAGGA AAGGGTTTGGAATGGGTTGCTCGCATAAGAAGTAAATATAATAATTATGCAACATATTATGCC GATTCAGTGAAAGACAGGTTCACCATCTCCAGAGATGATTCAAAAAACACTGCCTATCTACAA ATGAACAACTTGAAAACTGAGGACACTGCCGTGTACTACTGTGTGAGACATGGGAACTTCGGT AATAGCTACATATCCTACTGGGCTTACTGGGGCCAAGGGACTCTGGTCACCGTCTCCTCA |
| 32. | VL of H2C | QTVVTQEPSLTVSPGGTVTLTCGSSTGAVTSGYYPNWVQQKPGQAPRGLIGGTKFLAPGTPAR FSGSLLGGKAALTLSGVQPEDEAEYYCALWYSNRWVFGGGTKLTVL |
| 33. | VL of H2C | CAGACTGTTGTGACTCAGGAACCTTCACTCACCGTATCACCTGGTGGAACAGTCACACTCACT TGTGGCTCCTCGACTGGGGCTGTTACATCTGGCTACTACCCAAACTGGGTCCAACAAAAACCA GGTCAGGCACCCCGTGGTCTAATAGGTGGGACTAAGTTCCTCGCCCCCGGTACTCCTGCCAGA TTCTCAGGCTCCCTGCTTGGAGGCAAGGCTGCCCTCACCCTCTCAGGGGTACAGCCAGAGGAT GAGGCAGAATATTACTGTGCTCTATGGTACAGCAACCGCTGGGTGTTCGGTGGAGGAACCAAA CTGACTGTCCTA |
| 34. | VH-VL of H2C | EVQLVESGGGLVQPGGSLKLSCAASGFTFNKYAMNWVRQAPGKGLEWVARIRSKYNNYATYYA DSVKDRFTISRDDSKNTAYLQMNNLKTEDTAVYYCVRHGNFGNSYISYWAYWGQGTLVTVSSG GGGSGGGGSGGGGSQTVVTQEPSLTVSPGGTVTLTCGSSTGAVTSGYYPNWVQQKPGQAPRGL IGGTKFLAPGTPARFSGSLLGGKAALTLSGVQPEDEAEYYCALWYSNRWVFGGGTKLTVL |
| 35. | VH-VL of H2C | GAGGTGCAGCTGGTCGAGTCTGGAGGAGGATTGGTGCAGCCTGGAGGGTCATTGAAACTCTCA TGTGCAGCCTCTGGATTCACCTTCAATAAGTACGCCATGAACTGGGTCCGCCAGGCTCCAGGA AAGGGTTTGGAATGGGTTGCTCGCATAAGAAGTAAATATAATAATTATGCAACATATTATGCC GATTCAGTGAAAGACAGGTTCACCATCTCCAGAGATGATTCAAAAAACACTGCCTATCTACAA ATGAACAACTTGAAAACTGAGGACACTGCCGTGTACTACTGTGTGAGACATGGGAACTTCGGT AATAGCTACATATCCTACTGGGCTTACTGGGGCCAAGGGACTCTGGTCACCGTCTCCTCAGGT GGTGGTGGTTCTGGCGGCGGCGGCTCCGGTGGTGGTGGTTCTCAGACTGTTGTGACTCAGGAA CCTTCACTCACCGTATCACCTGGTGGAACAGTCACACTCACTTGTGGCTCCTCGACTGGGGCT GTTACATCTGGCTACTACCCAAACTGGGTCCAACAAAAACCAGGTCAGGCACCCCGTGGTCTA ATAGGTGGGACTAAGTTCCTCGCCCCCGGTACTCCTGCCAGATTCTCAGGCTCCCTGCTTGGA GGCAAGGCTGCCCTCACCCTCTCAGGGGTACAGCCAGAGGATGAGGCAGAATATTACTGTGCT CTATGGTACAGCAACCGCTGGGTGTTCGGTGGAGGAACCAAACTGACTGTCCTA |
| 36. | CDR-L1 of H1E | GSSTGAVTSGYYPN |

| | | |
|---|---|---|
| 37. | CDR-L2 of H1E | GTKFLAP |
| 38. | CDR-L3 of H1E | ALWYSNRWV |
| 39. | CDR-H1 of H1E | SYAMN |
| 40. | CDR-H2 of H1E | RIRSKYNNYATYYADSVKG |
| 41. | CDR-H3 of H1E | HGNFGNSYLSFWAY |
| 42. | VH of H1E | EVQLVESGGGLEQPGGSLKLSCAASGFTFNSYAMNWVRQAPGKGLEWVARIRSKYNNYATYYA<br>DSVKGRFTISRDDSKNTAYLQMNNLKTEDTAVYYCVRHGNFGNSYLSFWAYWGQGTLVTVSS |
| 43. | VH of H1E | GAGGTGCAGCTGGTCGAGTCTGGAGGAGGATTGGAGCAGCCTGGAGGGTCATTGAAACTCTCA<br>TGTGCAGCCTCTGGATTCACCTTCAATTCGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAGGGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACCTATCCTTCTGGGCTTACTGGGGCCAAGGGACTCTGGTCACCGTCTCCTC |
| 44. | VL of H1E | QTVVTQEPSLTVSPGGTVTLTCGSSTGAVTSGYYPNWVQQKPGQAPRGLIGGTKFLAPGTPAR<br>FSGSLLGGKAALTLSGVQPEDEAEYYCALWYSNRWVFGGGTKLTVL |
| 45. | VL of H1E | CAGACTGTTGTGACTCAGGAACCTTCACTCACCGTATCACCTGGTGGAACAGTCACACTCACT<br>TGTGGCTCCTCGACTGGGGCTGTTACATCTGGCTACTACCCAAACTGGGTCCAACAAAAACCA<br>GGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGAT<br>GAGGCAGAATATTACTGTGCTCTATGGTACAGCAACCGCTGGGTGTTCGGTGGAGGAACCAAA<br>CTGACTGTCCTA |
| 46. | VH-VL of H1E | EVQLVESGGGLEQPGGSLKLSCAASGFTFNSYAMNWVRQAPGKGLEWVARIRSKYNNYATYYA<br>DSVKGRFTISRDDSKNTAYLQMNNLKTEDTAVYYCVRHGNFGNSYLSFWAYWGQGTLVTVSSG<br>GGGSGGGGSGGGGSQTVVTQEPSLTVSPGGTVTLTCGSSTGAVTSGYYPNWVQQKPGQAPRGL<br>IGGTKFLAPGTPARFSGSLLGGKAALTLSGVQPEDEAEYYCALWYSNRWVFGGGTKLTVL |
| 47. | VH-VL of H1E | GAGGTGCAGCTGGTCGAGTCTGGAGGAGGATTGGAGCAGCCTGGAGGGTCATTGAAACTCTCA<br>TGTGCAGCCTCTGGATTCACCTTCAATTCGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAGGGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACCTATCCTTCTGGGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA |

| | | |
|---|---|---|
| | | CCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 48. | CDR-L1 of G4H | GSSTGAVTSGYYPN |
| 49. | CDR-L2 of G4H | GTKFLAP |
| 50. | CDR-L3 of G4H | ALWYSNRWV |
| 51. | CDR-H1 of G4H | RYAMN |
| 52. | CDR-H2 of G4H | RIRSKYNNYATYYADSVKG |
| 53. | CDR-H3 of G4H | HGNFGNSYLSYFAY |
| 54. | VH of G4H | EVQLVESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSS |
| 55. | VH of G4H | GAGGTGCAGCTGGTCGAGTCTGGAGGAGGATTGGTGCAGCCTGGAGGGTCATTGAAACTCTCATGTGCAGCCTCTGGATTCACCTTCAATCGCTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGGGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACTTATCCTACTTCGCTTACTGGGGCCAAGGGACTCTGGTCACCGTCTCCTCA |
| 56. | VL of G4H | QTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 57. | VL of G4H | CAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGCTCTATGGTACAGCAACCGCTGGGTGTTCGGTGGAGGAACCAAACTGACTGTCCTA |
| 58. | VH-VL of G4H | EVQLVESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSSGGGGSGGGGSGGGGSQTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 59. | VH-VL of G4H | GAGGTGCAGCTGGTCGAGTCTGGAGGAGGATTGGTGCAGCCTGGAGGGTCATTGAAACTCTCA |

| | | |
|---|---|---|
| | | TGTGCAGCCTCTGGATTCACCTTCAATCGCTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAGGGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACTTATCCTACTTCGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA<br>CCTTCACTCACCGTATCACCTGGTGGAACAGTCACACTCACTTGTGGCTCCTCGACTGGGGCT<br>GTTACATCTGGCTACTACCCAAACTGGGTCCAACAAAAACCAGGTCAGGCACCCCGTGGTCTA<br>ATAGGTGGGACTAAGTTCCTCGCCCCCGGTACTCCTGCCAGATTCTCAGGCTCCCTGCTTGGA<br>GGCAAGGCTGCCCTCACCCTCTCAGGGGTACAGCCAGAGGATGAGGCAGAATATTACTGTGCT<br>CTATGGTACAGCAACCGCTGGGTGTTCGGTGGAGGAACCAAACTGACTGTCCTA |
| 60. | CDR-L1 of A2J | RSSTGAVTSGYYPN |
| 61. | CDR-L2 of A2J | ATDMRPS |
| 62. | CDR-L3 of A2J | ALWYSNRWV |
| 63. | CDR-H1 of A2J | VYAMN |
| 64. | CDR-H2 of A2J | RIRSKYNNYATYYADSVKK |
| 65. | CDR-H3 of A2J | HGNFGNSYLSWWAY |
| 66. | VH of A2J | EVQLVESGGGLVQPGGSLKLSCAASGFTFNVYAMNWVRQAPGKGLEWVARIRSKYNNYATYYA<br>DSVKKRFTISRDDSKNTAYLQMNNLKTEDTAVYYCVRHGNFGNSYLSWWAYWGQGTLVTVSS |
| 67. | VH of A2J | GAGGTGCAGCTGGTCGAGTCTGGAGGAGGATTGGTGCAGCCTGGAGGGTCATTGAAACTCTCA<br>TGTGCAGCCTCTGGATTCACCTTCAATGTCTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAAAGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACTTATCCTGGTGGGCTTACTGGGGCCAAGGGACTCTGGTCACCGTCTCCTCA |
| 68. | VL of A2J | QTVVTQEPSLTVSPGGTVTLTCRSSTGAVTSGYYPNWVQQKPGQAPRGLIGATDMRPSGTPAR<br>FSGSLLGGKAALTLSGVQPEDEAEYYCALWYSNRWVFGGGTKLTVL |
| 69. | VL of A2J | CAGACTGTTGTGACTCAGGAACCTTCACTCACCGTATCACCTGGTGGAACAGTCACACTCACT<br>TGTCGCTCCTCGACTGGGGCTGTTACATCTGGCTACTACCCAAACTGGGTCCAACAAAAACCA<br>GGTCAGGCACCCCGTGGTCTAATAGGTGCCACTGACATGAGGCCCTCTGGTACTCCTGCCAGA<br>TTCTCAGGCTCCCTGCTTGGAGGCAAGGCTGCCCTCACCCTCTCAGGGGTACAGCCAGAGGAT<br>GAGGCAGAATATTACTGTGCTCTATGGTACAGCAACCGCTGGGTGTTCGGTGGAGGAACCAAA |

| | | |
|---|---|---|
| | | CTGACTGTCCTA |
| 70. | VH-VL of A2J | EVQLVESGGGLVQPGGSLKLSCAASGFTFNVYAMNWVRQAPGKGLEWVARIRSKYNNYATYYA<br>DSVKKRFTISRDDSKNTAYLQMNNLKTEDTAVYYCVRHGNFGNSYLSWWAYWGQGTLVTVSSG<br>GGGSGGGGSGGGGSQTVVTQEPSLTVSPGGTVTLTCRSSTGAVTSGYYPNWVQQKPGQAPRGL<br>IGATDMRPSGTPARFSGSLLGGKAALTLSGVQPEDEAEYYCALWYSNRWVFGGGTKLTVL |
| 71. | VH-VL of A2J | GAGGTGCAGCTGGTCGAGTCTGGAGGAGGATTGGTGCAGCCTGGAGGGTCATTGAAACTCTCA<br>TGTGCAGCCTCTGGATTCACCTTCAATGTCTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAAAGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACTTATCCTGGTGGGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA<br>CCTTCACTCACCGTATCACCTGGTGGAACAGTCACACTCACTTGTCGCTCCTCGACTGGGGCT<br>GTTACATCTGGCTACTACCCAAACTGGGTCCAACAAAAACCAGGTCAGGCACCCCGTGGTCTA<br>ATAGGTGCCACTGACATGAGGCCCTCTGGTACTCCTGCCAGATTCTCAGGCTCCCTGCTTGGA<br>GGCAAGGCTGCCCTCACCCTCTCAGGGGTACAGCCAGAGGATGAGGCAGAATATTACTGTGCT<br>CTATGGTACAGCAACCGCTGGGTGTTCGGTGGAGGAACCAAACTGACTGTCCTA |
| 72. | CDR-L1 of E1L | GSSTGAVTSGYYPN |
| 73. | CDR-L2 of E1L | GTKFLAP |
| 74. | CDR-L3 of E1L | ALWYSNRWV |
| 75. | CDR-H1 of E1L | KYAMN |
| 76. | CDR-H2 of E1L | RIRSKYNNYATYYADSVKS |
| 77. | CDR-H3 of E1L | HGNFGNSYTSYYAY |
| 78. | VH of E1L | EVQLVESGGGLVQPGGSLKLSCAASGFTFNKYAMNWVRQAPGKGLEWVARIRSKYNNYATYYA<br>DSVKSRFTISRDDSKNTAYLQMNNLKTEDTAVYYCVRHGNFGNSYTSYYAYWGQGTLVTVSS |
| 79. | VH of E1L | GAGGTGCAGCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAATCGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACACATCCTACTACGCTTACTGGGGCCAAGGGACTCTGGTCACCGTCTCCTCA |
| 80. | VL of E1L | QTVVTQEPSLTVSPGGTVTLTCGSSTGAVTSGYYPNWVQQKPGQAPRGLIGGTKFLAPGTPAR |

| | | |
|---|---|---|
| | | FSGSLLGGKAALTLSGVQPEDEAEYYCALWYSNRWVFGGGTKLTVL |
| 81. | VL of E1L | CAGACTGTTGTGACTCAGGAACCTTCACTCACCGTATCACCTGGTGGAACAGTCACACTCACT<br>TGTGGCTCCTCGACTGGGGCTGTTACATCTGGCTACTACCCAAACTGGGTCCAACAAAAACCA<br>GGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGAT<br>GAGGCAGAATATTACTGTGCTCTATGGTACAGCAACCGCTGGGTGTTCGGTGGAGGAACCAAA<br>CTGACTGTCCTA |
| 82. | VH-VL of E1L | EVQLVESGGGLVQPGGSLKLSCAASGFTFNKYAMNWVRQAPGKGLEWVARIRSKYNNYATYYA<br>DSVKSRFTISRDDSKNTAYLQMNNLKTEDTAVYYCVRHGNFGNSYTSYYAYWGQGTLVTVSSG<br>GGGSGGGGSGGGGSQTVVTQEPSLTVSPGGTVTLTCGSSTGAVTSGYYPNWVQQKPGQAPRGL<br>IGGTKFLAPGTPARFSGSLLGGKAALTLSGVQPEDEAEYYCALWYSNRWVFGGGTKLTVL |
| 83. | VH-VL of E1L | GAGGTGCAGCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAATCGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACACATCCTACTACGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA<br>CCTTCACTCACCGTATCACCTGGTGGAACAGTCACACTCACTTGTGGCTCCTCGACTGGGGCT<br>GTTACATCTGGCTACTACCCAAACTGGGTCCAACAAAAACCAGGTCAGGCACCCCGTGGTCTA<br>ATAGGTGGGACTAAGTTCCTCGCCCCCGGTACTCCTGCCAGATTCTCAGGCTCCCTGCTTGGA<br>GGCAAGGCTGCCCTCACCCTCTCAGGGGTACAGCCAGAGGATGAGGCAGAATATTACTGTGCT<br>CTATGGTACAGCAACCGCTGGGTGTTCGGTGGAGGAACCAAACTGACTGTCCTA |
| 84. | CDR-L1 of E2M | RSSTGAVTSGYYPN |
| 85. | CDR-L2 of E2M | ATDMRPS |
| 86. | CDR-L3 of E2M | ALWYSNRWV |
| 87. | CDR-H1 of E2M | GYAMN |
| 88. | CDR-H2 of E2M | RIRSKYNNYATYYADSVKE |
| 89. | CDR-H3 of E2M | HRNFGNSYLSWFAY |
| 90. | VH of E2M | EVQLVESGGGLVQPGGSLKLSCAASGFTFNGYAMNWVRQAPGKGLEWVARIRSKYNNYATYYA<br>DSVKERFTISRDDSKNTAYLQMNNLKTEDTAVYYCVRHRNFGNSYLSWFAYWGQGTLVTVSS |
| 91. | VH of E2M | GAGGTGCAGCTGGTCGAGTCTGGAGGAGGATTGGTGCAGCCTGGAGGGTCATTGAAACTCTCA |

| | | |
|---|---|---|
| | | TGTGCAGCCTCTGGATTCACCTTCAATGGCTACGCCATGAACTGGGTCCGCCAGGCTCCAGGA AAGGGTTTGGAATGGGTTGCTCGCATAAGAAGTAAATATAATAATTATGCAACATATTATGCC GATTCAGTGAAAGAGAGGTTCACCATCTCCAGAGATGATTCAAAAAACACTGCCTATCTACAA ATGAACAACTTGAAAACTGAGGACACTGCCGTGTACTACTGTGTGAGACATAGGAACTTCGGT AATAGCTACTTATCCTGGTTCGCTTACTGGGGCCAAGGGACTCTGGTCACCGTCTCCTCA |
| 92. | VL of E2M | QTVVTQEPSLTVSPGGTVTLTCRSSTGAVTSGYYPNWVQQKPGQAPRGLIGATDMRPSGTPAR FSGSLLGGKAALTLSGVQPEDEAEYYCALWYSNRWVFGGGTKLTVL |
| 93. | VL of E2M | CAGACTGTTGTGACTCAGGAACCTTCACTCACCGTATCACCTGGTGGAACAGTCACACTCACT TGTCGCTCCTCGACTGGGGCTGTTACATCTGGCTACTACCCAAACTGGGTCCAACAAAAACCA GGTCAGGCACCCCGTGGTCTAATAGGTGCCACTGACATGAGGCCCTCTGGTACTCCTGCCAGA TTCTCAGGCTCCCTGCTTGGAGGCAAGGCTGCCCTCACCCTCTCAGGGGTACAGCCAGAGGAT GAGGCAGAATATTACTGTGCTCTATGGTACAGCAACCGCTGGGTGTTCGGTGGAGGAACCAAA CTGACTGTCCTA |
| 94. | VH-VL of E2M | EVQLVESGGGLVQPGGSLKLSCAASGFTFNGYAMNWVRQAPGKGLEWVARIRSKYNNYATYYA DSVKERFTISRDDSKNTAYLQMNNLKTEDTAVYYCVRHRNFGNSYLSWFAYWGQGTLVTVSSG GGGSGGGGSGGGGSQTVVTQEPSLTVSPGGTVTLTCRSSTGAVTSGYYPNWVQQKPGQAPRGL IGATDMRPSGTPARFSGSLLGGKAALTLSGVQPEDEAEYYCALWYSNRWVFGGGTKLTVL |
| 95. | VH-VL of E2M | GAGGTGCAGCTGGTCGAGTCTGGAGGAGGATTGGTGCAGCCTGGAGGGTCATTGAAACTCTCA TGTGCAGCCTCTGGATTCACCTTCAATGGCTACGCCATGAACTGGGTCCGCCAGGCTCCAGGA AAGGGTTTGGAATGGGTTGCTCGCATAAGAAGTAAATATAATAATTATGCAACATATTATGCC GATTCAGTGAAAGAGAGGTTCACCATCTCCAGAGATGATTCAAAAAACACTGCCTATCTACAA ATGAACAACTTGAAAACTGAGGACACTGCCGTGTACTACTGTGTGAGACATAGGAACTTCGGT AATAGCTACTTATCCTGGTTCGCTTACTGGGGCCAAGGGACTCTGGTCACCGTCTCCTCAGGT GGTGGTGGTTCTGGCGGCGGCGGCTCCGGTGGTGGTGGTTCTCAGACTGTTGTGACTCAGGAA CCTTCACTCACCGTATCACCTGGTGGAACAGTCACACTCACTTGTCGCTCCTCGACTGGGGCT GTTACATCTGGCTACTACCCAAACTGGGTCCAACAAAAACCAGGTCAGGCACCCCGTGGTCTA ATAGGTGCCACTGACATGAGGCCCTCTGGTACTCCTGCCAGATTCTCAGGCTCCCTGCTTGGA GGCAAGGCTGCCCTCACCCTCTCAGGGGTACAGCCAGAGGATGAGGCAGAATATTACTGTGCT CTATGGTACAGCAACCGCTGGGTGTTCGGTGGAGGAACCAAACTGACTGTCCTA |
| 96. | CDR-L1 of F7O | GSSTGAVTSGYYPN |
| 97. | CDR-L2 of F7O | GTKFLAP |
| 98. | CDR-L3 of F7O | ALWYSNRWV |

| | | |
|---|---|---|
| 99. | CDR-H1 of F7O | VYAMN |
| 100. | CDR-H2 of F7O | RIRSKYNNYATYYADSVKK |
| 101. | CDR-H3 of F7O | HGNFGNSYISWWAY |
| 102. | VH of F7O | EVQLVESGGGLVQPGGSLKLSCAASGFTFNVYAMNWVRQAPGKGLEWVARIRSKYNNYATYYA<br>DSVKKRFTISRDDSKNTAYLQMNNLKTEDTAVYYCVRHGNFGNSYISWWAYWGQGTLVTVSS |
| 103. | VH of F7O | GAGGTGCAGCTGGTCGAGTCTGGAGGAGGATTGGTGCAGCCTGGAGGGTCATTGAAACTCTCA<br>TGTGCAGCCTCTGGATTCACCTTCAATGTGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAAAGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACATATCCTGGTGGGCTTACTGGGGCCAAGGGACTCTGGTCACCGTCTCCTCA |
| 104. | VL of F7O | QTVVTQEPSLTVSPGGTVTLTCGSSTGAVTSGYYPNWVQQKPGQAPRGLIGGTKFLAPGTPAR<br>FSGSLLGGKAALTLSGVQPEDEAEYYCALWYSNRWVFGGGTKLTVL |
| 105. | VL of F7O | CAGACTGTTGTGACTCAGGAACCTTCACTCACCGTATCACCTGGTGGAACAGTCACACTCACT<br>TGTGGCTCCTCGACTGGGGCTGTTACATCTGGCTACTACCCAAACTGGGTCCAACAAAAACCA<br>GGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGAT<br>GAGGCAGAATATTACTGTGCTCTATGGTACAGCAACCGCTGGGTGTTCGGTGGAGGAACCAAA<br>CTGACTGTCCTA |
| 106. | VH-VL of F7O | EVQLVESGGGLVQPGGSLKLSCAASGFTFNVYAMNWVRQAPGKGLEWVARIRSKYNNYATYYA<br>DSVKKRFTISRDDSKNTAYLQMNNLKTEDTAVYYCVRHGNFGNSYISWWAYWGQGTLVTVSSG<br>GGGSGGGGSGGGGSQTVVTQEPSLTVSPGGTVTLTCGSSTGAVTSGYYPNWVQQKPGQAPRGL<br>IGGTKFLAPGTPARFSGSLLGGKAALTLSGVQPEDEAEYYCALWYSNRWVFGGGTKLTVL |
| 107. | VH-VL of F7O | GAGGTGCAGCTGGTCGAGTCTGGAGGAGGATTGGTGCAGCCTGGAGGGTCATTGAAACTCTCA<br>TGTGCAGCCTCTGGATTCACCTTCAATGTGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAAAGAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACATATCCTGGTGGGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA<br>CCTTCACTCACCGTATCACCTGGTGGAACAGTCACACTCACTTGTGGCTCCTCGACTGGGGCT<br>GTTACATCTGGCTACTACCCAAACTGGGTCCAACAAAAACCAGGTCAGGCACCCCGTGGTCTA |

| | | |
|---|---|---|
| | | ATAGGTGGGACTAAGTTCCTCGCCCCCGGTACTCCTGCCAGATTCTCAGGCTCCCTGCTTGGA<br>GGCAAGGCTGCCCTCACCCTCTCAGGGGTACAGCCAGAGGATGAGGCAGAATATTACTGTGCT<br>CTATGGTACAGCAACCGCTGGGTGTTCGGTGGAGGAACCAAACTGACTGTCCTA |
| 108. | CDR-L1 of F12Q | GSSTGAVTSGNYPN |
| 109. | CDR-L2 of F12Q | GTKFLAP |
| 110. | CDR-L3 of F12Q | VLWYSNRWV |
| 111. | CDR-H1 of F12Q | SYAMN |
| 112. | CDR-H2 of F12Q | RIRSKYNNYATYYADSVKG |
| 113. | CDR-H3 of F12Q | HGNFGNSYVSWWAY |
| 114. | VH of F12Q | EVQLVESGGGLVQPGGSLKLSCAASGFTFNSYAMNWVRQAPGKGLEWVARIRSKYNNYATYYA<br>DSVKGRFTISRDDSKNTAYLQMNNLKTEDTAVYYCVRHGNFGNSYVSWWAYWGQGTLVTVSS |
| 115. | VH of F12Q | GAGGTGCAGCTGGTCGAGTCTGGAGGAGGATTGGTGCAGCCTGGAGGGTCATTGAAACTCTCA<br>TGTGCAGCCTCTGGATTCACCTTCAATAGCTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAGGCAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACGTTTCCTGGTGGGCTTACTGGGGCCAAGGGACTCTGGTCACCGTCTCCTCA |
| 116. | VL of F12Q | QTVVTQEPSLTVSPGGTVTLTCGSSTGAVTSGNYPNWVQQKPGQAPRGLIGGTKFLAPGTPAR<br>FSGSLLGGKAALTLSGVQPEDEAEYYCVLWYSNRWVFGGGTKLTVL |
| 117. | VL of F12Q | CAGACTGTTGTGACTCAGGAACCTTCACTCACCGTATCACCTGGTGGAACAGTCACACTCACT<br>TGTGGCTCCTCGACTGGGGCTGTTACATCTGGCAACTACCCAAACTGGGTCCAACAAAAACCA<br>GGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGAT<br>GAGGCAGAATATTACTGTGTTCTATGGTACAGCAACCGCTGGGTGTTCGGTGGAGGAACCAAA<br>CTGACTGTCCTA |
| 118. | VH-VL of F12Q | EVQLVESGGGLVQPGGSLKLSCAASGFTFNSYAMNWVRQAPGKGLEWVARIRSKYNNYATYYA<br>DSVKGRFTISRDDSKNTAYLQMNNLKTEDTAVYYCVRHGNFGNSYVSWWAYWGQGTLVTVSSG<br>GGGSGGGGSGGGGSQTVVTQEPSLTVSPGGTVTLTCGSSTGAVTSGNYPNWVQQKPGQAPRGL<br>IGGTKFLAPGTPARFSGSLLGGKAALTLSGVQPEDEAEYYCVLWYSNRWVFGGGTKLTVL |
| 119. | VH-VL of F12Q | GAGGTGCAGCTGGTCGAGTCTGGAGGAGGATTGGTGCAGCCTGGAGGGTCATTGAAACTCTCA<br>TGTGCAGCCTCTGGATTCACCTTCAATAGCTACGCCATGAACTGGGTCCGCCAGGCTCCAGGA<br>AAGGGTTTGGAATGGGTTGCTCGCATAAGAAGTAAATATAATAATTATGCAACATATTATGCC |

| | | |
|---|---|---|
| | | GATTCAGTGAAAGGCAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACGTTTCCTGGTGGGCTTACTGGGGCCAAGGGACTCTGGTCACCGTCTCCTCAGGTGGTGGTGGTTCTGGCGGCGGCGGCTCCGGTGGTGGTGGTTCTCAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 120. | CDR-L1 of I2C | GSSTGAVTSGNYPN |
| 121. | CDR-L2 of I2C | GTKFLAP |
| 122. | CDR-L3 of I2C | VLWYSNRWV |
| 123. | CDR-H1 of I2C | KYAMN |
| 124. | CDR-H2 of I2C | RIRSKYNNYATYYADSVKD |
| 125. | CDR-H3 of I2C | HGNFGNSYISYWAY |
| 126. | VH of I2C | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSS |
| 127. | VH of I2C | GAGGTGCAGCTGGTCGAGTCTGGAGGAGGATTGGTGCAGCCTGGAGGGTCATTGAAACTCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACATATCCTACTGGGCTTACTGGGGCCAAGGGACTCTGGTCACCGTCTCCTCA |
| 128. | VL of I2C | QTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 129. | VL of I2C | CAGACTGTTGTGACTCAGGAACCTTCACTCACCGTATCACCTGGTGGAACAGTCACACTCACTTGTGGCTCCTCGACTGGGGCTGTTACATCTGGCAACTACCCAAACTGGGTCCAACAAAAACCAGGTCAGGCACCCCGTGGTCTAATAGGTGGGACTAAGTTCCTCGCCCCCGGTACTCCTGCCAGATTCTCAGGCTCCCTGCTTGGAGGCAAGGCTGCCCTCACCCTCTCAGGGGTACAGCCAGAGGATGAGGCAGAATATTACTGTGTTCTATGGTACAGCAACCGCTGGGTGTTCGGTGGAGGAACCAAACTGACTGTCCTA |
| 130. | VH-VL of I2C | EVQLVESGGGLVQPGGSLKLSCAASGFTFNKYAMNWVRQAPGKGLEWVARIRSKYNNYATYYA |

| | | |
|---|---|---|
| | | DSVKDRFTISRDDSKNTAYLQMNNLKTEDTAVYYCVRHGNFGNSYISYWAYWGQGTLVTVSSG<br>GGGSGGGGSGGGGSQTVVTQEPSLTVSPGGTVTLTCGSSTGAVTSGNYPNWVQQKPGQAPRGL<br>IGGTKFLAPGTPARFSGSLLGGKAALTLSGVQPEDEAEYYCVLWYSNRWVFGGGTKLTVL |
| 131. | VH-VL of I2C | GAGGTGCAGCTGGTCGAGTCTGGAGGAGGATTGGTGCAGCCTGGAGGGTCATTGAAACTCTCA<br>TGTGCAGCCTCTGGATTCACCTTCAATAAGTACGCCATGAACTGGGTCCGCCAGGCTCCAGGA<br>AAGGGTTTGGAATGGGTTGCTCGCATAAGAAGTAAATATAATAATTATGCAACATATTATGCC<br>GATTCAGTGAAAGACAGGTTCACCATCTCCAGAGATGATTCAAAAAACACTGCCTATCTACAA<br>ATGAACAACTTGAAAACTGAGGACACTGCCGTGTACTACTGTGTGAGACATGGGAACTTCGGT<br>AATAGCTACATATCCTACTGGGCTTACTGGGGCCAAGGGACTCTGGTCACCGTCTCCTCAGGT<br>GGTGGTGGTTCTGGCGGCGGCGGCTCCGGTGGTGGTGGTTCTCAGACTGTTGTGACTCAGGAA<br>CCTTCACTCACCGTATCACCTGGTGGAACAGTCACACTCACTTGTGGCTCCTCGACTGGGGCT<br>GTTACATCTGGCAACTACCCAAACTGGGTCCAACAAAAACCAGGTCAGGCACCCCGTGGTCTA<br>ATAGGTGGGACTAAGTTCCTCGCCCCCGGTACTCCTGCCAGATTCTCAGGCTCCCTGCTTGGA<br>GGCAAGGCTGCCCTCACCCTCTCAGGGGTACAGCCAGAGGATGAGGCAGAATATTACTGTGTT<br>CTATGGTACAGCAACCGCTGGGTGTTCGGTGGAGGAACCAAACTGACTGTCCTA |
| 132. | CDH19 14302 x I2C-LFcBY<br>SEQ ID 1572 of PCT/EP2014/051550 | QRFVTGHFGGLYPANGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |
| 133. | CDH19 14302 x I2C-LFcBY-156<br>SEQ ID 1573 of PCT/EP2014/051550 | QRFVTGHFGGLYPANGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGS |

| | | |
|---|---|---|
| | | QRFCTGHFGGLHPCNGGGGGSGGGSRDWDFDVFGGGTPVGGHHHHHH |
| 134. | CDH19 14302 CC x I2C-LFcBY<br>SEQ ID 2219 of PCT/EP2014/051550 | QRFVTGHFGGLYPANGGGGGSQVQLVESGGGVVQPGGSLRLSCAASGFTFSSYGMHWVRQAPG<br>KCLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGCGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGS QRFCTGHFGGLHPCNGHHHHHH |
| 135. | CDH19 14302 CC x I2C-LFcBY-156<br>SEQ ID 2220 of PCT/EP2014/051550 | QRFVTGHFGGLYPANGGGGGSQVQLVESGGGVVQPGGSLRLSCAASGFTFSSYGMHWVRQAPG<br>KCLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGCGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGS QRFCTGHFGGLHPCNG GGGGS GGGS<br>RDWDFDVFGGGTPVGGHHHHHH |
| 136. | CH19_2G6_302xI2C6 | QVQLVESGGGVVQPGGSLRLSCAASGFTFSSYGMHWVRQAPGKGLEWVAFIWYDGSNKYYADS<br>VKDRFTISRDNSKNTLYLQMNSLRAEDTAVYYCARRAGIIGTIGYYYGMDVWGQGTTVTVSSG<br>GGGSGGGGSGGGGSSYELTQPPSVSVSPGQTASITCSGDRLGEKYTSWYQQRPGQSPLLVIYQ<br>DTKRPSGIPERFSGSNSGNTATLTISGTQAMDEADYYCQAWESSTVVFGGGTKLTVLSGGGGS<br>EVQLVESGGGLVQPGGSLKLSCAASGFTFNKYAMNWVRQAPGKGLEWVARIRSKYNNYATYYA<br>DSVKDRFTISRDDSKNTAYLQMNNLKTEDTAVYYCVRHGNFGNSYISYWAYWGQGTLVTVSSG<br>GGGSGGGGSGGGGSQTVVTQEPSLTVSPGGTVTLTCGSSTGAVTSGNYPNWVQQKPGQAPRGL<br>IGGTKFLAPGTPARFSGSLLGGKAALTLSGVQPEDEAEYYCVLWYSNRWVFGGGTKLTVLHHH<br>HHH |
| 137. | CH19_2G6_302xI2C6-156 | QVQLVESGGGVVQPGGSLRLSCAASGFTFSSYGMHWVRQAPGKGLEWVAFIWYDGSNKYYADS<br>VKDRFTISRDNSKNTLYLQMNSLRAEDTAVYYCARRAGIIGTIGYYYGMDVWGQGTTVTVSSG<br>GGGSGGGGSGGGGSSYELTQPPSVSVSPGQTASITCSGDRLGEKYTSWYQQRPGQSPLLVIYQ<br>DTKRPSGIPERFSGSNSGNTATLTISGTQAMDEADYYCQAWESSTVVFGGGTKLTVLSGGGGS |

| | | |
|---|---|---|
| | | EVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSGGGSRDWDFDVFGGGTPVGGHHHHHH |
| 138. | CH19_2G6_302xI2C6-D3 | QVQLVESGGGVVQPGGSLRLSCAASGFTFSSYGMHWVRQAPGKGLEWVAFIWYDGSNKYYADSVKDRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSEEPQNLIKQNCELFEQLGEYKFQNALLVRYTKKVPQVSTPTLVEVSRNLGKVGSKCCKHPEAKRMPCAEDYLSVVLNQLCVLHEKTPVSDRVTKCCTESLVNRRPCFSALEVDETYVPKEFNAETFTFHADICTLSEKERQIKKQTALVELVKHKPKATKEQLKAVMDDFAAFVEKCCKADDKETCFAEEGKKLVAASQAALGLDYHHHHHH |
| 139. | CH19_2G6_302xI2C6-LH-FcB-CH | QRFVTGHFGGLHPANGGGGGSQVQLVESGGGVVQPGGSLRLSCAASGFTFSSYGMHWVRQAPGKGLEWVAFIWYDGSNKYYADSVKDRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 140. | CH19_2G6_302xI2C6-LH-FcB-LH | QRFVTGHFGGLHPANGGGGGSQVQLVESGGGVVQPGGSLRLSCAASGFTFSSYGMHWVRQAPGKGLEWVAFIWYDGSNKYYADSVKDRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGA |

| | | |
|---|---|---|
| | | VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 141. | CH19_2G6_302xI2C6-LY-FcB-LH | QRFVTGHFGGLYPANGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 142. | CH19_2G6_302xI2C6-HALBwD | QVQLVESGGGVVQPGGSLRLSCAASGFTFSSYGMHWVRQAPGKGLEWVAFIWYDGSNKYYADS<br>VKDRFTISRDNSKNTLYLQMNSLRAEDTAVYYCARRAGIIGTIGYYYGMDVWGQGTTVTVSSG<br>GGGSGGGGSGGGGSSYELTQPPSVSVSPGQTASITCSGDRLGEKYTSWYQQRPGQSPLLVIYQ<br>DTKRPSGIPERFSGSNSGNTATLTISGTQAMDEADYYCQAWESSTVVFGGGTKLTVLSGGGGS<br>EVQLVESGGGLVQPGGSLKLSCAASGFTFNKYAMNWVRQAPGKGLEWVARIRSKYNNYATYYA<br>DSVKDRFTISRDDSKNTAYLQMNNLKTEDTAVYYCVRHGNFGNSYISYWAYWGQGTLVTVSSG<br>GGGSGGGGSGGGGSQTVVTQEPSLTVSPGGTVTLTCGSSTGAVTSGNYPNWVQQKPGQAPRGL<br>IGGTKFLAPGTPARFSGSLLGGKAALTLSGVQPEDEAEYYCVLWYSNRWVFGGGTKLTVLPGG<br>DGSDAHKSEVAHRFKDLGEENFKALVLIAFAQYLQQCPFEDHVKLVNEVTEFAKTCVADESAE<br>NCDKSLHTLFGDKLCTVATLRETYGEMADCCAKQEPERNECFLQHKDDNPNLPRLVRPEVDVM<br>CTAFHDNEETFLKKYLYEIARRHPYFYAPELLFFAKRYKAAFTECCQAADKAACLLPKLDELR<br>DEGKASSAKQRLKCASLQKFGERAFKAWAVARLSQRFPKAEFAEVSKLVTDLTKVHTECCHGD<br>LLECADDRADLAKYICENQDSISSKLKECCEKPLLEKSHCIAEVENDEMPADLPSLAADFVES<br>KDVCKNYAEAKDVFLGMFLYEYARRHPDYSVVLLLRLAKTYETTLEKCCAAADPHECYAKVFD<br>EFKPLVEEPQNLIKQNCELFEQLGEYKFQNALLVRYTKKVPQVSTPTLVEVSRNLGKVGSKCC<br>KHPEAKRMPCAEDYLSVVLNQLCVLHEKTPVSDRVTKCCTESLVNRRPCFSALEVDETYVPKE<br>FNAETFTFHADICTLSEKERQIKKQTALVELVKHKPKATKEQLKAVMDDFAAFVEKCCKADDK<br>ETCFAEEGKKLVAASQAALGLHHHHHH |
| 143. | CH19_2G6_302xI2C6-CH-FcB-LH | QRFCTGHFGGLHPCNGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG |

| | | |
|---|---|---|
| | | EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 144. | CH19_2G6_302xI2C6-CH-FcB-LY | QRFCTGHFGGLHPCNGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLYPANGHHHHHH |
| 145. | CH19_2G6_302xI2C6-CH-FcB-CH | QRFCTGHFGGLHPCNGGGGGSQVQLVESGGGVVQPGGSLRLSCAASGFTFSSYGMHWVRQAPG<br>KGLEWVAFIWYDGSNKYYADSVKDRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |
| 146. | CH19_2G6_302_VKGxI2C6-LFcBY | QRFVTGHFGGLYPANGGGGGSQVQLVESGGGVVQPGGSLRLSCAASGFTFSSYGMHWVRQAPG<br>KGLEWVAFIWYDGSNKYYADSVKGRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |

| | | |
|---|---|---|
| 147. | CH19_0-E11xI2C6-LFcBY | QRFVTGHFGGLYPANGGGGGSQVQLQESGPGLVKPSQTLSLTCTVSGGSISRGGYYWSWIRQH<br>PGKGLEWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQFPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 148. | CH19_5-G4xI2C6-LFcBY | QRFVTGHFGGLYPANGGGGGSQVQLQESGPGLVKPSQTLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGTSNRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 149. | CH19_8-H6xI2C6-LFcBY | QRFVTGHFGGLYPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 150. | CH19_2-C11xI2C6-LFcBY | QRFVTGHFGGLYPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLRSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY |

| | | |
|---|---|---|
| | | ISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 151. | CH19_2-A10xI2C6-LFcBY | QRFVTGHFGGLYPANGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 152. | CH19_1-D11xI2C6-LFcBY | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 153. | CH19_9-F9xI2C6-LFcBY | QRFVTGHFGGLYPANGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 154. | CH19_1-H8xI2C6-LFcBY | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSY |

| | | |
|---|---|---|
| | | LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 155. | CH19_1-B12xI2C6-LFcBY | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGPGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 156. | CH19_0-C4xI2C6-LFcBY | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 157. | CH19_3-F2xI2C6-LFcBY | QRFVTGHFGGLYPANGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |

| | | |
|---|---|---|
| 158. | CH19_3-B10xI2C6-LFcBY | QRFVTGHFGGLYPANGGGGGSQVQLQESGPGLVKPSQTLSLTCTVSGGSISSGGYYWSWIRQHPGKGLDWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 159. | CH19_0-G4xI2C6-LFcBY | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 160. | CH19_0-H5xI2C6-LFcBY | QRFVTGHFGGLYPANGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 161. | CH19_0-B8xI2C6-LFcBY | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYI |

| | | |
|---|---|---|
| | | SYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 162. | CH19_2-D9xI2C6-LFcBY | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 163. | CH19_8-H7xI2C6-LFcBY | QRFVTGHFGGLYPANGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 164. | CH19_9-C2xI2C6-LFcBY | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 165. | CH19_3-D5xI2C6-LFcBY | QRFVTGHFGGLYPANGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSY |

| | | |
|---|---|---|
| | | LAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 166. | CH19_1-G11xI2C6-LFcBY | QRFVTGHFGGLYPANGGGGGSQVQLQESGPGLVKPSETLSLTCTVSGGSVSSGGYYWSWIRQPPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 167. | CH19_1-H11xI2C6-LFcBY | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 168. | CH19_9-F3xI2C6-LFcBY | QRFVTGHFGGLYPANGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |

| | | |
|---|---|---|
| 169. | CH19_2-G6xI2C6-LFcBY | QRFVTGHFGGLYPANGGGGGSQVQLQESGPGLVKPSETLSLTCTVSGGSISSYYWSWIRQPPG<br>KGLEWIGYIYYSGSTNYNPSLKSRVTMSIDTSKNQFSLKLISVTAADTAVYYCARDQRRIVAA<br>GGYYYGMDVWGQGTTVTVSSGGGGSGGGGSGGGGSEIVLTQSPGTLSLSPGERATLSCRASQS<br>VSSSYLAWYQQKPGQAPRLLIYGASTRATGIPARFSGSGSGTDFTLTISRLEPEDFAVYYCQQ<br>YGSSPFTFGPGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFCTGHFGGLHPCNGHHHHHH |
| 170. | CH19_2-H7xI2C6-LFcBY | QRFVTGHFGGLYPANGGGGGSQVQLQESGPGLVKPSQTLSLTCTVSGGSISSNDYYWSWIRQH<br>PGKGLEWIGYIYYSGSTFYNPSLKSRGAISVDTSKNQFSLKLTSVTAADTAVYYCARGVYRTG<br>AFDIWGQGTMVTVSSGGGGSGGGGSGGGGSDIQMTQSPSSLSASVGDRVTITCRASQSISSYL<br>NWYQQKPGKAPKSLIYAASSLQSGVPSKFSGSGSGTDFTLTISSLQPEDIATYYCQQSYSTPQ<br>AFGGGTKVDIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 171. | CH19_5-B3xI2C6-LFcBY | QRFVTGHFGGLYPANGGGGGSQVQLVQSGAEVKKPGASVKVSCKASGYTFTYYGISWVRQAPG<br>QGLEWMGWINPYGGETNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 172. | CH19_5-E10xI2C6-LFcBY | QRFVTGHFGGLYPANGGGGGSQVQLVQSGAEVKKPGASVKVSCKASGYTFTYYGISWVRQAPG<br>QGLEWMGWINPYSGATNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY |

| | | |
|---|---|---|
| | | ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 173. | CH19_6-G10xI2C6-LFcBY | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 174. | CH19_4-H8xI2C6-LFcBY | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSVGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 175. | CH19_2-E4xI2C6- LFcBY | QRFVTGHFGGLYPANGGGGGSQVQLVQSGAEVKKPGASVKVSCKASGYTFTSYGISWVRQAPG<br>QGLEWMGWINPYTGNRNYAQKVQDRVTMTTDTSTNTAYMELSSLRSDDTAVYYCARGSGGFDY<br>WGQGTLVTVSSGGGGSGGGGSGGGGSDIVMTQSPLSLPVTLGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 176. | CH19_6-B8xI2C6- LFcBY | QRFVTGHFGGLYPANGGGGGSQVQLVQSGAEVKKPGASVKVSCKASGYTFTYYGISWVRQAPG<br>QGLEWMGWINPYTGKTNYAQKLQDRVTMTTDTSTNTAYMELRSLRSDDTAVYYCARGSGGFDY<br>WGQGTLVTVSSGGGGSGGGGSGGGGSDIVMTQTPLSLPVTPGEPASISCRSSQSLLHSVGYNY |

| | | |
|---|---|---|
| | | LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 177. | CH19_0-B4xI2C6-LFcBY | QRFVTGHFGGLYPANGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 178. | CH19_9-F1xI2C6-LFcBY | QRFVTGHFGGLYPANGGGGGSQVQLVQSGAEVKEPGASVKVSCKASGYTFTYYGISWVRQAPG<br>QGLEWMGWINPYTGNRNYAQKVQDRVTMTTDTSTNTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 179. | CH19_4-A7xI2C6-LFcBY | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |

| | | |
|---|---|---|
| 180. | CH19_6-E12xI2C6-LFcBY | QRFVTGHFGGLYPANGGGGGSQVQLVQSGAEVKKPGASVKVSCKASGYTFTSYGITWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLSVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 181. | CH19_6-C12xI2C6-LFcBY | QRFVTGHFGGLYPANGGGGGSQVQLVQSGAEVKKPGASVKVSCKASGYTFTSYGISWVRQAPGQGLEWMGWINPYTGNRNYAQKFQGRVTMTTDTSTNTAYMELSSLRSEDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 182. | CH19_6-A7xI2C6-LFcBY | QRFVTGHFGGLYPANGGGGGSQVQLVQSGAEVKKPGASVKVSCKASGYSFTSYGISWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 183. | CH19_6-G8xI2C6-LFcBY | QRFVTGHFGGLYPANGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSY |

| | | |
|---|---|---|
| | | ISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 184. | CH19_6-F9xI2C6-LFcBY | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 185. | CH19_0-C11xI2C6-LFcBY | QRFVTGHFGGLYPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 186. | CH19_8-F6xI2C6-LFcBY | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 187. | CH19_0-G9xI2C6-LFcBY | QRFVTGHFGGLYPANGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQ |

| | | |
|---|---|---|
| | | DISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 188. | CH19_1-E11xI2C6-LFcBY | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 189. | CH19_0-F5xI2C6-LFcBY | QRFVTGHFGGLYPANGGGGGSQVQLVESGGGVVQPGRSLRLSCAASGFTFSSYGMQWVRQAPGKGLEWVAVIWYSGSNKYYASSVKGRFTISRDNSKNTLYLQMNSLRAEDTAVYYCARDGYPIFYRYYGMDVWGQGTTVTVSSGGGGSGGGGSGGGGSDIQMTQSPSSLSASVGDRVTITCRASQGISNYLAWYQQKPGKVPKLLIYAASTLQSGVPSRFSGSGSGTDFTLTISSLQPEDVATYYCQKYNSAPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 190. | CH19_1-E1xI2C6-LFcBY | QRFVTGHFGGLYPANGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |

| | | |
|---|---|---|
| 191. | CH19_1-E6xI2C6-LFcBY | QRFVTGHFGGLYPANGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPFTFGPGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 192. | CH19_2G6_302_VKGxI2C6-LFcBY-156 | QRFVTGHFGGLYPANGGGGGSQVQLVESGGGVVQPGGSLRLSCAASGFTFSSYGMHWVRQAPGKGLEWVAFIWYDGSNKYYADSVKGRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 193. | CH19_0-E11xI2C6-LFcBY-156 | QRFVTGHFGGLYPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 194. | CH19_5-G4xI2C6-LFcBY-156 | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSP |

| | | |
|---|---|---|
| | | LTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 195. | CH19_8-H6xI2C6-LFcBY-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 196. | CH19_2-C11xI2C6-LFcBY-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLRSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 197. | CH19_2-A10xI2C6-LFcBY-156 | QRFVTGHFGGLYPANGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTS |

| | | |
|---|---|---|
| | | GNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 198. | CH19_1-D11xI2C6-LFcBY-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 199. | CH19_9-F9xI2C6-LFcBY-156 | QRFVTGHFGGLYPANGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 200. | CH19_1-H8xI2C6-LFcBY-156 | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |

| | | |
|---|---|---|
| 201. | CH19_1-B12xI2C6-LFcBY-156 | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGPGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 202. | CH19_0-C4xI2C6-LFcBY-156 | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 203. | CH19_3-F2xI2C6-LFcBY-156 | QRFVTGHFGGLYPANGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 204. | CH19_3-B10xI2C6-LFcBY-156 | QRFVTGHFGGLYPANGGGGGSQVQLQESGPGLVKPSQTLSLTCTVSGGSISSGGYYWSWIRQH<br>PGKGLDWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQSPGTLSLSPGERATLSCRASQSVSSSY |

| | | |
|---|---|---|
| | | LAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 205. | CH19_0-G4xI2C6-LFcBY-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 206. | CH19_0-H5xI2C6-LFcBY-156 | QRFVTGHFGGLYPANGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 207. | CH19_0-B8xI2C6-LFcBY-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYI |

| | | |
|---|---|---|
| | | SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 208. | CH19_2-D9xI2C6-LFcBY-156 | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 209. | CH19_8-H7xI2C6-LFcBY-156 | QRFVTGHFGGLYPANGGGGGSQVQLQESGPGLVKPSQTLSLTCTVSGGSISSGGYYWSWVRQH<br>PGKGLEWIGYIFYSGRTYYNPSLKSRVIISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 210. | CH19_9-C2xI2C6-LFcBY-156 | QRFVTGHFGGLYPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH |

| | | |
|---|---|---|
| | | HHHH |
| 211. | CH19_3-D5xI2C6-LFcBY-156 | QRFVTGHFGGLYPANGGGGGSQVQLQESGPGLVKPSQTLSLTCTVSGGSISSGGYYWSWIRQH<br>PGKGLEWIGYIFYSGRTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 212. | CH19_1-G11xI2C6-LFcBY-156 | QRFVTGHFGGLYPANGGGGGSQVQLQESGPGLVKPSETLSLTCTVSGGSVSSGGYYWSWIRQP<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 213. | CH19_1-H11xI2C6-LFcBY-156 | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGQGTRLEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 214. | CH19_9-F3xI2C6-LFcBY-156 | QRFVTGHFGGLYPANGGGGGSQVQLQESGPGLVKPSQTLSLTCTVSGGSISSGGYYWSWIRQH<br>PGKGLEWIGYIFYSGKTYYNPSLKSRVSISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 215. | CH19_2-G6xI2C6-LFcBY-156 | QRFVTGHFGGLYPANGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 216. | CH19_2-H7xI2C6-LFcBY-156 | QRFVTGHFGGLYPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 217. | CH19_5-B3xI2C6-LFcBY-156 | QRFVTGHFGGLYPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 218. | CH19_5-E10xI2C6-LFcBY-156 | QRFVTGHFGGLYPANGGGGGSQVQLVQSGAEVKKPGASVKVSCKASGYTFTYYGISWVRQAPG<br>QGLEWMGWINPYSGATNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 219. | CH19_6-G10xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 220. | CH19_4-H8xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSVGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFCTGHFGGLHPCNGGGGGSGGGSRDWDFDVFGGGTPVGGHH<br>HHHH |
| 221. | CH19_2-E4xI2C6-LFcBY-156 | QRFVTGHFGGLYPANGGGGGSQVQLVQSGAEVKKPGASVKVSCKASGYTFTSYGISWVRQAPG<br>QGLEWMGWINPYTGNRNYAQKVQDRVTMTTDTSTNTAYMELSSLRSDDTAVYYCARGSGGFDY<br>WGQGTLVTVSSGGGGSGGGGSGGGGSDIVMTQSPLSLPVTLGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 222. | CH19_6-B8xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKLQDRVTMTTDTSTNTAYMELRSLRSDDTAVYYCARGSGGFDY<br>WGQGTLVTVSSGGGGSGGGGSGGGGSDIVMTQTPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 223. | CH19_0-B4xI2C6-LFcBY-156 | QRFVTGHFGGLYPANGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 224. | CH19_9-F1xI2C6-LFcBY- | QRFVTGHFGGLYPANGGGGGSQVQLVQSGAEVKEPGASVKVSCKASGYTFTYYGISWVRQAPG |

| | | |
|---|---|---|
| | 156 | QGLEWMGWINPYTGNRNYAQKVQDRVTMTTDTSTNTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 225. | CH19_4-A7xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 226. | CH19_6-E12xI2C6-LFcBY-156 | QRFVTGHFGGLYPANGGGGGSQVQLVQSGAEVKKPGASVKVSCKASGYTFTSYGITWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLSVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 227. | CH19_6-C12xI2C6-LFcBY-156 | QRFVTGHFGGLYPANGGGGGSQVQLVQSGAEVKKPGASVKVSCKASGYTFTSYGISWVRQAPG<br>QGLEWMGWINPYTGNRNYAQKFQGRVTMTTDTSTNTAYMELSSLRSEDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP |

| | | |
|---|---|---|
| | | FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 228. | CH19_6-A7xI2C6-LFcBY-156 | QRFVTGHFGGLYPANGGGGGSQVQLVQSGAEVKKPGASVKVSCKASGYSFTSYGISWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 229. | CH19_6-G8xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKVQGRVTMTTDTSTSTAYMELRNLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 230. | CH19_6-F9xI2C6-LFcBY-156 | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS |

| | | |
|---|---|---|
| | | GNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 231. | CH19_0-C11xI2C6-LFcBY-156 | QRFVTGHFGGLYPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 232. | CH19_8-F6xI2C6-LFcBY-156 | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 233. | CH19_0-G9xI2C6-LFcBY-156 | QRFVTGHFGGLYPANGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |

| | | |
|---|---|---|
| 234. | CH19_1-E11xI2C6-LFcBY-156 | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 235. | CH19_0-F5xI2C6-LFcBY-156 | QRFVTGHFGGLYPANGGGGGSQVQLVESGGGVVQPGRSLRLSCAASGFTFSSYGMQWVRQAPGKGLEWVAVIWYSGSNKYYASSVKGRFTISRDNSKNTLYLQMNSLRAEDTAVYYCARDGYPIFYRYYGMDVWGQGTTVTVSSGGGGSGGGGSGGGGSDIQMTQSPSSLSASVGDRVTITCRASQGISNYLAWYQQKPGKVPKLLIYAASTLQSGVPSRFSGSGSGTDFTLTISSLQPEDVATYYCQKYNSAPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 236. | CH19_1-E1xI2C6-LFcBY-156 | QRFVTGHFGGLYPANGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 237. | CH19_1-E6xI2C6-LFcBY-156 | QRFVTGHFGGLYPANGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVS |

| | | |
|---|---|---|
| | | SSYLAWYQQKPGQAPRLLIYGASSRATGIPDRFSGSGSGTDFTLTISRLEPEDFAVYYCQQYG<br>SSPFTFGPGTRLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGGGGGSGGGSRDWDFDVFGGGTPVG<br>GHHHHHH |
| 238. | CH19_2G6_302_VKGxI2C6-LH-FcB-CH | QRFVTGHFGGLHPANGGGGGSQVQLVESGGGVVQPGGSLRLSCAASGFTFSSYGMHWVRQAPG<br>KGLEWVAFIWYDGSNKYYADSVKGRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |
| 239. | CH19_0-E11xI2C6-LH-FcB-CH | QRFVTGHFGGLHPANGGGGGSQVQLQESGPGLVKPSQTLSLTCTVSGGSISRGGYYWSWIRQH<br>PGKGLEWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQFPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 240. | CH19_5-G4xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGTSNRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFCTGHFGGLHPCNGHHHHHH |
| 241. | CH19_8-H6xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 242. | CH19_2-C11xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLRSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 243. | CH19_2-A10xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYSGSTFYNPSLRSRVTISVDTSKNQFSLKLSSVTAADTAVYYCARDSSSSR<br>ALDYWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDSSP<br>RTFGQGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 244. | CH19_1-D11xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFCTGHFGGLHPCNGHHHHHH |
| 245. | CH19_9-F9xI2C6-LH-FcB-CH | QRFVTGHFGGLHPANGGGGGSQVQLQESGPGLVKPSETLSLTCTVSGGSISSGGYYWSWIRQH PGKGLEWIGYIFYRGRTYYNPSLKSRVTISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP TFGGGTKVEIKSGGGGSEVQLVESGGGLVQPGGSLKLSCAASGFTFNKYAMNWVRQAPGKGLE WVARIRSKYNNYATYYADSVKDRFTISRDDSKNTAYLQMNNLKTEDTAVYYCVRHGNFGNSYI SYWAYWGQGTLVTVSSGGGGSGGGGSGGGGSQTVVTQEPSLTVSPGGTVTLTCGSSTGAVTSG NYPNWVQQKPGQAPRGLIGGTKFLAPGTPARFSGSLLGGKAALTLSGVQPEDEAEYYCVLWYS NRWVFGGGTKLTVLGGGGSQRFCTGHFGGLHPCNGHHHHHH |
| 246. | CH19_1-H8xI2C6-LH-FcB-CH | QRFVTGHFGGLHPANGGGGGSQVQLQESGPGLVKPSQTLSLTCTVSGGSISSGGYYWSWIRQH PGKGLEWIGYIFYRGRTYYNPSLKSRVTISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP LTFGGGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFCTGHFGGLHPCNGHHHHHH |
| 247. | CH19_1-B12xI2C6-LH-FcB-CH | QRFVTGHFGGLHPANGGGGGSQVQLQESGPGLVKPSQTLSLTCTVSGGSISSGGYYWSWIRQH PGKGLEWIGYIYYRGRTYYNPSLKSRVTISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP TFGPGTKVEIKSGGGGSEVQLVESGGGLVQPGGSLKLSCAASGFTFNKYAMNWVRQAPGKGLE WVARIRSKYNNYATYYADSVKDRFTISRDDSKNTAYLQMNNLKTEDTAVYYCVRHGNFGNSYI SYWAYWGQGTLVTVSSGGGGSGGGGSGGGGSQTVVTQEPSLTVSPGGTVTLTCGSSTGAVTSG NYPNWVQQKPGQAPRGLIGGTKFLAPGTPARFSGSLLGGKAALTLSGVQPEDEAEYYCVLWYS NRWVFGGGTKLTVLGGGGSQRFCTGHFGGLHPCNGHHHHHH |
| 248. | CH19_0-C4xI2C6-LH-FcB-CH | QRFVTGHFGGLHPANGGGGGSQVQLQESGPGLVKPSQTLSLTCTVSGGSISSGGYYWSWIRQH PGKGLEWIGYIYYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 249. | CH19_3-F2xI2C6-LH-FcB-CH | QRFVTGHFGGLHPANGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 250. | CH19_3-B10xI2C6-LH-FcB-CH | QRFVTGHFGGLHPANGGGGGSQVQLQESGPGLVKPSQTLSLTCTVSGGSISSGGYYWSWIRQH<br>PGKGLDWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 251. | CH19_0-G4xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASTRATGIPDRFSGSGSGTDFTLTISRLEPEDFAVYYCQQYGSSP<br>SFGQGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFCTGHFGGLHPCNGHHHHHH |
| 252. | CH19_0-H5xI2C6-LH-FcB-CH | QRFVTGHFGGLHPANGGGGGSQVQLQESGPGLVRPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDTSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 253. | CH19_0-B8xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGHHHHHH |
| 254. | CH19_2-D9xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 255. | CH19_8-H7xI2C6-LH-FcB-CH | QRFVTGHFGGLHPANGGGGGSQVQLQESGPGLVKPSQTLSLTCTVSGGSISSGGYYWSWVRQH<br>PGKGLEWIGYIFYSGRTYYNPSLKSRVIISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 256. | CH19_9-C2xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 257. | CH19_3-D5xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 258. | CH19_1-G11xI2C6-LH-FcB-CH | QRFVTGHFGGLHPANGGGGGSQVQLQESGPGLVKPSETLSLTCTVSGGSVSSGGYYWSWIRQP<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 259. | CH19_1-H11xI2C6-LH-FcB-CH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 260. | CH19_9-F3xI2C6-LH-FcB-CH | QRFVTGHFGGLHPANGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 261. | CH19_2-G6xI2C6-LH-FcB-CH | QRFVTGHFGGLHPANGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 262. | CH19_2-H7xI2C6-LH-FcB-CH | QRFVTGHFGGLHPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFCTGHFGGLHPCNGHHHHHH |
| 263. | CH19_5-B3xI2C6-LH-FcB-CH | QRFVTGHFGGLHPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 264. | CH19_5-E10xI2C6-LH-FcB-CH | QRFVTGHFGGLHPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 265. | CH19_6-G10xI2C6-LH-FcB-CH | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 266. | CH19_4-H8xI2C6-LH-FcB-CH | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSVGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 267. | CH19_2-E4xI2C6-LH-FcB-CH | QRFVTGHFGGLHPANGGGGGSQVQLVQSGAEVKKPGASVKVSCKASGYTFTSYGISWVRQAPGQGLEWMGWINPYTGNRNYAQKVQDRVTMTTDTSTNTAYMELSSLRSDDTAVYYCARGSGGFDYWGQGTLVTVSSGGGGSGGGGSGGGGSDIVMTQSPLSLPVTLGQPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 268. | CH19_6-B8xI2C6-LH-FcB-CH | QRFVTGHFGGLHPANGGGGGSQVQLVQSGAEVKKPGASVKVSCKASGYTFTYYGISWVRQAPGQGLEWMGWINPYTGKTNYAQKLQDRVTMTTDTSTNTAYMELRSLRSDDTAVYYCARGSGGFDYWGQGTLVTVSSGGGGSGGGGSGGGGSDIVMTQTPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 269. | CH19_0-B4xI2C6-LH-FcB-CH | QRFVTGHFGGLHPANGGGGGSQVQLVQSGAEVKKPGASVKVSCKASGYTFTTYGISWVRQAPGQGLEWMGWINPYTGKTNYAQKLQGRVTMATDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 270. | CH19_9-F1xI2C6-LH-FcB-CH | QRFVTGHFGGLHPANGGGGGSQVQLVQSGAEVKEPGASVKVSCKASGYTFTYYGISWVRQAPGQGLEWMGWINPYTGNRNYAQKVQDRVTMTTDTSTNTAYMELRSLRSDDTAVYYCARGSGGFDY |

| | | |
|---|---|---|
| | | WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 271. | CH19_4-A7xI2C6-LH-FcB-CH | QRFVTGHFGGLHPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 272. | CH19_6-E12xI2C6-LH-FcB-CH | QRFVTGHFGGLHPANGGGGGSQVQLVQSGAEVKKPGASVKVSCKASGYTFTSYGITWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLSVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 273. | CH19_6-C12xI2C6-LH-FcB-CH | QRFVTGHFGGLHPANGGGGGSQVQLVQSGAEVKKPGASVKVSCKASGYTFTSYGISWVRQAPG<br>QGLEWMGWINPYTGNRNYAQKFQGRVTMTTDTSTNTAYMELSSLRSEDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFCTGHFGGLHPCNGHHHHHH |
| 274. | CH19_6-A7xI2C6-LH-FcB-CH | QRFVTGHFGGLHPANGGGGGSQVQLVQSGAEVKKPGASVKVSCKASGYSFTSYGISWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 275. | CH19_6-G8xI2C6-LH-FcB-CH | QRFVTGHFGGLHPANGGGGGSQVQLVQSGAEVKKPGASVKVSCKASGYTFTYYGISWVRQAPG<br>QGLEWMGWINPYTGKTNYAQKVQGRVTMTTDTSTSTAYMELRNLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 276. | CH19_6-F9xI2C6-LH-FcB-CH | QRFVTGHFGGLHPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGHHHHHH |
| 277. | CH19_0-C11xI2C6-LH-FcB-CH | QRFVTGHFGGLHPANGGGGGSQVQLVESGGGVVQPGRSLRLSCAASGFTFRNYAMHWVRQAPG<br>KGLEWVAVISYSGNNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTDFTFTISSLQPEDIATYYCQQ<br>YDNLPTFGQGTRLEIKSGGGGSEVQLVESGGGLVQPGGSLKLSCAASGFTFNKYAMNWVRQAP |

| | | |
|---|---|---|
| | | GKGLEWVARIRSKYNNYATYYADSVKDRFTISRDDSKNTAYLQMNNLKTEDTAVYYCVRHGNF GNSYISYWAYWGQGTLVTVSSGGGGSGGGGSGGGGSQTVVTQEPSLTVSPGGTVTLTCGSSTG AVTSGNYPNWVQQKPGQAPRGLIGGTKFLAPGTPARFSGSLLGGKAALTLSGVQPEDEAEYYC VLWYSNRWVFGGGTKLTVLGGGGSQRFCTGHFGGLHPCNGHHHHHH |
| 278. | CH19_8-F6xI2C6-LH-FcB-CH | QRFVTGHFGGLHPANGGGGGSQVQLVESGGGVVQPGRSLRLSCVASGFTFRNYAMHWVRQAPG KGLEWVAVISYSGNNKYYASSVKGRFTISRDNSKNTLYLQLNSLRAEDTAVYYCAREFYYDST GYDYSTPGDYWGQGTLVTVSSGGGGSGGGGSGGGGSDIQMTQSPSSLSASVGDRVTITCRASQ SISSYLNWYQQKPGKAPKLLIYDASSLQSGVPSRFSGSGSGTEFTLTISSLQAEDVAVYYCQQ YYSTPLTFGGGTKVDIKSGGGGSEVQLVESGGGLVQPGGSLKLSCAASGFTFNKYAMNWVRQA PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFCTGHFGGLHPCNGHHHHHH |
| 279. | CH19_0-G9xI2C6-LH-FcB-CH | QRFVTGHFGGLHPANGGGGGSQVQLVESGGGVVQPGRSLRLSCAASGFTFRNYAMHWVRQAPG KGLEWVAGISYSGTNKYYASSVKGRFTISRDNSKNTLYLQLNSLRAEDTAVYYCAREFYYDST GYDYSTPGDYWGQGTLVTVSSGGGGSGGGGSGGGGSDIQMTQSPSSLSASVGDRVTITCQASQ DISNYLNWYQQKPGKAPKLLIYDASNLETGVPSRFSGSGSGTEFTLTISSLQPEDIATYYCQQ YDNLPLTFGGGTKVDIKSGGGGSEVQLVESGGGLVQPGGSLKLSCAASGFTFNKYAMNWVRQA PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFCTGHFGGLHPCNGHHHHHH |
| 280. | CH19_1-E11xI2C6-LH-FcB-CH | QRFVTGHFGGLHPANGGGGGSQVQLVESGGGVVQPGRSLRLSCVASGFTFRNYAMHWVRQAPG KGLEWVAVISYSGNNKYYASSVKGRFTISRDNSKNTLFLQLNSLRAEDTAVYYCAREFYYDST GYDYSTPGNYWGQGTLVTVSSGGGGSGGGGSGGGGSDIQMTQSPSSLSASVGDRVTITCQASQ DISNYLNWYQQKPGKAPKLLIYDASNLETGVPSRFSGSGSGTDFTFTISSLQPEDIATYYCQQ YVNLPLTFGGGTKLEIKSGGGGSEVQLVESGGGLVQPGGSLKLSCAASGFTFNKYAMNWVRQA PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFCTGHFGGLHPCNGHHHHHH |
| 281. | CH19_0-F5xI2C6-LH-FcB-CH | QRFVTGHFGGLHPANGGGGGSQVQLVESGGGVVQPGRSLRLSCAASGFTFSSYGMQWVRQAPG KGLEWVAVIWYSGSNKYYASSVKGRFTISRDNSKNTLYLQMNSLRAEDTAVYYCARDGYPIFY |

| | | |
|---|---|---|
| | | RYYGMDVWGQGTTVTVSSGGGGSGGGGSGGGGSDIQMTQSPSSLSASVGDRVTITCRASQGIS<br>NYLAWYQQKPGKVPKLLIYAASTLQSGVPSRFSGSGSGTDFTLTISSLQPEDVATYYCQKYNS<br>APLTFGGGTKVEIKSGGGGSEVQLVESGGGLVQPGGSLKLSCAASGFTFNKYAMNWVRQAPGK<br>GLEWVARIRSKYNNYATYYADSVKDRFTISRDDSKNTAYLQMNNLKTEDTAVYYCVRHGNFGN<br>SYISYWAYWGQGTLVTVSSGGGGSGGGGSGGGGSQTVVTQEPSLTVSPGGTVTLTCGSSTGAV<br>TSGNYPNWVQQKPGQAPRGLIGGTKFLAPGTPARFSGSLLGGKAALTLSGVQPEDEAEYYCVL<br>WYSNRWVFGGGTKLTVLGGGGSQRFCTGHFGGLHPCNGHHHHHH |
| 282. | CH19_1-E1xI2C6-LH-FcB-CH | QRFVTGHFGGLHPANGGGGGSQVQLVESGGGVVQPGRSLRLSCAASGFTFSSYGMHWVRQAPG<br>KGLEWVAVISYSGSNKYYASSVKGRFTISRDNSKNTLYLQMNSLRAEDTAVYYCARDGYPIFY<br>RYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDLAVYYCQQYG<br>TSPLTFGGGTKLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |
| 283. | CH19_1-E6xI2C6-LH-FcB-CH | QRFVTGHFGGLHPANGGGGGSQVQLVESGGGVVQPGRSLRLSCAASGFTFSSFGMHWVRQAPG<br>KGLEWVAFIWYSGSNKYYASSVKGRVTISRDNSKNTLYLQMNSLRAEDTAVYYCARDGYPIFY<br>YYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDFAVYYCQQYG<br>SSPFTFGPGTRLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFCTGHFGGLHPCNGHHHHHH |
| 284. | CH19_2G6_302_VKGxI2C6-LH-FcB-LH | QRFVTGHFGGLHPANGGGGGSQVQLVESGGGVVQPGGSLRLSCAASGFTFSSYGMHWVRQAPG<br>KGLEWVAFIWYDGSNKYYADSVKGRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV |

| | | |
|---|---|---|
| | | LWYSNRWVFGGGTKLTVLGGGGSQRFVTGHFGGLHPANGHHHHHH |
| 285. | CH19_0-E11xI2C6-LH-FcB-LH | QRFVTGHFGGLHPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 286. | CH19_5-G4xI2C6-LH-FcB-LH | QRFVTGHFGGLHPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 287. | CH19_8-H6xI2C6-LH-FcB-LH | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 288. | CH19_2-C11xI2C6-LH-FcB-LH | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLRSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 289. | CH19_2-A10xI2C6-LH-FcB-LH | QRFVTGHFGGLHPANGGGGGSQVQLQESGPGLVKPSQTLSLTCTVSGGSISSGGYYWSWIRQH<br>PGKGLEWIGYIYYSGSTFYNPSLRSRVTISVDTSKNQFSLKLSSVTAADTAVYYCARDSSSSR<br>ALDYWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDSSP<br>RTFGQGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 290. | CH19_1-D11xI2C6-LH-FcB-LH | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 291. | CH19_9-F9xI2C6-LH-FcB-LH | QRFVTGHFGGLHPANGGGGGSQVQLQESGPGLVKPSETLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 292. | CH19_1-H8xI2C6-LH-FcB-LH | QRFVTGHFGGLHPANGGGGGSQVQLQESGPGLVKPSQTLSLTCTVSGGSISSGGYYWSWIRQH<br>PGKGLEWIGYIFYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 293. | CH19_1-B12xI2C6-LH-FcB-LH | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 294. | CH19_0-C4xI2C6-LH-FcB-LH | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 295. | CH19_3-F2xI2C6-LH-FcB-LH | QRFVTGHFGGLHPANGGGGGSQVQLQESGPGLVKPSQTLSLTCTISGGSISSGGYYWSWIRQHPGKGLEWIGYIFYSGRTYYNPSLKSRVTISVDTSKNQFSLKLTSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLHPANGHHHHHH |
| 296. | CH19_3-B10xI2C6-LH-FcB-LH | QRFVTGHFGGLHPANGGGGGSQVQLQESGPGLVKPSQTLSLTCTVSGGSISSGGYYWSWIRQHPGKGLDWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 297. | CH19_0-G4xI2C6-LH-FcB-LH | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 298. | CH19_0-H5xI2C6-LH-FcB-LH | QRFVTGHFGGLHPANGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 299. | CH19_0-B8xI2C6-LH-FcB-LH | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLE |

| | | |
|---|---|---|
| | | WVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 300. | CH19_2-D9xI2C6-LH-FcB-LH | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 301. | CH19_8-H7xI2C6-LH-FcB-LH | QRFVTGHFGGLHPANGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 302. | CH19_9-C2xI2C6-LH-FcB-LH | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 303. | CH19_3-D5xI2C6-LH-FcB-LH | QRFVTGHFGGLHPANGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 304. | CH19_1-G11xI2C6-LH-FcB-LH | QRFVTGHFGGLHPANGGGGSQVQLQESGPGLVKPSETLSLTCTVSGGSVSSGGYYWSWIRQP<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 305. | CH19_1-H11xI2C6-LH-FcB-LH | QRFVTGHFGGLHPANGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGQGTRLEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 306. | CH19_9-F3xI2C6-LH-FcB-LH | QRFVTGHFGGLHPANGGGGSQVQLQESGPGLVKPSQTLSLTCTVSGGSISSGGYYWSWIRQH<br>PGKGLEWIGYIFYSGKTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>ITFGQGTRLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 307. | CH19_2-G6xI2C6-LH-FcB-LH | QRFVTGHFGGLHPANGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 308. | CH19_2-H7xI2C6-LH-FcB-LH | QRFVTGHFGGLHPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 309. | CH19_5-B3xI2C6-LH-FcB-LH | QRFVTGHFGGLHPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 310. | CH19_5-E10xI2C6-LH-FcB-LH | QRFVTGHFGGLHPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 311. | CH19_6-G10xI2C6-LH-FcB-LH | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 312. | CH19_4-H8xI2C6-LH-FcB-LH | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSVGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 313. | CH19_2-E4xI2C6-LH-FcB-LH | QRFVTGHFGGLHPANGGGGGSQVQLVQSGAEVKKPGASVKVSCKASGYTFTSYGISWVRQAPGQGLEWMGWINPYTGNRNYAQKVQDRVTMTTDTSTNTAYMELSSLRSDDTAVYYCARGSGGFDYWGQGTLVTVSSGGGGSGGGGSGGGGSDIVMTQSPLSLPVTLGQPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 314. | CH19_6-B8xI2C6-LH-FcB-LH | QRFVTGHFGGLHPANGGGGGSQVQLVQSGAEVKKPGASVKVSCKASGYTFTYYGISWVRQAPGQGLEWMGWINPYTGKTNYAQKLQDRVTMTTDTSTNTAYMELRSLRSDDTAVYYCARGSGGFDY |

| | | |
|---|---|---|
| | | WGQGTLVTVSSGGGGSGGGGSGGGGSDIVMTQTPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 315. | CH19_0-B4xI2C6-LH-FcB-LH | QRFVTGHFGGLHPANGGGGGSQVQLVQSGAEVKKPGASVKVSCKASGYTFTTYGISWVRQAPGQGLEWMGWINPYTGKTNYAQKLQGRVTMATDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 316. | CH19_9-F1xI2C6-LH-FcB-LH | QRFVTGHFGGLHPANGGGGGSQVQLVQSGAEVKEPGASVKVSCKASGYTFTYYGISWVRQAPGQGLEWMGWINPYTGNRNYAQKVQDRVTMTTDTSTNTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 317. | CH19_4-A7xI2C6-LH-FcB-LH | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 318. | CH19_6-E12xI2C6-LH-FcB-LH | QRFVTGHFGGLHPANGGGGGSQVQLVQSGAEVKKPGASVKVSCKASGYTFTSYGITWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLSVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 319. | CH19_6-C12xI2C6-LH-FcB-LH | QRFVTGHFGGLHPANGGGGGSQVQLVQSGAEVKKPGASVKVSCKASGYTFTSYGISWVRQAPGQGLEWMGWINPYTGNRNYAQKFQGRVTMTTDTSTNTAYMELSSLRSEDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 320. | CH19_6-A7xI2C6-LH-FcB-LH | QRFVTGHFGGLHPANGGGGGSQVQLVQSGAEVKKPGASVKVSCKASGYSFTSYGISWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 321. | CH19_6-G8xI2C6-LH-FcB-LH | QRFVTGHFGGLHPANGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 322. | CH19_6-F9xI2C6-LH-FcB-LH | QRFVTGHFGGLHPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 323. | CH19_0-C11xI2C6-LH-FcB-LH | QRFVTGHFGGLHPANGGGGGSQVQLVESGGGVVQPGRSLRLSCAASGFTFRNYAMHWVRQAPG<br>KGLEWVAVISYSGNNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTDFTFTISSLQPEDIATYYCQQ<br>YDNLPTFGQGTRLEIKSGGGGSEVQLVESGGGLVQPGGSLKLSCAASGFTFNKYAMNWVRQAP<br>GKGLEWVARIRSKYNNYATYYADSVKDRFTISRDDSKNTAYLQMNNLKTEDTAVYYCVRHGNF<br>GNSYISYWAYWGQGTLVTVSSGGGGSGGGGSGGGGSQTVVTQEPSLTVSPGGTVTLTCGSSTG<br>AVTSGNYPNWVQQKPGQAPRGLIGGTKFLAPGTPARFSGSLLGGKAALTLSGVQPEDEAEYYC<br>VLWYSNRWVFGGGTKLTVLGGGGSQRFVTGHFGGLHPANGHHHHHH |
| 324. | CH19_8-F6xI2C6-LH-FcB-LH | QRFVTGHFGGLHPANGGGGGSQVQLVESGGGVVQPGRSLRLSCVASGFTFRNYAMHWVRQAPG<br>KGLEWVAVISYSGNNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCRASQ<br>SISSYLNWYQQKPGKAPKLLIYDASSLQSGVPSRFSGSGSGTEFTLTISSLQAEDVAVYYCQQ<br>YYSTPLTFGGGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFVTGHFGGLHPANGHHHHHH |
| 325. | CH19_0-G9xI2C6-LH-FcB-LH | QRFVTGHFGGLHPANGGGGGSQVQLVESGGGVVQPGRSLRLSCAASGFTFRNYAMHWVRQAPG<br>KGLEWVAGISYSGTNKYYASSVKGRFTISRDNSKNTLYLQLNSLRAEDTAVYYCAREFYYDST |

| | | |
|---|---|---|
| | | GYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 326. | CH19_1-E11xI2C6-LH-FcB-LH | QRFVTGHFGGLHPANGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 327. | CH19_0-F5xI2C6-LH-FcB-LH | QRFVTGHFGGLHPANGGGGGSQVQLVESGGGVVQPGRSLRLSCAASGFTFSSYGMQWVRQAPGKGLEWVAVIWYSGSNKYYASSVKGRFTISRDNSKNTLYLQMNSLRAEDTAVYYCARDGYPIFYRYYGMDVWGQGTTVTVSSGGGGSGGGGSGGGGSDIQMTQSPSSLSASVGDRVTITCRASQGISNYLAWYQQKPGKVPKLLIYAASTLQSGVPSRFSGSGSGTDFTLTISSLQPEDVATYYCQKYNSAPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 328. | CH19_1-E1xI2C6-LH-FcB-LH | QRFVTGHFGGLHPANGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCV |

| | | |
|---|---|---|
| | | LWYSNRWVFGGGTKLTVLGGGGSQRFVTGHFGGLHPANGHHHHHH |
| 329. | CH19_1-E6xI2C6-LH-FcB-LH | QRFVTGHFGGLHPANGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPFTFGPGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 330. | CH19_2G6_302_VKGxI2C6-LY-FcB-LH | QRFVTGHFGGLYPANGGGGGSQVQLVESGGGVVQPGGSLRLSCAASGFTFSSYGMHWVRQAPGKGLEWVAFIWYDGSNKYYADSVKGRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 331. | CH19_0-E11xI2C6-LY-FcB-LH | QRFVTGHFGGLYPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 332. | CH19_5-G4xI2C6-LY-FcB-LH | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 333. | CH19_8-H6xI2C6-LY-FcB-LH | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 334. | CH19_2-C11xI2C6-LY-FcB-LH | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLRSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 335. | CH19_2-A10xI2C6-LY-FcB-LH | QRFVTGHFGGLYPANGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 336. | CH19_1-D11xI2C6-LY-FcB-LH | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 337. | CH19_9-F9xI2C6-LY-FcB-LH | QRFVTGHFGGLYPANGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 338. | CH19_1-H8xI2C6-LY-FcB-LH | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 339. | CH19_1-B12xI2C6-LY-FcB-LH | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLHPANGHHHHHH |
| 340. | CH19_0-C4xI2C6-LY-FcB-LH | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 341. | CH19_3-F2xI2C6-LY-FcB-LH | QRFVTGHFGGLYPANGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 342. | CH19_3-B10xI2C6-LY-FcB-LH | QRFVTGHFGGLYPANGGGGGSQVQLQESGPGLVKPSQTLSLTCTVSGGSISSGGYYWSWIRQH<br>PGKGLDWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 343. | CH19_0-G4xI2C6-LY-FcB-LH | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASTRATGIPDRFSGSGSGTDFTLTISRLEPEDFAVYYCQQYGSSP<br>SFGQGTKVEIKSGGGGSEVQLVESGGGLVQPGGSLKLSCAASGFTFNKYAMNWVRQAPGKGLE |

| | | |
|---|---|---|
| | | WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 344. | CH19_0-H5xI2C6-LY-FcB-LH | QRFVTGHFGGLYPANGGGGGSQVQLQESGPGLVRPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDTSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 345. | CH19_0-B8xI2C6-LY-FcB-LH | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGHHHHHH |
| 346. | CH19_2-D9xI2C6-LY-FcB-LH | QRFVTGHFGGLYPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 347. | CH19_8-H7xI2C6-LY-FcB-LH | QRFVTGHFGGLYPANGGGGGSQVQLQESGPGLVKPSQTLSLTCTVSGGSISSGGYYWSWVRQH<br>PGKGLEWIGYIFYSGRTYYNPSLKSRVIISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP LTFGGGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGHHHHHH |
| 348. | CH19_9-C2xI2C6-LY-FcB-LH | QRFVTGHFGGLYPANGGGGGSQVQLQESGPGLVKPSQTLSLTCTVSGGSISSGGYYWSWIRQH PGKGLEWIGYIFYSGRTYYNPSLKSRVTISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP LTFGGGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGHHHHHH |
| 349. | CH19_3-D5xI2C6-LY-FcB-LH | QRFVTGHFGGLYPANGGGGGSQVQLQESGPGLVKPSQTLSLTCTVSGGSISSGGYYWSWIRQH PGKGLEWIGYIFYSGRTYYNPSLKSRVSISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP LTFGGGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGHHHHHH |
| 350. | CH19_1-G11xI2C6-LY-FcB-LH | QRFVTGHFGGLYPANGGGGGSQVQLQESGPGLVKPSETLSLTCTVSGGSVSSGGYYWSWIRQP PGKGLEWIGYIYYRGRTYYNPSLKSRVTISVDTSKNQFSLKLSSVTAADTAVYYCARVYSNYG WFDPWGQGTLVTVSSGGGGSGGGGSGGGGSEIVLTQSPGTLSLSPGERATLSCRASQSVSSSY LAWYQQKPGQAPRLLIYGASSRATGIPDRFSGSGSGTDFTLTISRLEPEDFAVYYCQQYGSSP LTFGGGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 351. | CH19_1-H11xI2C6-LY-FcB-LH | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 352. | CH19_9-F3xI2C6-LY-FcB-LH | QRFVTGHFGGLYPANGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 353. | CH19_2-G6xI2C6-LY-FcB-LH | QRFVTGHFGGLYPANGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 354. | CH19_2-H7xI2C6-LY-FcB-LH | QRFVTGHFGGLYPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLE |

| | | |
|---|---|---|
| | | WVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 355. | CH19_5-B3xI2C6-LY-FcB-LH | QRFVTGHFGGLYPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 356. | CH19_5-E10xI2C6-LY-FcB-LH | QRFVTGHFGGLYPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 357. | CH19_6-G10xI2C6-LY-FcB-LH | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 358. | CH19_4-H8xI2C6-LY-FcB-LH | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDY |

| | | |
|---|---|---|
| | | WGQGTLVTVSSGGGGSGGGGSGGGGSDIVMTQTPLSLSVTPGEPASISCRSSQSLLHSVGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 359. | CH19_2-E4xI2C6-LY-FcB-LH | QRFVTGHFGGLYPANGGGGGSQVQLVQSGAEVKKPGASVKVSCKASGYTFTSYGISWVRQAPG<br>QGLEWMGWINPYTGNRNYAQKVQDRVTMTTDTSTNTAYMELSSLRSDDTAVYYCARGSGGFDY<br>WGQGTLVTVSSGGGGSGGGGSGGGGSDIVMTQSPLSLPVTLGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 360. | CH19_6-B8xI2C6-LY-FcB-LH | QRFVTGHFGGLYPANGGGGGSQVQLVQSGAEVKKPGASVKVSCKASGYTFTYYGISWVRQAPG<br>QGLEWMGWINPYTGKTNYAQKLQDRVTMTTDTSTNTAYMELRSLRSDDTAVYYCARGSGGFDY<br>WGQGTLVTVSSGGGGSGGGGSGGGGSDIVMTQTPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 361. | CH19_0-B4xI2C6-LY-FcB-LH | QRFVTGHFGGLYPANGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 362. | CH19_9-F1xI2C6-LY-FcB-LH | QRFVTGHFGGLYPANGGGGGSQVQLVQSGAEVKEPGASVKVSCKASGYTFTYYGISWVRQAPGQGLEWMGWINPYTGNRNYAQKVQDRVTMTTDTSTNTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 363. | CH19_4-A7xI2C6-LY-FcB-LH | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 364. | CH19_6-E12xI2C6-LY-FcB-LH | QRFVTGHFGGLYPANGGGGGSQVQLVQSGAEVKKPGASVKVSCKASGYTFTSYGITWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLSVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 365. | CH19_6-C12xI2C6-LY-FcB-LH | QRFVTGHFGGLYPANGGGGGSQVQLVQSGAEVKKPGASVKVSCKASGYTFTSYGISWVRQAPGQGLEWMGWINPYTGNRNYAQKFQGRVTMTTDTSTNTAYMELSSLRSEDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 366. | CH19_6-A7xI2C6-LY-FcB-LH | QRFVTGHFGGLYPANGGGGGSQVQLVQSGAEVKKPGASVKVSCKASGYSFTSYGISWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 367. | CH19_6-G8xI2C6-LY-FcB-LH | QRFVTGHFGGLYPANGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 368. | CH19_6-F9xI2C6-LY-FcB-LH | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 369. | CH19_0-C11xI2C6-LY-FcB-LH | QRFVTGHFGGLYPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDST |

| | | |
|---|---|---|
| | | GYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 370. | CH19_8-F6xI2C6-LY-FcB-LH | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 371. | CH19_0-G9xI2C6-LY-FcB-LH | QRFVTGHFGGLYPANGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 372. | CH19_1-E11xI2C6-LY-FcB-LH | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYY |

| | | |
|---|---|---|
| | | CVLWYSNRWVFGGGTKLTVLGGGGSQRFVTGHFGGLHPANGHHHHHH |
| 373. | CH19_0-F5xI2C6-LY-FcB-LH | QRFVTGHFGGLYPANGGGGGSQVQLVESGGGVVQPGRSLRLSCAASGFTFSSYGMQWVRQAPG<br>KGLEWVAVIWYSGSNKYYASSVKGRFTISRDNSKNTLYLQMNSLRAEDTAVYYCARDGYPIFY<br>RYYGMDVWGQGTTVTVSSGGGGSGGGGSGGGGSDIQMTQSPSSLSASVGDRVTITCRASQGIS<br>NYLAWYQQKPGKVPKLLIYAASTLQSGVPSRFSGSGSGTDFTLTISSLQPEDVATYYCQKYNS<br>APLTFGGGTKVEIKSGGGGSEVQLVESGGGLVQPGGSLKLSCAASGFTFNKYAMNWVRQAPGK<br>GLEWVARIRSKYNNYATYYADSVKDRFTISRDDSKNTAYLQMNNLKTEDTAVYYCVRHGNFGN<br>SYISYWAYWGQGTLVTVSSGGGGSGGGGSGGGGSQTVVTQEPSLTVSPGGTVTLTCGSSTGAV<br>TSGNYPNWVQQKPGQAPRGLIGGTKFLAPGTPARFSGSLLGGKAALTLSGVQPEDEAEYYCVL<br>WYSNRWVFGGGTKLTVLGGGGSQRFVTGHFGGLHPANGHHHHHH |
| 374. | CH19_1-E1xI2C6-LY-FcB-LH | QRFVTGHFGGLYPANGGGGGSQVQLVESGGGVVQPGRSLRLSCAASGFTFSSYGMHWVRQAPG<br>KGLEWVAVISYSGSNKYYASSVKGRFTISRDNSKNTLYLQMNSLRAEDTAVYYCARDGYPIFY<br>RYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDLAVYYCQQYG<br>TSPLTFGGGTKLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 375. | CH19_1-E6xI2C6-LY-FcB-LH | QRFVTGHFGGLYPANGGGGGSQVQLVESGGGVVQPGRSLRLSCAASGFTFSSFGMHWVRQAPG<br>KGLEWVAFIWYSGSNKYYASSVKGRVTISRDNSKNTLYLQMNSLRAEDTAVYYCARDGYPIFY<br>YYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDFAVYYCQQYG<br>SSPFTFGPGTRLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 376. | CH19_2G6_302_VKGxI2C6-LH-FcB-CH-156 | QRFVTGHFGGLHPANGGGGGSQVQLVESGGGVVQPGGSLRLSCAASGFTFSSYGMHWVRQAPG<br>KGLEWVAFIWYDGSNKYYADSVKGRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG |

| | | |
|---|---|---|
| | | KGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 377. | CH19_0-E11xI2C6-LH-FcB-CH-156 | QRFVTGHFGGLHPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 378. | CH19_5-G4xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 379. | CH19_8-H6xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFCTGHFGGLHPCNGGGGGSGGGSRDWDFDVFGGGTPVGGHH<br>HHHH |
| 380. | CH19_2-C11xI2C6-LH-FcB-CH-156 | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLRSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 381. | CH19_2-A10xI2C6-LH-FcB-CH-156 | QRFVTGHFGGLHPANGGGGGSQVQLQESGPGLVKPSQTLSLTCTVSGGSISSGGYYWSWIRQH<br>PGKGLEWIGYIYYSGSTFYNPSLRSRVTISVDTSKNQFSLKLSSVTAADTAVYYCARDSSSSR<br>ALDYWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDSSP<br>RTFGQGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 382. | CH19_1-D11xI2C6-LH-FcB-CH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 383. | CH19_9-F9xI2C6-LH-FcB- | QRFVTGHFGGLHPANGGGGGSQVQLQESGPGLVKPSETLSLTCTVSGGSISSGGYYWSWIRQH |

| | | |
|---|---|---|
| | CH-156 | PGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 384. | CH19_1-H8xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 385. | CH19_1-B12xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 386. | CH19_0-C4xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSP |

| | | |
|---|---|---|
| | | TFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 387. | CH19_3-F2xI2C6-LH-FcB-CH-156 | QRFVTGHFGGLHPANGGGGGSQVQLQESGPGLVKPSQTLSLTCTISGGSISSGGYYWSWIRQHPGKGLEWIGYIFYSGRTYYNPSLKSRVTISVDTSKNQFSLKLTSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 388. | CH19_3-B10xI2C6-LH-FcB-CH-156 | QRFVTGHFGGLHPANGGGGGSQVQLQESGPGLVKPSQTLSLTCTVSGGSISSGGYYWSWIRQHPGKGLDWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 389. | CH19_0-G4xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSG |

| | | |
|---|---|---|
| | | NYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 390. | CH19_0-H5xI2C6-LH-FcB-CH-156 | QRFVTGHFGGLHPANGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 391. | CH19_0-B8xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 392. | CH19_2-D9xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |

| | | |
|---|---|---|
| 393. | CH19_8-H7xI2C6-LH-FcB-CH-156 | QRFVTGHFGGLHPANGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 394. | CH19_9-C2xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 395. | CH19_3-D5xI2C6-LH-FcB-CH-156 | QRFVTGHFGGLHPANGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 396. | CH19_1-G11xI2C6-LH-FcB-CH-156 | QRFVTGHFGGLHPANGGGGGSQVQLQESGPGLVKPSETLSLTCTVSGGSVSSGGYYWSWIRQPPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSY |

| | | |
|---|---|---|
| | | LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 397. | CH19_1-H11xI2C6-LH-FcB-CH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGQGTRLEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFCTGHFGGLHPCNGGGGGSGGGSRDWDFDVFGGGTPVGGHHH<br>HHH |
| 398. | CH19_9-F3xI2C6-LH-FcB-CH-156 | QRFVTGHFGGLHPANGGGGGSQVQLQESGPGLVKPSQTLSLTCTVSGGSISSGGYYWSWIRQH<br>PGKGLEWIGYIFYSGKTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>ITFGQGTRLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFCTGHFGGLHPCNGGGGGSGGGSRDWDFDVFGGGTPVGGHH<br>HHHH |
| 399. | CH19_2-G6xI2C6-LH-FcB-CH-156 | QRFVTGHFGGLHPANGGGGGSQVQLQESGPGLVKPSETLSLTCTVSGGSISSYYWSWIRQPPG<br>KGLEWIGYIYYSGSTNYNPSLKSRVTMSIDTSKNQFSLKLISVTAADTAVYYCARDQRRIVAA<br>GGYYYGMDVWGQGTTVTVSSGGGGSGGGGSGGGGSEIVLTQSPGTLSLSPGERATLSCRASQS<br>VSSSYLAWYQQKPGQAPRLLIYGASTRATGIPARFSGSGSGTDFTLTISRLEPEDFAVYYCQQ<br>YGSSPFTFGPGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN |

| | | |
|---|---|---|
| | | FGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 400. | CH19_2-H7xI2C6-LH-FcB-CH-156 | QRFVTGHFGGLHPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 401. | CH19_5-B3xI2C6-LH-FcB-CH-156 | QRFVTGHFGGLHPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 402. | CH19_5-E10xI2C6-LH-FcB-CH-156 | QRFVTGHFGGLHPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHH |

| | | |
|---|---|---|
| | | HHHH |
| 403. | CH19_6-G10xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 404. | CH19_4-H8xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSVGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 405. | CH19_2-E4xI2C6-LH-FcB-CH-156 | QRFVTGHFGGLHPANGGGGGSQVQLVQSGAEVKKPGASVKVSCKASGYTFTSYGISWVRQAPGQGLEWMGWINPYTGNRNYAQKVQDRVTMTTDTSTNTAYMELSSLRSDDTAVYYCARGSGGFDYWGQGTLVTVSSGGGGSGGGGSGGGGSDIVMTQSPLSLPVTLGQPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 406. | CH19_6-B8xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKLQDRVTMTTDTSTNTAYMELRSLRSDDTAVYYCARGSGGFDY |

| | | |
|---|---|---|
| | | WGQGTLVTVSSGGGGSGGGGSGGGGSDIVMTQTPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 407. | CH19_0-B4xI2C6-LH-FcB-CH-156 | QRFVTGHFGGLHPANGGGGGSQVQLVQSGAEVKKPGASVKVSCKASGYTFTTYGISWVRQAPGQGLEWMGWINPYTGKTNYAQKLQGRVTMATDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 408. | CH19_9-F1xI2C6-LH-FcB-CH-156 | QRFVTGHFGGLHPANGGGGGSQVQLVQSGAEVKEPGASVKVSCKASGYTFTYYGISWVRQAPGQGLEWMGWINPYTGNRNYAQKVQDRVTMTTDTSTNTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 409. | CH19_4-A7xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFCTGHFGGLHPCNGGGGGSGGGSRDWDFDVFGGGTPVGGHH HHHH |
| 410. | CH19_6-E12xI2C6-LH-FcB-CH-156 | QRFVTGHFGGLHPANGGGGGSQVQLVQSGAEVKKPGASVKVSCKASGYTFTSYGITWVRQAPG QGLEWMGWINPYTGNRNYAQKVQGRVTMTTDTSTSTAYMELRSLRSDDTAVYYCARGSGGFDY WGQGTLVTVSSGGGGSGGGGSGGGGSDIVMTQSPLSLSVTPGEPASISCRSSQSLLHSVGYNY LDWYLQKPGQSPQLLIYLGSNRASGVPDRFSGSGSGTDFTLKISRVEAEDVGVYYCMQALQTP FTFGPGTKVDIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFCTGHFGGLHPCNGGGGGSGGGSRDWDFDVFGGGTPVGGHH HHHH |
| 411. | CH19_6-C12xI2C6-LH-FcB-CH-156 | QRFVTGHFGGLHPANGGGGGSQVQLVQSGAEVKKPGASVKVSCKASGYTFTSYGISWVRQAPG QGLEWMGWINPYTGNRNYAQKFQGRVTMTTDTSTNTAYMELSSLRSEDTAVYYCARGSGGFDY WGQGTLVTVSSGGGGSGGGGSGGGGSDIVMTQSPLSLPVTPGEPASISCRSSQSLLHSVGYNY LDWYLQKPGQSPQLLIYLGSNRASGVPDRFSGSGSGTDFTLKISRVEAEDVGVYYCMQALQTP FTFGPGTKVDIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFCTGHFGGLHPCNGGGGGSGGGSRDWDFDVFGGGTPVGGHH HHHH |
| 412. | CH19_6-A7xI2C6-LH-FcB-CH-156 | QRFVTGHFGGLHPANGGGGGSQVQLVQSGAEVKKPGASVKVSCKASGYSFTSYGISWVRQAPG QGLEWMGWINPYTGNRNYAQKVQGRVTMTTDTSTSTAYMELRSLRSDDTAVYYCARGSGGFDY WGQGTLVTVSSGGGGSGGGGSGGGGSDIVMTQSPLSLPVTPGEPASISCRSSQSLLHSVGYNY LDWYLQKPGQSPQLLIYLGSNRASGVPDRFSGSGSGTDFTLKISRVEAEDVGVYYCMQALQTP FTFGPGTKVEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFCTGHFGGLHPCNGGGGGSGGGSRDWDFDVFGGGTPVGGHHHHHH |
| 413. | CH19_6-G8xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 414. | CH19_6-F9xI2C6-LH-FcB-CH-156 | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 415. | CH19_0-C11xI2C6-LH-FcB-CH-156 | QRFVTGHFGGLHPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 416. | CH19_8-F6xI2C6-LH-FcB- | QRFVTGHFGGLHPANGGGGGSQVQLVESGGGVVQPGRSLRLSCVASGFTFRNYAMHWVRQAPG |

| | | |
|---|---|---|
| | CH-156 | KGLEWVAVISYSGNNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCRASQ<br>SISSYLNWYQQKPGKAPKLLIYDASSLQSGVPSRFSGSGSGTEFTLTISSLQAEDVAVYYCQQ<br>YYSTPLTFGGGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFCTGHFGGLHPCNGGGGGSGGGSRDWDFDVFGGGTP<br>VGGHHHHHH |
| 417. | CH19_0-G9xI2C6-LH-FcB-CH-156 | QRFVTGHFGGLHPANGGGGGSQVQLVESGGGVVQPGRSLRLSCAASGFTFRNYAMHWVRQAPG<br>KGLEWVAGISYSGTNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTEFTLTISSLQPEDIATYYCQQ<br>YDNLPLTFGGGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFCTGHFGGLHPCNGGGGGSGGGSRDWDFDVFGGGTP<br>VGGHHHHHH |
| 418. | CH19_1-E11xI2C6-LH-FcB-CH-156 | QRFVTGHFGGLHPANGGGGGSQVQLVESGGGVVQPGRSLRLSCVASGFTFRNYAMHWVRQAPG<br>KGLEWVAVISYSGNNKYYASSVKGRFTISRDNSKNTLFLQLNSLRAEDTAVYYCAREFYYDST<br>GYDYSTPGNYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTDFTFTISSLQPEDIATYYCQQ<br>YVNLPLTFGGGTKLEIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFCTGHFGGLHPCNGGGGGSGGGSRDWDFDVFGGGTP<br>VGGHHHHHH |
| 419. | CH19_0-F5xI2C6-LH-FcB-CH-156 | QRFVTGHFGGLHPANGGGGGSQVQLVESGGGVVQPGRSLRLSCAASGFTFSSYGMQWVRQAPG<br>KGLEWVAVIWYSGSNKYYASSVKGRFTISRDNSKNTLYLQMNSLRAEDTAVYYCARDGYPIFY<br>RYYGMDVWGQGTTVTVSSGGGGSGGGGSGGGGSDIQMTQSPSSLSASVGDRVTITCRASQGIS<br>NYLAWYQQKPGKVPKLLIYAASTLQSGVPSRFSGSGSGTDFTLTISSLQPEDVATYYCQKYNS |

| | | |
|---|---|---|
| | | APLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 420. | CH19_1-E1xI2C6-LH-FcB-CH-156 | QRFVTGHFGGLHPANGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 421. | CH19_1-E6xI2C6-LH-FcB-CH-156 | QRFVTGHFGGLHPANGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPFTFGPGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 422. | CH19_2G6_302_VKGxI2C6-LH-FcB-LH-156 | QRFVTGHFGGLHPANGGGGGSQVQLVESGGGVVQPGGSLRLSCAASGFTFSSYGMHWVRQAPGKGLEWVAFIWYDGSNKYYADSVKGRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGA |

| | | |
|---|---|---|
| | | VTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 423. | CH19_0-E11xI2C6-LH-FcB-LH-156 | QRFVTGHFGGLHPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 424. | CH19_5-G4xI2C6-LH-FcB-LH-156 | QRFVTGHFGGLHPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 425. | CH19_8-H6xI2C6-LH-FcB-LH-156 | QRFVTGHFGGLHPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |

| | | |
|---|---|---|
| 426. | CH19_2-C11xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLRSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 427. | CH19_2-A10xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYSGSTFYNPSLRSRVTISVDTSKNQFSLKLSSVTAADTAVYYCARDSSSSR<br>ALDYWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDSSP<br>RTFGQGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 428. | CH19_1-D11xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 429. | CH19_9-F9xI2C6-LH-FcB-LH-156 | QRFVTGHFGGLHPANGGGGGSQVQLQESGPGLVKPSETLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY |

| | | |
|---|---|---|
| | | LAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 430. | CH19_1-H8xI2C6-LH-FcB-LH-156 | QRFVTGHFGGLHPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 431. | CH19_1-B12xI2C6-LH-FcB-LH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 432. | CH19_0-C4xI2C6-LH-FcB-LH-156 | QRFVTGHFGGLHPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYI |

| | | |
|---|---|---|
| | | SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGGGGGSGGGSRDWDFDVFGGGTPVGGHHH<br>HHH |
| 433. | CH19_3-F2xI2C6-LH-FcB-LH-156 | QRFVTGHFGGLHPANGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGGGGGSGGGSRDWDFDVFGGGTPVGGHHH<br>HHH |
| 434. | CH19_3-B10xI2C6-LH-FcB-LH-156 | QRFVTGHFGGLHPANGGGGGSQVQLQESGPGLVKPSQTLSLTCTVSGGSISSGGYYWSWIRQH<br>PGKGLDWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 435. | CH19_0-G4xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASTRATGIPDRFSGSGSGTDFTLTISRLEPEDFAVYYCQQYGSSP<br>SFGQGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGGGGGSGGGSRDWDFDVFGGGTPVGGHHH |

| | | |
|---|---|---|
| | | HHH |
| 436. | CH19_0-H5xI2C6-LH-FcB-LH-156 | QRFVTGHFGGLHPANGGGGGSQVQLQESGPGLVRPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYDTSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 437. | CH19_0-B8xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGGGGGSGGGSRDWDFDVFGGGTPVGGHHH<br>HHH |
| 438. | CH19_2-D9xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 439. | CH19_8-H7xI2C6-LH-FcB-LH-156 | QRFVTGHFGGLHPANGGGGGSQVQLQESGPGLVKPSQTLSLTCTVSGGSISSGGYYWSWVRQH<br>PGKGLEWIGYIFYSGRTYYNPSLKSRVIISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 440. | CH19_9-C2xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 441. | CH19_3-D5xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIFYSGRTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 442. | CH19_1-G11xI2C6-LH-FcB-LH-156 | QRFVTGHFGGLHPANGGGGGSQVQLQESGPGLVKPSETLSLTCTVSGGSVSSGGYYWSWIRQP<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 443. | CH19_1-H11xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGQGTRLEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLHPANGGGGGSGGGSRDWDFDVFGGGTPVGGHHH<br>HHH |
| 444. | CH19_9-F3xI2C6-LH-FcB-LH-156 | QRFVTGHFGGLHPANGGGGGSQVQLQESGPGLVKPSQTLSLTCTVSGGSISSGGYYWSWIRQH<br>PGKGLEWIGYIFYSGKTYYNPSLKSRVSISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>ITFGQGTRLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 445. | CH19_2-G6xI2C6-LH-FcB-LH-156 | QRFVTGHFGGLHPANGGGGGSQVQLQESGPGLVKPSETLSLTCTVSGGSISSYYWSWIRQPPG<br>KGLEWIGYIYYSGSTNYNPSLKSRVTMSIDTSKNQFSLKLISVTAADTAVYYCARDQRRIVAA<br>GGYYYGMDVWGQGTTVTVSSGGGGSGGGGSGGGGSEIVLTQSPGTLSLSPGERATLSCRASQS<br>VSSSYLAWYQQKPGQAPRLLIYGASTRATGIPARFSGSGSGTDFTLTISRLEPEDFAVYYCQQ<br>YGSSPFTFGPGTKVDIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY |

| | | |
|---|---|---|
| | | CVLWYSNRWVFGGGTKLTVLGGGGSQRFVTGHFGGLHPANGGGGGSGGGSRDWDFDVFGGGTPVGGHHHHHH |
| 446. | CH19_2-H7xI2C6-LH-FcB-LH-156 | QRFVTGHFGGLHPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 447. | CH19_5-B3xI2C6-LH-FcB-LH-156 | QRFVTGHFGGLHPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 448. | CH19_5-E10xI2C6-LH-FcB-LH-156 | QRFVTGHFGGLHPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 449. | CH19_6-G10xI2C6-LH- | QRFVTGHFGGLHPANGGGGGSQVQLVQSGAEVKKPGASVKVSCKASGYTFTYYGISWVRQAPG |

| | | |
|---|---|---|
| | FcB-LH-156 | QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 450. | CH19_4-H8xI2C6-LH-FcB-LH-156 | QRFVTGHFGGLHPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSVGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 451. | CH19_2-E4xI2C6-LH-FcB-LH-156 | QRFVTGHFGGLHPANGGGGGSQVQLVQSGAEVKKPGASVKVSCKASGYTFTSYGISWVRQAPG<br>QGLEWMGWINPYTGNRNYAQKVQDRVTMTTDTSTNTAYMELSSLRSDDTAVYYCARGSGGFDY<br>WGQGTLVTVSSGGGGSGGGGSGGGGSDIVMTQSPLSLPVTLGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 452. | CH19_6-B8xI2C6-LH-FcB-LH-156 | QRFVTGHFGGLHPANGGGGGSQVQLVQSGAEVKKPGASVKVSCKASGYTFTYYGISWVRQAPG<br>QGLEWMGWINPYTGKTNYAQKLQDRVTMTTDTSTNTAYMELRSLRSDDTAVYYCARGSGGFDY<br>WGQGTLVTVSSGGGGSGGGGSGGGGSDIVMTQTPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP |

| | | |
|---|---|---|
| | | FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 453. | CH19_0-B4xI2C6-LH-FcB-LH-156 | QRFVTGHFGGLHPANGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 454. | CH19_9-F1xI2C6-LH-FcB-LH-156 | QRFVTGHFGGLHPANGGGGGSQVQLVQSGAEVKEPGASVKVSCKASGYTFTYYGISWVRQAPG<br>QGLEWMGWINPYTGNRNYAQKVQDRVTMTTDTSTNTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 455. | CH19_4-A7xI2C6-LH-FcB-LH-156 | QRFVTGHFGGLHPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS |

| | | |
|---|---|---|
| | | GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 456. | CH19_6-E12xI2C6-LH-FcB-LH-156 | QRFVTGHFGGLHPANGGGGGSQVQLVQSGAEVKKPGASVKVSCKASGYTFTSYGITWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLSVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 457. | CH19_6-C12xI2C6-LH-FcB-LH-156 | QRFVTGHFGGLHPANGGGGGSQVQLVQSGAEVKKPGASVKVSCKASGYTFTSYGISWVRQAPG<br>QGLEWMGWINPYTGNRNYAQKFQGRVTMTTDTSTNTAYMELSSLRSEDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 458. | CH19_6-A7xI2C6-LH-FcB-LH-156 | QRFVTGHFGGLHPANGGGGGSQVQLVQSGAEVKKPGASVKVSCKASGYSFTSYGISWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |

| | | |
|---|---|---|
| 459. | CH19_6-G8xI2C6-LH-FcB-LH-156 | QRFVTGHFGGLHPANGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 460. | CH19_6-F9xI2C6-LH-FcB-LH-156 | QRFVTGHFGGLHPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 461. | CH19_0-C11xI2C6-LH-FcB-LH-156 | QRFVTGHFGGLHPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 462. | CH19_8-F6xI2C6-LH-FcB-LH-156 | QRFVTGHFGGLHPANGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQ |

| | | |
|---|---|---|
| | | SISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 463. | CH19_0-G9xI2C6-LH-FcB-LH-156 | QRFVTGHFGGLHPANGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 464. | CH19_1-E11xI2C6-LH-FcB-LH-156 | QRFVTGHFGGLHPANGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 465. | CH19_0-F5xI2C6-LH-FcB-LH-156 | QRFVTGHFGGLHPANGGGGGSQVQLVESGGGVVQPGRSLRLSCAASGFTFSSYGMQWVRQAPGKGLEWVAVIWYSGSNKYYASSVKGRFTISRDNSKNTLYLQMNSLRAEDTAVYYCARDGYPIFYRYYGMDVWGQGTTVTVSSGGGGSGGGGSGGGGSDIQMTQSPSSLSASVGDRVTITCRASQGISNYLAWYQQKPGKVPKLLIYAASTLQSGVPSRFSGSGSGTDFTLTISSLQPEDVATYYCQKYNSAPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGN |

| | | |
|---|---|---|
| | | SYISYWAYWGQGTLVTVSSGGGGSGGGGSGGGGSQTVVTQEPSLTVSPGGTVTLTCGSSTGAV TSGNYPNWVQQKPGQAPRGLIGGTKFLAPGTPARFSGSLLGGKAALTLSGVQPEDEAEYYCVL WYSNRWVFGGGTKLTVLGGGGSQRFVTGHFGGLHPANGGGGGSGGGSRDWDFDVFGGGTPVGG HHHHHH |
| 466. | CH19_1-E1xI2C6-LH-FcB-LH-156 | QRFVTGHFGGLHPANGGGGGSQVQLVESGGGVVQPGRSLRLSCAASGFTFSSYGMHWVRQAPG KGLEWVAVISYSGSNKYYASSVKGRFTISRDNSKNTLYLQMNSLRAEDTAVYYCARDGYPIFY RYYGMDVWGQGTTVTVSSGGGGSGGGGSGGGGSEIVLTQSPGTLSLSPGERATLSCRASQSVS SSYLAWYQQKPGQAPRLLIYGASSRATGIPDRFSGSGSGTDFTLTISRLEPEDLAVYYCQQYG TSPLTFGGGTKLEIKSGGGGSEVQLVESGGGLVQPGGSLKLSCAASGFTFNKYAMNWVRQAPG KGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGA VTSGNYPNWVQQKPGQAPRGLIGGTKFLAPGTPARFSGSLLGGKAALTLSGVQPEDEAEYYCV LWYSNRWVFGGGTKLTVLGGGGSQRFVTGHFGGLHPANGGGGGSGGGSRDWDFDVFGGGTPVG GHHHHHH |
| 467. | CH19_1-E6xI2C6-LH-FcB-LH-156 | QRFVTGHFGGLHPANGGGGGSQVQLVESGGGVVQPGRSLRLSCAASGFTFSSFGMHWVRQAPG KGLEWVAFIWYSGSNKYYASSVKGRVTISRDNSKNTLYLQMNSLRAEDTAVYYCARDGYPIFY YYYGMDVWGQGTTVTVSSGGGGSGGGGSGGGGSEIVLTQSPGTLSLSPGERATLSCRASQSVS SSYLAWYQQKPGQAPRLLIYGASSRATGIPDRFSGSGSGTDFTLTISRLEPEDFAVYYCQQYG SSPFTFGPGTRLEIKSGGGGSEVQLVESGGGLVQPGGSLKLSCAASGFTFNKYAMNWVRQAPG KGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGA VTSGNYPNWVQQKPGQAPRGLIGGTKFLAPGTPARFSGSLLGGKAALTLSGVQPEDEAEYYCV LWYSNRWVFGGGTKLTVLGGGGSQRFVTGHFGGLHPANGGGGGSGGGSRDWDFDVFGGGTPVG GHHHHHH |
| 468. | CH19_2G6_302_VKGxI2C6-LY-FcB-LH-156 | QRFVTGHFGGLYPANGGGGGSQVQLVESGGGVVQPGGSLRLSCAASGFTFSSYGMHWVRQAPG KGLEWVAFIWYDGSNKYYADSVKGRFTISRDNSKNTLYLQMNSLRAEDTAVYYCARRAGIIGT IGYYYGMDVWGQGTTVTVSSGGGGSGGGGSGGGGSSYELTQPPSVSVSPGQTASITCSGDRLG EKYTSWYQQRPGQSPLLVIYQDTKRPSGIPERFSGSNSGNTATLTISGTQAMDEADYYCQAWE SSTVVFGGGTKLTVLSGGGGSEVQLVESGGGLVQPGGSLKLSCAASGFTFNKYAMNWVRQAPG KGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGA VTSGNYPNWVQQKPGQAPRGLIGGTKFLAPGTPARFSGSLLGGKAALTLSGVQPEDEAEYYCV LWYSNRWVFGGGTKLTVLGGGGSQRFVTGHFGGLHPANGGGGGSGGGSRDWDFDVFGGGTPVG |

| | | |
|---|---|---|
| | | GHHHHHH |
| 469. | CH19_0-E11xI2C6-LY-FcB-LH-156 | QRFVTGHFGGLYPANGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQFPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 470. | CH19_5-G4xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGTSNRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 471. | CH19_8-H6xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 472. | CH19_2-C11xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLRSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 473. | CH19_2-A10xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 474. | CH19_1-D11xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 475. | CH19_9-F9xI2C6-LY-FcB-LH-156 | QRFVTGHFGGLYPANGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLE |

| | | |
|---|---|---|
| | | WVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 476. | CH19_1-H8xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 477. | CH19_1-B12xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 478. | CH19_0-C4xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLHPANGGGGGSGGGSRDWDFDVFGGGTPVGGHHHHHH |
| 479. | CH19_3-F2xI2C6-LY-FcB-LH-156 | QRFVTGHFGGLYPANGGGGGSQVQLQESGPGLVKPSQTLSLTCTISGGSISSGGYYWSWIRQHPGKGLEWIGYIFYSGRTYYNPSLKSRVTISVDTSKNQFSLKLTSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 480. | CH19_3-B10xI2C6-LY-FcB-LH-156 | QRFVTGHFGGLYPANGGGGGSQVQLQESGPGLVKPSQTLSLTCTVSGGSISSGGYYWSWIRQHPGKGLDWIGYIFYRGRTYYNPSLKSRVTISVDTSKNQFSLKLSSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 481. | CH19_0-G4xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 482. | CH19_0-H5xI2C6-LY-FcB- | QRFVTGHFGGLYPANGGGGGSQVQLQESGPGLVRPSQTLSLTCTVSGGSISSGGYYWSWIRQH |

| | | |
|---|---|---|
| | LH-156 | PGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 483. | CH19_0-B8xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 484. | CH19_2-D9xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 485. | CH19_8-H7xI2C6-LY-FcB-LH-156 | QRFVTGHFGGLYPANGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSP |

| | | |
|---|---|---|
| | | LTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 486. | CH19_9-C2xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 487. | CH19_3-D5xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 488. | CH19_1-G11xI2C6-LY-FcB-LH-156 | QRFVTGHFGGLYPANGGGGGSQVQLQESGPGLVKPSETLSLTCTVSGGSVSSGGYYWSWIRQPPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTS |

| | | |
|---|---|---|
| | | GNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 489. | CH19_1-H11xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 490. | CH19_9-F3xI2C6-LY-FcB-LH-156 | QRFVTGHFGGLYPANGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 491. | CH19_2-G6xI2C6-LY-FcB-LH-156 | QRFVTGHFGGLYPANGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |

| | | |
|---|---|---|
| 492. | CH19_2-H7xI2C6-LY-FcB-LH-156 | QRFVTGHFGGLYPANGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 493. | CH19_5-B3xI2C6-LY-FcB-LH-156 | QRFVTGHFGGLYPANGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 494. | CH19_5-E10xI2C6-LY-FcB-LH-156 | QRFVTGHFGGLYPANGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 495. | CH19_6-G10xI2C6-LY-FcB-LH-156 | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNY |

| | | |
|---|---|---|
| | | LDWYLQKPGQSPQLLIYLGSYRASGVPDRFSGSGSGTDFTLKISRVEAEDVGVYYCMQALQTP FTFGPGTKVDIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGGGGGSGGGSRDWDFDVFGGGTPVGGHH HHHH |
| 496. | CH19_4-H8xI2C6-LY-FcB-LH-156 | QRFVTGHFGGLYPANGGGGGSQVQLVQSGAEVKKPGASVKVSCKASGYTFTYYGISWVRQAPG QGLEWMGWINPYTGKTNYAQKLQGRVTMTTDTSTSTAYMELRSLRSDDTAVYYCARGSGGFDY WGQGTLVTVSSGGGGSGGGGSGGGGSDIVMTQTPLSLSVTPGEPASISCRSSQSLLHSVGYNY LDWYLQKPGQSPQLLIYLGSYRASGVPDRFSGSGSGTDFTLKISRVEAEDVGVYYCMQALQTP FTFGPGTKVDIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGGGGGSGGGSRDWDFDVFGGGTPVGGHH HHHH |
| 497. | CH19_2-E4xI2C6-LY-FcB-LH-156 | QRFVTGHFGGLYPANGGGGGSQVQLVQSGAEVKKPGASVKVSCKASGYTFTSYGISWVRQAPG QGLEWMGWINPYTGNRNYAQKVQDRVTMTTDTSTNTAYMELSSLRSDDTAVYYCARGSGGFDY WGQGTLVTVSSGGGGSGGGGSGGGGSDIVMTQSPLSLPVTLGQPASISCRSSQSLLHSVGYNY LDWYLQKPGQSPQLLIYLGSNRASGVPDRFSGSGSGTDFTLKISRVEAEDVGVYYCMQALQTP FTFGPGTKLEIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY ISYWAYWGQGTLVTVSSGGGGSGGGGSGGGGSQTVVTQEPSLTVSPGGTVTLTCGSSTGAVTS GNYPNWVQQKPGQAPRGLIGGTKFLAPGTPARFSGSLLGGKAALTLSGVQPEDEAEYYCVLWY SNRWVFGGGTKLTVLGGGGSQRFVTGHFGGLHPANGGGGGSGGGSRDWDFDVFGGGTPVGGHH HHHH |
| 498. | CH19_6-B8xI2C6-LY-FcB-LH-156 | QRFVTGHFGGLYPANGGGGGSQVQLVQSGAEVKKPGASVKVSCKASGYTFTYYGISWVRQAPG QGLEWMGWINPYTGKTNYAQKLQDRVTMTTDTSTNTAYMELRSLRSDDTAVYYCARGSGGFDY WGQGTLVTVSSGGGGSGGGGSGGGGSDIVMTQTPLSLPVTPGEPASISCRSSQSLLHSVGYNY LDWYLQKPGQSPQLLIYLGSNRASGVPDRFSGSGSGTDFTLKISRVEAEDVGVYYCMQALQTP FTFGPGTKVDIKSGGGGSEVQLVESGGGLVQPGGSLKLSCAASGFTFNKYAMNWVRQAPGKGL EWVARIRSKYNNYATYYADSVKDRFTISRDDSKNTAYLQMNNLKTEDTAVYYCVRHGNFGNSY |

| | | |
|---|---|---|
| | | ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 499. | CH19_0-B4xI2C6-LY-FcB-LH-156 | QRFVTGHFGGLYPANGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 500. | CH19_9-F1xI2C6-LY-FcB-LH-156 | QRFVTGHFGGLYPANGGGGGSQVQLVQSGAEVKEPGASVKVSCKASGYTFTYYGISWVRQAPG<br>QGLEWMGWINPYTGNRNYAQKVQDRVTMTTDTSTNTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 501. | CH19_4-A7xI2C6-LY-FcB-LH-156 | QRFVTGHFGGLYPANGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH |

| | | |
|---|---|---|
| | | HHHH |
| 502. | CH19_6-E12xI2C6-LY-FcB-LH-156 | QRFVTGHFGGLYPANGGGGGSQVQLVQSGAEVKKPGASVKVSCKASGYTFTSYGITWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLSVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 503. | CH19_6-C12xI2C6-LY-FcB-LH-156 | QRFVTGHFGGLYPANGGGGGSQVQLVQSGAEVKKPGASVKVSCKASGYTFTSYGISWVRQAPG<br>QGLEWMGWINPYTGNRNYAQKFQGRVTMTTDTSTNTAYMELSSLRSEDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 504. | CH19_6-A7xI2C6-LY-FcB-LH-156 | QRFVTGHFGGLYPANGGGGGSQVQLVQSGAEVKKPGASVKVSCKASGYSFTSYGISWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGGGGGSGGGSRDWDFDVFGGGTPVGGHH<br>HHHH |
| 505. | CH19_6-G8xI2C6-LY-FcB-LH-156 | QRFVTGHFGGLYPANGGGGGSQVQLVQSGAEVKKPGASVKVSCKASGYTFTYYGISWVRQAPG<br>QGLEWMGWINPYTGKTNYAQKVQGRVTMTTDTSTSTAYMELRNLRSDDTAVYYCARGSGGFDY |

| | | |
|---|---|---|
| | | WGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 506. | CH19_6-F9xI2C6-LY-FcB-LH-156 | QRFVTGHFGGLYPANGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 507. | CH19_0-C11xI2C6-LY-FcB-LH-156 | QRFVTGHFGGLYPANGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 508. | CH19_8-F6xI2C6-LY-FcB-LH-156 | QRFVTGHFGGLYPANGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQA |

| | | |
|---|---|---|
| | | PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFVTGHFGGLHPANGGGGGSGGGSRDWDFDVFGGGTP VGGHHHHHH |
| 509. | CH19_0-G9xI2C6-LY-FcB-LH-156 | QRFVTGHFGGLYPANGGGGGSQVQLVESGGGVVQPGRSLRLSCAASGFTFRNYAMHWVRQAPG KGLEWVAGISYSGTNKYYASSVKGRFTISRDNSKNTLYLQLNSLRAEDTAVYYCAREFYYDST GYDYSTPGDYWGQGTLVTVSSGGGGSGGGGSGGGGSDIQMTQSPSSLSASVGDRVTITCQASQ DISNYLNWYQQKPGKAPKLLIYDASNLETGVPSRFSGSGSGTEFTLTISSLQPEDIATYYCQQ YDNLPLTFGGGTKVDIKSGGGGSEVQLVESGGGLVQPGGSLKLSCAASGFTFNKYAMNWVRQA PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFVTGHFGGLHPANGGGGGSGGGSRDWDFDVFGGGTP VGGHHHHHH |
| 510. | CH19_1-E11xI2C6-LY-FcB-LH-156 | QRFVTGHFGGLYPANGGGGGSQVQLVESGGGVVQPGRSLRLSCVASGFTFRNYAMHWVRQAPG KGLEWVAVISYSGNNKYYASSVKGRFTISRDNSKNTLFLQLNSLRAEDTAVYYCAREFYYDST GYDYSTPGNYWGQGTLVTVSSGGGGSGGGGSGGGGSDIQMTQSPSSLSASVGDRVTITCQASQ DISNYLNWYQQKPGKAPKLLIYDASNLETGVPSRFSGSGSGTDFTFTISSLQPEDIATYYCQQ YVNLPLTFGGGTKLEIKSGGGGSEVQLVESGGGLVQPGGSLKLSCAASGFTFNKYAMNWVRQA PGKGLEWVARIRSKYNNYATYYADSVKDRFTISRDDSKNTAYLQMNNLKTEDTAVYYCVRHGN FGNSYISYWAYWGQGTLVTVSSGGGGSGGGGSGGGGSQTVVTQEPSLTVSPGGTVTLTCGSST GAVTSGNYPNWVQQKPGQAPRGLIGGTKFLAPGTPARFSGSLLGGKAALTLSGVQPEDEAEYY CVLWYSNRWVFGGGTKLTVLGGGGSQRFVTGHFGGLHPANGGGGGSGGGSRDWDFDVFGGGTP VGGHHHHHH |
| 511. | CH19_0-F5xI2C6-LY-FcB-LH-156 | QRFVTGHFGGLYPANGGGGGSQVQLVESGGGVVQPGRSLRLSCAASGFTFSSYGMQWVRQAPG KGLEWVAVIWYSGSNKYYASSVKGRFTISRDNSKNTLYLQMNSLRAEDTAVYYCARDGYPIFY RYYGMDVWGQGTTVTVSSGGGGSGGGGSGGGGSDIQMTQSPSSLSASVGDRVTITCRASQGIS NYLAWYQQKPGKVPKLLIYAASTLQSGVPSRFSGSGSGTDFTLTISSLQPEDVATYYCQKYNS APLTFGGGTKVEIKSGGGGSEVQLVESGGGLVQPGGSLKLSCAASGFTFNKYAMNWVRQAPGK GLEWVARIRSKYNNYATYYADSVKDRFTISRDDSKNTAYLQMNNLKTEDTAVYYCVRHGNFGN SYISYWAYWGQGTLVTVSSGGGGSGGGGSGGGGSQTVVTQEPSLTVSPGGTVTLTCGSSTGAV TSGNYPNWVQQKPGQAPRGLIGGTKFLAPGTPARFSGSLLGGKAALTLSGVQPEDEAEYYCVL |

| | | |
|---|---|---|
| | | WYSNRWVFGGGTKLTVLGGGGSQRFVTGHFGGLHPANGGGGGSGGGSRDWDFDVFGGGTPVGGHHHHHH |
| 512. | CH19_1-E1xI2C6-LY-FcB-LH-156 | QRFVTGHFGGLYPANGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 513. | CH19_1-E6xI2C6-LY-FcB-LH-156 | QRFVTGHFGGLYPANGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPFTFGPGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGGGGGSGGGSRDWDFDVFGGGTPVGGHHHHHH |
| 514. | CH19_2G6_302_VKGxI2C6-CH-FcB-LH | QRFCTGHFGGLHPCNGGGGGSQVQLVESGGGVVQPGGSLRLSCAASGFTFSSYGMHWVRQAPGKGLEWVAFIWYDGSNKYYADSVKGRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 515. | CH19_0-E11xI2C6-CH-FcB-LH | QRFCTGHFGGLHPCNGGGGGSQVQLQESGPGLVKPSQTLSLTCTVSGGSISRGGYYWSWIRQHPGKGLEWIGYIFYRGRTYYNPSLKSRVTISVDTSKNQFSLKLSSVTAADTAVYYCARVNSNYG |

| | | |
|---|---|---|
| | | WFDPWGQGTLVTVSSGGGGSGGGGSGGGGSEIVLTQFPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 516. | CH19_5-G4xI2C6-CH-FcB-LH | QRFCTGHFGGLHPCNGGGGGSQVQLQESGPGLVKPSQTLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGTSNRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 517. | CH19_8-H6xI2C6-CH-FcB-LH | QRFCTGHFGGLHPCNGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 518. | CH19_2-C11xI2C6-CH-FcB-LH | QRFCTGHFGGLHPCNGGGGGSQVQLQESGPGLVKPSQTLSLTCTVSGGSISSGGYYWSWIRQH<br>PGKGLEWIGYIFYSGRTYYNPSLKSRVTISVDTSKNQFSLKLRSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 519. | CH19_2-A10xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 520. | CH19_1-D11xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 521. | CH19_9-F9xI2C6-CH-FcB-LH | QRFCTGHFGGLHPCNGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 522. | CH19_1-H8xI2C6-CH-FcB-LH | QRFCTGHFGGLHPCNGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 523. | CH19_1-B12xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 524. | CH19_0-C4xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 525. | CH19_3-F2xI2C6-CH-FcB-LH | QRFCTGHFGGLHPCNGGGGGSQVQLQESGPGLVKPSQTLSLTCTISGGSISSGGYYWSWIRQHPGKGLEWIGYIFYSGRTYYNPSLKSRVTISVDTSKNQFSLKLTSVTAADTAVYYCARVNSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 526. | CH19_3-B10xI2C6-CH-FcB-LH | QRFCTGHFGGLHPCNGGGGGSQVQLQESGPGLVKPSQTLSLTCTVSGGSISSGGYYWSWIRQHPGKGLDWIGYIFYRGRTYYNPSLKSRVTISVDTSKNQFSLKLSSVTAADTAVYYCARVNSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 527. | CH19_0-G4xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASTRATGIPDRFSGSGSGTDFTLTISRLEPEDFAVYYCQQYGSSPSFGQGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 528. | CH19_0-H5xI2C6-CH-FcB-LH | QRFCTGHFGGLHPCNGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 529. | CH19_0-B8xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLHPANGHHHHHH |
| 530. | CH19_2-D9xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 531. | CH19_8-H7xI2C6-CH-FcB-LH | QRFCTGHFGGLHPCNGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 532. | CH19_9-C2xI2C6-CH-FcB-LH | QRFCTGHFGGLHPCNGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 533. | CH19_3-D5xI2C6-CH-FcB-LH | QRFCTGHFGGLHPCNGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 534. | CH19_1-G11xI2C6-CH-FcB-LH | QRFCTGHFGGLHPCNGGGGGSQVQLQESGPGLVKPSETLSLTCTVSGGSVSSGGYYWSWIRQPPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 535. | CH19_1-H11xI2C6-CH-FcB-LH | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 536. | CH19_9-F3xI2C6-CH-FcB-LH | QRFCTGHFGGLHPCNGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 537. | CH19_2-G6xI2C6-CH-FcB-LH | QRFCTGHFGGLHPCNGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAA |

| | | |
|---|---|---|
| | | GGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 538. | CH19_2-H7xI2C6-CH-FcB-LH | QRFCTGHFGGLHPCNGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 539. | CH19_5-B3xI2C6-CH-FcB-LH | QRFCTGHFGGLHPCNGGGGGSQVQLVQSGAEVKKPGASVKVSCKASGYTFTYYGISWVRQAPGQGLEWMGWINPYGGETNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 540. | CH19_5-E10xI2C6-CH-FcB-LH | QRFCTGHFGGLHPCNGGGGGSQVQLVQSGAEVKKPGASVKVSCKASGYTFTYYGISWVRQAPGQGLEWMGWINPYSGATNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 541. | CH19_6-G10xI2C6-CH-FcB-LH | QRFCTGHFGGLHPCNGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSNGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 542. | CH19_4-H8xI2C6-CH-FcB-LH | QRFCTGHFGGLHPCNGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGEPASISCRSSQSLLHSVGYNYLDWYLQKPGQSPQLLIYLGSYRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 543. | CH19_2-E4xI2C6-CH-FcB-LH | QRFCTGHFGGLHPCNGGGGGSQVQLVQSGAEVKKPGASVKVSCKASGYTFTSYGISWVRQAPGQGLEWMGWINPYTGNRNYAQKVQDRVTMTTDTSTNTAYMELSSLRSDDTAVYYCARGSGGFDYWGQGTLVTVSSGGGGSGGGGSGGGGSDIVMTQSPLSLPVTLGQPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 544. | CH19_6-B8xI2C6-CH-FcB-LH | QRFCTGHFGGLHPCNGGGGGSQVQLVQSGAEVKKPGASVKVSCKASGYTFTYYGISWVRQAPGQGLEWMGWINPYTGKTNYAQKLQDRVTMTTDTSTNTAYMELRSLRSDDTAVYYCARGSGGFDYWGQGTLVTVSSGGGGSGGGGSGGGGSDIVMTQTPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 545. | CH19_0-B4xI2C6-CH-FcB-LH | QRFCTGHFGGLHPCNGGGGGSQVQLVQSGAEVKKPGASVKVSCKASGYTFTTYGISWVRQAPG<br>QGLEWMGWINPYTGKTNYAQKLQGRVTMATDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 546. | CH19_9-F1xI2C6-CH-FcB-LH | QRFCTGHFGGLHPCNGGGGGSQVQLVQSGAEVKEPGASVKVSCKASGYTFTYYGISWVRQAPG<br>QGLEWMGWINPYTGNRNYAQKVQDRVTMTTDTSTNTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 547. | CH19_4-A7xI2C6-CH-FcB-LH | QRFCTGHFGGLHPCNGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLHPANGHHHHHH |
| 548. | CH19_6-E12xI2C6-CH-FcB-LH | QRFCTGHFGGLHPCNGGGGGSQVQLVQSGAEVKKPGASVKVSCKASGYTFTSYGITWVRQAPG<br>QGLEWMGWINPYTGNRNYAQKVQGRVTMTTDTSTSTAYMELRSLRSDDTAVYYCARGSGGFDY |

| | | |
|---|---|---|
| | | WGQGTLVTVSSGGGGSGGGGSGGGGSDIVMTQSPLSLSVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 549. | CH19_6-C12xI2C6-CH-FcB-LH | QRFCTGHFGGLHPCNGGGGGSQVQLVQSGAEVKKPGASVKVSCKASGYTFTSYGISWVRQAPGQGLEWMGWINPYTGNRNYAQKFQGRVTMTTDTSTNTAYMELSSLRSEDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 550. | CH19_6-A7xI2C6-CH-FcB-LH | QRFCTGHFGGLHPCNGGGGGSQVQLVQSGAEVKKPGASVKVSCKASGYSFTSYGISWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 551. | CH19_6-G8xI2C6-CH-FcB-LH | QRFCTGHFGGLHPCNGGGGGSQVQLVQSGAEVKKPGASVKVSCKASGYTFTYYGISWVRQAPGQGLEWMGWINPYTGKTNYAQKVQGRVTMTTDTSTSTAYMELRNLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLHPANGHHHHHH |
| 552. | CH19_6-F9xI2C6-CH-FcB-LH | QRFCTGHFGGLHPCNGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQTPLSLSVTPGQPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 553. | CH19_0-C11xI2C6-CH-FcB-LH | QRFCTGHFGGLHPCNGGGGGSQVQLVESGGGVVQPGRSLRLSCAASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYDNLPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 554. | CH19_8-F6xI2C6-CH-FcB-LH | QRFCTGHFGGLHPCNGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLHPANGHHHHHH |
| 555. | CH19_0-G9xI2C6-CH-FcB-LH | QRFCTGHFGGLHPCNGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQA |

| | | |
|---|---|---|
| | | PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFVTGHFGGLHPANGHHHHHH |
| 556. | CH19_1-E11xI2C6-CH-FcB-LH | QRFCTGHFGGLHPCNGGGGGSQVQLVESGGGVVQPGRSLRLSCVASGFTFRNYAMHWVRQAPG<br>KGLEWVAVISYSGNNKYYASSVKGRFTISRDNSKNTLFLQLNSLRAEDTAVYYCAREFYYDST<br>GYDYSTPGNYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTDFTFTISSLQPEDIATYYCQQ<br>YVNLPLTFGGGTKLEIKSGGGGSEVQLVESGGGLVQPGGSLKLSCAASGFTFNKYAMNWVRQA<br>PGKGLEWVARIRSKYNNYATYYADSVKDRFTISRDDSKNTAYLQMNNLKTEDTAVYYCVRHGN<br>FGNSYISYWAYWGQGTLVTVSSGGGGSGGGGSGGGGSQTVVTQEPSLTVSPGGTVTLTCGSST<br>GAVTSGNYPNWVQQKPGQAPRGLIGGTKFLAPGTPARFSGSLLGGKAALTLSGVQPEDEAEYY<br>CVLWYSNRWVFGGGTKLTVLGGGGSQRFVTGHFGGLHPANGHHHHHH |
| 557. | CH19_0-F5xI2C6-CH-FcB-LH | QRFCTGHFGGLHPCNGGGGGSQVQLVESGGGVVQPGRSLRLSCAASGFTFSSYGMQWVRQAPG<br>KGLEWVAVIWYSGSNKYYASSVKGRFTISRDNSKNTLYLQMNSLRAEDTAVYYCARDGYPIFY<br>RYYGMDVWGQGTTVTVSSGGGGSGGGGSGGGGSDIQMTQSPSSLSASVGDRVTITCRASQGIS<br>NYLAWYQQKPGKVPKLLIYAASTLQSGVPSRFSGSGSGTDFTLTISSLQPEDVATYYCQKYNS<br>APLTFGGGTKVEIKSGGGGSEVQLVESGGGLVQPGGSLKLSCAASGFTFNKYAMNWVRQAPGK<br>GLEWVARIRSKYNNYATYYADSVKDRFTISRDDSKNTAYLQMNNLKTEDTAVYYCVRHGNFGN<br>SYISYWAYWGQGTLVTVSSGGGGSGGGGSGGGGSQTVVTQEPSLTVSPGGTVTLTCGSSTGAV<br>TSGNYPNWVQQKPGQAPRGLIGGTKFLAPGTPARFSGSLLGGKAALTLSGVQPEDEAEYYCVL<br>WYSNRWVFGGGTKLTVLGGGGSQRFVTGHFGGLHPANGHHHHHH |
| 558. | CH19_1-E1xI2C6-CH-FcB-LH | QRFCTGHFGGLHPCNGGGGGSQVQLVESGGGVVQPGRSLRLSCAASGFTFSSYGMHWVRQAPG<br>KGLEWVAVISYSGSNKYYASSVKGRFTISRDNSKNTLYLQMNSLRAEDTAVYYCARDGYPIFY<br>RYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDLAVYYCQQYG<br>TSPLTFGGGTKLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 559. | CH19_1-E6xI2C6-CH-FcB-LH | QRFCTGHFGGLHPCNGGGGGSQVQLVESGGGVVQPGRSLRLSCAASGFTFSSFGMHWVRQAPG<br>KGLEWVAFIWYSGSNKYYASSVKGRVTISRDNSKNTLYLQMNSLRAEDTAVYYCARDGYPIFY |

| | | |
|---|---|---|
| | | YYYGMDVWGQGTTVTVSSGGGGSGGGGSGGGGSEIVLTQSPGTLSLSPGERATLSCRASQSVS<br>SSYLAWYQQKPGQAPRLLIYGASSRATGIPDRFSGSGSGTDFTLTISRLEPEDFAVYYCQQYG<br>SSPFTFGPGTRLEIKSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLHPANGHHHHHH |
| 560. | CH19_2G6_302_VKGxI2C6-CH-FcB-LY | QRFCTGHFGGLHPCNGGGGGSQVQLVESGGGVVQPGGSLRLSCAASGFTFSSYGMHWVRQAPG<br>KGLEWVAFIWYDGSNKYYADSVKGRFTISRDNSKNTLYLQMNSLRAEDTAVYYCARRAGIIGT<br>IGYYYGMDVWGQGTTVTVSSGGGGSGGGGSGGGGSSYELTQPPSVSVSPGQTASITCSGDRLG<br>EKYTSWYQQRPGQSPLLVIYQDTKRPSGIPERFSGSNSGNTATLTISGTQAMDEADYYCQAWE<br>SSTVVFGGGTKLTVLSGGGGSEVQLVESGGGLVQPGGSLKLSCAASGFTFNKYAMNWVRQAPG<br>KGLEWVARIRSKYNNYATYYADSVKDRFTISRDDSKNTAYLQMNNLKTEDTAVYYCVRHGNFG<br>NSYISYWAYWGQGTLVTVSSGGGGSGGGGSGGGGSQTVVTQEPSLTVSPGGTVTLTCGSSTGA<br>VTSGNYPNWVQQKPGQAPRGLIGGTKFLAPGTPARFSGSLLGGKAALTLSGVQPEDEAEYYCV<br>LWYSNRWVFGGGTKLTVLGGGGSQRFVTGHFGGLYPANGHHHHHH |
| 561. | CH19_0-E11xI2C6-CH-FcB-LY | QRFCTGHFGGLHPCNGGGGGSQVQLQESGPGLVKPSQTLSLTCTVSGGSISRGGYYWSWIRQH<br>PGKGLEWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQFPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 562. | CH19_5-G4xI2C6-CH-FcB-LY | QRFCTGHFGGLHPCNGGGGGSQVQLQESGPGLVKPSQTLSLTCTVSGGSISSGGYYWSWIRQH<br>PGKGLEWIGYIFYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGTSNRATGIPDRFSGSGSGTDFTLTISRLEPEDFAVYYCQQYGSSP<br>LTFGGGTKLEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLYPANGHHHHHH |
| 563. | CH19_8-H6xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 564. | CH19_2-C11xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYSGRTYYNPSLKSRVTISVDTSKNQFSLKLRSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 565. | CH19_2-A10xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYSGSTFYNPSLRSRVTISVDTSKNQFSLKLSSVTAADTAVYYCARDSSSSRALDYWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDSSPRTFGQGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 566. | CH19_1-D11xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKLEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 567. | CH19_9-F9xI2C6-CH-FcB-LY | QRFCTGHFGGLHPCNGGGGGSQVQLQESGPGLVKPSETLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 568. | CH19_1-H8xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 569. | CH19_1-B12xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 570. | CH19_0-C4xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLYPANGHHHHHH |
| 571. | CH19_3-F2xI2C6-CH-FcB-LY | QRFCTGHFGGLHPCNGGGGGSQVQLQESGPGLVKPSQTLSLTCTISGGSISSGGYYWSWIRQH<br>PGKGLEWIGYIFYSGRTYYNPSLKSRVTISVDTSKNQFSLKLTSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>TFGGGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS<br>NRWVFGGGTKLTVLGGGGSQRFVTGHFGGLYPANGHHHHHH |
| 572. | CH19_3-B10xI2C6-CH-FcB-LY | QRFCTGHFGGLHPCNGGGGGSQVQLQESGPGLVKPSQTLSLTCTVSGGSISSGGYYWSWIRQH<br>PGKGLDWIGYIFYRGRTYYNPSLKSRVTISVDTSKNQFSLKLSSVTAADTAVYYCARVNSNYG<br>WFDPWGQGTLVTVSSGGGGSGGGGSGGGGSEIVLTQSPGTLSLSPGERATLSCRASQSVSSSY<br>LAWYQQKPGQAPRLLIYGASSRATGIPDRFSGSGSGTDFTLTISRLEPEDFAVYYCQQYGSSP<br>LTFGGGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 573. | CH19_0-G4xI2C6-CH-FcB-LY | QRFCTGHFGGLHPCNGGGGGSQVQLQESGPGLVKPSQTLSLTCTVSGGSISSGGYYWSWIRQH<br>PGKGLEWIGYIYYRGRTYYNPSLKSRVTISVDTSKNQFSLKLSSVTAADTAVYYCARVYSNYG<br>WFDPWGQGTLVTVSSGGGGSGGGGSGGGGSEIVLTQSPGTLSLSPGERATLSCRASQSVSSSY<br>LAWYQQKPGQAPRLLIYGASTRATGIPDRFSGSGSGTDFTLTISRLEPEDFAVYYCQQYGSSP<br>SFGQGTKVEIKSGGGGSEVQLVESGGGLVQPGGSLKLSCAASGFTFNKYAMNWVRQAPGKGLE<br>WVARIRSKYNNYATYYADSVKDRFTISRDDSKNTAYLQMNNLKTEDTAVYYCVRHGNFGNSYI<br>SYWAYWGQGTLVTVSSGGGGSGGGGSGGGGSQTVVTQEPSLTVSPGGTVTLTCGSSTGAVTSG<br>NYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLYPANGHHHHHH |
| 574. | CH19_0-H5xI2C6-CH-FcB-LY | QRFCTGHFGGLHPCNGGGGGSQVQLQESGPGLVRPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYDTSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 575. | CH19_0-B8xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 576. | CH19_2-D9xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 577. | CH19_8-H7xI2C6-CH-FcB-LY | QRFCTGHFGGLHPCNGGGGGSQVQLQESGPGLVKPSQTLSLTCTVSGGSISSGGYYWSWVRQHPGKGLEWIGYIFYSGRTYYNPSLKSRVIISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 578. | CH19_9-C2xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYSGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 579. | CH19_3-D5xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYSGRTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 580. | CH19_1-G11xI2C6-CH-FcB-LY | QRFCTGHFGGLHPCNGGGGGSQVQLQESGPGLVKPSETLSLTCTVSGGSVSSGGYYWSWIRQPPGKGLEWIGYIYYRGRTYYNPSLKSRVTISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 581. | CH19_1-H11xI2C6-CH-FcB-LY | QRFCTGHFGGLHPCNGGGGGSQVQLQESGPGLVKPSQTLSLTCTVSGGSISSGGYYWSWIRQHPGKGLEWIGYIYYRGRTYYNPSLKSRVTISVDTSKNQFSLKLSSVTAADTAVYYCARVYSNYG |

| | | |
|---|---|---|
| | | WFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPTFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 582. | CH19_9-F3xI2C6-CH-FcB-LY | QRFCTGHFGGLHPCNGGGGGSQVQLQESGPGLVKPSQTLSLTCTVSGGSISSGGYYWSWIRQHPGKGLEWIGYIFYSGKTYYNPSLKSRVSISVDTSKNQFSLKLSSVTAADTAVYYCARVYSNYGWFDPWGQGTLVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 583. | CH19_2-G6xI2C6-CH-FcB-LY | QRFCTGHFGGLHPCNGGGGGSQVQLQESGPGLVKPSETLSLTCTVSGGSISSYYWSWIRQPPGKGLEWIGYIYYSGSTNYNPSLKSRVTMSIDTSKNQFSLKLISVTAADTAVYYCARDQRRIVAAGGYYYGMDVWGQGTTVTVSSGGGGSGGGGSGGGGSEIVLTQSPGTLSLSPGERATLSCRASQSVSSSYLAWYQQKPGQAPRLLIYGASTRATGIPARFSGSGSGTDFTLTISRLEPEDFAVYYCQQYGSSPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 584. | CH19_2-H7xI2C6-CH-FcB-LY | QRFCTGHFGGLHPCNGGGGGSQVQLQESGPGLVKPSQTLSLTCTVSGGSISSNDYYWSWIRQHPGKGLEWIGYIYYSGSTFYNPSLKSRGAISVDTSKNQFSLKLTSVTAADTAVYYCARGVYRTGAFDIWGQGTMVTVSSGGGGSGGGGSGGGGSDIQMTQSPSSLSASVGDRVTITCRASQSISSYLNWYQQKPGKAPKSLIYAASSLQSGVPSKFSGSGSGTDFTLTISSLQPEDIATYYCQQSYSTPQAFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYS |

| | | |
|---|---|---|
| | | NRWVFGGGTKLTVLGGGGSQRFVTGHFGGLYPANGHHHHHH |
| 585. | CH19_5-B3xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYGGETNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 586. | CH19_5-E10xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYSGATNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 587. | CH19_6-G10xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSNGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 588. | CH19_4-H8xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGEPASISCRSSQSLLHSVGYNY<br>LDWYLQKPGQSPQLLIYLGSYRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL |

| | | |
|---|---|---|
| | | EWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 589. | CH19_2-E4xI2C6-CH-FcB-LY | QRFCTGHFGGLHPCNGGGGGSQVQLVQSGAEVKKPGASVKVSCKASGYTFTSYGISWVRQAPGQGLEWMGWINPYTGNRNYAQKVQDRVTMTTDTSTNTAYMELSSLRSDDTAVYYCARGSGGFDYWGQGTLVTVSSGGGGSGGGGSGGGGSDIVMTQSPLSLPVTLGQPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 590. | CH19_6-B8xI2C6-CH-FcB-LY | QRFCTGHFGGLHPCNGGGGGSQVQLVQSGAEVKKPGASVKVSCKASGYTFTYYGISWVRQAPGQGLEWMGWINPYTGKTNYAQKLQDRVTMTTDTSTNTAYMELRSLRSDDTAVYYCARGSGGFDYWGQGTLVTVSSGGGGSGGGGSGGGGSDIVMTQTPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 591. | CH19_0-B4xI2C6-CH-FcB-LY | QRFCTGHFGGLHPCNGGGGGSQVQLVQSGAEVKKPGASVKVSCKASGYTFTTYGISWVRQAPGQGLEWMGWINPYTGKTNYAQKLQGRVTMATDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 592. | CH19_9-F1xI2C6-CH-FcB-LY | QRFCTGHFGGLHPCNGGGGGSQVQLVQSGAEVKEPGASVKVSCKASGYTFTYYGISWVRQAPGQGLEWMGWINPYTGNRNYAQKVQDRVTMTTDTSTNTAYMELRSLRSDDTAVYYCARGSGGFDY |

| | | |
|---|---|---|
| | | WGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 593. | CH19_4-A7xI2C6-CH-FcB-LY | QRFCTGHFGGLHPCNGGGGGSQVQLVQSGAEVKKPGASVKVSCKASGYTFTYYGISWVRQAPGQGLEWMGWINPYTGKTNYAQKLQGRVTMTTDTSTSTAYMELRSLRSDDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 594. | CH19_6-E12xI2C6-CH-FcB-LY | QRFCTGHFGGLHPCNGGGGGSQVQLVQSGAEVKKPGASVKVSCKASGYTFTSYGITWVRQAPGQGLEWMGWINPYTGNRNYAQKVQGRVTMTTDTSTSTAYMELRSLRSDDTAVYYCARGSGGFDYWGQGTLVTVSSGGGGSGGGGSGGGGSDIVMTQSPLSLSVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 595. | CH19_6-C12xI2C6-CH-FcB-LY | QRFCTGHFGGLHPCNGGGGGSQVQLVQSGAEVKKPGASVKVSCKASGYTFTSYGISWVRQAPGQGLEWMGWINPYTGNRNYAQKFQGRVTMTTDTSTNTAYMELSSLRSEDTAVYYCARGSGGFDYWGQGTLVTVSSGGGGSGGGGSGGGGSDIVMTQSPLSLPVTPGEPASISCRSSQSLLHSVGYNYLDWYLQKPGQSPQLLIYLGSNRASGVPDRFSGSGSGTDFTLKISRVEAEDVGVYYCMQALQTPFTFGPGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWY |

| | | |
|---|---|---|
| | | SNRWVFGGGTKLTVLGGGGSQRFVTGHFGGLYPANGHHHHHH |
| 596. | CH19_6-A7xI2C6-CH-FcB-LY | QRFCTGHFGGLHPCNGGGGGSQVQLVQSGAEVKKPGASVKVSCKASGYSFTSYGISWVRQAPG<br>QGLEWMGWINPYTGNRNYAQKVQGRVTMTTDTSTSTAYMELRSLRSDDTAVYYCARGSGGFDY<br>WGQGTLVTVSSGGGGSGGGGSGGGGSDIVMTQSPLSLPVTPGEPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVEIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 597. | CH19_6-G8xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYTGKTNYAQKVQGRVTMTTDTSTSTAYMELRNLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 598. | CH19_6-F9xI2C6-CH-FcB-LY | QRFCTGHFGGLHPCNGGGGGSQVQLVQSGAEVKKPGASVKVSCKASGYTFTYYGISWVRQAPG<br>QGLEWMGWINPYTGKTNYAQKLQGRVTMTTDTSTSTAYMELRSLRSDDTAVYYCARGSGGFDY<br>WGQGTLVTVSSGGGGSGGGGSGGGGSDIVMTQTPLSLSVTPGQPASISCRSSQSLLHSVGYNY<br>LDWYLQKPGQSPQLLIYLGSNRASGVPDRFSGSGSGTDFTLKISRVEAEDVGVYYCMQALQTP<br>FTFGPGTKVDIKSGGGGSEVQLVESGGGLVQPGGSLKLSCAASGFTFNKYAMNWVRQAPGKGL<br>EWVARIRSKYNNYATYYADSVKDRFTISRDDSKNTAYLQMNNLKTEDTAVYYCVRHGNFGNSY<br>ISYWAYWGQGTLVTVSSGGGGSGGGGSGGGGSQTVVTQEPSLTVSPGGTVTLTCGSSTGAVTS<br>GNYPNWVQQKPGQAPRGLIGGTKFLAPGTPARFSGSLLGGKAALTLSGVQPEDEAEYYCVLWY<br>SNRWVFGGGTKLTVLGGGGSQRFVTGHFGGLYPANGHHHHHH |
| 599. | CH19_0-C11xI2C6-CH-FcB-LY | QRFCTGHFGGLHPCNGGGGGSQVQLVESGGGVVQPGRSLRLSCAASGFTFRNYAMHWVRQAPG<br>KGLEWVAVISYSGNNKYYASSVKGRFTISRDNSKNTLYLQLNSLRAEDTAVYYCAREFYYDST<br>GYDYSTPGDYWGQGTLVTVSSGGGGSGGGGSGGGGSDIQMTQSPSSLSASVGDRVTITCQASQ<br>DISNYLNWYQQKPGKAPKLLIYDASNLETGVPSRFSGSGSGTDFTFTISSLQPEDIATYYCQQ<br>YDNLPTFGQGTRLEIKSGGGGSEVQLVESGGGLVQPGGSLKLSCAASGFTFNKYAMNWVRQAP |

| | | |
|---|---|---|
| | | GKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 600. | CH19_8-F6xI2C6-CH-FcB-LY | QRFCTGHFGGLHPCNGGGGGSQVQLVESGGGVVQPGRSLRLSCVASGFTFRNYAMHWVRQAPGKGLEWVAVISYSGNNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCRASQSISSYLNWYQQKPGKAPKLLIYDASSLQSGVPSRFSGSGSGTEFTLTISSLQAEDVAVYYCQQYYSTPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 601. | CH19_0-G9xI2C6-CH-FcB-LY | QRFCTGHFGGLHPCNGGGGGSQVQLVESGGGVVQPGRSLRLSCAASGFTFRNYAMHWVRQAPGKGLEWVAGISYSGTNKYYASSVKGRFTISRDNSKNTLYLQLNSLRAEDTAVYYCAREFYYDSTGYDYSTPGDYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTEFTLTISSLQPEDIATYYCQQYDNLPLTFGGGTKVDIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 602. | CH19_1-E11xI2C6-CH-FcB-LY | QRFCTGHFGGLHPCNGGGGGSQVQLVESGGGVVQPGRSLRLSCVASGFTFRNYAMHWVRQAPGKGLEWVAVISYSGNNKYYASSVKGRFTISRDNSKNTLFLQLNSLRAEDTAVYYCAREFYYDSTGYDYSTPGNYWGQGTLVTVSSGGGGSGGGGSGGGGSDIQMTQSPSSLSASVGDRVTITCQASQDISNYLNWYQQKPGKAPKLLIYDASNLETGVPSRFSGSGSGTDFTFTISSLQPEDIATYYCQQYVNLPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 603. | CH19_0-F5xI2C6-CH-FcB-LY | QRFCTGHFGGLHPCNGGGGGSQVQLVESGGGVVQPGRSLRLSCAASGFTFSSYGMQWVRQAPGKGLEWVAVIWYSGSNKYYASSVKGRFTISRDNSKNTLYLQMNSLRAEDTAVYYCARDGYPIFY |

| | | |
|---|---|---|
| | | RYYGMDVWGQGTTVTVSSGGGGSGGGGSGGGGSDIQMTQSPSSLSASVGDRVTITCRASQGISNYLAWYQQKPGKVPKLLIYAASTLQSGVPSRFSGSGSGTDFTLTISSLQPEDVATYYCQKYNSAPLTFGGGTKVEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 604. | CH19_1-E1xI2C6-CH-FcB-LY | QRFCTGHFGGLHPCNGGGGGSQVQLVESGGGVVQPGRSLRLSCAASGFTFSSYGMHWVRQAPGKGLEWVAVISYSGSNKYYASSVKGRFTISRDNSKNTLYLQMNSLRAEDTAVYYCARDGYPIFYRYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDLAVYYCQQYGTSPLTFGGGTKLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 605. | CH19_1-E6xI2C6-CH-FcB-LY | QRFCTGHFGGLHPCNGGGGGSQVQLVESGGGVVQPGRSLRLSCAASGFTFSSFGMHWVRQAPGKGLEWVAFIWYSGSNKYYASSVKGRVTISRDNSKNTLYLQMNSLRAEDTAVYYCARDGYPIFYYYYGMDVWGQGTTVTVSSGGGGSGGGGSGGGGSEIVLTQSPGTLSLSPGERATLSCRASQSVSSSYLAWYQQKPGQAPRLLIYGASSRATGIPDRFSGSGSGTDFTLTISRLEPEDFAVYYCQQYGSSPFTFGPGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 606. | CH19_2G6_302xI2C6-LH-FcB-CH-156 | QRFVTGHFGGLHPANGGGGGSQVQLVESGGGVVQPGGSLRLSCAASGFTFSSYGMHWVRQAPGKGLEWVAFIWYDGSNKYYADSVKDRFTISRDNSKNTLYLQMNSLRAEDTAVYYCARRAGIIGTIGYYYGMDVWGQGTTVTVSSGGGGSGGGGSGGGGSSYELTQPPSVSVSPGQTASITCSGDRLGEKYTSWYQQRPGQSPLLVIYQDTKRPSGIPERFSGSNSGNTATLTISGTQAMDEADYYCQAWESSTVVFGGGTKLTVLSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCV |

| | | |
|---|---|---|
| | | LWYSNRWVFGGGTKLTVLGGGGSQRFCTGHFGGLHPCNGGGGGSGGGSRDWDFDVFGGGTPVG GHHHHHH |
| 607. | CH19_2G6_302xI2C6-LH-FcB-LH-156 | QRFVTGHFGGLHPANGGGGGSQVQLVESGGGVVQPGGSLRLSCAASGFTFSSYGMHWVRQAPG KGLEWVAFIWYDGSNKYYADSVKDRFTISRDNSKNTLYLQMNSLRAEDTAVYYCARRAGIIGT IGYYYGMDVWGQGTTVTVSSGGGGSGGGGSGGGGSSYELTQPPSVSVSPGQTASITCSGDRLG EKYTSWYQQRPGQSPLLVIYQDTKRPSGIPERFSGSNSGNTATLTISGTQAMDEADYYCQAWE SSTVVFGGGTKLTVLSGGGGSEVQLVESGGGLVQPGGSLKLSCAASGFTFNKYAMNWVRQAPG KGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGA VTSGNYPNWVQQKPGQAPRGLIGGTKFLAPGTPARFSGSLLGGKAALTLSGVQPEDEAEYYCV LWYSNRWVFGGGTKLTVLGGGGSQRFVTGHFGGLHPANGGGGGSGGGSRDWDFDVFGGGTPVG GHHHHHH |
| 608. | CH19_2G6_302xI2C6-LY-FcB-LH-156 | QRFVTGHFGGLYPANGGGGGSQVQLVESGGGVVQPGGSLRLSCAASGFTFSSYGMHWVRQAPG KGLEWVAFIWYDGSNKYYADSVKDRFTISRDNSKNTLYLQMNSLRAEDTAVYYCARRAGIIGT IGYYYGMDVWGQGTTVTVSSGGGGSGGGGSGGGGSSYELTQPPSVSVSPGQTASITCSGDRLG EKYTSWYQQRPGQSPLLVIYQDTKRPSGIPERFSGSNSGNTATLTISGTQAMDEADYYCQAWE SSTVVFGGGTKLTVLSGGGGSEVQLVESGGGLVQPGGSLKLSCAASGFTFNKYAMNWVRQAPG KGLEWVARIRSKYNNYATYYADSVKDRFTISRDDSKNTAYLQMNNLKTEDTAVYYCVRHGNFG NSYISYWAYWGQGTLVTVSSGGGGSGGGGSGGGGSQTVVTQEPSLTVSPGGTVTLTCGSSTGA VTSGNYPNWVQQKPGQAPRGLIGGTKFLAPGTPARFSGSLLGGKAALTLSGVQPEDEAEYYCV LWYSNRWVFGGGTKLTVLGGGGSQRFVTGHFGGLHPANGGGGGSGGGSRDWDFDVFGGGTPVG GHHHHHH |
| 609. | Albumin binding peptide | RDWDFDVFGGGTPVGG |
| 610. | Human CD3ε extracellular domain | QDGNEEMGGITQTPYKVSISGTTVILTCPQYPGSEILWQHNDKNIGGDEDDKNIGSDEDHLSL KEFSELEQSGYYVCYPRGSKPEDANFYLYLRARVCENCMEMD |
| 611. | Human Cadherin-19 | MNCYLLLRFMLGIPLLWPCLGATENSQTKKVKQPVRSHLRVKRGWVWNQFFVPEEMNTTSHHIGQLRSDLDNGNNS FQYKLLGAGAGSTFIIDERTGDIYAIQKLDREERSLYILRAQVIDIATGRAVEPESEFVIKVSDINDNEPKFLDEP YEAIVPEMSPEGTLVIQVTASDADDPSSGNNARLLYSLLQGQPYFSVEPTTGVIRISSKMDRELQDEYWVIIQAKD MIGQPGALSGTTSVLIKLSDVNDNKPIFKESLYRLTVSESAPTGTSIGTIMAYDNDIGENAEMDYSIEEDDSQTFD IITNHETQEGIVILKKKVDFEHQNHYGIRAKVKNHHVPEQLMKYHTEASTTFIKIQVEDVDEPPLFLLPYYVFEVF EETPQGSFVGVVSATDPDNRKSPIRYSITRSKVFNINDNGTITTSNSLDREISAWYNLSITATEKYNIEQISSIPL YVQVLNINDHAPEFSQYYETYVCENAGSGQVIQTISAVDRDESIEEHHFYFNLSVEDTNNSSFTIIDNQDNTAVIL TNRTGFNLQEEPVFYISILIADNGIPSLTSTNTLTIHVCDCGDSGSTQTCQYQELVLSMGFKTEVIIAILICIMII FGFIFLTLGLKQRRKQILFPEKSEDFRENIFQYDDEGGGEEDTEAFDIAELRSSTIMRERKTRKTTSAEIRSLYRQ |

| | | |
|---|---|---|
| | | SLQVGPDSAIFRKFILEKLEEANTDPCAPPFDSLQTYAFEGTGSLAGSLSSLESAVSDQDESYDYLNELGPRFKRLACMFGSAVQSNN |
| 612. | Cyno Cadherin-19 Macaca fascicularis | MNCYLLLPFMLGIPLLWPCLGATENSQTKKVQQPVGSHLRVKRGWVWNQFFVPEEMNTTSHHVGRLRSDLDNGNNSFQYKLLGAGAGSTFIIDERTGDIYAIEKLDREERSLYILRAQVIDITTGRAVEPESEFVIKVSDINDNEPKFLDEPYEAIVPEMSPEGTLVIQVTASDADDPSSGNNARLLYSLLQGQPYFSVEPTTGVIRISSKMDRELQDEYWVIIQAKDMIGQPGALSGTTSVLIKLSDVNDNKPIFKESLYRLTVSESAPTGTSIGTIMAYDNDIGENAEMDYSIEEDDSQTFDIITNHETQEGIVILKKKVNFEHQNHYGIRAKVKNHHVDEQLMKYHTEASTTFIKIQVEDVDEPPLFLLPYYIFEIFEETPQGSFVGVVSATDPDNRKSPIRYSITRSKVFNIDDNGTITTTNSLDREISAWYNLSITATEKYNIEQISSIPVYVQVLNINDHAPEFSQYYESYVCENAGSGQVIQTISAVDRDESIEEHHFYFNLSVEDTNSSSFTIIDNQDNTAVILTNRTGFNLQEEPIFYISILIADNGIPSLTSTNTLTIHVCDCDDSGSTQTCQYQELMLSMGFKTEVIIAILICIMVIFGFIFLTLGLKQRRKQILFPEKSEDFRENIFRYDDEGGGEEDTEAFDVAALRSSTIMRERKTRKTTSAEIRSLYRQSLQVGPDSAIFRKFILEKLEEADTDPCAPPFDSLQTYAFEGTGSLAGSLSSLESAVSDQDESYDYLNELGPRFKRLACMFGSAVQSNN |

| SEQ ID NO | Designation | Format | Sequence |
|---|---|---|---|
| 613. | CD33_E11xI2C6-LFcBY | VH CDR1 | NYGMN |
| 614. | CD33_E11xI2C6-LFcBY | VH CDR2 | WINTYTGEPTYADKFQG |
| 615. | CD33_E11xI2C6-LFcBY | VH CDR3 | WSWSDGYYVYFDY |
| 616. | CD33_E11xI2C6-LFcBY | VL CDR1 | KSSQSVLDSSTNKNSLA |
| 617. | CD33_E11xI2C6-LFcBY | VL CDR2 | WASTRES |
| 618. | CD33_E11xI2C6-LFcBY | VL CDR3 | QQSAHFPIT |
| 619. | CD33_E11xI2C6-LFcBY | VH | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSS |
| 620. | CD33_E11xI2C6-LFcBY | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |

| | | | |
|---|---|---|---|
| 621. | CD33_E11xI2C6-LFcBY | scFv | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |
| 622. | CD33_E11xI2C6-LFcBY | bispecific molecule | QRFVTGHFGGLYPANGGGGSQVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 623. | CD33_E11_CCxI2C6-_LFcBY | VH CDR1 | NYGMN |
| 624. | CD33_E11_CCxI2C6-_LFcBY | VH CDR2 | WINTYTGEPTYADKFQG |
| 625. | CD33_E11_CCxI2C6-_LFcBY | VH CDR3 | WSWSDGYYVYFDY |
| 626. | CD33_E11_CCxI2C6-_LFcBY | VL CDR1 | KSSQSVLDSSTNKNSLA |
| 627. | CD33_E11_CCxI2C6-_LFcBY | VL CDR2 | WASTRES |
| 628. | CD33_E11_CCxI2C6-_LFcBY | VL CDR3 | QQSAHFPIT |
| 629. | CD33_E11_CCxI2C6-_LFcBY | VH | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSS |
| 630. | CD33_E11_CCxI2C6-_LFcBY | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |

| | | | |
|---|---|---|---|
| 631. | CD33_E11_CCxI2C6-_LFcBY | scFv | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |
| 632. | CD33_E11_CCxI2C6-_LFcBY | bispecific molecule | QRFVTGHFGGLYPANGGGGSQVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGHHHHHH |
| 633. | CD33_E11xI2C6-CH-FcB-LY | VH CDR1 | NYGMN |
| 634. | CD33_E11xI2C6-CH-FcB-LY | VH CDR2 | WINTYTGEPTYADKFQG |
| 635. | CD33_E11xI2C6-CH-FcB-LY | VH CDR3 | WSWSDGYYVYFDY |
| 636. | CD33_E11xI2C6-CH-FcB-LY | VL CDR1 | KSSQSVLDSSTNKNSLA |
| 637. | CD33_E11xI2C6-CH-FcB-LY | VL CDR2 | WASTRES |
| 638. | CD33_E11xI2C6-CH-FcB-LY | VL CDR3 | QQSAHFPIT |
| 639. | CD33_E11xI2C6-CH-FcB-LY | VH | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSS |
| 640. | CD33_E11xI2C6-CH-FcB-LY | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |

| | | | |
|---|---|---|---|
| 641. | CD33_E11xI2C6-CH-FcB-LY | scFv | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |
| 642. | CD33_E11xI2C6-CH-FcB-LY | bispecific molecule | QRFCTGHFGGLHPCNGGGGGSQVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 643. | CD33_E11_CCxI2C6-CH-FcB-LY | VH CDR1 | NYGMN |
| 644. | CD33_E11_CCxI2C6-CH-FcB-LY | VH CDR2 | WINTYTGEPTYADKFQG |
| 645. | CD33_E11_CCxI2C6-CH-FcB-LY | VH CDR3 | WSWSDGYYVYFDY |
| 646. | CD33_E11_CCxI2C6-CH-FcB-LY | VL CDR1 | KSSQSVLDSSTNKNSLA |
| 647. | CD33_E11_CCxI2C6-CH-FcB-LY | VL CDR2 | WASTRES |
| 648. | CD33_E11_CCxI2C6-CH-FcB-LY | VL CDR3 | QQSAHFPIT |
| 649. | CD33_E11_CCxI2C6-CH-FcB-LY | VH | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSS |
| 650. | CD33_E11_CCxI2C6-CH-FcB-LY | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |

| | | | |
|---|---|---|---|
| 651. | CD33_E11_CCxI2C6-CH-FcB-LY | scFv | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |
| 652. | CD33_E11_CCxI2C6-CH-FcB-LY | bispecific molecule | QRFCTGHFGGLHPCNGGGGGSQVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGHHHHHH |
| 653. | CD33_E11xI2C6-LFcBY-156 | VH CDR1 | NYGMN |
| 654. | CD33_E11xI2C6-LFcBY-156 | VH CDR2 | WINTYTGEPTYADKFQG |
| 655. | CD33_E11xI2C6-LFcBY-156 | VH CDR3 | WSWSDGYYVYFDY |
| 656. | CD33_E11xI2C6-LFcBY-156 | VL CDR1 | KSSQSVLDSSTNKNSLA |
| 657. | CD33_E11xI2C6-LFcBY-156 | VL CDR2 | WASTRES |
| 658. | CD33_E11xI2C6-LFcBY-156 | VL CDR3 | QQSAHFPIT |
| 659. | CD33_E11xI2C6-LFcBY-156 | VH | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSS |
| 660. | CD33_E11xI2C6-LFcBY-156 | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |

| | | | |
|---|---|---|---|
| 661. | CD33_E11xI2C6-LFcBY-156 | scFv | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |
| 662. | CD33_E11xI2C6-LFcBY-156 | bispecific molecule | QRFVTGHFGGLYPANGGGGGSQVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 663. | CD33_E11_CCxI2C6-LFcBY-156 | VH CDR1 | NYGMN |
| 664. | CD33_E11_CCxI2C6-LFcBY-156 | VH CDR2 | WINTYTGEPTYADKFQG |
| 665. | CD33_E11_CCxI2C6-LFcBY-156 | VH CDR3 | WSWSDGYYVYFDY |
| 666. | CD33_E11_CCxI2C6-LFcBY-156 | VL CDR1 | KSSQSVLDSSTNKNSLA |
| 667. | CD33_E11_CCxI2C6-LFcBY-156 | VL CDR2 | WASTRES |
| 668. | CD33_E11_CCxI2C6-LFcBY-156 | VL CDR3 | QQSAHFPIT |
| 669. | CD33_E11_CCxI2C6-LFcBY-156 | VH | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSS |
| 670. | CD33_E11_CCxI2C6-LFcBY-156 | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |

| | | | |
|---|---|---|---|
| 671. | CD33_E11_CCxI2C6-LFcBY-156 | scFv | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |
| 672. | CD33_E11_CCxI2C6-LFcBY-156 | bispecific molecule | QRFVTGHFGGLYPANGGGGGSQVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFCTGHFGGLHPCNGGGGGSGGGSRDWDFDVFGGGTPVGGHHHHHH |
| 673. | CD33_E11xI2C6-CH-FcB-LY-156 | VH CDR1 | NYGMN |
| 674. | CD33_E11xI2C6-CH-FcB-LY-156 | VH CDR2 | WINTYTGEPTYADKFQG |
| 675. | CD33_E11xI2C6-CH-FcB-LY-156 | VH CDR3 | WSWSDGYYVYFDY |
| 676. | CD33_E11xI2C6-CH-FcB-LY-156 | VL CDR1 | KSSQSVLDSSTNKNSLA |
| 677. | CD33_E11xI2C6-CH-FcB-LY-156 | VL CDR2 | WASTRES |
| 678. | CD33_E11xI2C6-CH-FcB-LY-156 | VL CDR3 | QQSAHFPIT |
| 679. | CD33_E11xI2C6-CH-FcB-LY-156 | VH | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSS |
| 680. | CD33_E11xI2C6-CH-FcB-LY-156 | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |

| | | | |
|---|---|---|---|
| 681. | CD33_E11xI2C6-CH-FcB-LY-156 | scFv | QVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIK |
| 682. | CD33_E11xI2C6-CH-FcB-LY-156 | bispecific molecule | QRFCTGHFGGLHPCNGGGGGSQVQLVQSGAEVKKPGESVKVSCKASGYTFTNYGMNWVKQAPGQGLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGQGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGGGGGSGGGSRDWDFDVFGGGTPVGGHHHHHH |
| 683. | CD33_E11_CCxI2C6-CH-FcB-LY-156 | VH CDR1 | NYGMN |
| 684. | CD33_E11_CCxI2C6-CH-FcB-LY-156 | VH CDR2 | WINTYTGEPTYADKFQG |
| 685. | CD33_E11_CCxI2C6-CH-FcB-LY-156 | VH CDR3 | WSWSDGYYVYFDY |
| 686. | CD33_E11_CCxI2C6-CH-FcB-LY-156 | VL CDR1 | KSSQSVLDSSTNKNSLA |
| 687. | CD33_E11_CCxI2C6-CH-FcB-LY-156 | VL CDR2 | WASTRES |
| 688. | CD33_E11_CCxI2C6-CH-FcB-LY-156 | VL CDR3 | QQSAHFPIT |
| 689. | CD33_E11_CCxI2C6-CH-FcB-LY-156 | VH | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSS |
| 690. | CD33_E11_CCxI2C6-CH-FcB-LY-156 | VL | DIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |

| | | | |
|---|---|---|---|
| 691. | CD33_E11_CCxI2C6-CH-FcB-LY-156 | scFv | QVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIK |
| 692. | CD33_E11_CCxI2C6-CH-FcB-LY-156 | bispecific molecule | QRFCTGHFGGLHPCNGGGGGSQVQLVQSGAEVKKPGESVKVSCKASGYTFTNYGMNWVKQAPGQCLEWMGWINTYTGEPTYADKFQGRVTMTTDTSTSTAYMEIRNLGGDDTAVYYCARWSWSDGYYVYFDYWGQGTSVTVSSGGGGSGGGGSGGGGSDIVMTQSPDSLTVSLGERTTINCKSSQSVLDSSTNKNSLAWYQQKPGQPPKLLLSWASTRESGIPDRFSGSGSGTDFTLTIDSPQPEDSATYYCQQSAHFPITFGCGTRLEIKSGGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVLGGGGSQRFVTGHFGGLYPANGGGGGSGGGSRDWDFDVFGGGTPVGGHHHHHH |

## Claims

1. A bispecific single chain antibody construct binding to a target cell surface antigen via a first binding domain and to the T cell surface antigen CD3 via a second binding domain, the construct comprising two FcRn binding peptides, wherein:

   (a) a first FcRn binding peptide consists of the amino acid sequence **QRFVTGHFGGL$X_1$PANG** (SEQ ID NO: 1) whereas $X_1$ is Y or H; and
   (b) a second FcRn binding peptide consists of the amino acid sequence **TGHFGGLHP** (SEQ ID NO: 4);

   wherein the bispecific single chain antibody construct does not have an amino acid sequence as depicted in SEQ ID NOs: **132-135.**

2. A bispecific single chain antibody construct binding to a target cell surface antigen via a first binding domain and to the T cell surface antigen CD3 via a second binding domain, the construct comprising two FcRn binding peptides, wherein:

   (a) a first FcRn binding peptide consists of the amino acid sequence **QRFVTGHFGGL$X_1$PANG** (SEQ ID NO: 1) whereas $X_1$ is Y or H; and
   (b) a second FcRn binding peptide consists of the amino acid sequence **QRFVTGHFGGLHPANG** (SEQ ID NO: 3) or **QRFCTGHFGGLHPCNG** (SEQ ID NO: 5).

3. A bispecific single chain antibody construct binding to a target cell surface antigen via a first binding domain and to the T cell surface antigen CD3 via a second binding domain, the construct comprising an FcRn binding peptide at the N-terminus and an FcRn binding peptide at the C-terminus, wherein:

   (a) the FcRn binding peptide at the N-terminus consists of the amino acid sequence **QRFVTGHFGGLYPANG** (SEQ ID NO: 2) and at the C-terminus consists of the amino acid sequence **QRFCTGHFGGLHPCNG** (SEQ ID NO: 5);
   (b) the FcRn binding peptide at the N-terminus consists of the amino acid sequence **QRFVTGHFGGLYPANG** (SEQ ID NO: 2) and at the C-terminus consists of the amino acid sequence **QRFVTGHFGGLHPANG** (SEQ ID NO: 3);
   (c) the FcRn binding peptide at the N-terminus consists of the amino acid sequence **QRFVTGHFGGLHPANG** (SEQ ID NO: 3) and at the C-terminus consists of the amino acid sequence **QRFCTGHFGGLHPCNG** (SEQ ID NO: 5);
   (d) the FcRn binding peptide at the N-terminus consists of the amino acid sequence **QRFVTGHFGGLHPANG** (SEQ ID NO: 3) and at the C-terminus consists of the amino acid sequence **QRFVTGHFGGLHPANG** (SEQ ID NO: 3);
   (e) the FcRn binding peptide at the N-terminus consists of the amino acid sequence **QRFCTGHFGGLHPCNG** (SEQ ID NO: 5) and at the C-terminus consists of the amino acid sequence **QRFVTGHFGGLYPANG** (SEQ ID NO: 2); and
   (f) the FcRn binding peptide at the N-terminus consists of the amino acid sequence **QRFCTGHFGGLHPCNG** (SEQ ID NO: 5) and at the C-terminus consists of the amino acid sequence **QRFVTGHFGGLHPANG** (SEQ ID NO: 3).

4. The antibody construct according to any one of the preceding claims, wherein the FcRn binding peptides are linked to the antibody construct via one or more peptide linker(s).

5. The antibody construct according to claim 4, wherein the peptide linker has the amino acid sequence $(GGGGS)_n$ (SEQ ID NO: $6)_n$ wherein n is an integer in the range of 1 to 5.

6. The antibody construct according to any one of the preceding claims, wherein the construct comprises a further domain binding to serum albumin.

7. The antibody construct according to any one of the preceding claims, wherein the target cell surface antigen is a tumor antigen.

8. The antibody construct according to claim 7, wherein the tumor antigen is selected from the group of consisting of CDH19, mesothelin (MSLN), DLL3, FLT3, CD33, CD20 and EGFRvIII.

**9.** The antibody construct according to any one of the preceding claims, wherein the second binding domain binds to an epitope of human and *Callithrix jacchus, Saguinus oedipus* or *Saimiri sciureus* CD3ε chain, wherein the epitope is part of an amino acid sequence comprised in the group consisting of SEQ ID NOs: 7, 8, 9, and 10 and comprises at least the amino acid sequence Gln-Asp-Gly-Asn-Glu (SEQ ID NO: 11).

**10.** The antibody construct according to claim 9, wherein the second binding domain comprises a VL region having CDR-L1-L3 and a VH region having CDR-H1-H3 selected from the group consisting of:

(a) CDR-L1-L3 as depicted in SEQ ID NOs: 12-14 and CDR-H1-H3 as depicted in SEQ ID NOs: 15-17;
(b) CDR-L1-L3 as depicted in SEQ ID NOs: 24-26 and CDR-H1-H3 as depicted in SEQ ID NOs: 27-29;
(c) CDR-L1-L3 as depicted in SEQ ID NOs: 36-38 and CDR-H1-H3 as depicted in SEQ ID NOs: 39-41;
(d) CDR-L1-L3 as depicted in SEQ ID NOs: 48-50 and CDR-H1-H3 as depicted in SEQ ID NOs: 51-53;
(e) CDR-L1-L3 as depicted in SEQ ID NOs: 60-62 and CDR-H1-H3 as depicted in SEQ ID NOs: 63-65;
(f) CDR-L1-L3 as depicted in SEQ ID NOs: 72-74 and CDR-H1-H3 as depicted in SEQ ID NOs: 75-77;
(g) CDR-L1-L3 as depicted in SEQ ID NOs: 84-86 and CDR-H1-H3 as depicted in SEQ ID NOs: 87-89;
(h) CDR-L1-L3 as depicted in SEQ ID NOs: 96-98 and CDR-H1-H3 as depicted in SEQ ID NOs: 99-101;
(i) CDR-L1-L3 as depicted in SEQ ID NOs: 108-110 and CDR-H1-H3 as depicted in SEQ ID NOs: 111-113;
(j) CDR-L1-L3 as depicted in SEQ ID NOs: 120-122 and CDR-H1-H3 as depicted in SEQ ID NOs: 123-125;
(k) CDR-L1-L3 as depicted in SEQ ID NOs: 616-618 and CDR-H1-H3 as depicted in SEQ ID NOs: 613-615;
(l) CDR-L1-L3 as depicted in SEQ ID NOs: 626-628 and CDR-H1-H3 as depicted in SEQ ID NOs: 623-625;
(m) CDR-L1-L3 as depicted in SEQ ID NOs: 636-638 and CDR-H1-H3 as depicted in SEQ ID NOs: 633-635;
(n) CDR-L1-L3 as depicted in SEQ ID NOs: 646-648 and CDR-H1-H3 as depicted in SEQ ID NOs: 643-645;
(o) CDR-L1-L3 as depicted in SEQ ID NOs: 656-658 and CDR-H1-H3 as depicted in SEQ ID NOs: 653-655;
(p) CDR-L1-L3 as depicted in SEQ ID NOs: 666-668 and CDR-H1-H3 as depicted in SEQ ID NOs: 663-665;
(q) CDR-L1-L3 as depicted in SEQ ID NOs: 676-678 and CDR-H1-H3 as depicted in SEQ ID NOs: 673-675; and
(r) CDR-L1-L3 as depicted in SEQ ID NOs: 686-688 and CDR-H1-H3 as depicted in SEQ ID NOs: 683-685.

**11.** The antibody construct according to claim 9 or 10, wherein the second binding domain comprises pairs of VH and VL chains selected from the group consisting of:

(a) a VH-chain as depicted in SEQ ID NO: 18 and a VL-chain as depicted in SEQ ID NO: 20;
(b) a VH-chain as depicted in SEQ ID NO: 30 and a VL-chain as depicted in SEQ ID NO: 32;
(c) a VH-chain as depicted in SEQ ID NO: 42 and a VL-chain as depicted in SEQ ID NO: 44;
(d) a VH-chain as depicted in SEQ ID NO: 54 and a VL-chain as depicted in SEQ ID NO: 56;
(e) a VH-chain as depicted in SEQ ID NO: 66 and a VL-chain as depicted in SEQ ID NO: 68;
(f) a VH-chain as depicted in SEQ ID NO: 78 and a VL-chain as depicted in SEQ ID NO: 80;
(g) a VH-chain as depicted in SEQ ID NO: 90 and a VL-chain as depicted in SEQ ID NO: 92;
(h) a VH-chain as depicted in SEQ ID NO: 102 and a VL-chain as depicted in SEQ ID NO: 104;
(i) a VH-chain as depicted in SEQ ID NO: 114 and a VL-chain as depicted in SEQ ID NO: 116;
(j) a VH-chain as depicted in SEQ ID NO: 126 and a VL-chain as depicted in SEQ ID NO: 128;
(k) a VH-chain as depicted in SEQ ID NO: 619 and a VL-chain as depicted in SEQ ID NO: 620;
(l) a VH-chain as depicted in SEQ ID NO: 629 and a VL-chain as depicted in SEQ ID NO: 630;
(m) a VH-chain as depicted in SEQ ID NO: 639 and a VL-chain as depicted in SEQ ID NO: 640;
(n) a VH-chain as depicted in SEQ ID NO: 649 and a VL-chain as depicted in SEQ ID NO: 650;
(o) a VH-chain as depicted in SEQ ID NO: 659 and a VL-chain as depicted in SEQ ID NO: 660;
(p) a VH-chain as depicted in SEQ ID NO: 669 and a VL-chain as depicted in SEQ ID NO: 670;
(q) a VH-chain as depicted in SEQ ID NO: 679 and a VL-chain as depicted in SEQ ID NO: 680; and
(r) a VH-chain as depicted in SEQ ID NO: 689 and a VL-chain as depicted in SEQ ID NO: 690.

**12.** The antibody construct according to any of claims 9 to 11, wherein the second binding domain comprises an amino acid sequence as depicted in SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 621, SEQ ID NO: 631, SEQ ID NO: 641, SEQ ID NO: 651, SEQ ID NO: 661, SEQ ID NO: 671, SEQ ID NO: 681, or SEQ ID NO: 691.

**13.** The antibody construct according to any of claims 1 and 4-12, wherein the domains are arranged from the N-terminus to the C-terminus a follows:

(FcRn binding peptide consisting of SEQ ID NO:1, whereas $X_1$ is Y or H)-(VH chain of the first binding domain) - (VL chain of the first binding domain)-(VH chain of the second binding domain) - (VL chain of the second binding domain)-(FcRn binding peptide consisting of SEQ ID NO: 4)
or
(FcRn binding peptide consisting of SEQ ID NO:4)-(VH chain of the first binding domain) - (VL chain of the first binding domain)-(VH chain of the second binding domain) - (VL chain of the second binding domain) - (FcRn binding peptide consisting of SEQ ID NO: 1, whereas $X_1$ is Y or H)

**14.** A polynucleotide encoding an antibody construct as defined in any one of claims 1 to 13.

**15.** A vector comprising a polynucleotide as defined in claim 14.

**16.** A host cell transformed or transfected with the polynucleotide as defined in claim 14 or with the vector as defined in claim 15.

**17.** A process for the production of an antibody construct according to any one of claims 1 to 13, said process comprising culturing a host cell as defined in claim 16 under conditions allowing the expression of the antibody construct as defined in any one of claims 1 to 13 and recovering the produced antibody construct from the culture.

**18.** A pharmaceutical composition comprising an antibody construct according to any one of claims 1 to 13, or produced according to the process of claim 17.

**19.** The antibody construct according to any one of claims 1 to 13, or produced according to the process of claim 17, for use in the prevention, treatment or amelioration of a disease selected from a proliferative disease, a tumorous disease, a viral disease or an immunological disorder.

**20.** A kit comprising an antibody construct according to any one of claims 1 to 13, or produced according to the process of claim 17, a vector as defined in claim 15, and/or a host cell as defined in claim 16.

**Patentansprüche**

**1.** Bispezifisches Einzelketten-Antikörperkonstrukt, das über eine erste Bindungsdomäne an ein Zielzelloberflächenantigen und über eine zweite Bindungsdomäne an das T-Zelloberflächenantigen CD3 bindet, wobei das Konstrukt zwei FcRn-Bindungspeptide umfasst, wobei:

(a) ein erstes FcRn-Bindungspeptid aus der Aminosäuresequenz **QRFVTGHFGGL$X_1$PANG** (SEQ ID NO: 1) besteht, wobei $X_1$ Y oder H ist; und
(b) ein zweites FcRn-Bindungspeptid aus der Aminosäuresequenz **TGHFGGLHP** (SEQ ID NO: 4) besteht;
wobei das bispezifische Einzelketten-Antikörperkonstrukt keine Aminosäuresequenz aufweist wie in SEQ ID NO: **132-135** dargestellt.

**2.** Bispezifisches Einzelketten-Antikörperkonstrukt, das über eine erste Bindungsdomäne an ein Zielzelloberflächenantigen und über eine zweite Bindungsdomäne an das T-Zelloberflächenantigen CD3 bindet, wobei das Konstrukt zwei FcRn-Bindungspeptide umfasst, wobei:

(a) ein erstes FcRn-Bindungspeptid aus der Aminosäuresequenz **QRFVTGHFGGL$X_1$PANG** (SEQ ID NO: 1) besteht, wobei $X_1$ Y oder H ist; und
(b) ein zweites FcRn-Bindungspeptid aus der Aminosäuresequenz **QRFVTGHFGGLHPANG** (SEQ ID NO: 3) oder **QRFCTGHFGGLHPCNG** (SEQ ID NO: 5) besteht.

**3.** Bispezifisches Einzelketten-Antikörperkonstrukt, das über eine erste Bindungsdomäne an ein Zielzelloberflächenantigen und über eine zweite Bindungsdomäne an das T-Zelloberflächenantigen CD3 bindet, wobei das Konstrukt ein FcRn-Bindungspeptid am N-Terminus und ein FcRn-Bindungspeptid am C-Terminus umfasst, wobei:

(a) das FcRn-Bindungspeptid am N-Terminus aus der Aminosäuresequenz **QRFVTGHFGGLYPANG** (SEQ ID NO: 2) und am C-Terminus aus der Aminosäuresequenz **QRFCTGHFGGLHPCNG** (SEQ ID NO: 5) besteht;
(b) das FcRn-Bindungspeptid am N-Terminus aus der Aminosäuresequenz **QRFVTGHFGGLYPANG** (SEQ ID NO: 2) und am C-Terminus besteht aus der Aminosäuresequenz **QRFVTGHFGGLHPANG** (SEQ ID NO: 3) besteht;
(c) das FcRn-Bindungspeptid am N-Terminus aus der Aminosäuresequenz **QRFVTGHFGGLHPANG** (SEQ ID NO: 3) und am C-Terminus besteht aus der Aminosäuresequenz **QRFCTGHFGGLHPCNG** (SEQ ID NO: 5) besteht;
(d) das FcRn-Bindungspeptid am N-Terminus aus der Aminosäuresequenz **QRFVTGHFGGLHPANG** (SEQ ID NO: 3) und am C-Terminus besteht aus der Aminosäuresequenz **QRFVTGHFGGLHPANG** (SEQ ID NO: 3) besteht;
(e) das FcRn-Bindungspeptid am N-Terminus aus der Aminosäuresequenz **QRFCTGHFGGLHPCNG** (SEQ ID NO: 5) und am C-Terminus besteht aus der Aminosäuresequenz **QRFVTGHFGGLYPANG** (SEQ ID NO: 2) besteht; und
(f) das FcRn-Bindungspeptid am N-Terminus aus der Aminosäuresequenz **QRFCTGHFGGLHPCNG** (SEQ ID NO: 5) und am C-Terminus aus der Aminosäuresequenz **QRFVTGHFGGLHPANG** (SEQ ID NO: 3) besteht.

**4.** Antikörperkonstrukt nach einem der vorhergehenden Ansprüche, wobei die FcRn-Bindungspeptide über einen oder mehrere Peptidlinker mit dem Antikörperkonstrukt verknüpft sind.

**5.** Antikörperkonstrukt nach Anspruch 4, wobei der Peptidlinker die Aminosäuresequenz $(GGGGS)_n$ (SEQ ID NO: $6)_n$ aufweist, wobei n eine ganze Zahl im Bereich von 1 bis 5 ist.

**6.** Antikörperkonstrukt nach einem der vorhergehenden Ansprüche, wobei das Konstrukt eine weitere Domäne umfasst, die an Serumalbumin bindet.

**7.** Antikörperkonstrukt nach einem der vorhergehenden Ansprüche, wobei das Zielzelloberflächenantigen ein Tumorantigen ist.

**8.** Antikörperkonstrukt nach Anspruch 7, wobei das Tumorantigen ausgewählt ist aus der Gruppe bestehend aus CDH19, Mesothelin (MSLN), DLL3, FLT3, CD33, CD20 und EGFRvIII.

**9.** Antikörperkonstrukt nach einem der vorhergehenden Ansprüche, wobei die zweite Bindungsdomäne an ein Epitop der CD3ε-Kette von Mensch und *Callithrix jacchus, Saguinus oedipus* oder *Saimiri sciureus* bindet, wobei das Epitop Teil einer Aminosäuresequenz ist, die enthalten ist in der Gruppe bestehend aus SEQ ID NO: 7, 8, 9 und 10 und mindestens die Aminosäuresequenz Gin-Asp-Gly-Asn-Glu (SEQ ID NO: 11) umfasst.

**10.** Antikörperkonstrukt nach Anspruch 9, wobei die zweite Bindungsdomäne eine VL-Region mit CDR-L1-L3 und eine VH-Region mit CDR-H1-H3 umfasst, ausgewählt aus der Gruppe bestehend aus:

(a) CDR-L1-L3, wie dargestellt in SEQ ID NO: 12-14 sowie CDR-H1-H3, wie dargestellt in SEQ ID NO: 15-17;
(b) CDR-L1-L3, wie dargestellt in SEQ ID NO: 24-26 sowie CDR-H1-H3, wie dargestellt in SEQ ID NO: 27-29;
(c) CDR-L1-L3, wie dargestellt in SEQ ID NO: 36-38 sowie CDR-H1-H3, wie dargestellt in SEQ ID NO: 39-41;
(d) CDR-L1-L3, wie dargestellt in SEQ ID NO: 48-50 sowie CDR-H1-H3, wie dargestellt in SEQ ID NO: 51-53;
(e) CDR-L1-L3, wie dargestellt in SEQ ID NO: 60-62 sowie CDR-H1-H3, wie dargestellt in SEQ ID NO: 63-65;
(f) CDR-L1-L3, wie dargestellt in SEQ ID NO: 72-74 sowie CDR-H1-H3, wie dargestellt in SEQ ID NO: 75-77;
(g) CDR-L1-L3, wie dargestellt in SEQ ID NO: 84-86 sowie CDR-H1-H3, wie dargestellt in SEQ ID NO: 87-89;
(h) CDR-L1-L3, wie dargestellt in SEQ ID NO: 96-98 sowie CDR-H1-H3, wie dargestellt in SEQ ID NO: 99-101;
(i) CDR-L1-L3, wie dargestellt in SEQ ID NO: 108-110 sowie CDR-H1-H3, wie dargestellt in SEQ ID NO: 111-113;
(j) CDR-L1-L3, wie dargestellt in SEQ ID NO: 120-122 sowie CDR-H1-H3, wie dargestellt in SEQ ID NO: 123-125;
(k) CDR-L1-L3, wie dargestellt in SEQ ID NO: 616-618 sowie CDR-H1-H3, wie dargestellt in SEQ ID NO: 613-615;
(l) CDR-L1-L3, wie dargestellt in SEQ ID NO: 626-628 sowie CDR-H1-H3, wie dargestellt in SEQ ID NO: 623-625;
(m) CDR-L1-L3, wie dargestellt in SEQ ID NO: 636-638 sowie CDR-H1-H3, wie dargestellt in SEQ ID NO: 633-635;
(n) CDR-L1-L3, wie dargestellt in SEQ ID NO: 646-648 sowie CDR-H1-H3, wie dargestellt in SEQ ID NO: 643-645;
(o) CDR-L1-L3, wie dargestellt in SEQ ID NO: 656-658 sowie CDR-H1-H3, wie dargestellt in SEQ ID NO: 653-655;
(p) CDR-L1-L3, wie dargestellt in SEQ ID NO: 666-668 sowie CDR-H1-H3, wie dargestellt in SEQ ID NO: 663-665;

(q) CDR-L1-L3, wie dargestellt in SEQ ID NO: 676-678 sowie CDR-H1-H3, wie dargestellt in SEQ ID NO: 673-675; und
(r) CDR-L1-L3, wie dargestellt in SEQ ID NO: 686-688 sowie CDR-H1-H3, wie dargestellt in SEQ ID NO: 683-685.

**11.** Antikörperkonstrukt nach Anspruch 9 oder 10, wobei die zweite Bindungsdomäne Paare von VH- und VL-Ketten umfasst, ausgewählt aus der Gruppe bestehend aus:

(a) einer VH-Kette wie dargestellt in SEQ ID NO: 18 und einer VL-Kette wie dargestellt in SEQ ID NO: 20;
(b) einer VH-Kette wie dargestellt in SEQ ID NO: 30 und einer VL-Kette wie dargestellt in SEQ ID NO: 32;
(c) einer VH-Kette wie dargestellt in SEQ ID NO: 42 und einer VL-Kette wie dargestellt in SEQ ID NO: 44;
(d) einer VH-Kette wie dargestellt in SEQ ID NO: 54 und einer VL-Kette wie dargestellt in SEQ ID NO: 56;
(e) einer VH-Kette wie dargestellt in SEQ ID NO: 66 und einer VL-Kette wie dargestellt in SEQ ID NO: 68;
(f) einer VH-Kette wie dargestellt in SEQ ID NO: 78 und einer VL-Kette wie dargestellt in SEQ ID NO: 80;
(g) einer VH-Kette wie dargestellt in SEQ ID NO: 90 und einer VL-Kette wie dargestellt in SEQ ID NO: 92;
(h) einer VH-Kette wie dargestellt in SEQ ID NO: 102 und einer VL-Kette wie dargestellt in SEQ ID NO: 104;
(i) einer VH-Kette wie dargestellt in SEQ ID NO: 114 und einer VL-Kette wie dargestellt in SEQ ID NO: 116;
(j) einer VH-Kette wie dargestellt in SEQ ID NO: 126 und einer VL-Kette wie dargestellt in SEQ ID NO: 128;
(k) einer VH-Kette wie dargestellt in SEQ ID NO: 619 und einer VL-Kette wie dargestellt in SEQ ID NO: 620;
(l) einer VH-Kette wie dargestellt in SEQ ID NO: 629 und einer VL-Kette wie dargestellt in SEQ ID NO: 630;
(m) einer VH-Kette wie dargestellt in SEQ ID NO: 639 und einer VL-Kette wie dargestellt in SEQ ID NO: 640;
(n) einer VH-Kette wie dargestellt in SEQ ID NO: 649 und einer VL-Kette wie dargestellt in SEQ ID NO: 650;
(o) einer VH-Kette wie dargestellt in SEQ ID NO: 659 und einer VL-Kette wie dargestellt in SEQ ID NO: 660;
(p) einer VH-Kette wie dargestellt in SEQ ID NO: 669 und einer VL-Kette wie dargestellt in SEQ ID NO: 670;
(q) einer VH-Kette wie dargestellt in SEQ ID NO: 679 und einer VL-Kette wie dargestellt in SEQ ID NO: 680; und
(r) einer VH-Kette wie dargestellt in SEQ ID NO: 689 und einer VL-Kette wie dargestellt in SEQ ID NO: 690.

**12.** Antikörperkonstrukt nach einem der Ansprüche 9 bis 11, wobei die zweite Bindungsdomäne eine Aminosäuresequenz umfasst, wie dargestellt in SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 621, SEQ ID NO: 631, SEQ ID NO: 641, SEQ ID NO: 651, SEQ ID NO: 661, SEQ ID NO: 671, SEQ ID NO: 681 oder SEQ ID NO: 691.

**13.** Antikörperkonstrukt nach einem der Ansprüche 1 und 4-12, wobei die Domänen vom N-Terminus zum C-Terminus wie folgt angeordnet sind:

(FcRn-Bindungspeptid, bestehend aus SEQ ID NO: 1, wobei $X_1$ Y oder H ist) - (VH-Kette der ersten Bindungsdomäne) - (VL-Kette der ersten Bindungsdomäne) - (VH-Kette der zweiten Bindungsdomäne) - (VL-Kette der zweiten Bindungsdomäne) - (FcRn-Bindungspeptid, bestehend aus SEQ ID NO: 4)
oder
(FcRn-Bindungspeptid, bestehend aus SEQ ID NO: 4) - (VH-Kette der ersten Bindungsdomäne) - (VL-Kette der ersten Bindungsdomäne) - (VH-Kette der zweiten Bindungsdomäne) - (VL-Kette der zweiten Bindungsdomäne) - (FcRn-Bindungspeptid, bestehend aus SEQ ID NO: 1, wobei $X_1$ Y oder H ist) .

**14.** Polynukleotid, das für ein Antikörperkonstrukt kodiert, wie definiert in einem der Ansprüche 1 bis 13.

**15.** Vektor, umfassend ein Polynukleotid, wie definiert in Anspruch 14.

**16.** Wirtszelle, transformiert oder transfiziert mit dem Polynukleotid nach Anspruch 14 oder mit dem Vektor nach Anspruch 15.

**17.** Verfahren zum Erzeugen eines Antikörperkonstrukts nach einem der Ansprüche 1 bis 13, wobei das Verfahren das Kultivieren einer Wirtszelle nach Anspruch 16 unter Bedingungen umfasst, die die Expression des Antikörperkonstrukts nach einem der Ansprüche 1 bis 13 und das Wiedergewinnen des erzeugten Antikörperkonstrukts aus der Kultur ermöglichen.

**18.** Pharmazeutische Zusammensetzung, umfassend ein Antikörperkonstrukt nach einem der Ansprüche 1 bis 13 oder erzeugt nach dem Verfahren nach Anspruch 17.

**19.** Antikörperkonstrukt nach einem der Ansprüche 1 bis 13 oder erzeugt nach dem Verfahren nach Anspruch 17, vorzugsweise zur Verwendung bei der Prävention, Behandlung oder Linderung einer Erkrankung, ausgewählt aus einer proliferativen Erkrankung, einer Tumorerkrankung, einer viralen Erkrankung oder einer immunologischen Störung.

**20.** Kit, umfassend ein Antikörperkonstrukt nach einem der Ansprüche 1 bis 13, oder erzeugt nach dem Verfahren nach Anspruch 17, einen wie in Anspruch 15 definierten Vektor und/oder eine wie in Anspruch 16 definierte Wirtszelle.

## Revendications

**1.** Construction d'anticorps monocaténaire bispécifique se liant à un antigène de surface cellulaire cible via un premier domaine de liaison et à l'antigène de surface des cellules T CD3 via un deuxième domaine de liaison, la construction comprenant deux peptides de liaison FcRn,

(a) un premier peptide de liaison FcRn étant constitué de la séquence d'acides aminés **QRFVTGHFGGL$X_1$PANG** (SEQ ID NO: 1) tandis que $X_1$ est Y ou H ; et
(b) un deuxième peptide de liaison FcRn étant constitué de la séquence d'acides aminés **TGHFGGLHP** (SEQ ID NO: 4) ;

la construction d'anticorps monocaténaire bispécifique ne possédant pas une séquence d'acides aminés telle que décrite dans les SEQ ID NO: **132-135.**

**2.** Construction d'anticorps monocaténaire bispécifique se liant à un antigène de surface cellulaire cible via un premier domaine de liaison et à l'antigène de surface des cellules T CD3 via un deuxième domaine de liaison, la construction comprenant deux peptides de liaison FcRn,

(a) un premier peptide de liaison FcRn étant constitué de la séquence d'acides aminés **QRFVTGHFGGL$X_1$PANG** (SEQ ID NO: 1) tandis que $X_1$ est Y ou H ; et
(b) un deuxième peptide de liaison FcRn étant constitué de la séquence d'acides aminés **QRFVTGHFGGLHPANG** (SEQ ID NO: 3) ou **QRFCTGHFGGLHPCNG** (SEQ ID NO: 5).

**3.** Construction d'anticorps monocaténaire bispécifique se liant à un antigène de surface cellulaire cible via un premier domaine de liaison et à l'antigène de surface des cellules T CD3 via un deuxième domaine de liaison, la construction comprenant un peptide de liaison FcRn au niveau de la terminaison N et un peptide de liaison FcRn au niveau de la terminaison C,

(a) le peptide de liaison FcRn au niveau de la terminaison N étant constitué de la séquence d'acides aminés **QRFVTGHFGGLYPANG** (SEQ ID NO: 2) et au niveau de la terminaison C étant constitué de la séquence d'acides aminés **QRFCTGHFGGLHPCNG** (SEQ ID NO: 5) ;
(b) le peptide de liaison FcRn au niveau de la terminaison N étant constitué de la séquence d'acides aminés **QRFVTGHFGGLYPANG** (SEQ ID NO: 2) et au niveau de la terminaison C étant constitué de la séquence d'acides aminés **QRFVTGHFGGLHPANG** (SEQ ID NO: 3) ;
(c) le peptide de liaison FcRn au niveau de la terminaison N étant constitué de la séquence d'acides aminés **QRFVTGHFGGLHPANG** (SEQ ID NO: 3) et au niveau de la terminaison C étant constitué de la séquence d'acides aminés **QRFCTGHFGGLHPCNG** (SEQ ID NO: 5) ;
(d) le peptide de liaison FcRn au niveau de la terminaison N étant constitué de la séquence d'acides aminés **QRFVTGHFGGLHPANG** (SEQ ID NO: 3) et au niveau de la terminaison C étant constitué de la séquence d'acides aminés **QRFVTGHFGGLHPANG** (SEQ ID NO: 3) ;
(e) le peptide de liaison FcRn au niveau de la terminaison N étant constitué de la séquence d'acides aminés **QRFCTGHFGGLHPCNG** (SEQ ID NO: 5) et au niveau de la terminaison C étant constitué de la séquence d'acides aminés **QRFVTGHFGGLYPANG** (SEQ ID NO: 2) ; et
(f) le peptide de liaison FcRn au niveau de la terminaison N étant constitué de la séquence d'acides aminés **QRFCTGHFGGLHPCNG** (SEQ ID NO: 5) et au niveau de la terminaison C étant constitué de la séquence d'acides aminés **QRFVTGHFGGLHPANG** (SEQ ID NO: 3).

**4.** Construction d'anticorps selon l'une quelconque des revendications précédentes, les peptides de liaison FcRn étant liés à la construction d'anticorps via un ou plusieurs lieurs peptidiques.

**5.** Construction d'anticorps selon la revendication 4, le lieur peptidique ayant la séquence d'acides aminés $(GGGGS)_n$ $(SEQ\ ID\ NO:\ 6)_n$, n étant un entier dans la plage de 1 à 5.

**6.** Construction d'anticorps selon l'une quelconque des revendications précédentes, la construction comprenant un domaine supplémentaire se liant à l'albumine sérique.

**7.** Construction d'anticorps selon l'une quelconque des revendications précédentes, l'antigène de surface cellulaire cible étant un antigène tumoral.

**8.** Construction d'anticorps selon la revendication 7, l'antigène tumoral étant choisi dans le groupe constitué par CDH19, mésotheline (MSLN), DLL3, FLT3, CD33, CD20 et EGFRvIII.

**9.** Construction d'anticorps selon l'une quelconque des revendications précédentes, le deuxième domaine de liaison se liant à un épitope d'humain et la chaîne CD3ε de *Callithrix jacchus, Saguinus oedipus* ou *Saimiri sciureus,* l'épitope étant une partie d'une séquence d'acides aminés comprise dans le groupe constitué par les SEQ ID NO: 7, 8, 9, et 10 et comprenant au moins la séquence d'acides aminés Gln-Asp-Gly-Asn-Glu (SEQ ID NO: 11).

**10.** Construction d'anticorps selon la revendication 9, le deuxième domaine de liaison comprenant une région VL ayant CDR-L1-L3 et une région VH ayant CDR-H1-H3 choisies dans le groupe constitué par :

(a) CDR-L1-L3 telle que décrite dans SEQ ID NO: 12-14 et CDR-H1-H3 telle que décrite dans SEQ ID NO: 15-17 ;
(b) CDR-L1-L3 telle que décrite dans SEQ ID NO: 24-26 et CDR-H1-H3 telle que décrite dans SEQ ID NO: 27-29 ;
(c) CDR-L1-L3 telle que décrite dans SEQ ID NO: 36-38 et CDR-H1-H3 telle que décrite dans SEQ ID NO: 39-41 ;
(d) CDR-L1-L3 telle que décrite dans SEQ ID NO: 48-50 et CDR-H1-H3 telle que décrite dans SEQ ID NO: 51-53 ;
(e) CDR-L1-L3 telle que décrite dans SEQ ID NO: 60-62 et CDR-H1-H3 telle que décrite dans SEQ ID NO: 63-65 ;
(f) CDR-L1-L3 telle que décrite dans SEQ ID NO: 72-74 et CDR-H1-H3 telle que décrite dans SEQ ID NO: 75-77 ;
(g) CDR-L1-L3 telle que décrite dans SEQ ID NO: 84-86 et CDR-H1-H3 telle que décrite dans SEQ ID NO: 87-89 ;
(h) CDR-L1-L3 telle que décrite dans SEQ ID NO: 96-98 et CDR-H1-H3 telle que décrite dans SEQ ID NO: 99-101 ;
(i) CDR-L1-L3 telle que décrite dans SEQ ID NO: 108-110 et CDR-H1-H3 telle que décrite dans SEQ ID NO: 111-113 ;
(j) CDR-L1-L3 telle que décrite dans SEQ ID NO: 120-122 et CDR-H1-H3 telle que décrite dans SEQ ID NO: 123-125 ;
(k) CDR-L1-L3 telle que décrite dans SEQ ID NO: 616-618 et CDR-H1-H3 telle que décrite dans SEQ ID NO: 613-615 ;
(l) CDR-L1-L3 telle que décrite dans SEQ ID NO: 626-628 et CDR-H1-H3 telle que décrite dans SEQ ID NO: 623-625 ;
(m) CDR-L1-L3 telle que décrite dans SEQ ID NO: 636-638 et CDR-H1-H3 telle que décrite dans SEQ ID NO: 633-635 ;
(n) CDR-L1-L3 telle que décrite dans SEQ ID NO: 646-648 et CDR-H1-H3 telle que décrite dans SEQ ID NO: 643-645 ;
(o) CDR-L1-L3 telle que décrite dans SEQ ID NO: 656-658 et CDR-H1-H3 telle que décrite dans SEQ ID NO: 653-655 ;
(p) CDR-L1-L3 telle que décrite dans SEQ ID NO: 666-668 et CDR-H1-H3 telle que décrite dans SEQ ID NO: 663-665 ;
(q) CDR-L1-L3 telle que décrite dans SEQ ID NO: 676-678 et CDR-H1-H3 telle que décrite dans SEQ ID NO: 673-675 ; et
(r) CDR-L1-L3 telle que décrite dans SEQ ID NO: 686-688 et CDR-H1-H3 telle que décrite dans SEQ ID NO: 683-685.

**11.** Construction d'anticorps selon la revendication 9 ou 10, le deuxième domaine de liaison comprenant des paires de chaînes VH et VL choisies dans le groupe constitué par :

(a) une chaîne VH telle que décrite dans SEQ ID NO: 18 et une chaîne VL telle que décrite dans SEQ ID NO: 20 ;
(b) une chaîne VH telle que décrite dans SEQ ID NO: 30 et une chaîne VL telle que décrite dans SEQ ID NO: 32 ;
(c) une chaîne VH telle que décrite dans SEQ ID NO: 42 et une chaîne VL telle que décrite dans SEQ ID NO: 44 ;
(d) une chaîne VH telle que décrite dans SEQ ID NO: 54 et une chaîne VL telle que décrite dans SEQ ID NO: 56 ;
(e) une chaîne VH telle que décrite dans SEQ ID NO: 66 et une chaîne VL telle que décrite dans SEQ ID NO: 68 ;

(f) une chaîne VH telle que décrite dans SEQ ID NO: 78 et une chaîne VL telle que décrite dans SEQ ID NO: 80 ;
(g) une chaîne VH telle que décrite dans SEQ ID NO: 90 et une chaîne VL telle que décrite dans SEQ ID NO: 92 ;
(h) une chaîne VH telle que décrite dans SEQ ID NO: 102 et une chaîne VL telle que décrite dans SEQ ID NO: 104 ;
(i) une chaîne VH telle que décrite dans SEQ ID NO: 114 et une chaîne VL telle que décrite dans SEQ ID NO: 116 ;
(j) une chaîne VH telle que décrite dans SEQ ID NO: 126 et une chaîne VL telle que décrite dans SEQ ID NO: 128 ;
(k) une chaîne VH telle que décrite dans SEQ ID NO: 619 et une chaîne VL telle que décrite dans SEQ ID NO: 620 ;
(l) une chaîne VH telle que décrite dans SEQ ID NO: 629 et une chaîne VL telle que décrite dans SEQ ID NO: 630 ;
(m) une chaîne VH telle que décrite dans SEQ ID NO: 639 et une chaîne VL telle que décrite dans SEQ ID NO: 640 ;
(n) une chaîne VH telle que décrite dans SEQ ID NO: 649 et une chaîne VL telle que décrite dans SEQ ID NO: 650 ;
(o) une chaîne VH telle que décrite dans SEQ ID NO: 659 et une chaîne VL telle que décrite dans SEQ ID NO: 660 ;
(p) une chaîne VH telle que décrite dans SEQ ID NO: 669 et une chaîne VL telle que décrite dans SEQ ID NO: 670 ;
(q) une chaîne VH telle que décrite dans SEQ ID NO: 679 et une chaîne VL telle que décrite dans SEQ ID NO: 680 ; et
(r) une chaîne VH telle que décrite dans SEQ ID NO: 689 et une chaîne VL telle que décrite dans SEQ ID NO: 690.

**12.** Construction d'anticorps selon l'une quelconque des revendications 9 à 11, le deuxième domaine de liaison comprenant une séquence d'acides aminés telle que décrite dans SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 621, SEQ ID NO: 631, SEQ ID NO: 641, SEQ ID NO: 651, SEQ ID NO: 661, SEQ ID NO: 671, SEQ ID NO: 681, ou SEQ ID NO: 691.

**13.** Construction d'anticorps selon l'une quelconque des revendications 1 et 4-12, les domaines étant agencés depuis la terminaison N vers la terminaison C comme suit :

(peptide de liaison FcRn constitué par SEQ ID NO:1, tandis que $X_1$ est Y ou H)-(chaîne VH du premier domaine de liaison) - (chaîne VL du premier domaine de liaison)-(chaîne VH du deuxième domaine de liaison) - (chaîne VL du deuxième domaine de liaison)-(peptide de liaison FcRn constitué par SEQ ID NO: 4)
ou
(peptide de liaison FcRn constitué par SEQ ID NO:4)-(chaîne VH du premier domaine de liaison) - (chaîne VL du premier domaine de liaison)-(chaîne VH du deuxième domaine de liaison) - (chaîne VL du deuxième domaine de liaison)-(peptide de liaison FcRn constitué par SEQ ID NO: 1, tandis que $X_1$ est Y ou H)

**14.** Polynucléotide codant pour une construction d'anticorps telle que définie dans l'une quelconque des revendications 1 à 13.

**15.** Vecteur comprenant un polynucléotide tel que défini dans la revendication 14.

**16.** Cellule hôte transformée ou transfectée par le polynucléotide tel que défini dans la revendication 14 ou par le vecteur tel que défini dans la revendication 15.

**17.** Procédé pour la production d'une construction d'anticorps selon l'une quelconque des revendications 1 à 13, ledit procédé comprenant la culture d'une cellule hôte telle que définie dans la revendication 16 dans des conditions permettant l'expression de la construction d'anticorps telle que définie dans l'une quelconque des revendications 1 à 13 et la récupération de la construction d'anticorps produite depuis la culture.

**18.** Composition pharmaceutique comprenant une construction d'anticorps selon l'une quelconque des revendications 1 à 13, ou produite selon le procédé selon la revendication 17.

**19.** Construction d'anticorps selon l'une quelconque des revendications 1 à 13, ou produite selon le procédé selon la revendication 17, pour une utilisation dans la prévention, le traitement ou l'amélioration d'une maladie choisie parmi une maladie proliférative, une maladie tumorale, une maladie virale ou un trouble immunologique.

**20.** Kit comprenant une construction d'anticorps selon l'une quelconque des revendications 1 à 13, ou produite selon le procédé selon la revendication 17, un vecteur tel que défini dans la revendication 15 et/ou une cellule hôte telle que définie dans la revendication 16.

# Figure 1

**A** BiTE: c-terminal cyclic FcRnBP, c-terminal cyclic FcRnBP

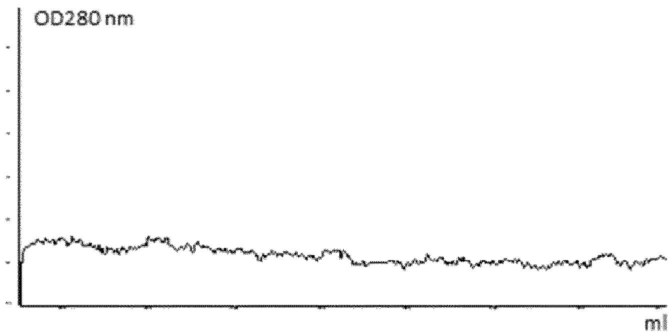


No Elution of BiTE protein

**B** BiTE: n-terminal linear FcRnBP, c-terminal cyclic FcRnBP

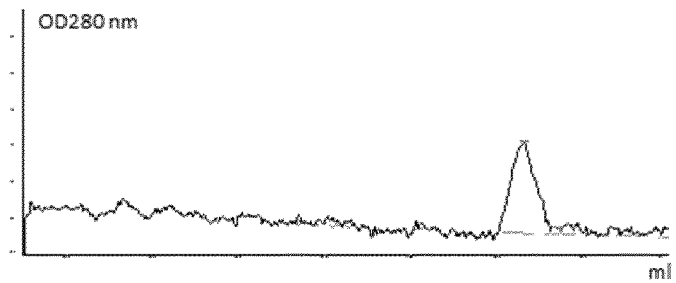


Elution of BiTE protein

Figure 2(1)

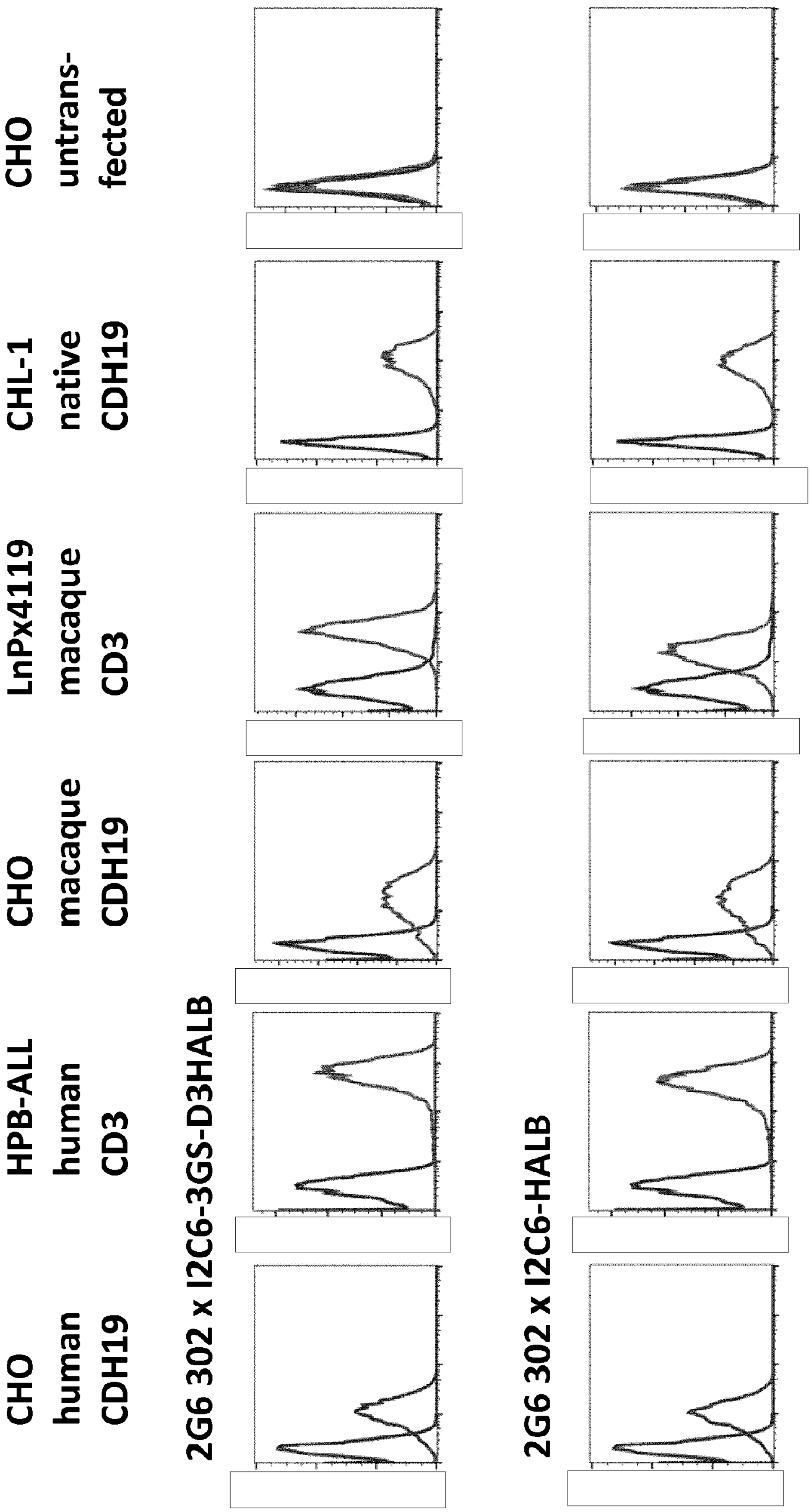
CHO human CDH19
HPB-ALL human CD3
CHO macaque CDH19
LnPx4119 macaque CD3
CHL-1 native CDH19
CHO untrans-fected
2G6 302 x I2C6-3GS-D3HALB
2G6 302 x I2C6-HALB

Figure 2(2)

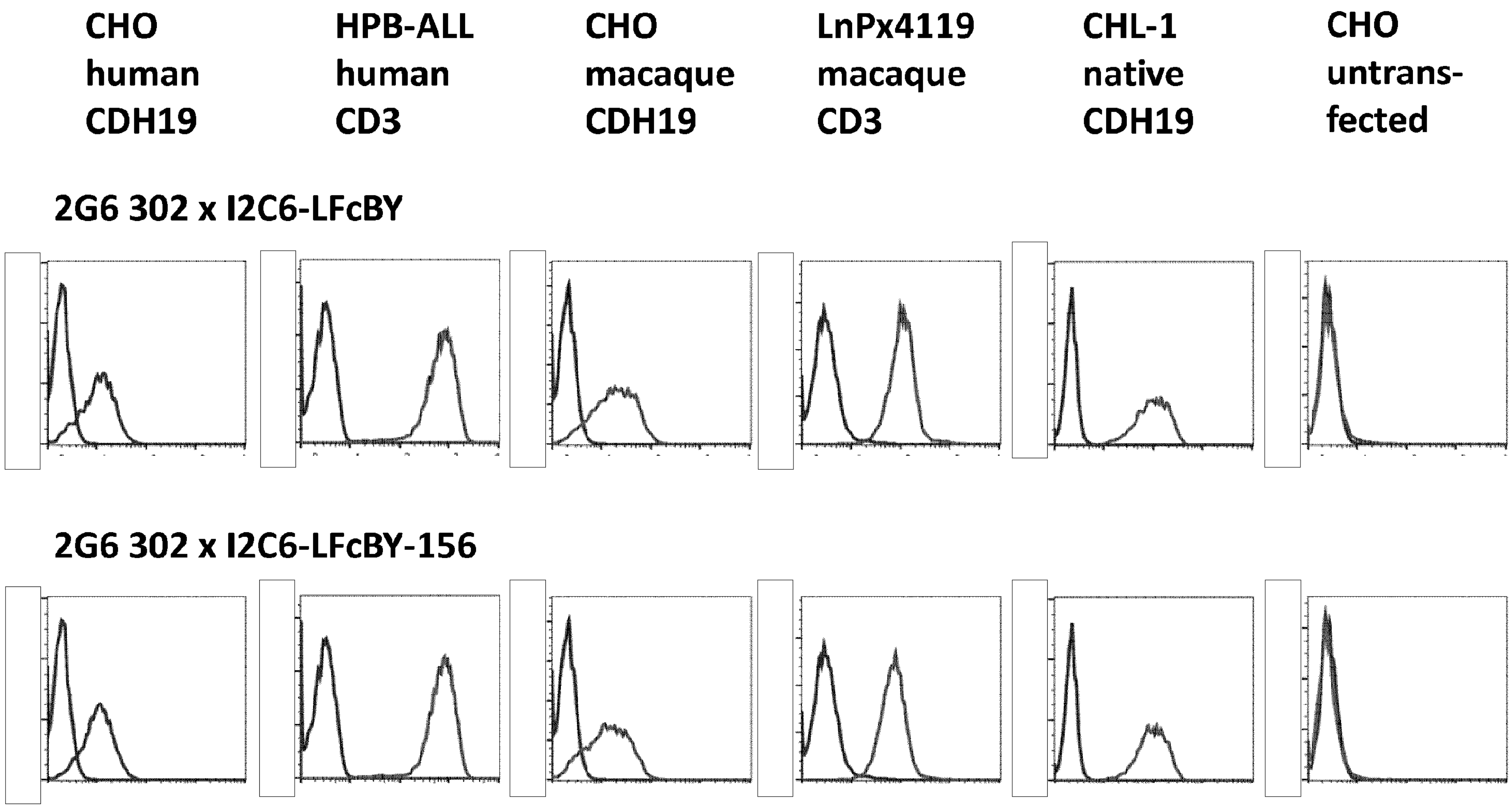
CHO human CDH19
HPB-ALL human CD3
CHO macaque CDH19
LnPx4119 macaque CD3
CHL-1 native CDH19
CHO untrans-fected
2G6 302 x I2C6-LFcBY
2G6 302 x I2C6-LFcBY-156

Figure 2(3)

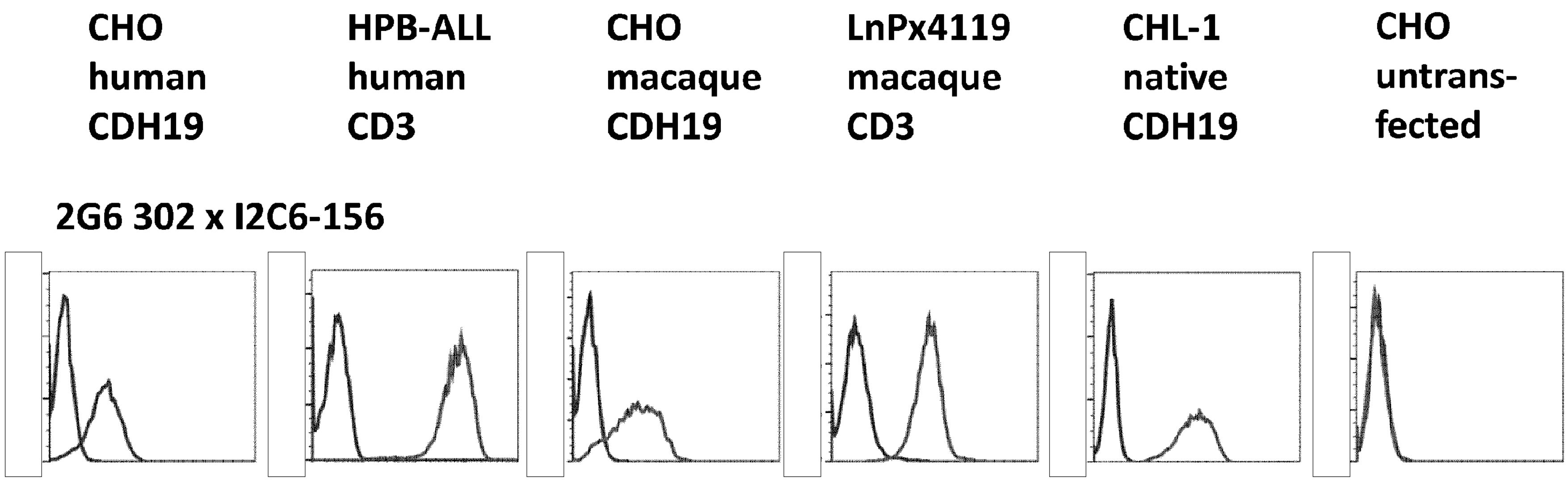
CHO
human
CDH19
HPB-ALL
human
CD3
CHO
macaque
CDH19
LnPx4119
macaque
CD3
CHL-1
native
CDH19
CHO
untrans-
fected
2G6 302 x I2C6-156

Figure 3

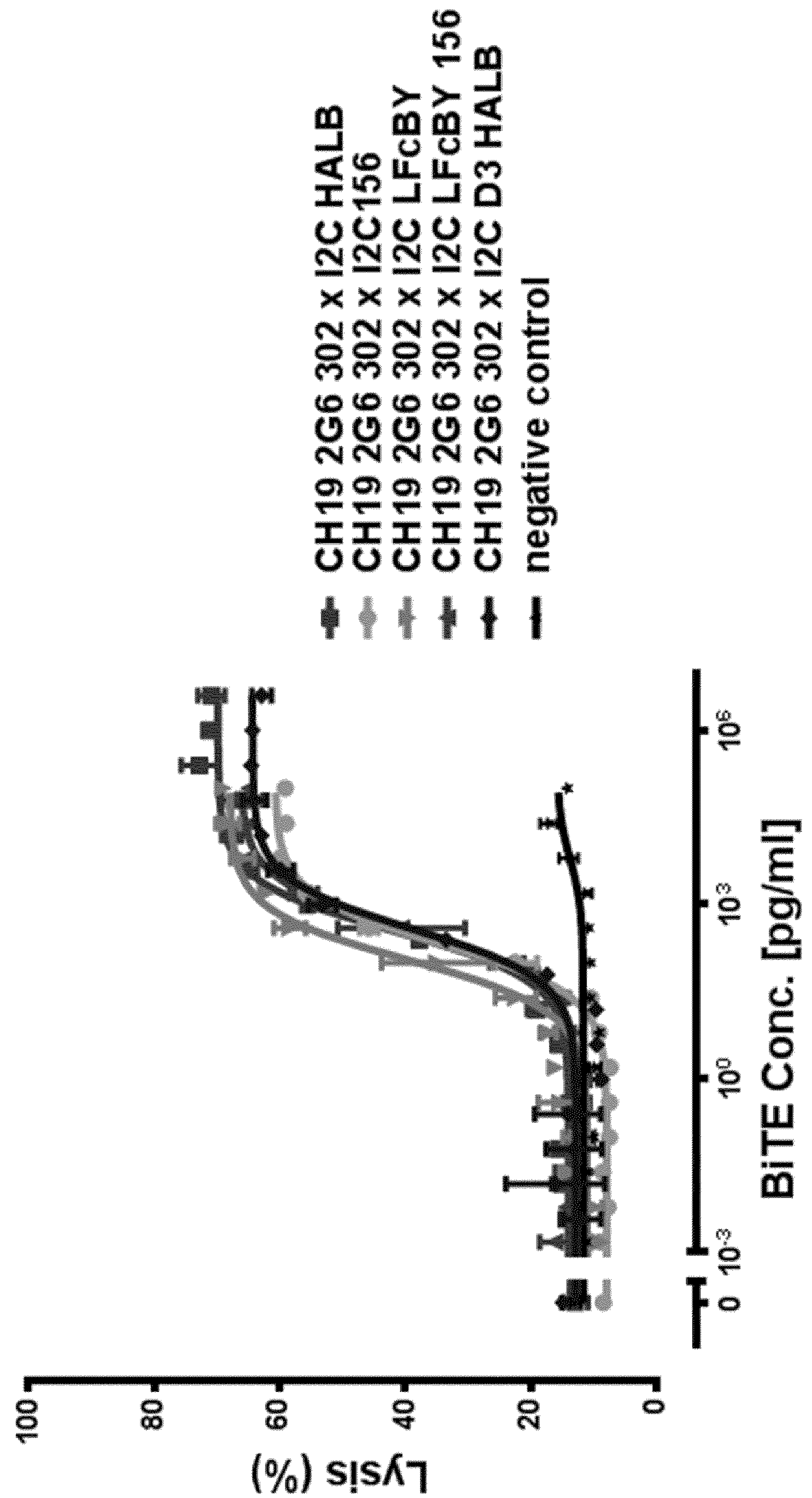

CH19 2G6 302 x I2C HALB
CH19 2G6 302 x I2C156
CH19 2G6 302 x I2C LFcBY
CH19 2G6 302 x I2C LFcBY 156
CH19 2G6 302 x I2C D3 HALB
negative control
Lysis (%)
100
80
60
40
20
0
0
$10^{-3}$
$10^{0}$
$10^{3}$
$10^{6}$
BiTE Conc. [pg/ml]

Figure 4

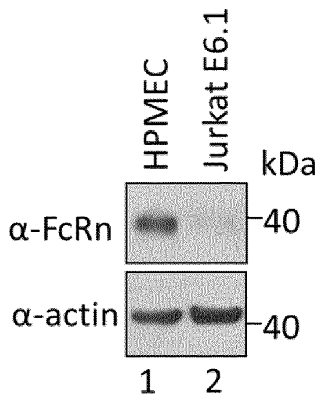


Figure 5

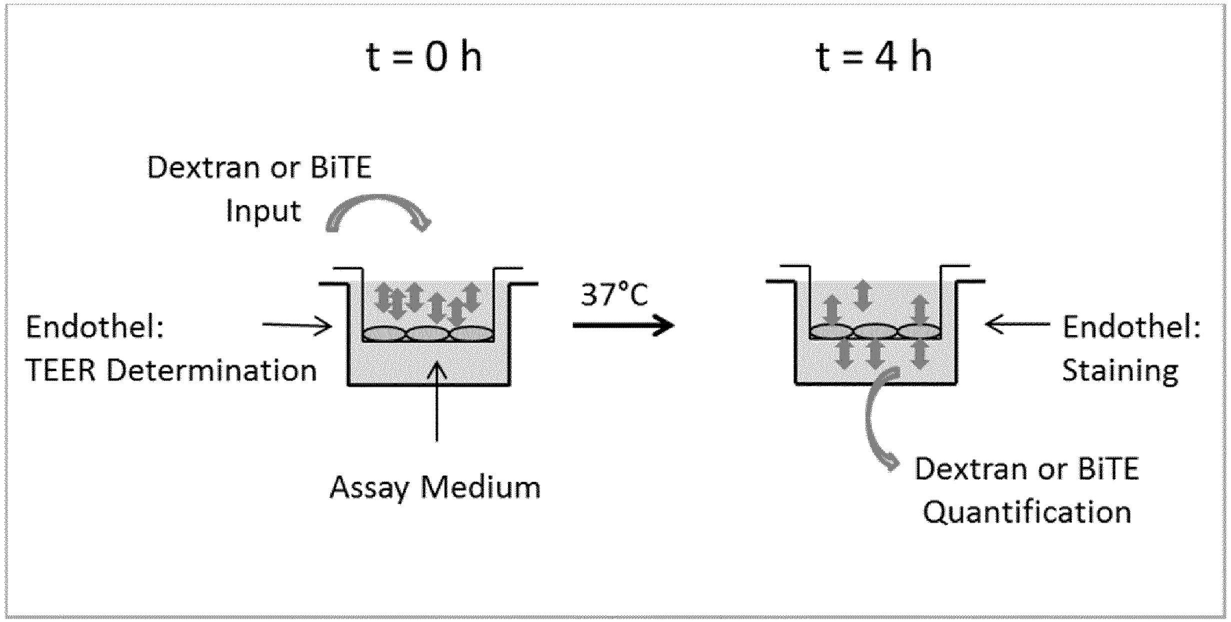

Figure 6

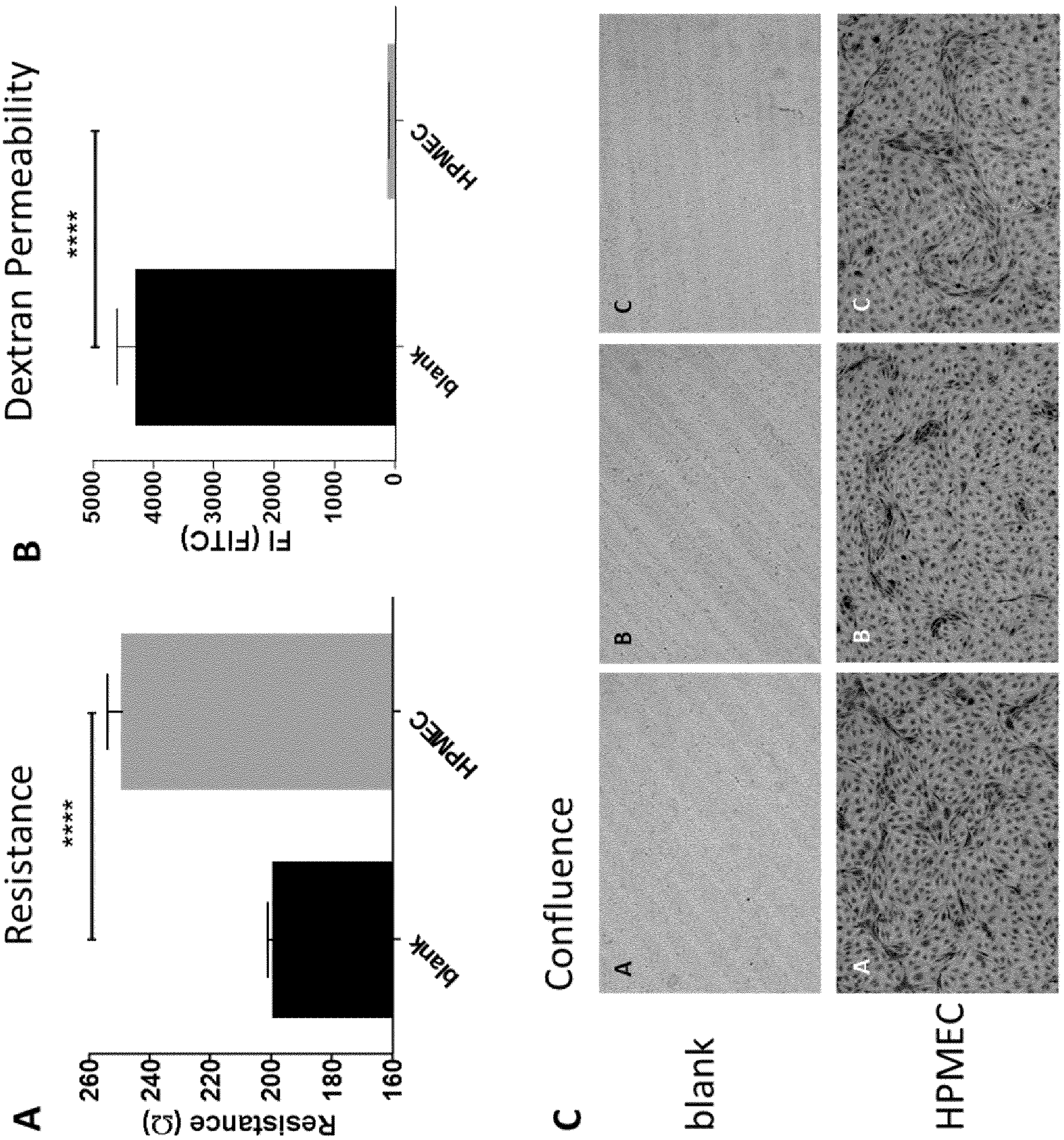
A
Resistance
Resistance (Ω)
260
240
220
200
180
160
****
blank
HPMEC
B
Dextran Permeability
FI (FITC)
5000
4000
3000
2000
1000
0
****
blank
HPMEC
C
Confluence
blank
HPMEC
A
B
C
A
B
C

# Figure 7

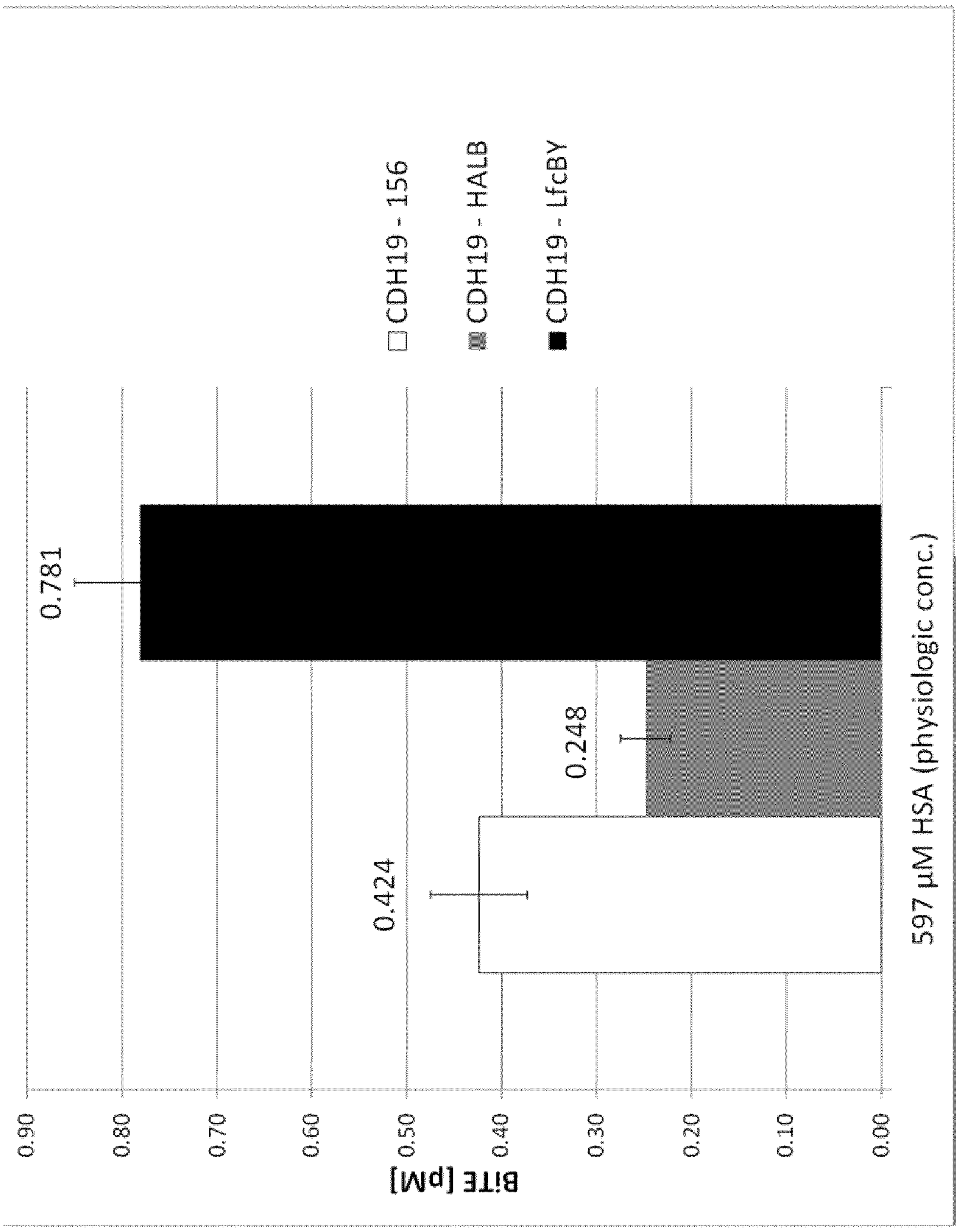


| BiTE Antibodies | P-Value Summary |
|---|---|
| CDH19-156 vs. CDH19-HALB | * |
| CDH19-156 vs. CDH19-LfcBY | *** |
| CDH19-HALB vs. CDH19-LfcBY | **** |

# Figure 8

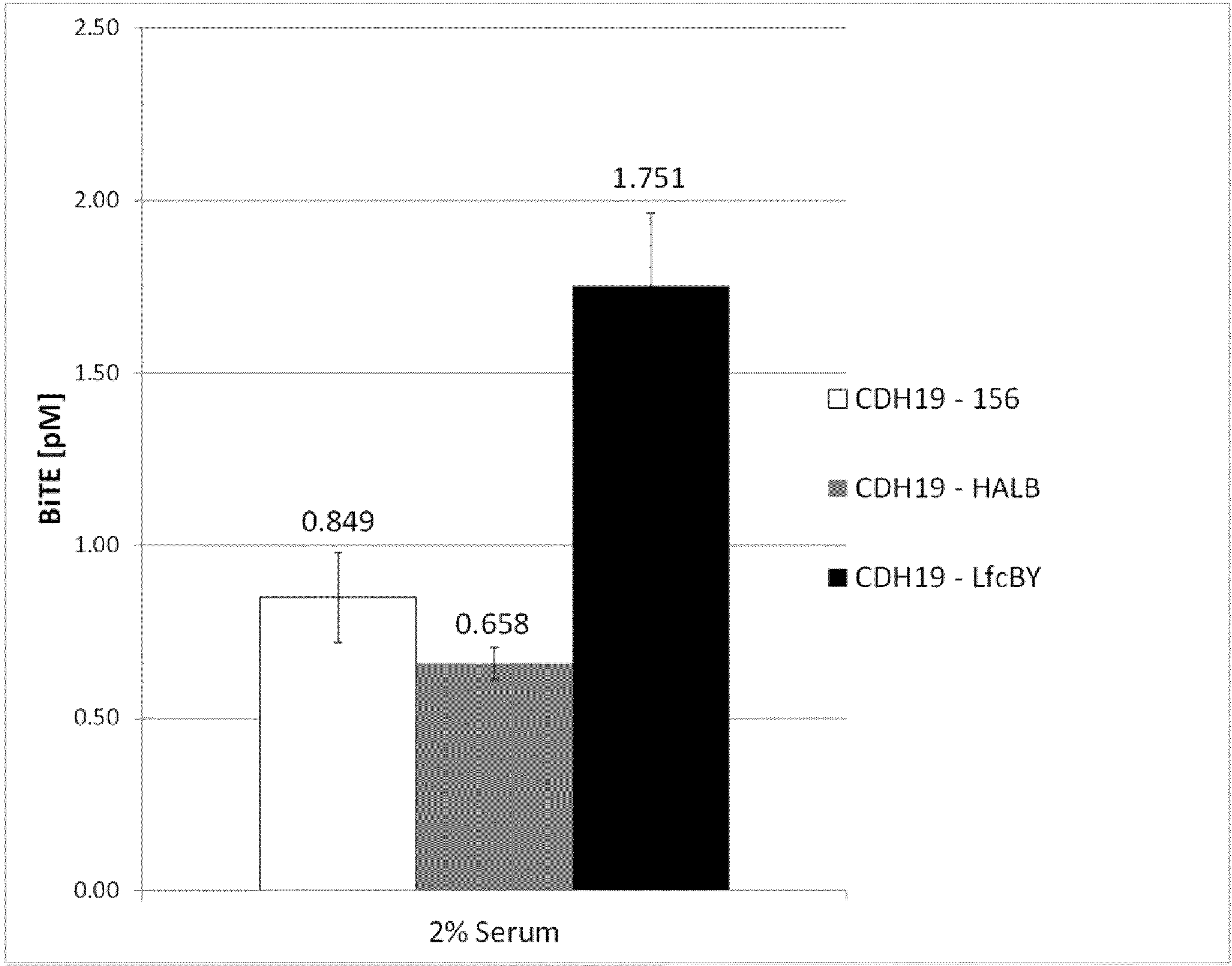


| BiTE Antibodies | P-Value Summary |
|---|---|
| CDH19-156 vs. CDH19-HALB | ns |
| CDH19-156 vs. CDH19-LfcBY | *** |
| CDH19-HALB vs. CDH19-LfcBY | *** |

# Figure 9

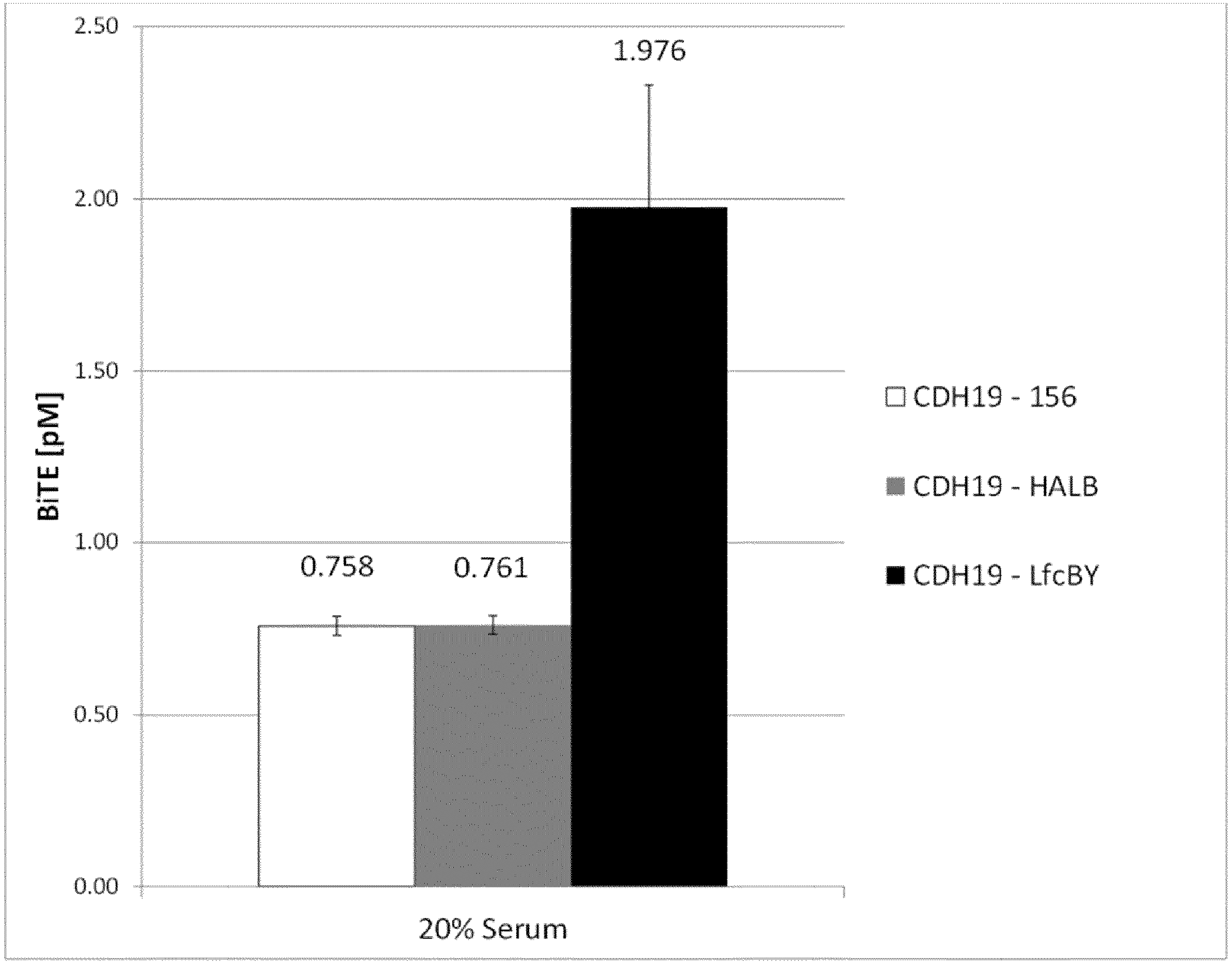


| BiTE Antibodies | P-Value Summary |
|---|---|
| CDH19-156 vs. CDH19-HALB | ns |
| CDH19-156 vs. CDH19-LfcBY | *** |
| CDH19-HALB vs. CDH19-LfcBY | *** |

# Figure 10

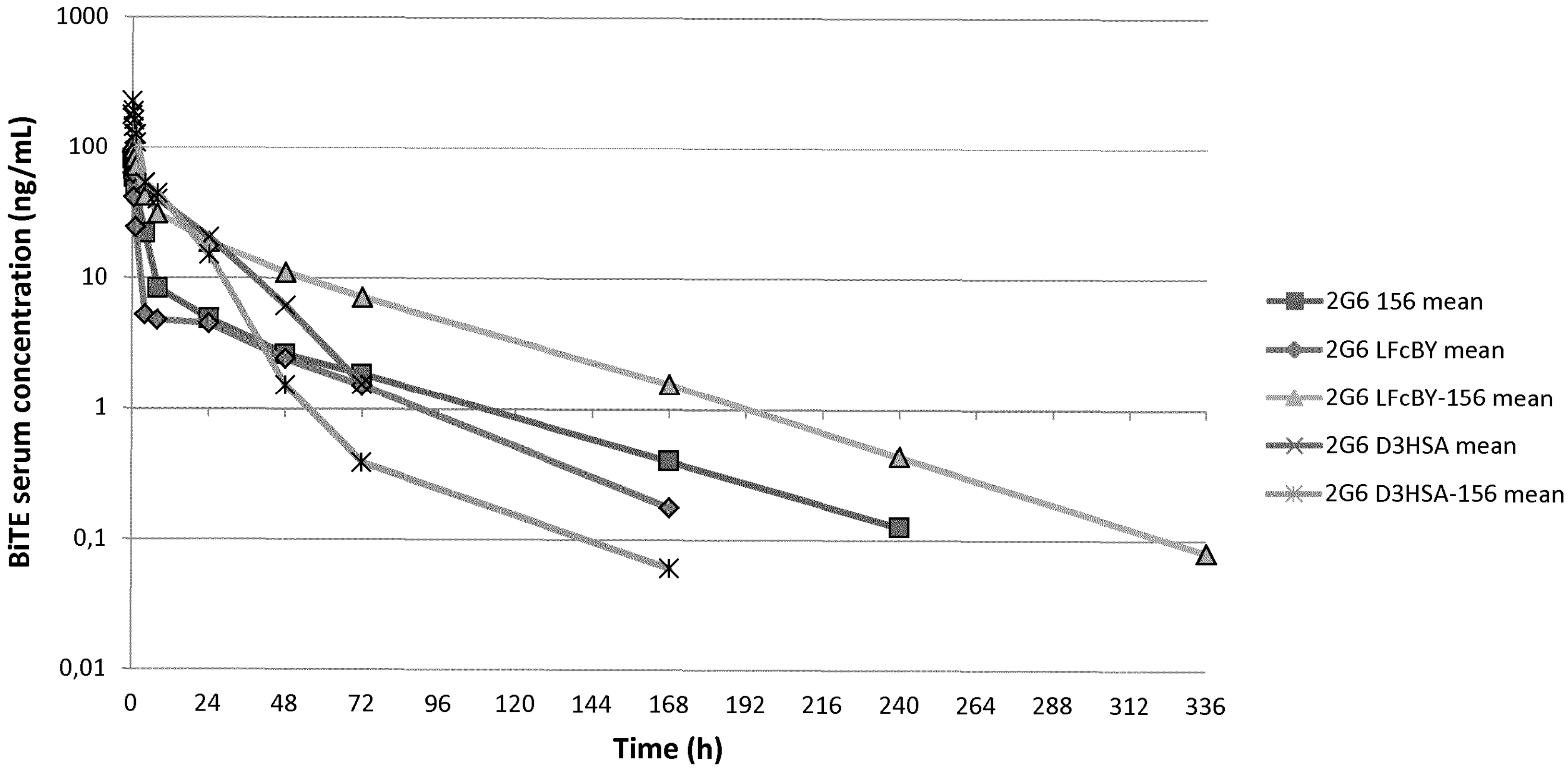


| BiTE | Dose (µg/kg) | $AUC_{inf}$ (ng·h/mL) | $V_{ss}$ (mL/kg) | CL (mL/h/kg) | MRT (h) | $t_{1/2}$ (h) |
|---|---|---|---|---|---|---|
| **2G6 156 mean** | **6** | **568** | **446** | **11.3** | **42.3** | **44.3** |
| **2G6 LFcBy mean** | **6** | **366** | **594** | **16.1** | **39.8** | **31.2** |
| **2G6 LFcBy-156 mean*** | **6** | **1796** | **193** | **4.3** | **48.7** | **40.3** |
| **2G6 D3HSA mean** | **6** | **1383** | **80.7** | **4.9** | **18.1** | **13.5** |
| **2G6 D3HSA-156 mean*** | **6** | **1216** | **58.9** | **5.2** | **12.3** | **17.3** |

Figure 11

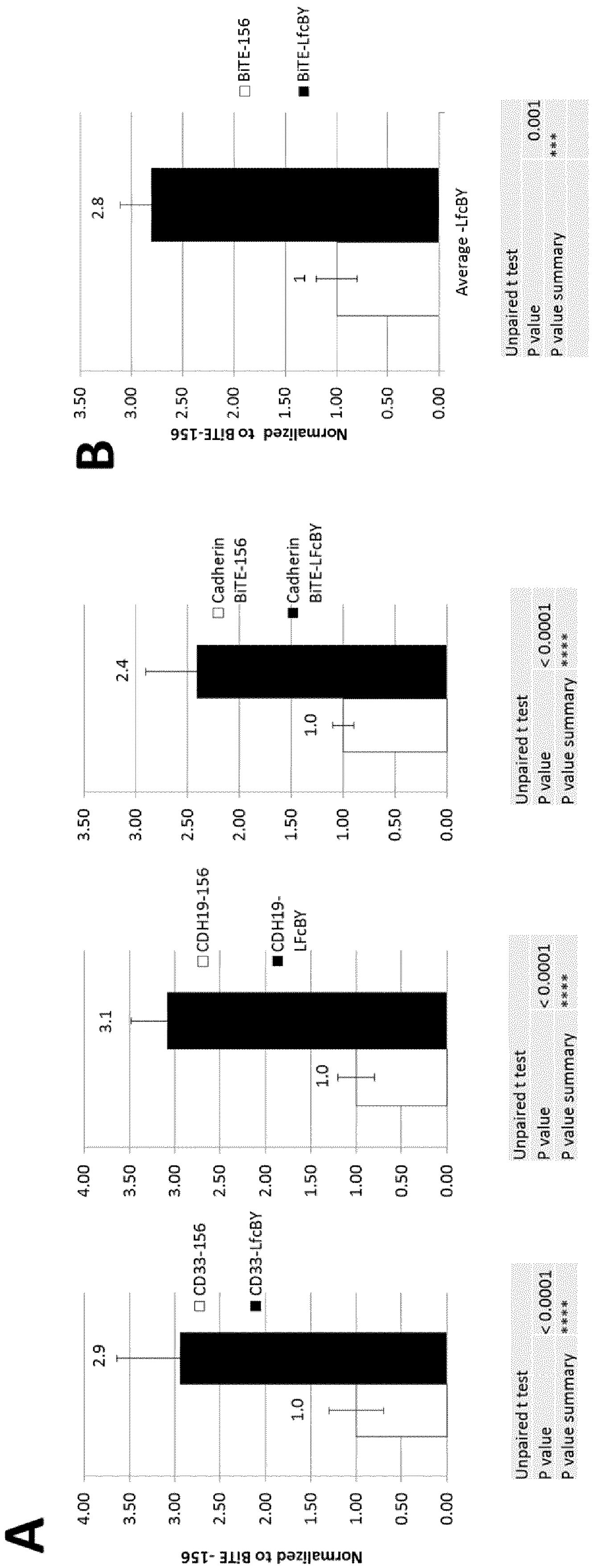
A
Normalized to BiTE -156
CD33-156
CD33-LfcBY
1.0
2.9
Unpaired t test
P value < 0.0001
P value summary ****
CDH19-156
CDH19-LFcBY
1.0
3.1
Unpaired t test
P value < 0.0001
P value summary ****
Cadherin BiTE-156
Cadherin BiTE-LFcBY
1.0
2.4
Unpaired t test
P value < 0.0001
P value summary ****
B
Normalized to BiTE-156
BiTE-156
BiTE-LfcBY
1
2.8
Average -LfcBY
Unpaired t test
P value 0.001
P value summary ***

Figure 12

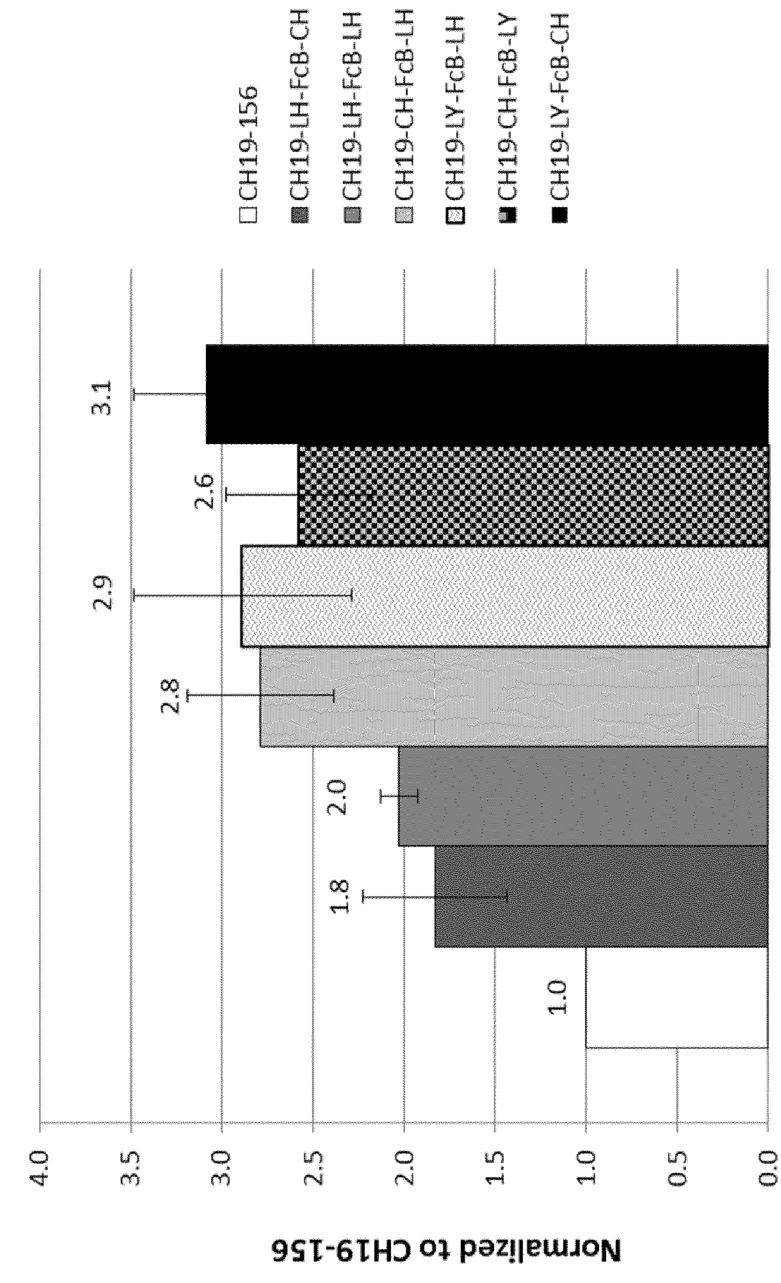


| | |
|---|---|
| ANOVA summary | |
| P value | < 0.0001 |
| P value summary | **** |

| | |
|---|---|
| CH19-156 vs. CH19-LH-FcB-CH | ** |
| CH19-156 vs. CH19-LH-FcB-LH | *** |
| CH19-156 vs. CH19-CH-FcB-LH | **** |
| CH19-156 vs. CH19-LY-FcB-LH | **** |
| CH19-156 vs. CH19-CH-FcB-LY | **** |
| CH19-156 vs. CH19-LY-FcB-CH | **** |

# Figure 13

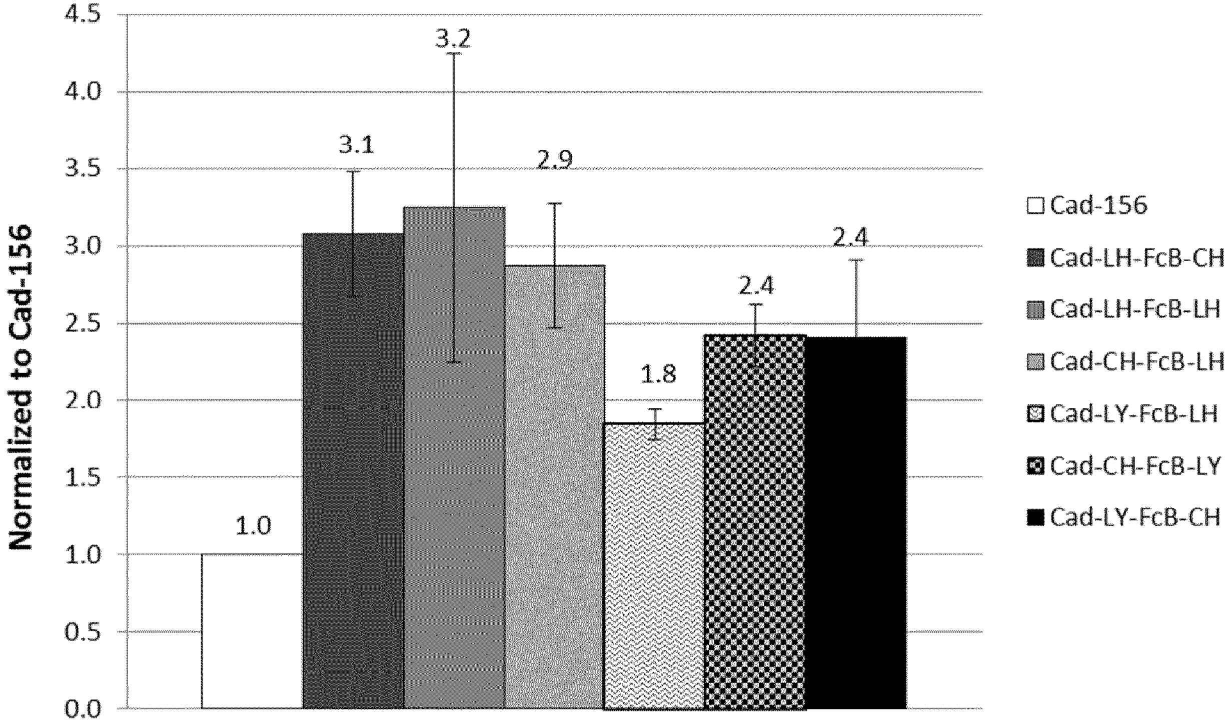


| | |
|---|---|
| ANOVA summary | |
| P value | < 0.0001 |
| P value summary | **** |

| | |
|---|---|
| Cad-156 vs. Cad-LH-FcB-CH | **** |
| Cad-156 vs. Cad-LH-FcB-LH | **** |
| Cad-156 vs. Cad-CH-FcB-LH | **** |
| Cad-156 vs. Cad-LY-FcB-LH | * |
| Cad-156 vs. Cad-CH-FcB-LY | **** |
| Cad-156 vs. Cad-LY-FcB-CH | **** |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006071441 A2
- US 4816567 A
- US 5223409 A, Ladner
- US 20030070185 A
- WO 9634096 A
- WO 9633735 A
- US 5648260 A
- US 4816397 A, Boss
- EP 0171496 A, Tanaguchi
- EP 0173494 A
- GB 2177096 A
- WO 9852976 A
- WO 0034317 A
- US 6300064 B
- US 5585089 A
- US 5693761 A
- US 5693762 A
- US 5859205 A
- US 6407213 B
- US 5225539 A
- EP 239400 A
- US 759620
- US 466008
- US 610515
- US 919297
- US 922649
- US 031801
- US 112848
- US 234145
- US 376279
- US 430938
- US 464584
- US 464582
- US 463191
- US 462837
- US 486853
- US 486857
- US 486859
- US 462513
- US 724752
- US 6162963 A
- US 6150584 A
- US 6114598 A
- US 6075181 A
- US 5939598 A
- JP 3068180 B
- JP 3068506 B
- JP 3068507 B
- EP 0463151 B1
- WO 9402602 A
- WO 9824893 A
- WO 0076310 A
- WO 0347336 A
- US 5545807 A, Surani
- US 5545806 A
- US 5625825 A
- US 5625126 A
- US 5633425 A
- US 5661016 A
- US 5770429 A
- US 5789650 A
- US 5814318 A
- US 5877397 A
- US 5874299 A
- US 6255458 B, Lonberg and Kay
- US 5591669 A
- US 6023010 A, Krimpenfort and Berns
- US 5612205 A
- US 5721367 A
- US 5789215 A, Berns
- US 5643763 A, Choi and Dunn
- US 574748
- US 575962
- US 810279
- US 853408
- US 904068
- US 990860
- US 053131
- US 096762
- US 155301
- US 161739
- US 165699
- US 209741
- EP 0546073 B1
- WO 9203918 A
- WO 9222645 A
- WO 9222647 A
- WO 9222670 A
- WO 9312227 A
- WO 9400569 A
- WO 9425585 A
- WO 9614436 A
- WO 9713852 A
- WO 9824884 A
- US 5981175 A
- EP 773288 A
- EP 843961 A
- US 5476996 A

- US 5698767 A
- US 5958765 A
- US 5565332 A
- US 4751180 A
- US 4935233 A
- WO 8809344 A
- WO 9954440 A
- US 4946778 A
- US 3969287 A
- US 3691016 A
- US 4195128 A
- US 4247642 A
- US 4229537 A
- US 4330440 A
- WO 8705330 A
- US 4640835 A
- US 4496689 A
- US 4301144 A
- US 4670417 A
- US 4791192 A
- US 4179337 A
- WO 9215673 A, Renilla
- WO 9507463 A
- WO 9814605 A
- WO 9826277 A
- WO 9949019 A
- US 5292658 A
- US 5418155 A
- US 5683888 A
- US 5741668 A
- US 5777079 A
- US 5804387 A
- US 5874304 A
- US 5876995 A
- US 5925558 A
- WO 9410308 A
- WO 2007042261 A
- WO 2008119567 A
- US 4475196 A
- US 4439196 A
- US 4447224 A
- US 4447 A
- US 233 A
- US 4486194 A
- US 4487603 A
- US 4596556 A
- US 4790824 A
- US 4941880 A
- US 5064413 A
- US 5312335 A
- US 5383851 A
- US 5399163 A


**Non-patent literature cited in the description**


- **BERTUCCI et al.** *Anticancer Research,* 2007, vol. 27, 3441-3450
- **SOCKOLOSKY et al.** *PNAS,* 2012, vol. 109 (40), 16095-16100
- **SOCKOLOSKY.** *PNAS,* 2012, vol. 109 (40), 16095
- **HARLOW ; LANE.** Antibodies a laboratory manual. CSHL Press, 1988
- Using Antibodies: a laboratory manual. CSHL Press, 1999
- **KONTERMANN ; DÜBEL.** Antibody Engineering. Springer, 2010
- **LITTLE.** Recombinant Antibodies for Immunotherapy. Cambridge University Press, 2009
- **KOEHLER et al.** *Nature,* 1975, vol. 256, 495
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **SMITH.** *Science,* 1985, vol. 228, 1315-1317
- **CLACKSON et al.** *Nature,* 1991, vol. 352, 624-628
- **MARKS et al.** *J. Mol. Biol.,* 1991, vol. 222, 581-597
- **GREEN et al.** *Nature Genetics,* vol. 7, 13-21
- **HAWKINS et al.** *J. Mol. Biol.,* 1992, vol. 254, 889-896
- **LOWMAN et al.** *Biochemistry,* 1991, vol. 30, 10832-10837
- **MORRISON et al.** *Proc. Natl. Acad. Sci. USA,* 1984, vol. 81, 6851-6855
- **MORRISON et al.** *, Proc. Natl. Acad. ScL U.S.A.,* 1985, vol. 81, 6851
- **TAKEDA et al.** *Nature,* 1985, vol. 314, 452
- **TOMLINSON et al.** *J. Mol. Biol.,* 1992, vol. 227, 776-798
- **COOK, G.P. et al.** *Immunol. Today,* 1995, vol. 16 (5), 237-242
- **TOMLINSON et al.** *EMBO J.,* 1995, vol. 14 (14), 4628-4638
- **TOMLINSON, LA. et al.** *MRC Centre for Protein Engineering*
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **MORRISON.** *Science,* 1985, vol. 229, 1202-1207
- **OI et al.** *BioTechniques,* 1986, vol. 4, 214
- **TENG et al.** *Proc. Natl. Acad. Sci. U.S.A.,* 1983, vol. 80, 7308-7312

- **KOZBOR et al.** *Immunology Today,* 1983, vol. 4, 7279
- **OLSSON et al.** *, Meth. Enzymol,* 1982, vol. 92, 3-16
- **WARD et al.** *Nature,* 1989, vol. 341, 544-546
- **GREEN et al.** *Nature Genetics,* 1994, vol. 7, 13-21
- **MENDEZ et al.** *Nature Genetics,* 1997, vol. 15, 146-156
- **GREEN ; JAKOBOVITS.** *J. Exp. Med.,* 1998, vol. 188, 483-495
- **CHOTHIA et al.** *J. Mol. Biol,* 1987, vol. 196, 901-917
- **MACCALLUM et al.** *J. Mol. Biol,* 1996, vol. 262, 732
- Protein Sequence and Structure Analysis of Antibody Variable Domains. Antibody Engineering Lab Manual. Springer-Verlag
- **CHOTHIA ; LESK.** *J. Mol. Biol.,* 1987, vol. 196, 901
- **CHOTHIA et al.** *Nature,* 1989, vol. 342, 877
- **MARTIN ; THORNTON.** *J. Mol. Biol,* 1996, vol. 263, 800
- Antibodies: A Laboratory Manual. Cold Spring Harbor Laboratory, 1988
- Immunoglobulin Genes. Academic Press, 1995
- **SONGSIVILAI ; LACHMANN.** *Clin. Exp. Immunol,* 1990, vol. 79, 315-321
- **DALL'ACQUA et al.** *Biochem,* 1998, vol. 37, 9266-9273
- **CHEADLE et al.** *Mol Immunol,* 1992, vol. 29, 21-30
- **RAAG ; WHITLOW.** *FASEB,* 1995, vol. 9 (1), 73-80
- **SAMBROOK et al.** Molecular Cloning: A Laboratory Manual. Cold Spring Harbor Laboratory Press, 2001
- **MACK.** *J. Immunol.,* 1997, vol. 158, 3965-3970
- **MACK.** *PNAS,* 1995, vol. 92, 7021-7025
- **KUFER.** *Cancer Immunol. Immunother.,* 1997, vol. 45, 193-197
- **LÖFFLER.** *Blood,* 2000, vol. 95 (6), 2098-2103
- **BRÜHL.** *Immunol,* 2001, vol. 166, 2420-2426
- **KIPRIYANOV.** *J. Mol. Biol.,* 1999, vol. 293, 41-56
- **KUFER P. et al.** *Trends in Biotechnology,* 2004, vol. 22 (5), 238-244
- **HOLLINGER ; PHILIPP et al.** *Proceedings of the National Academy of Sciences of the United States of America,* July 1993, vol. 90 (14), 6444-8
- **T. E. CREIGHTON.** Proteins: Structure and Molecular Properties. W. H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **DUSKIN et al.** *J. Biol. Chem.,* 1982, vol. 257, 3105
- **CHALFIE et al.** *Science,* 1994, vol. 263, 802-805
- **STAUBER.** *Biotechniques,* 1998, vol. 24, 462-471
- **HEIM et al.** *Curr. Biol.,* 1996, vol. 6, 178-182
- **ICHIKI et al.** *J. Immunol.,* 1993, vol. 150, 5408-5417
- **NOLAN et al.** *Proc. Natl. Acad. Sci. U.S.A.,* 1988, vol. 85, 2603-2607
- **LANDSCHULZ et al.** *Science,* 1988, vol. 240, 1759
- **HOPPE et al.** *FEBS Letters,* 1994, vol. 344, 191
- **FANSLOW et al.** *Semin. Immunol.,* 1994, vol. 6, 267-78
- **CUNNINGHAM ; WELLS.** *Science,* 1989, vol. 244, 1081-1085
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat. Acad. Sci. U.S.A.,* 1988, vol. 85, 2444
- **DEVEREUX et al.** *Nucl. Acid Res.,* 1984, vol. 12, 387-395
- Current Methods in Sequence Comparison and Analysis. Macromolecule Sequencing and Synthesis, Selected Methods and Applications. Alan R. Liss, Inc, 1988, 127-149
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **KARIN et al.** *Proc. Natl. Acad. Sci. U.S.A.,* 1993, vol. 90, 5873-5787
- **ALTSCHUL et al.** *Methods in Enzymology,* 1996, vol. 266, 460-480
- **ALTSCHUL et al.** *Nucl. Acids Res.,* 1993, vol. 25, 3389-3402
- **HWANG ; FOOTE.** Immunogenicity of engineered antibodies. *Methods,* 2005, vol. 36, 3-10
- **HIATT et al.** *Nature,* 1989, vol. 342, 76-78
- **OWEN et al.** *Bio/Technology,* 1992, vol. 10, 790-794
- **ARTSAENKO et al.** *The Plant J,* 1995, vol. 8, 745-750

- **FECKER et al.** *Plant Mol Biol,* 1996, vol. 32, 979-986
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** *, Annals N. Y Acad. Sci.,* 1982, vol. 383, 44-68
- **CARTER et al.** *Bio/Technology,* 1992, vol. 10, 163-167
- **KNAPPE A et al.** *Blood,* 2000, vol. 95, 3256-3261